(19) Europäisches Patentamt
European Patent Office
Office européen des brevets

(11) EP 1 945 799 B1

(12) EUROPEAN PATENT SPECIFICATION

(45) Date of publication and mention of the grant of the patent:
**09.11.2016 Bulletin 2016/45**

(21) Application number: **06774001.9**

(22) Date of filing: **23.06.2006**

(51) Int Cl.:
***G06F 19/18*** *(2011.01)* ***G06F 19/28*** *(2011.01)*
***C12Q 1/68*** *(2006.01)*

(86) International application number:
**PCT/US2006/024805**

(87) International publication number:
**WO 2007/002586 (04.01.2007 Gazette 2007/01)**

(54) **METHODS FOR GENOTYPE SCREENING**

GENOTYP-SCREENING-VERFAHREN

PROCÉDÉS DE CRIBLAGE DE GÉNOTYPES

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.06.2005 US 166990**

(43) Date of publication of application:
**23.07.2008 Bulletin 2008/30**

(73) Proprietor: **Transnetyx, Inc.**
**Cordova, TN 38116 (US)**

(72) Inventor: **HODGE, Timothy A.**
**Eads, Tennessee 38028 (US)**

(74) Representative: **Boult Wade Tennant**
**Verulam Gardens**
**70 Gray's Inn Road**
**London WC1X 8BT (GB)**

(56) References cited:
**WO-A-02/20842**
**WO-A2-03/046141**
**US-A1- 2002 177 137**
**US-A1- 2003 165 922**

- **Caroline Turner ET AL: "Genomic DNA Purification from Cigarette Butts and Buccal Swabs Using the DNA IQ(TM) System", Promega Application Notes, 1 May 2003 (2003-05-01), pages 1-4, XP055090105, Retrieved from the Internet: URL:http://france.promega.com/~/media/files/resources/application notes/genetic identity/an106 genomic dna purification from cigarette butts and buccal swabs using the dna iq system.pdf?la=fr-FR [retrieved on 2013-11-26]**
- **GREENSPOON S A ET AL: "APPLICATION OF THE BIOMEK(R) 2000 LABORATORY AUTOMATION WORKSTATION AND THE DNA IQTM SYSTEM TO THE EXTRACTION OF FORENSIC CASEWORK SAMPLES", JOURNAL OF FORENSIC SCIENCES, CALLAGHAN AND CO, CHICAGO, IL, US, vol. 49, no. 1, 1 January 2004 (2004-01-01), pages 29-39, XP008068213, ISSN: 0022-1198, DOI: 10.1520/JFS2003179**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the Invention:

This invention relates to methods for genotype screening. More specifically, this invention relates to various methods to detect or screen for at least one designated genetic sequences in a plurality of biological samples.

Background of the Invention:

Genomic modification resulting from mutations in the DNA of an organism can be transferred to the progeny if such mutations are present in the gametes of the organism, referred to as germ-line mutations. These mutations may arise from genetic manipulation of the DNA using recombinant DNA technology or may be introduced by challenging the DNA by chemical or physical means. DNA introduced via recombinant DNA technology can be derived from many sources, including but not limited to DNA from viruses, mycoplasm, bacteria, fungi, yeast, and chordates including mammals such as humans.

Recombinant DNA technology allows for the introduction, deletion or replacement of DNA of an organism. Random introduction of DNA into a cell can be achieved by technologies such as transfection (including electroporation, lipofection), injection (pronuclear injection, nuclear transplantation) or transduction (viral infection). Random mutations (point mutations, deletions, amplifications) can be generated by treatment of cells with chemical mutagens or submitting them to physical insult such as X-irradiation or linear energy transfer irradiation (LET). Targeted addition, deletion or replacement of DNA in an organism (either inducible or non-inducible) is achieved via homologous recombination. Inducible systems employ sequence-specific recombinases such as Cre-LoxP (US patent numbers 5,654,182 and 5,677,177) and FLP/FRT (US patent number 5,527,695).

Transgenic organisms are organisms that carry DNA sequences (be it genes or gene segments) derived from another or the same species, stably integrated randomly into their genome. Transgenic mammals are generally created by microinjection of DNA into the pronucleus of fertilized eggs, a technique in which the number of DNA copies or the integration site of the DNA into the host genome is uncontrollable. A transgenic line or strain refers to an organism that transmits the foreign DNA sequences to its offspring.

Genotype screening is used to determine if a genome possesses specific genetic sequences that exist endogenously or have been modified, mutated or genetically engineered. Genomic nucleic acid is screened for these modifications, mutations or endogenous conditions. Genomic nucleic acid is challenging to work with because of its size. The genomic nucleic acid includes both coding and noncoding regions. Therefore, the genomic nucleic acid contains exons and introns, promoter and gene regulation regions, telomeres, origins or replication and nonfunctional intergenic nucleic acid. The genomic nucleic acid is a double stranded molecule which is methylated. cDNA and PCR-amplicons differs in that the molecules are much smaller. Additionally, biochemical modification events, such as methylation, do not occur with the smaller molecules. Shena, M (2000) DNA Microarrays: A Practical Approach. Oxford University Press, New York, NY.

Genotype screening is currently done manually. The present manual system is time-consuming and can provide variable results depending on the laboratory and even depending on skill of laboratory workers. Presently, a researcher using Southern blot technology may require greater than a week to screen a tissue sample for a transgene or a targeted mutation.

In an alternative technology, up to thirty PCR (polymerase chain reaction) can be conducted in an Eppendorf microtube® (Brinkmann Instruments, Westbury, NY) and separated on a gel. This process in most laboratories requires 3 to 7 days. A need exists in the industry to provide a system and method for more accurate, faster and high volume genotype screening.

Additionally, as researchers continue to use transgenic species in research specific information about the progeny of the transgenic species is of vital importance. An emerging technique in mouse mutant breeding is producing 'homozygous' transgenic conditions. During the initial creation of transgenic animals the transgene sequence integrates randomly into the host genome. Moreover, the number of transgene insertions also varies. Once the transgene is established in the genome, some investigators are interested in having this/these transgene(s) on the corresponding chromosome. The preferred mechanism for getting both chromosomes to have the transgene(s), is by breeding two transgenic animal from the same strain together. The goal is to identify homozygous animals that can then be bred to each other to ensure continual homozygous progeny. Typically, such transgenic animals are difficult to genotype by traditional PCR methods as accurate quantification is not possible with fragment-based analysis.

WO02/20842 and US2002/0177137 relate to methods, apparatus and systems for transgenic and targeted mutagenesis screening of genomic DNA.

US5705628 relates to a method of separating polynucleotides by reversible, nonspecific binding to a solid surface coated with a functional group.

Turner et al., Promega Application Notes, 2003, pages 1-4 discusses the purification of genomic DNA from cigarette butts and buccal swabs using the DNA IQ™ system.

Greenspoon et al., Journal of Forensic Sciences, 2004, vol. 49, pages 29-39 discusses the application of the BioMek® 2000 Laboratory Automation Workstation and the DNA IQ™ system to the extraction of DNA from forensic casework samples.

**Summary of the Invention:**

The present invention is defined in the claims. It provides a unique solution to the above-described problems by providing a method for rapid genotype screening. In particular, this invention provides a method to rapidly report screening results to a remote user from a screening laboratory for a plurality of biological samples. Efficient screening of a plurality of biological samples can be achieved by placing the sample to be screened in a well of a microwell container. The biological samples in the microwell containers are lysed to release at least a portion of intact genomic nucleic acid and cellular debris. A standard concentration of purified genomic nucleic acid is obtained by saturating the binding ability of the magnetic particles and by regulating the amount of genomic nucleic acid released. The purified genomic nucleic acid are screened to obtain screening results. The screening comprises: (i) adding at least one probe and primer set corresponding to one of said at least one designated genetic sequence to said biological sample in at least one well of a microwell container, (ii) adding at least one probe and primer set corresponding to a reference sequence to said biological nucleic acid sample in said at least one well of a microwell container, (iii) screening said biological sample in said at least one well of a microwell container to obtain screening results, wherein one of said screening results is a probe value, and (iv) comparing the probe values for said probe corresponding to said designated genetic sequence with said probe value corresponding to said reference sequence to detect said at least one designated genetic sequence. The screening results may be reported to a remote user according to some aspects of the invention. These screening results can include information on whether a designated genetic sequence is present in an organism and the zygosity of designated genetic sequences. Additionally, the zygosity of a transgene can be quantitatively determined and reported to a remote user.

Additionally, rapid screening can be obtained by using methods to evaluate the validity of the data obtained from screening. This method to evaluate the screening results comprises: (a) dispensing an aliquot of a standard concentration of a biological nucleic acid sample, wherein said biological sample is not human embryonic tissue or human embryonic stem cells, separated from a measured amount of magnetic particles saturated with the nucleic acid from the strain into at least one well of a microwell container, (b) adding at least one probe and primer set corresponding to a designated genetic sequence to said biological nucleic acid sample and at least one probe and primer set corresponding to a reference sequence to said at least one well of a microwell container, (c) screening said biological nucleic acid sample in said at least one well of said microwell container to obtain screening results wherein one of said screening results is a probe value, and (d) comparing the probe values for said probe corresponding to said designated genetic sequence with said probe value corresponding to said reference sequence to detect said at least one designated genetic sequence.

Brief Description of the Drawings:

A more complete understanding of the invention and its advantages will be apparent from the following Description of the Preferred Embodiment(s) taken in conjunction with the accompanying drawings, wherein:

**FIG.** 1 is an illustrative overview of the remote automated testing procedures of the present invention.

**FIG.** 2 is a block diagram of one embodiment of the system.

**FIG. 3** is a block diagram of the ordering procedure.

**FIG. 4** is a block diagram of account registration.

**FIGS. 5-6** illustrate the survey of work and sample identification sections.

**FIG. 7A** is a block diagram of the laboratory process system.

**FIG. 7B** is a block diagram of the laboratory process system.

**FIG. 7C** is a block diagram of the laboratory process system.

**FIG. 7D** is a block diagram of the laboratory process system.

**FIG. 8** is a block diagram of standard laboratory stations.

**FIG. 9** is a screen display illustrating a document on the transgenic screening laboratory **20**'s web site relating to an outcome file.

**FIG. 10** is a graphical representation of the results.

**FIG. 11** is a graphical representation of signal magnitude.

**FIG. 12** is a graphical representation of signal magnitude.

**FIG. 13** is a graphical representation of signal magnitude.

**FIGS. 14** and **15** illustrate a preferred device for performing the functions of a Lysing Station and an Automated Accessioning Station as described herein, including an oven (**FIG**. **15)** for incubating the samples.

**FIG. 16** illustrates a preferred device for performing the functions of an Isolation/Purification Station as described herein.

**FIG. 17** illustrates a preferred device for drying samples.

**FIG. 18** illustrates a preferred device for performing the functions of a Screening Station as described herein.

**FIG. 19** illustrates a preferred device for performing the functions of a Detection Station as described herein.

Detailed Description of the Preferred Embodiments:

The present invention provides a method for high volume genotype screening. This invention provides a method for rapid identification of an organism, whose genome possesses specific genetic sequences that exist endogenously or has been modified, mutated or genetically engineered.

1. Definitions:

The following terms and acronyms are used throughout the detailed description.
**Alox5-KO**

TGCCCAGCGGTCCTATCTAGAGGTCATTCTCTCCACAGAGCGAGTCAAGAACCACTG
GCAGGAAGACCTCATGTTTGGCTACCAGTTCCTGAATGGCTGCAACCCAGTAATTCT
ACCGGGTAGGGGAGGCGCTTTTCCCAAGGCAGTCTGGAGCATGCGCTTTAGCAGCCC
CGCTGGCACTTGGCGCTACACAAGTGGCCTCTGGCCTCGCACACATTCCACATCCAC

CGGTAGCGCCAACCGGCTCCGTTCTTTGGTGGCCCCTTCGCGCCACCTTCTACTCCTC
CCCTAGTCAGGAAGTTCCCCCCCGCCCCGCAGCTCGCGTCGTGCAGGACGTGACAAA
TGGAAGTAGCACGTCTCACTAGTCTCGTGCAGATGGACAGCACCGCTGAGCAATGG
AAGCGGGTAGGCCTTTGGGGCAGCGGCCAATAGCAGCTTTGCTCCTTCGCTTTCTGG
GCTCAGAGGCTGGGAAGGGGTGGGTCCGGGGGCGGGCTCAGGG (SEQ ID NO. 1)

| | |
|---|---|
| ***Forward Primer Seq.:*** | TTGGCTACCAGTTCCTGAATGG (SEQ ID NO. 2) |
| ***Reverse Primer Seq.:*** | CAGACTGCCTTGGGAAAAGC (SEQ ID NO. 3) |
| ***Probe:*** | CTGCAACCCAGTAATTC (SEQ ID NO. 4) |

**ALOX5-WT**

AAGAACCACTGGCAGGAAGACCTCATGTTTGGCTACCAGTTCCTGAATGGCTGCAAC
CCAGTACTCATCAAGCGCTGCACAGCGTTGCCCCCGAAGCTCCCAGTGACCACAGAG
ATGGTGGAGTGCAGCCTAGAGCGGCAGCTCAGTTTAGAACA (SEQ ID NO. 5)

| | |
|---|---|
| ***Forward Primer Seq.:*** | TTGGCTACCAGTTCCTGAATGG (SEQ ID NO. 6) |
| ***Reverse Primer Seq.:*** | CTGTGGTCACTGGGAGCTT (SEQ ID NO. 7) |
| ***Probe:*** | CTGCAACCCAGTACTCAT (SEQ ID NO. 8) |

**APC Min**

TATCATGTCTCCCGGCTCAAGTCTGCCATCCCTTCACGTTAGGAAACAGAAAGCTCT
AGAAGCTGAGCTAGATGCTCAGCATTTATCAGAAACCTTCGACAACATTGACAACCT
AAGTCCCAAGGCCTCTCACCGGAGTAAGCAGAGACACAAGCAGAATCTTTATGGTG
ACTATGCTTTTGACGCCAATCGACATGATGATAGTAGGTCAGACAATTTCAATACTG
GAAACATGACTGTTCTTTCACCATATTTAAATACTACGGTATTGCCCAGCTCTTCTTC
CTCAAGGGGAAGTTTAGACAGTTCTCGTTCTGAGAAAGACAGAAGTTAGGAGAGAG
AGCGAGGTATTGGCCTCAGTGCTTACCATCCAACAACAGAAAATGCAGGAACCTCAT
CAAAACGAGGTCTGCAGATCACTACCACTGCAGCCCAGATAGCCAAAGTTATGGAA
GAAGTATCAGCCATTCATACCTCCCAGGACGACAGAAGTTCTGCTTCTACCACCGAG
TTCCATTGTGTGGCAGACGACAGGAGTGCGGCACGAAGAAGCTCTGCCTNNNNNNN
NNNNNNNNNNNNNNNNNNNCTTCACTAAGTCGGAAAATTCAAATAGGACATGCTCTA
TGCCTTATGCCAAAGTGGAATATAAACGATCTTCAAATGACAGTTTAAATA
GTGTCACTAGTA (SEQ ID NO. 9)

| | |
|---|---|
| ***Forward Primer:*** | GGGAAGTTTAGACAGTTCTCGTTCT (SEQ ID NO. 10) |
| ***Reverse Primer:*** | GTAAGCACTGAGGCCAATACCT (SEQ ID NO. 11) |
| ***Probe 1:*** | CTCTCTCCAAACTTC (SEQ ID NO. 12) |
| ***Probe 2:*** | TCTCTCTCCTAACTTC (SEQ ID NO. 13) |

**Bgal**

GTTGAGAATGAGTACGGGTCCTACTTTGCCTGCGATTACGACTACCTACGCTTCCTG
GTGCACCGCTTCCGCTACCATCTGGGTAATGACGTCATTCTCTTCACCACCGACGGA
GCAAGTGAAAAAATGCTGAAGTGTGGGACCCTGCAGGACCTGTACGCCACAGTGGA
TTTTGGAACAG (SEQ ID NO. 14)

| | |
|---|---|
| ***Forward Primer Seq.:*** | CACCGCTTCCGCTACCAT (SEQ ID NO. 15) |
| ***Reverse Primer Seq.:*** | GCTCCGTCGGTGGTGAAG (SEQ ID NO. 16) |
| ***Probe:*** | CTGGGTAATGACGTCATTCT (SEQ ID NO. 17) |

**complementary** - chemical affinity between nitrogenous bases as a result of hydrogen bonding. Responsible for the base pairing between nucleic acid strands. Klug, W.S. and Cummings, M.R. (1997) Concepts of Genetics, fifth ed., Prentice-Hall, Upper Saddle River, NJ.

**copy number** - the number of transgenes that have randomly integrated into the genome.

***Cjun* - (housekeeping or reference sequence)**

GACCGGTAACAAGTGGCCGGGAGCGAACTTTTGCAAATCTCTTCTGCGCCTTAAGGC
TGCCACCGAGACTGTAAAGAAAAGGGAGAAGAGGAACCTATACTCATACCAGTTCG
CACAGGCGGCTGAAGTTGGGCGAGCGCTAGCCGCGGCTGCCTAGCGTCCCCCTCCCC
CTCACAGCGGAGGAGGGGACAGTTGTCGGAGGCCGGGCGGCAGAGCCCGATCGCGG
GCTTCCACCGAGAATTCCGTGACGACTGGTCAGCACCGCCGGAGAGCCGCTGTTGCT
GGGACTGGTCTGCGGGCTCCAAGGAACCGCTGCTCCCCGAGAGCGCTCCGTGAGTG
ACCGCGACTTTTCAAAGCTCGGCATCGCGCGGGAGCCTACCAACGTGAGTGCTAGCG
GAGTCTTAACCCTGCGCTCCCTGGAGCGAACTGGGGAGGAGGGCTCAGGGGGAAGC
ACTGCCGTCTGGAGCGCACGCTCCTAAACAAACTTTGTTACAGAAGCGGGGACGCGC
GGGTATCCCCCCGCTTCCCGGCGCGCTGTTGCGGCCCCGAAACTTCTGCGCACAGCC
CAGGCTAACCCCGCGTGAAGTGACGGACCGTTCTATGACTGCAAAGATGGAAACGA
CCTTCTACGACGATGCCCTCAACGCCTCGTTCCTCCAGTCCGAGAGCGGTGCCTACG
GCTACAGTAACCCTAAGATCCTAAAACAGAGCATGACCTTGAACCTGGCCGACCCG
GTGGGCAGTCTGAAGCCGCACCTCCGCGCCAAGAACTCGGACCTTCTCACGTCGCCC

GACGTCGGGCTGCTCAAGCTGGCGTCGCCGGAGCTGGAGCGCCTGATCATCCAGTCC AGCAATGGGCACATCACCACTACACCGACCCCCACCCAGTTCTTGTGCCCCAAGAAC GTGACCGACGAGCAGGAGGGCTTCGCCGAGGGCTTCGTGCGCGCCCTGGCTGAACT GCATAGCCAGAACACGCTTCCCAGTGTCACCTCCGCGGCACAGCCGGTCAGCGGGG CGGGCATGGTGGCTCCCGCGGTGGCCTCAGTAGCAGGCGCTGGCGGCGGTGGTGGC TACAGCGCCAGCCTGCACAGTGAGCCTCCGGTCTACGCCAACCTCAGCAACTTCAAC CCGGGTGCGCTGAGCAGCGGCGGTGGGGCGCCCTCCTATGGCGCGGCCGGGCTGGC CTTTCCCTCGCAGCCGCAGCAGCAGCAGCAGCCGCCTCAGCCGCCGCACCACTTGCC CCAACAGATCCCGGTGCAGCACCCGCGGCTGCAAGCCCTGAAGGAAGAGCCGCAGA CCGTGCCGGAGATGCCGGGAGAGACGCCGCCCCTGTCCCCTATCGACATGGAGTCTC AGGAGCGGATCAAGGCAGAGAGGAAGCGCATGAGGAACCGCATTGCCGCCTCCAAG TGCCGGAAAAGGAAGCTGGAGCGGATCGCTCGGCTAGAGGAAAAAGTGAAAACCTT GAAAGCGCAAAACTCCGAGCTGGCATCCACGGCCAACATGCTCAGGGAACAGGTGG CACAGCTTAAGCAGAAAGTCATGAACCACGTTAACAGTGGGTGCCAACTCATGCTA ACGCAGCAGTTGCAAACGTTTTGAGAACAGACTGTCAGGGCTGAGGGGCAATGGAA GAAAAAAAATAACAGAGACAAACTTGAGAACTTGACTGGTTGCGACAGAGAAAAA AAAAGTGTCCGAGTACTGAAGCCAAGGGTACACAAGATGGACTGGGTTGCGACCTG ACGGCGCCCCCAGTGTGCTGGAGTGGGAAGGACGTGGCGCGCCTGGCTTTGGCGTG GAGCCAGAGAGCAGCGGCCTATTGGCCGGCAGACTTTGCGGACGGGCTGTGCCCGC GCGCGACCAGAACGATGGACTTTTCGTTAACATTGACCAAGAACTGCATGGACCTAA CATTCGATCTCATTCAGTATTAAAGGGGGGTGGGAGGGGTTACAAACTGCAATAGA GACTGTAGATTGCTTCTGTAGTGCTCCTTAACACAAAGCAGGGAGGGCTGGGAAGG GGGGGGAGGCTTGTAAGTGCCAGGCTAGACTGCAGATGAACTCCCCTGGCCTGCCTC TCTCAACTGTGTATGTACATATATATTTTTTTTTAATTTGATGAAAGCTGATTACTGTC AATAAACAGCTTCCTGCCTTTGTAAGTTATTCCATGTTTGTTTGTTTGGGTGTCCTGC CC (SEQ ID NO. 18)

***Forward Primer:*** GAGTGCTAGCGGAGTCTTAACC (SEQ ID NO. 19)
***Reverse Primer:*** CTCCAGACGGCAGTGCTT (SEQ ID NO. 20)
***Probe:*** AAGCACTGCCGTCTGGAG (SEQ ID NO. 21)

**Cre**

ATGCCCAAGAAGAAGAGGAAGGTGTCCAATTTACTGACCGTACACCAAAATTTGCCT
GCATTACCGGTCGATGCAACGAGTGATGAGGTTCGCAAGAACCTGATGGACATGTTC
AGGGATCGCCAGGCGTTTTCTGAGCATACCTGGAAAATGCTTCTGTCCGTTTGCCGG
TCGTGGGCGGCATGGTGCAAGTTGAATAACCGGAAATGGTTTCCCGCAGAACCTGA
AGATGTTCGCGATTATCTTCTATATCTTCAGGCGCGCGGTCTGGCAGTAAAAACTAT
CCAGCAACATTTGGGCCAGCTAAACATGCTTCATCGTCGGTCCGGGCTGCCACGACC
AAGTGACAGCAATGCTGTTTCACTGGTTATGCGGCGGATCCGAAAAGAAAACGTTG
ATGCCGGTGAACGTGCAAAACAGGCTCTAGCGTTCGAACGCACTGATTTCGACCAGG
TTCGTTCACTCATGGAAAATAGCGATCGCTGCCAGGATATACGTAATCTGGCATTTC
TGGGGATTGCTTATAACACCCTGTTACGTATAGCCGAAATTGCCAGGATCAGGGTTA
AAGATATCTCACGTACTGACGGTGGGAGAATGTTAATCCATATTGGCAGAACGAAA
ACGCTGGTTAGCACCGCAGGTGTAGAGAAGGCACTTAGCCTGGGGGTAACTAAACT
GGTCGAGCGATGGATTTCCGTCTCTGGTGTAGCTGATGATCCGAATAACTACCTGTTT
TGCCGGGTCAGAAAAAATGGTGTTGCCGCGCCATCTGCCACCAGCCAGCTATCAACT
CGCGCCCTGGAAGGGATTTTTGAAGCAACTCATCGATTGATTTACGGCGCTAAGGAT
GACTCTGGTCAGAGATACCTGGCCTGGTCTGGACACAGTGCCCGTGTCGGAGCCGCG
CGAGATATGGCCCGCGCTGGAGTTTCAATACCGGAGATCATGCAAGCTGGTGGCTGG
ACCAATGTAAATATTGTCATGAACTATATCCGTAACCTGGATAGTGAAACAGGGGCA
ATGGTGCGCCTGCTGGAAGATGGCGATTAGCCATTAACGCGTAAATGATTGCTATAA
TTATTTGATAT (SEQ ID: NO. 22)

***Forward Primer:*** TTAATCCATATTGGCAGAACGAAAACG (SEQ ID: NO. 23)
***Reverse Primer:*** CAGGCTAAGTGCCTTCTCTACA (SEQ ID: NO. 24)
***Probe:*** CCTGCGGTGCTAACC (SEQ ID: NO. 25)

**designated genetic sequence** - includes a transgenic insert, a selectable marker, microsatellite loci, recombinant site or any gene or gene segment.

**DNA (deoxyribonucleic acid) -** One of the two main types of nucleic acid, consisting of a long, unbranched macromolecule formed from one, or more commonly, two, strands of linked deoxyribonucleotides, the 3"-phosphate group of each constituent deoxyribonucleotide being joined in 3', 5'-phosphodiester linkage to the 5'-hydroxyl group of the deoxyribose moiety of the next one. Oxford Dictionary of Biochemistry and Molecular Biology; p. 182.

**embryonic stem cells (ES cells) -** a cell of the early embryo that can replicate indefinitely and which can differentiate into other cells; stem cells serve as a continuous source of new cells.

**genome -** all the genetic material in the chromosomes of a particular organism; its size is generally given as its total number of base pairs.

**genomic nucleic acid -** The genomic nucleic acid includes both coding and noncoding regions. Therefore, the genomic nucleic acid contains exons and introns, promoter and gene regulation regions, telomeres, origins or replication and nonfunctional intergenic nucleic acid. The genomic nucleic acid is a double stranded molecule which is methylated. cDNA and PCR-amplicons differs in that the molecules are much smaller. Additionally, biochemical modification events, such as methylation, do not occur with the smaller molecules. Shena, M (2000) DNA Microarrays: A Practical Approach. Oxford University Press, New York, NY.

**genotype -** genetic constitution of an individual cell or organism that can include at least one designated gene sequence.

**hemizygous** - a situation within a cell or organism where only one copy of a gene, group of genes or genetic sequence is present instead of two copies in a diploid genome.

**heterozygosity** - the state of having two different genes (alleles) at one or more corresponding loci on homologous chromosomes.

**homozygosity** - The state of having the same genes (alleles) at one or more corresponding homologous chromosomes.

**HumanTTTy8**

AAAGAAGAGCAGCACGTCATACCCAAGACCAACATCTCTCAGTGTTTCACGCTAACC
CAAGGAGAGACACTAGCAGTCTTCTCTGCAGGACCCCTTGAATTTACATTGAATTCC
ATCCCCAGCCGAGCAGGTGCTTAAAGTCAACAGGGGACACTCCATTTTCTTGGAATT
TCATTCTGGCAAAGAGGGTGTGAGCAGCAATAAG (SEQ ID NO. 26)

| | |
|---|---|
| ***Forward Primer Seq.:*** | GCAGGACCCCTTGAATTTACATTGA (SEQ ID NO. 27) |
| ***Reverse Primer Seq.:*** | TGGAGTGTCCCCTGTTGACT (SEQ ID NO. 28) |
| ***Probe:*** | CCGAGCAGGTGCTTAA (SEQ ID NO. 29) |

**Hygromycin**

ATGAAAAAGCCTGAACTCACCGCGACGTCTGTCGAGAAGTTTCTGATCGAAAAGTTC
GACAGCGTCTCCGACCTGATGCAGCTCTCGGAGGGCGAAGAATCTCGTGCTTTCAGC
TTCGATGTAGGAGGGCGTGGATATGTCCTGCGGGTAAATAGCTGCGCCGATGGTTTC
TACAAAGATCGTTATGTTTATCGGCACTTTGCATCGGCCGCGCTCCCGATTCCGGAA
GTGCTTGACATTGGGGAATTCAGCGAGAGCCTGACCTATTGCATCTCCCGCCGTGCA
CAGGGTGTCACGTTGCAAGACCTGCCTGAAACCGAACTGCCCGCTGTTCTGCAGCCG
GTCGCGGAGGCCATGGATGCGATCGCTGCGGCCGATCTTAGCCAGACGAGCGGGTT
CGGCCCATTCGGACCGCAAGGAATCGGTCAATACACTACATGGCGTGATTTCATATG
CGCGATTGCTGATCCCCATGTGTATCACTGGCAAACTGTGATGGACGACACCGTCAG
TGCGTCCGTCGCGCAGGCTCTCGATGAGCTGATGCTTTGGGCCGAGGACTGCCCCGA
AGTCCGGCACCTCGTGCACGCGGATTTCGGCTCCAACAATGTCCTGACGGACAATGG
CCGCATAACAGCGGTCATTGACTGGAGCGAGGCGATGTTCGGGGATTCCCAATACG
AGGTCGCCAACATCTTCTTCTGGAGGCCGTGGTTGGCTTGTATGGAGCAGCAGACGC
GCTACTTCGAGCGGAGGCATCCGGAGCTTGCAGGATCGCCGCGGCTCCGGGCGTATA
TGCTCCGCATTGGTCTTGACCAACTCTATCAGAGCTTGGTTGACGGCAATTTCGATGA
TGCAGCTTGGGCGCAGGGTCGATGCGACGCAATCGTCCGATCCGGAGCCGGGACTG
TCGGGCGTACACAAATCGCCCGCAGAAGCGCGGCCGTCTGGACCGATGGCTGTGTA
GAAGTACTCGCCGATAGTGGAAACCGACGCCCCAGCACTCGTCCGAGGGCAAAGGA
ATAG (SEQ ID: No. 30)

***Forward Primer:*** CGCAAGGAATCGGTCAATACACTA (SEQ ID NO.: 31)
***Reverse Primer:*** CACAGTTTGCCAGTGATACACATG (SEQ ID NO.: 32)
***Probe:*** CATGGCGTGATTTCAT (SEQ ID NO.: 33)

**internet -** a collection of interconnected (public and/or private) networks that are linked together by a set of standard protocols to form a global, distributed network. The World Wide Web (hereinafter web) refers to both a distributed collection of interlinked, user viewable hypertext documents (commonly referred to as web pages) that are accessible via the Internet and the user and server software components which provide user access to such documents using standard Internet protocols.

**line** - A line is a group of organisms bred for a genotype (i.e. at least one designated genetic sequence).

**MHV**

TATAAGAGTGATTGGCGTCCGTACGTACCCTCTCAACTCTAAAACTCTTGTAGTTTAA

ATCTAATCTAAACTTTATAAACGGCACTTCCTGCGTGTCCATGCCCGCGGGCCTGGTC
TTGTCATAGTGCTGACATTTGTAGTTCCTTGACTTTCGTTCTCTGCCAGTGACGTGTC
CATTCGGCGCCAGCAGCCCACCCATAGGTTGCATAATGGCAAAGATGGGCAAATAC
GGTCTCGGCTTCAAATGGGCCCCAGAATTTCCATGGATGCTTCCGAACGCATCGGAG
AAGTTGGGTAACCCTGAGAGGTCAGAGGAGGATGGGTTTTGCCCCTCTGCTGCGCAA
GAACCGAAAGTTAAAGGAAAAACTTTGGTTAATCACGTGAGGGTGAATTGTAGCCG
GCTTCCAGCTTTGGAATGCTGTGTTCAGTCTGCCATAATCCGTGATATTTTTGTAGAT
GAGGATCCCCAGAAGGTGGAGGCCTCAACTATGATGGCATTGCAGTTCGGTAGTGCC
GTCTTGGTTAAGCCATCCAAGCGCTTGTCTATTCAGGCATGGACTAATTTGGGTGTGC
TTCCCAAAACAGCTGCCATGGGGTTGTTCAAGCGCGTCTGCCTGTGTAACACCAGGG
AGTGCTCTTGTGACGCCCACGTGGCCTTTCACCTTTTTACGGTCCAACCCGATGGTGT
ATGCCTGGGTAATGGCCGTTTTATAGGCTGGTTCGTTCCAGTCACAGCCATACCGGA
GTATGCGAAGCAGTGGTTGCAACCCTGGTCCATCCTTCTTCGTAAGGGTGGTAACAA
AGGGTCTGTGACATCCGGCCACTTCCGCCGCGCTGTTACCATGCCTGTGTATGACTTT
AATGTAGAGGATGCTTGTGAGGAGGTTCATCTTAACCCGAAGGGTAAGTACTCCTGC
AAGGCGTATGCTCTTCTTAAGGGCTATCGCGGTGTTAAGCCCATCCTGTTTGTGGACC
AGTATGGTTGCGACTATACTGGATGTCTCGCCAAGGGTCTTGAGGACTATGGCGATC
TCACCTTGAGTGAGATGAAGGAGTTGTTCCCTGTGTGGCGTGACTCCTTGGATAGTG
AAGTCCTTGTGGCTTGGCACGTTGATCGAGATCCTCGGGCTGCTATGCGTCTGCAGA
CTCTTGCTACTGTACGTTGCATTGATTATGTGGGCCAACCGACCGAGGATGTGGTGG
ATGGAGATGTGGTAGTGCGTGAGCCTGCTCATCTTCTCGCAGCCAATGCCATTGTTA
AAAGACTCCCCCGTTTGGTGGAGACTATGCTGTATACGGATTCGTCCGTTACAGAAT
TCTGTTATAAAACCAAGCTGTGTGAATGCGGTTTTATCACGCAGTTTGGCTATGTGG
ATTGTTGTGGTGACACCTGCGATTTTCGTGGGTGGGTTGCCGGCAATATGATGGATG
GCTTTCCATGTCCAGGGTGTACCAAAAATTATATGCCCTGGGAATTGGAGGCCCAGT
CATCAGGTGTTATACCAGAAGGAGGTGTTCTATTCACTCAGAGCACTGATACAGTGA
ATCGTGAGTCCTTTAAGCTCTACGGTCATGCTGTTGTGCCTTTTGGTTCTGCTGTGTA
TTGGAGCCCTTGCCCAGGTATGTGGCTTCCAGTAATTTGGTCTTCTGTTAAGTCATAC
TCTGGTTTGACTTATACAGGAGTAGTTGGTTGTAAGGCAATTGTTCAAGAGACAGAC
GCTATATGTCGTTCTCTGTATATGGATTATGTCCAGCACAAGTGTGGCAATCTCGAGC
AGAGAGCTATCCTTGGATTGGACGATGTCTATCATAGACAGTTGCTTGTGAATAGGG
GTGACTATAGTCTCCTCCTTGAGAATGTGGATTTGTTTGTTAAGCGGCGCGCTGAATT
TGCTTGCAAATTCGCCACCTGTGGAGATGGTCTTGTACCCCTCCTACTAGATGGTTTA
GTGCCCCGCAGTTATTATTTGATTAAGAGTGGTCAAGCTTTCACCTCTATGATGGTTA

ATTTTAGCCATGAGGTGACTGACATGTGTATGGACATGGCTTTATTGTTCATGCATGA
TGTTAAAGTGGCCACTAAGTATGTTAAGAAGGTTACTGGCAAACTGGCCGTGCGCTT
TAAAGCGTTGGGTGTAGCCGTTGTCAGAAAAATTACTGAATGGTTTGATTTAGCCGT
GGACATTGCTGCTAGTGCCGCTGGATGGCTTTGCTACCAGCTGGTAAATGGCTTATTT
GCAGTGGCCAATGGTGTTATAACCTTTGTACAGGAGGTGCCTGAGCTTGTCAAGAAT
TTTGTTGACAAGTTCAAGGCATTTTTCAAGGTTTTGATCGACTCTATGTCGGTTTCTA
TCTTGTCTGGACTTACTGTTGTCAAGACTGCCTCAAATAGGGTGTGTCTTGCTGGCAG
TAAGGTTTATGAAGTTGTGCAGAAATCTTTGTCTGCATATGTTATGCCTGTGGGTTGC
AGTGAAGCCACTTGTTTGGTGGGTGAGATTGAACCTGCAGTTTTTGAAGATGATGTT
GTTGATGTGGTTAAAGCCCCATTAACATATCAAGGCTGTTGTAAGCCACCCACTTCTT
TCGAGAAGATTTGTATTGTGGATAAATTGTATATGGCCAAGTGTGGTGATCAATTTT
ACCCTGTGGTTGTTGATAACGACACTGTTGGCGTGTTAGATCAGTGCTGGAGGTTTC
CCTGTGCGGGCAAGAAAGTCGAGTTTAACGACAAGCCCAAAGTCAGGAAGATACCC
TCCACCCGTAAGATTAAGATCACCTTCGCACTGGATGCGACCTTTGATAGTGTTCTTT
CGAAGGCGTGTTCAGAGTTTGAAGTTGATAAAGATGTTACATTGGATGAGCTGCTTG
ATGTTGTGCTTGACGCAGTTGAGAGTACGCTCAGCCCTTGTAAGGAGCATGATGTGA
TAGGCACAAAAGTTTGTGCTTTACTTGATAGGTTGGCAGGAGATTATGTCTATCTTTT
TGATGAGGGAGGCGATGAAGTGATCGCCCCGAGGATGTATTGTTCCTTTTCTGCTCC
TGATGATGAAGACTGCGTTGCAGCGGATGTTGTAGATGCAGATGAAAACCAAGATG
ATGATGCTGAAGACTCAGCAGTCCTTGTCGCTGATACCCAAGAAGAGGACGGCGTTG
CCAAGGGGCAGGTTGAGGCGGATTCGGAAATTTGCGTTGCGCATACTGGTAGTCAA
GAAGAATTGGCTGAGCCTGATGCTGTCGGATCTCAAACTCCCATCGCCTCTGCTGAG
GAAACCGAAGTCGGAGAGGCAAGCGACAGGGAAGGGATTGCTGAGGCGAAGGCAA
CTGTGTGTGCTGATGCTGTAGATGCCTGCCCCGATCAAGTGGAGGCATTTGAAATTG
AAAAGGTTGAAGACTCTATCTTGGATGAGCTTCAAACTGAACTTAATGCGCCAGCGG
ACAAGACCTATGAGGATGTCTTGGCATTCGATGCCGTATGCTCAGAGGCGTTGTCTG
CATTCTATGCTGTGCCGAGTGATGAGACGCACTTTAAAGTGTGTGGATTCTATTCGCC
TGCTATAGAGCGCACTAATTGTTGGCTGCGTTCTACTTTGATAGTAATGCAGAGTCTA
CCTTTGGAATTTAAAGACTTGGAGATGCAAAAGCTCTGGTTGTCTTACAAGGCCGGC
TATGACCAATGCTTTGTGGACAAACTAGTTAAGAGCGTGCCCAAGTCTATTATCCTT
CCACAAGGTGGTTATGTGGCAGATTTTGCCTATTTCTTTCTAAGCCAGTGTAGCTTTA
AAGCTTATGCTAACTGGCGTTGTTTAGAGTGTGACATGGAGTTAAAGCTTCAAGGCT
TGGACGCCATGTTTTTCTATGGGGACGTTGTGTCTCATATGTGCAAGTGTGGTAATAG
CATGACCTTGTTGTCTGCAGATATACCCTACACTTTGCATTTTGGAGTGCGAGATGAT

AAGTTTTGCGCTTTTTACACGCCAAGAAAGGTCTTTAGGGCTGCTTGTGCGGTAGAT
GTTAATGATTGTCACTCTATGGCTGTAGTAGAGGGCAAGCAAATTGATGGTAAAGTG
GTTACCAAATTTATTGGTGACAAATTTGATTTTATGGTGGGTTACGGGATGACATTTA
GTATGTCTCCTTTTGAACTCGCCCAGTTATATGGTTCATGTATAACACCAAATGTTTG
TTTTGTTAAAGGAGATGTTATAAAGGTTGTTCGCTTAGTTAATGCTGAAGTCATTGTT
AACCCTGCTAATGGGCGTATGGCTCATGGTGCAGGTGTTGCAGGTGCTATAGCTGAA
AAGGCGGGCAGTGCTTTTATTAAAGAAACCTCCGATATGGTGAAGGCTCAGGGCGTT
TGCCAGGTTGGTGAATGCTATGAATCTGCCGGTGGTAAGTTATGTAAAAAGGTGCTT
AACATTGTAGGGCCAGATGCGCGAGGGCATGGCAAGCAATGCTATTCACTTTTAGAG
CGTGCTTATCAGCATATTAATAAGTGTGACAATGTTGTCACTACTTTAATTTCGGCTG
GTATATTTAGTGTGCCTACTGATGTCTCCCTAACTTACTTACTTGGTGTAGTGACAAA
GAATGTCATTCTTGTCAGTAACAACCAGGATGATTTTGATGTGATAGAGAAGTGTCA
GGTGACCTCCGTTGCTGGTACCAAAGCGCTATCACTTCAATTGGCCAAAAATTTGTG
CCGTGATGTAAAGTTTGTGACGAATGCATGTAGTTCGCTTTTTAGTGAATCTTGCTTT
GTCTCAAGCTATGATGTGTTGCAGGAAGTTGAAGCGCTGCGACATGATATACAATTG
GATGATGATGCTCGTGTCTTTGTGCAGGCTAATATGGACTGTCTGCCCACAGACTGG
CGTCTCGTTAACAAATTTGATAGTGTTGATGGTGTTAGAACCATTAAGTATTTTGAAT
GCCCGGGCGGGATTTTTGTATCCAGCCAGGGCAAAAAGTTTGGTTATGTTCAGAATG
GTTCATTTAAGGAGGCGAGTGTTAGCCAAATAAGGGCTTTACTCGCTAATAAGGTTG
ATGTCTTGTGTACTGTTGATGGTGTTAACTTCCGCTCCTGCTGCGTAGCAGAGGGTGA
AGTTTTTGGCAAGACATTAGGTTCAGTCTTTTGTGATGGCATAAATGTCACCAAAGTT
AGGTGTAGTGCCATTTACAAGGGTAAGGTTTTCTTTCAGTACAGTGATTTGTCCGAG
GCAGATCTTGTGGCTGTTAAAGATGCCTTTGGTTTTGATGAACCACAACTGCTGAAG
TACTACACTATGCTTGGCATGTGTAAGTGGTCAGTAGTTGTTTGTGGCAATTATTTTG
CTTTCAAGCAGTCAAATAATAATTGCTATATAAATGTGGCATGTTTAATGCTGCAAC
ACTTGAGTTTAAAGTTTCCTAAGTGGCAATGGCAAGAGGCTTGGAACGAGTTCCGCT
CTGGTAAACCACTAAGGTTTGTGTCCTTGGTATTAGCAAAGGGCAGCTTTAAATTTA
ATGAACCTTCTGATTCTATCGATTTTATGCGTGTGGTGCTACGTGAAGCAGATTTGAG
TGGTGCCACGTGCAATTTGGAATTTGTTTGTAAATGTGGTGTGAAGCAAGAGCAGCG
CAAAGGTGTTGACGCTGTTATGCATTTTGGTACGTTGGATAAAGGTGATCTTGTCAG
GGGTTATAATATCGCATGTACGTGCGGTAGTAAACTTGTGCATTGCACCCAATTTAA
CGTACCATTTTTAATTTGCTCCAACACACCAGAGGGTAGGAAACTGCCCGACGATGT
TGTTGCAGCTAATATTTTTACTGGTGGTAGTGTGGGCCATTACACGCATGTGAAATGT
AAACCCAAGTACCAGCTTTATGATGCTTGTAATGTTAATAAGGTTTCGGAGGCTAAG

GGTAATTTTACCGATTGCCTCTACCTTAAAAATTTAAAGCAAACTTTTTCGTCTGTGC
TGACGACTTTTTATTTAGATGATGTAAAGTGTGTGGAGTATAAGCCAGATTTATCGC
AGTATTACTGTGAGTCTGGTAAATATTATACAAAACCCATTATTAAGGCCCAATTTA
GAACATTTGAGAAGGTTGATGGTGTCTATACCAACTTTAAATTGGTGGGACATAGTA
TTGCTGAAAAACTCAATGCTAAGCTGGGATTTGATTGTAATTCTCCCTTTGTGGAGTA
TAAAATTACAGAGTGGCCAACAGCTACTGGAGATGTGGTGTTGGCTAGTGATGATTT
GTATGTAAGTCGGTACTCAAGCGGGTGCATTACTTTTGGTAAACCGGTTGTCTGGCTT
GGCCATGAGGAAGCATCGCTGAAATCTCTCACATATTTTAATAGACCTAGTGTCGTT
TGTGAAAATAAATTTAATGTGTTGCCCGTTGATGTCAGTGAACCCACGGACAAGGGG
CCTGTGCCTGCTGCAGTCCTTGTTACCGGCGTCCCTGGAGCTGATGCGTCAGCTGGTG
CCGGTATTGCCAAGGAGCAAAAAGCCTGTGCTTCTGCTAGTGTGGAGGATCAGGTTG
TTACGGAGGTTCGTCAAGAGCCATCTGTTTCAGCTGCTGATGTCAAAGAGGTTAAAT
TGAATGGTGTTAAAAAGCCTGTTAAGGTGGAAGGTAGTGTGGTTGTTAATGATCCCA
CTAGCGAAACCAAAGTTGTTAAAAGTTTGTCTATTGTTGATGTCTATGATATGTTCCT
GACAGGGTGTAAGTATGTGGTTTGGACTGCTAATGAGTTGTCTCGACTAGTAAATTC
ACCGACTGTTAGGGAGTATGTGAAGTGGGGTAAGGGAAAGATTGTAACACCCGCTA
AGTTGTTGTTGTTAAGAGATGAGAAGCAAGAGTTCGTAGCGCCAAAAGTAGTCAAG
GCGAAAGCTATTGCCTGCTATTGTGCTGTGAAGTGGTTTCTCCTCTATTGTTTTAGTT
GGATAAAGTTTAATACTGATAATAAGGTTATATACACCACAGAAGTAGCTTCAAAGC
TTACTTTCAAGTTGTGCTGTTTGGCCTTTAAGAATGCCTTACAGACGTTTAATTGGAG
CGTTGTGTCTAGGGGCTTTTTCCTAGTTGCAACGGTCTTTTTATTATGGTTTAACTTTT
TGTATGCTAATGTTATTTTGAGTGACTTCTATTTGCCTAATATTGGGCCTCTCCCTAC
GTTTGTGGGACAGATAGTTGCGTGGTTTAAGACTACATTTGGTGTGTCAACCATCTGT
GATTTCTACCAGGTGACGGATTTGGGCTATAGAAGTTCGTTTTGTAATGGAAGTATG
GTATGTGAACTATGCTTCTCAGGTTTTGATATGCTGGACAACTATGATGCTATAAATG
TTGTTCAACACGTTGTAGATAGGCGTTTGTCCTTTGACTATATTAGCCTATTTAAATT
AGTAGTTGAGCTTGTAATCGGCTACTCTCTTTATACTGTGTGCTTCTACCCACTGTTT
GTCCTTATTGGAATGCAGTTGTTGACCACATGGTTGCCTGAATTCTTTATGCTGGAGA
CTATGCATTGGAGTGCTCGTTTGTTTGTGTTTGTTGCCAATATGCTTCCAGCTTTTACG
TTACTGCGATTTTACATCGTGGTGACAGCTATGTATAAGGTCTATTGTCTTTGTAGAC
ATGTTATGTATGGATGTAGTAAGCCTGGTTGCTTGTTTTGTTATAAGAGAAACCGTA
GTGTCCGTGTTAAGTGTAGCACCGTTGTTGGTGGTTCACTACGCTATTACGATGTAAT
GGCTAACGGCGGCACAGGTTTCTGTACAAAGCACCAGTGGAACTGTCTTAATTGCAA
TTCCTGGAAACCAGGCAATACATTCATAACTCATGAAGCAGCGGCGGACCTCTCTAA

GGAGTTGAAACGCCCTGTGAATCCAACAGATTCTGCTTATTACTCGGTCACAGAGGT TAAGCAGGTTGGTTGTTCCATGCGTTTGTTCTACGAGAGAGATGGACAGCGTGTTTA TGATGATGTTAATGCTAGTTTGTTTGTGGACATGAATGGTCTGCTGCATTCTAAAGTT AAAGGTGTGCCTGAAACGCATGTTGTGGTTGTTGAGAATGAAGCTGATAAAGCTGGT TTTCTCGGCGCCGCAGTGTTTTATGCACAATCGCTCTACAGACCTATGTTGATGGTGG AAAAGAAATTAATAACTACCGCCAACACTGGTTTGTCTGTTAGTCGAACTATGTTTG ACCTTTATGTAGATTCATTGCTGAACGTCCTCGACGTGGATCGCAAGAGTCTAACAA GTTTTGTAAATGCTGCGCACAACTCTCTAAAGGAGGGTGTTCAGCTTGAACAAGTTA TGGATACCTTTATTGGCTGTGCCCGACGTAAGTGTGCTATAGATTCTGATGTTGAAAC CAAGTCTATTACCAAGTCCGTCATGTCGGCAGTAAATGCTGGCGTTGATTTTACGGA TGAGAGTTGTAATAACTTGGTGCCTACCTATGTTAAAAGTGACACTATCGTTGCAGC CGATTTGGGTGTTCTTATTCAGAATAATGCTAAGCATGTACAGGCTAATGTTGCTAA AGCCGCTAATGTGGCTTGCATTTGGTCTGTGGATGCTTTTAACCAGCTATCTGCTGAC TTACAGCATAGGCTGCGAAAAGCATGTTCAAAAACTGGCTTGAAGATTAAGCTTACT TATAATAAGCAGGAGGCAAATGTTCCTATTTTAACTACACCGTTCTCTCTTAAAGGG GGCGCTGTTTTTAGTAGAATGTTACAATGGTTGTTTGTTGCTAATTTGATTTGTTTCAT TGTGTTGTGGGCCCTTATGCCAACATATGCAGTGCACAAATCGGATATGCAGTTGCC TTTATATGCCAGTTTTAAAGTTATAGATAATGGTGTGCTAAGGGATGTGTCTGTTACT GACGCATGCTTCGCAAACAAATTTAATCAATTTGATCAATGGTATGAGTCTACTTTTG GTCTTGCTTATTACCGCAACTCTAAGGCTTGTCCTGTTGTGGTTGCTGTAATAGATCA AGACATTGGCCATACCTTATTTAATGTTCCTACCACAGTTTTAAGATATGGATTTCAT GTGTTGCATTTTATAACCCATGCATTTGCTACTGATAGCGTGCAGTGTTACACGCCAC ATATGCAAATCCCCTATGATAATTTCTATGCTAGTGGTTGCGTGTTGTCATCCCTCTG TACTATGCTTGCGCATGCAGATGGAACCCCGCATCCTTATTGTTATACAGGGGGTGT TATGCACAATGCCTCTCTGTATAGTTCTTTGGCTCCTCATGTCCGTTATAACCTGGCT AGTTCAAATGGTTATATACGTTTTCCCGAAGTGGTTAGTGAAGGCATTGTGCGTGTT GTGCGCACTCGCTCTATGACCTACTGCAGGGTTGGTTTATGTGAGGAGGCCGAGGAG GGTATCTGCTTTAATTTTAATCGTTCATGGGTATTGAACAACCCGTATTATAGGGCCA TGCCTGGAACTTTTTGTGGTAGGAATGCTTTTGATTTAATACATCAAGTTTTAGGAGG ATTAGTGCGGCCTATTGATTTCTTTGCCTTAACGGCGAGTTCAGTGGCTGGTGCTATC CTTGCAATTATTGTCGTTTTGGCTTTCTATTATTTAATAAAGCTTAAACGTGCCTTTGG TGACTACACTAGTGTTGTGGTTATCAATGTAATTGTGTGGTGTATAAATTTCTGATG CTTTTTGTGTTTCAGGTTTATCCCACATTGTCTTGTTTATATGCTTGTTTTTATTTCTAC ACAACGCTTTATTTCCCTTCGGAGATAAGTGTTGTTATGCATTTGCAATGGCTTGTCA

TGTATGGTGCTATTATGCCCTTGTGGTTTTGCATTATTTACGTGGCAGTCGTTGTTTCA
AACCATGCATTGTGGTTGTTCTCTTACTGCCGCAAAATTGGTACCGAGGTTCGTAGTG
ACGGCACATTTGAGGAAATGGCCCTTACTACCTTTATGATTACTAAAGAATCTTATT
GTAAGTTGAAAAATTCTGTTTCTGATGTTGCTTTTAACAGGTACTTGAGTCTTTATAA
CAAGTATCGTTATTTTAGTGGCAAAATGGATACTGCCGCTTATAGAGAGGCTGCCTG
TTCACAACTGGCAAAGGCAATGGAAACATTTAACCATAATAATGGTAATGATGTTCT
CTATCAGCCTCCAACCGCCTCTGTTACTACATCATTTTTACAGTCTGGTATAGTGAAG
ATGGTGTCGCCCACCTCTAAAGTGGAGCCTTGTATTGTTAGTGTTACTTATGGTAACA
TGACACTTAATGGGTTGTGGTTGGATGATAAAGTTTATTGCCCAAGACATGTTATCT
GTTCTTCAGCTGACATGACAGACCCTGATTATCCTAATTTGCTTTGTAGAGTGACATC
AAGTGATTTTTGTGTTATGTCTGGTCGTATGAGCCTTACTGTAATGTCTTATCAAATG
CAGGGCTGCCAACTTGTTTTGACTGTTACACTGCAAAATCCTAACACGCCTAAGTAT
TCCTTCGGTGTTGTTAAGCCTGGTGAGACATTTACTGTACTGGCTGCATACAATGGCA
GACCTCAAGGAGCCTTCCATGTTACGCTTCGTAGTAGCCATACCATAAAGGGCTCCT
TTCTATGTGGATCCTGCGGTTCTGTAGGATATGTTTTAACTGGCGATAGTGTACGATT
TGTTTATATGCATCAGCTAGAGTTGAGTACTGGTTGTCATACCGGTACTGACTTTAGT
GGGAACTTTTATGGTCCCTATAGAGATGCGCAAGTTGTACAATTGCCTGTTCAGGAT
TATACGCAGACTGTTAATGTTGTAGCTTGGCTTTATGCTGCTATTTTTAACAGATGCA
ACTGGTTTGTGCAAAGTGATAGTTGTTCCCTGGAGGAGTTTAATGTTTGGGCTATGA
CCAATGGTTTTAGCTCAATCAAAGCCGATCTTGTCTTGGATGCGCTTGCTTCTATGAC
AGGCGTTACAGTTGAACAGGTGTTGGCCGCTATTAAGAGGCTGCATTCTGGATTCCA
GGGCAAACAAATTTTAGGTAGTTGTGTGCTTGAAGATGAGCTGACACCAAGTGATGT
TTATCAACAACTAGCTGGTGTCAAGCTACAGTCAAAGCGCACAAGAGTTATAAAAG
GTACATGTTGCTGGATATTGGCTTCAACGTTTTTGTTCTGTAGCATTATCTCAGCATT
TGTAAAATGGACTATGTTTATGTATGTTACTACCCATATGTTGGGAGTGACATTGTGT
GCACTTTGTTTTGTAAGCTTTGCTATGTTGTTGATCAAGCATAAGCATTTGTATTTAA
CTATGTATATTATGCCTGTGTTATGCACACTGTTTTACACCAACTATTTGGTTGTGTA
CAAACAGAGTTTTAGAGGTCTAGCTTATGCTTGGCTTTCACACTTTGTCCCTGCTGTA
GATTATACATATATGGATGAAGTTTTATATGGTGTTGTGTTGCTAGTAGCTATGGTGT
TTGTTACCATGCGTAGCATAAACCACGACGTCTTTTCTATTATGTTCTTGGTTGGTAG
ACTTGTCAGCCTGGTATCCATGTGGTATTTTGGAGCCAATTTAGAGGAAGAGGTACT
ATTGTTCCTCACATCCCTATTTGGCACGTACACATGGACTACTATGTTGTCATTGGCT
ACCGCTAAGGTTATTGCTAAATGGTTGGCTGTGAATGTCTTGTACTTCACAGACGTA
CCGCAAATTAAATTAGTTCTTTTGAGCTACTTGTGTATTGGTTATGTGTGTTGTTGTT

ATTGGGGAATCTTGTCACTCCTTAATAGCATTTTTAGGATGCCATTGGGCGTCTACAA
TTATAAAATCTCCGTTCAGGAGTTACGTTATATGAATGCTAATGGCTTGCGCCCACCT
AGAAATAGTTTTGAGGCCCTGATGCTTAATTTTAAGCTGTTGGGAATTGGTGGTGTG
CCAGTCATTGAAGTATCTCAAATTCAATCAAGATTGACGGATGTTAAATGTGCTAAT
GTTGTGTTGCTTAATTGCCTCCAGCACTTGCATATTGCATCTAATTCTAAGTTGTGGC
AGTATTGTAGTACTTTGCACAATGAAATACTGGCTACATCTGATTTGAGCGTGGCCTT
CGATAAGTTGGCTCAGCTCTTAGTTGTTTTATTTGCTAATCCAGCAGCAGTGGATAGC
AAGTGCCTTGCAAGTATTGAAGAAGTGAGCGATGATTACGTTCGCGACAATACTGTC
TTGCAAGCCTTACAGAGTGAATTTGTTAATATGGCTAGCTTCGTTGAGTATGAACTTG
CTAAGAAGAATCTAGATGAGGCTAAGGCTAGCGGCTCTGCCAATCAACAGCAGATT
AAGCAGCTAGAGAAGGCGTGTAATATTGCTAAGTCAGCATATGAGCGCGACAGAGC
TGTTGCTCGTAAGCTGGAACGTATGGCTGATTTAGCTCTTACAAACATGTATAAAGA
AGCTAGAATTAATGATAAGAAGAGTAAGGTAGTGTCTGCATTGCAAACCATGCTCTT
TAGTATGGTGCGTAAGCTAGATAACCAAGCTCTTAATTCTATTTTAGATAATGCAGTT
AAGGGTTGTGTACCTTTGAATGCAATACCATCATTGACTTCGAACACTCTGACTATA
ATAGTGCCAGATAAGCAGGTTTTTGATCAGGTTGTGGATAATGTGTATGTCACCTAT
GCTGGGAATGTATGGCATATACAGTTTATTCAAGATGCTGATGGTGCTGTTAAACAA
TTGAATGAGATAGATGTTAATTCAACCTGGCCTCTAGTCATTGCTGCAAATAGGCAT
AATGAAGTGTCTACTGTTGTTTTGCAGAACAATGAGTTGATGCCTCAGAAGTTGAGA
ACTCAGGTTGTCAATAGTGGCTCAGATATGAATTGTAATACTCCTACCCAGTGTTACT
ATAATACTACTGGCACGGGTAAGATTGTGTATGCTATACTTAGTGACTGTGATGGTC
TCAAGTACACTAAGATAGTAAAAGAAGATGGAAATTGTGTTGTTTTGGAATTGGATC
CTCCCTGTAAGTTTTCTGTTCAGGATGTGAAGGGCCTTAAAATTAAGTACCTTTACTT
TGTGAAGGGGTGTAATACACTGGCTAGAGGCTGGGTTGTAGGCACCTTATCCTCGAC
AGTGAGATTGCAGGCGGGTACGGCAACTGAGTATGCCTCCAACTCTGCAATACTGTC
GCTGTGTGCGTTTTCTGTAGATCCTAAGAAAACGTACTTGGATTATATAAAACAGGG
TGGAGTTCCCGTTACTAATTGTGTTAAGATGTTATGTGACCATGCTGGCACTGGTATG
GCCATTACTATTAAGCCGGAGGCAACCACTAATCAGGATTCTTATGGTGGTGCTTCC
GTTTGTATATATTGCCGCTCGCGTGTTGAACATCCAGATGTTGATGGATTGTGCAAAT
TACGCGGCAAGTTTGTCCAAGTGCCCTTAGGCATAAAAGATCCTGTGTCATATGTGT
TGACGCATGATGTTTGTCAGGTTTGTGGCTTTTGGCGAGATGGTAGCTGTTCCTGTGT
AGGCACAGGCTCCCAGTTTCAGTCAAAAGACACGAACTTTTTAAACGGGTTCGGGGT
ACAAGTGTAAATGCCCGTCTTGTACCCTGTGCCAGTGGCTTGGACACTGATGTTCAA
TTAAGGGCATTTGACATTTGTAATGCTAATCGAGCTGGCATTGGTTTGTATTATAAAG

TGAATTGCTGCCGCTTCCAGCGTGTAGATGAGGACGGCAACAAGTTGGATAAGTTCT
TTGTTGTTAAAAGAACTAATTTAGAAGTGTATAATAAGGAGAAAGAATGCTATGAGT
TGACAAAAGAATGCGGTGTTGTGGCTGAACACGAGTTCTTCACATTTGATGTGGAGG
GAAGTCGGGTACCACACATAGTCCGTAAAGATCTTTCAAAGTTTACTATGTTAGATC
TTTGCTATGCATTGCGTCATTTTGACCGCAATGATTGTTCAACTCTTAAGGAAATTCT
CCTTACATATGCTGAGTGTGAAGAGTCCTACTTCCAAAAGAAGGACTGGTATGATTT
TGTTGAGAATCCTGATATAATTAATGTGTATAAAAAGCTTGGTCCTATATTTAATAG
AGCCCTGCTTAACACTGCCAAGTTTGCAGACGCATTAGTGGAGGCAGGCTTAGTAGG
TGTTTTAACACTTGATAATCAAGATTTATATGGTCAATGGTATGACTTTGGAGATTTT
GTCAAGACAGTACCTGGTTGTGGTGTTGCCGTGGCAGACTCTTATTATTCATATATGA
TGCCAATGCTGACTATGTGTCATGCGTTGGATAGTGAGTTGTTTGTTAATGGTACTTA
TAGGGAGTTTGACCTTGTTCAGTATGATTTTACTGATTTCAAGCTAGAGCTCTTCACT
AAGTATTTTAAGCATTGGAGTATGACCTACCACCCGAACACCTGTGAGTGCGAGGAT
GACAGGTGCATTATTCATTGCGCCAATTTTAATATACTTTTTAGTATGGTCTTACCTA
AGACCTGTTTTGGGCCTCTTGTTAGGCAGATATTTGTGGATGGTGTTCCTTTCGTTGT
GTCGATCGGTTACCATTATAAAGAATTAGGTGTTGTTATGAATATGGATGTGGATAC
ACATCGTTATCGCTTGTCTCTTAAGGACTTGCTTTTGTATGCTGCAGACCCTGCCCTT
CATGTGGCGTCTGCTAGTGCACTGCTTGATTTGCGCACATGTTGTTTTAGCGTTGCAG
CTATTACAAGTGGCGTAAAATTTCAAACAGTTAAACCTGGAAATTTTAATCAGGATT
TTTATGAGTTTATTTTGAGTAAAGGCCTGCTTAAAGAGGGGAGCTCCGTTGATTTGA
AGCACTTCTTCTTTACGCAGGATGGTAATGCTGCTATTACTGATTATAATTATTACAA
GTATAATCTACCCACCATGGTGGATATTAAGCAGTTGTTGTTTGTTTTAGAAGTTGTT
AATAAGTATTTTGAGATCTATGAGGGTGGGTGTATACCCGCAACACAGGTCATTGTT
AATAATTATGATAAGAGTGCTGGCTATCCATTTAATAAATTTGGAAAGGCCAGGCTC
TATTATGAGGCATTATCATTTGAGGAGCAGGATGAAATTTATGCGTATACCAAACGC
AATGTCCTGCCGACCCTAACTCAAATGAATCTTAAATATGCTATTAGTGCTAAGAAT
AGGGCCCGCACCGTTGCTGGTGTCTCTATTCTCAGTACTATGACTGGCAGAATGTTTC
ATCAAAAGTGTCTAAAGAGTATAGCAGCTACTCGCGGTGTTCCTGTAGTTATAGGCA
CCACGAAGTTCTATGGCGGTTGGGATGATATGTTACGCCGCCTTATTAAAGATGTTG
ATAGTCCTGTACTCATGGGTTGGGACTATCCTAAATGTGATCGTGCTATGCCAAACA
TACTGCGTATTGTTAGTAGTTTGGTGCTAGCCCGTAAACATGATTCGTGCTGTTCGCA
TACGGATAGATTCTATCGTCTTGCGAACGAGTGCGCCCAAGTTTTGAGTGAAATTGT
TATGTGTGGTGGTTGTTATTATGTTAAACCAGGTGGCACTAGTAGTGGGGATGCAAC
CACTGCTTTTGCTAATTCTGTGTTTAACATTTGTCAAGCTGTTTCCGCCAATGTATGCT

CGCTTATGGCATGCAATGGACACAAAATTGAAGATTTGAGTATACGCGAGTTACAAA
AGCGCCTATACTCTAATGTCTATCGTGCGGACCATGTTGACCCCGCATTTGTTAGTGA
GTATTATGAGTTTTTAAATAAGCATTTTAGTATGATGATTTTGAGTGATGATGGTGTT
GTGTGTTATAATTCAGAGTTTGCGTCCAAGGGTTATATTGCTAATATAAGTGCCTTTC
AACAGGTATTATATTATCAAAATAATGTGTTTATGTCTGAGGCCAAATGTTGGGTAG
AAACAGACATCGAAAAGGGACCGCATGAATTTTGTTCTCAACATACAATGCTAGTCA
AGATGGATGGTGATGAAGTCTACCTTCCATACCCTGATCCTTCGAGAATCTTAGGAG
CAGGCTGTTTTGTTGATGATTTATTAAAGACTGATAGCGTTCTCTTGATAGAGCGTTT
CGTAAGTCTTGCAATTGATGCTTATCCTTTAGTATACCATGAGAACCCAGAGTATCA
AAATGTGTTCCGGGTATATTTAGAATATATAAAGAAGCTGTACAATGATCTCGGTAA
TCAGATCCTGGACAGCTACAGTGTTATTTTAAGTACTTGTGATGGTCAAAAGTTTACT
GATGAGACCTTTTACAAGAACATGTATTTAAGAAGTGCAGTGCTGCAAAGCGTTGGT
GCCTGCGTTGTCTGTAGTTCTCAAACATCATTACGTTGTGGCAGTTGCATACGCAAGC
CTTTGCTGTGTTGCAAATGCGCCTATGATCATGTTATGTCCACTGATCATAAATATGT
CCTGAGTGTGTCACCATATGTGTGTAATTCACCGGGATGTGATGTAAATGATGTTAC
CAAATTGTATTTAGGTGGTATGTCATATTATTGTGAGGACCATAAACCACAGTATTC
ATTCAAATTGGTGATGAATGGTATGGTTTTTGGTTTATATAAACAATCTTGTACTGGT
TCGCCCTACATAGAGGATTTTAATAAAATAGCTAGTTGCAAATGGACAGAAGTCGAT
GATTATGTGCTAGCTAATGAATGCACCGAACGCCTTAAATTGTTTGCCGCAGAAACG
CAGAAGGCCACAGAAGAGGCCTTTAAGCAATGTTATGCGTCAGCAACGATCCGTGA
GATCGTGAGCGATCGGGAGTTAATTTTATCTTGGGAAATTGGTAAAGTGAGACCACC
ACTTAATAAAAATTATGTTTTTACTGGCTACCATTTTACTAATAATGGTAAGACAGTT
TTAGGTGAGTATGTTTTTGATAAGAGTGAGTTGACTAATGGTGTGTACTATCGCGCC
ACAACCACTTATAAGTTATCTGTAGGTGATGTGTTCATTTTAACATCACACGCAGTGT
CTAGTTTAAGTGCTCCTACATTAGTACCGCAGGAGAATTATACTAGCATTCGTTTTGC
TAGTGTTTATAGTGTGCCTGAGACGTTTCAGAATAATGTGCCTAATTATCAGCACATT
GGAATGAAGCGCTATTGTACTGTACAGGGACCGCCTGGTACTGGTAAGTCCCATCTA
GCCATTGGGCTAGCTGTTTATTATTGTACAGCGCGCGTGGTGTATACCGCTGCTAGCC
ATGCTGCAGTTGACGCGCTGTGTGAAAAGGCACATAAATTTTTAAATATTAATGACT
GCACGCGTATTGTTCCTGCAAAGGTGCGTGTAGATTGTTATGATAAATTTAAGGTCA
ATGACACCACTCGCAAGTATGTGTTTACTACAATAAATGCATTACCTGAGTTGGTGA
CTGACATTATTGTCGTTGATGAAGTTAGTATGCTTACCAACTATGAGCTGTCTGTTAT
TAACAGTCGTGTTAGTGCTAAGCATTATGTGTATATTGGAGACCCTGCGCAGTTACC
TGCACCACGTGTGCTACTGAATAAGGGAACTCTAGAACCTAGATATTTTAATTCCGT

TACCAAGCTAATGTGTTGTTTGGGTCCAGATATTTTCTTGGGCACCTGTTATAGATGC CCTAAGGAGATTGTGGATACGGTGTCAGCCTTGGTTTATAATAATAAGCTGAAGGCT AAAAATGATAATAGCTCCATGTGCTTTAAGGTTTATTATAAGGGCCAGACTACACAT GAGAGTTCTAGTGCTGTTAATATGCAGCAAATACATTTAATTAGTAAGTTTTTAAAG GCAAACCCCAGTTGGAGTAACGCCGTATTTATTAGTCCTTATAATAGTCAGAACTAT GTTGCTAAGAGAGTCTTGGGATTACAAACCCAGACAGTAGACTCAGCGCAGGGTTCT GAATATGATTTTGTTATTTATTCACAGACTGCGGAAACAGCGCATTCTGTCAATGTA AATAGATTCAATGTTGCTATTACACGTGCTAAGAAGGGTATTCTCTGTGTCATGAGT AGTATGCAATTATTTGAGTCTCTTAATTTTACTACACTGACGTTGGATAAGATTAACA ATCCACGATTACAGTGTACTACAAATTTGTTTAAGGATTGTAGCAGGAGCTATGTAG GATATCACCCAGCCCATGCACCATCCTTTTTGGCAGTTGATGACAAATATAAGGTAG GCGGTGATTTAGCCGTTTGCCTTAATGTTGCTGATTCTGCTGTCACTTATTCGCGGCT TATATCACTCATGGGATTCAAGCTTGACTTGACCCTTGATGGTTATTGTAAGCTGTTT ATAACTAGAGATGAAGCTATCAAACGTGTTAGAGCCTGGGTTGGCTTCGATGCAGAA GGTGCCCATGCGATACGTGATAGCATTGGGACAAATTTCCCATTACAATTAGGCTTT TCGACTGGAATTGATTTTGTTGTCGAAGCCACTGGAATGTTTGCTGAGAGAGATGGT TATGTCTTTAAAAAGGCAGCCGCACGAGCTCCTCCTGGCGAACAATTTAAACACCTT ATCCCACTTATGTCAAGAGGGCAGAAATGGGATGTGGTTCGAATTAGAATAGTACA AATGTTGTCAGACCACCTAGCGGATTTGGCAGACAGTGTTGTACTTGTGACGTGGGC TGCCAGCTTTGAGCTCACATGTTTGCGATATTTCGCTAAAGTTGGAAGAGAAGTTGT GTGTAGTGTCTGCACCAAGCGTGCGACATGTTTTAATTCTAGAACTGGATACTATGG ATGCTGGCGACATAGTTATTCCTGTGATTACCTGTACAACCCACTAATAGTTGACATT CAACAGTGGGGATATACAGGATCTTTAACTAGCAATCATGATCCTATTTGCAGCGTG CATAAGGGTGCTCATGTTGCATCATCTGATGCTATCATGACCCGGTGTCTAGCTGTTC ATGATTGCTTTTGTAAGTCTGTTAATTGGAATTTAGAATACCCCATTATTTCAAATGA GGTCAGTGTTAATACCTCCTGCAGGTTATTGCAGCGCGTAATGTTTAGGGCTGCGAT GCTATGCAATAGGTATGATGTGTGTTATGACATTGGCAACCCTAAAGGTCTTGCCTG TGTCAAAGGATATGATTTTAAGTTTTATGATGCCTCCCCTGTTGTTAAGTCTGTTAAA CAGTTTGTTTATAAATACGAGGCACATAAAGATCAATTTTTAGATGGTTTGTGTATGT TTTGGAACTGCAATGTGGATAAGTATCCAGCGAATGCAGTTGTGTGTAGGTTTGACA CGCGTGTGTTGAACAAATTAAATCTCCCTGGCTGTAATGGTGGCAGTTTGTATGTTA ACAAACATGCATTCCACACCAGTCCCTTTACCCGGGCTGCCTTCGAGAATTTGAAGC CTATGCCTTTCTTTTATTATTCAGATACGCCCTGTGTGTATATGGAAGGCATGGAATC TAAGCAGGTCGATTATGTCCCATTGAGAAGCGCTACATGCATCACAAGATGCAATTT

AGGTGGCGCTGTTTGTTTAAAACATGCTGAGGAGTATCGTGAGTACCTTGAGTCTTA
CAATACGGCAACCACAGCGGGTTTTACTTTTTGGGTCTATAAGACTTTTGATTTTTAT
AACCTTTGGAATACTTTTACTAGGCTCCAAAGTTTAGAAAATGTAGTGTATAATTTG
GTCAATGCTGGACACTTTGATGGCCGGGCGGGTGAACTGCCTTGTGCTGTTATAGGT
GAGAAAGTCATTGCCAAGATTCAAAATGAGGATGTCGTGGTCTTTAAAAATAACAC
GCCATTCCCCACTAATGTGGCTGTCGAATTATTTGCTAAGCGCAGTATTCGGCCCCAC
CCCGAGCTTAAGCTCTTTAGAAATTTGAATATTGACGTGTGCTGGAGTCACGTCCTTT
GGGATTATGCTAAGGATAGTGTGTTTTGCAGTTCGACGTATAAGGTCTGCAAATACA
CAGATTTACAGTGCATTGAAAGCTTGAATGTACTTTTTGATGGTCGTGATAATGGTG
CTCTTGAAGCTTTTAAGAAGTGCCGGAATGGCGTCTACATTAACACGACAAAAATTA
AAAGTCTGTCGATGATTAAAGGCCCACAACGTGCCGATTTGAATGGCGTAGTTGTGG
AGAAAGTTGGAGATTCTGATGTGGAATTTTGGTTTGCTGTGCGTAAAGACGGTGACG
ATGTTATCTTCAGCCGTACAGGGAGCCTTGAACCGAGCCATTACCGGAGCCCACAAG
GTAATCCGGGTGGTAATCGCGTGGGTGATCTCAGCGGTAATGAAGCTCTAGCGCGTG
GCACTATCTTTACTCAAAGCAGATTATTATCTTCTTTCACACCTCGATCAGAGATGGA
GAAAGATTTTATGGATTTAGATGATGATGTGTTCATTGCAAAATATAGTTTACAGGA
CTACGCGTTTGAACACGTTGTTTATGGTAGTTTTAACCAGAAGATTATTGGAGGTTTG
CATTTGCTTATTGGCTTAGCCCGTAGGCAGCAAAAATCCAATCTGGTAATTCAAGAG
TTCGTGACATACGACTCTAGCATTCATTCGTACTTTATCACTGACGAGAACAGTGGT
AGTAGTAAGAGTGTGTGCACTGTTATTGATTTATTGTTAGATGATTTTGTGGACATTG
TAAAGTCCCTGAATCTAAAGTGTGTGAGTAAGGTTGTTAATGTTAATGTTGATTTTAA
AGATTTCCAGTTTATGTTGTGGTGCAATGAGGAGAAGGTCATGACTTTCTATCCTCGT
TTGCAGGCTGCTGCTGACTGGAAACCTGGTTATGTTATGCCTGTCTTATATAAGTATT
TGGAATCGCCTCTGGAAAGAGTAAACCTCTGGAATTATGGCAAGCCGATTACTTTAC
CTACAGGATGTATGATGAATGTTGCTAAGTATACTCAATTATGTCAATATTTGAGCA
CTACAACATTAGCAGTTCCGGCTAATATGCGTGTCTTACACCTTGGTGCCGGTTCGG
ATAAGGGTGTTGCCCCTGGGTCTGCAGTTCTTAGGCAGTGGCTACCAGCGGGAAGTA
TTCTTGTAGATAATGATGTGAATCCATTTGTGAGTGACAGTGTCGCCTCATATTATGG
AAATTGTATAACCTTACCCTTTGATTGTCAGTGGGATCTGATAATTTCTGATATGTAC
GACCCTCTTACTAAGAACATTGGGGAGTACAACGTGAGTAAAGATGGATTCTTTACT
TACCTCTGTCATTTAATTCGTGACAAGTTGGCTCTGGGTGGCAGTGTTGCCATAAAA
ATAACAGAGTTTTCTTGGAACGCTGAGTTATATAGTTTAATGGGGAAGTTTGCGTTCT
GGACAATCTTTTGCACCAACGTAAACGCCTCTTCAAGTGAAGGATTTTTGATTGGCA
TAAATTGGTTGAATAAGACCCGTACCGAAATTGACGGTAAAACCATGCATGCCAATT

ATCTGTTTTGGAGAAATAGTACAATGTGGAATGGAGGGGCTTACAGTCTCTTTGACA
TGAGTAAGTTCCCTTTGAAAGCGGCTGGTACGGCTGTTGTTAGCCTTAAACCAGACC
AAATAAATGACTTAGTCCTCTCCTTGATTGAGAAGGGCAAGTTATTAGTGCGTGATA
CACGCAAAGAAGTTTTTGTTGGCGATAGCCTAGTAAATGTCAAATAAATCTATACTT
GTCGTGGCTGTGAAAATGGCCTTTGCTGACAAGCCTAATCATTTCATAAACTTTCCCC
TGGCCCAATTTAGTGGCTTTATGGGTAAGTATTTAAAGCTACAGTCTCAACTTGTGG
AAATGGGTTTAGACTGTAAATTACAGAAGGCACCACATGTTAGTATTACCCTGCTTG
ATATTAAAGCAGACCAATACAAACAGGTGGAATTTGCAATACAAGAAATAATAGAT
GATCTGGCGGCATATGAGGGAGATATTGTCTTTGACAACCCTCACATGCTTGGCAGA
TGCCTTGTTCTTGATGTTAGAGGATTTGAAGAGTTGCATGAAGATATTGTTGAAATTC
TCCGCAGAAGGGGTTGCACGGCAGATCAATCCAGACACTGGATTCCGCACTGCACTG
TGGCCCAATTTGACGAAGAAAGAGAAACAAAAGGAATGCAATTCTATCATAAAGAA
CCCTTCTACCTCAAGCATAACAACCTATTAACGGATGCTGGGCTTGAGCTCGTGAAG
ATAGGTTCTTCCAAAATAGATGGGTTTTATTGTAGTGAACTGAGTGTTTGGTGTGGTG
AGAGGCTTTGTTATAAGCCTCCAACACCCAAATTCAGTGATATATTTGGCTATTGCTG
CATAGATAAAATACGTGGTGATTTAGAAATAGGAGACCTACCGCAGGATGATGAGG
AAGCGTGGGCCGAGCTAAGTTACCACTATCAAAGAAACACCTACTTCTTCAGACATG
TGCACGATAATAGCATCTATTTTCGTACCGTGTGTAGAATGAAGGGTTGTATGTGTT
GATTTGTTTTTACACTATTAGTGTAATAAGCTTATTATTTTGTTGAAAAGGGCAGGAT
GTGCATAGCTATGGCTCCTCGCACACTGCTTTTGCTGATTTGATGTCAGCTGGTGTTT
GGGTTCAATGAACCTCTTAACATCGTTTCACATTTAAATGATGACTGGTTTCTATTTG
GTGACAGTCGTTCTGACTGTACCTATGTAGAAAATAACGGTCATCCTAAATTAGATT
GGCTTGACCTCGACCCAAAGTTGTGTAATTCAGGAAAGATTTCCGCAAAGAGTGGTA
ACTCTCTCTTTAGGAGTTTTCACTTCACTGATTTTTACAATTATACGGGTGAGGGAGA
CCAAATTGTATTTTATGAAGGAGTTAATTTTAGTCCCAGCCATGGCTTTAAATGCCTG
GCTCATGGAGATAATAAAAGATGGATGGGCAATAAAGCTCGATTTTATGCCCGAGT
GTATGAGAAGATGGCCCAATATAGGAGCCTATCGTTTGTTAATGTGTCTTATGCCTA
TGGAGGTAATGCAAAGCCCGCCTCCATTTGCAAAGACAATACTTTAACACTCAATAA
CCCCACCTTCATATCGAAGGAGTCTAATTATGTTGATTATTACTATGAGAGTGAGGC
TAATTTCACACTAGAAGGTTGTGATGAATTTATAGTACCGCTCTGTGGTTTTAATGGC
CATTCCAAGGGCAGCTCTTCGGATGCTGCCAATAAATATTATACTGACTCTCAGAGT
TACTATAATATGGATATTGGTGTCTTATATGGGTTCAATTCGACCTTGGATGTTGGCA
ACACTGCTAAGGATCCGGGTCTTGATCTCACTTGCAGGTATCTTGCATTGACTCCTGG
TAATTATAAGGCTGTGTCCTTAGAATATTTGTTAAGCTTACCCTCAAAGGCTATTTGC

CTCCATAAGACAAAGCGCTTTATGCCTGTGCAGGTAGTTGACTCAAGGTGGAGTAGC
ATCCGCCAGTCAGACAATATGACCGCTGCAGCCTGTCAGCTGCCATATTGTTTCTTTC
GCAACACATCTGCGAATTATAGTGGTGGCACACATGATGCGCACCATGGTGATTTTC
ATTTCAGGCAGTTATTGTCTGGTTTGTTATATAATGTTTCCTGTATTGCCCAGCAGGG
TGCATTTCTTTATAATAATGTTAGTTCCTCTTGGCCAGCCTATGGGTACGGTCATTGT
CCAACGGCAGCTAACATTGGTTATATGGCACCTGTTTGTATCTATGACCCTCTCCCGG
TCATACTGCTAGGTGTGTTATTGGGTATAGCTGTGTTGATTATTGTGTTTTTGATGTTT
TATTTTATGACGGATAGCGGTGTTAGATTGCATGAGGCATAATCTAAACATGCTGTT
CGTGTTTATTCTATTTTTGCCCTCTTGTTTAGGGTATATTGGTGATTTTAGATGTATCC
AGCTTGTGAATTCAAACGGTGCTAATGTTAGTGCTCCAAGCATTAGCACTGAGACCG
TTGAAGTTTCACAAGGCCTGGGGACATATTATGTGTTAGATCGAGTTTATTTAAATG
CCACATTATTGCTTACTGGTTACTACCCGGTCGATGGTTCTAAGTTTAGAAACCTCGC
TCTTACGGGAACTAACTCAGTTAGCTTGTCGTGGTTTCAACCACCCTATTTAAGTCAG
TTTAATGATGGCATATTTGCGAAGGTGCAGAACCTTAAGACAAGTACGCCATCAGGT
GCAACTGCATATTTTCCTACTATAGTTATAGGTAGTTTGTTTGGCTATACTTCCTATA
CCGTTGTAATAGAGCCATATAATGGTGTTATAATGGCCTCAGTGTGCCAGTATACCA
TTTGTCTGTTACCTTACACTGATTGTAAGCCTAACACTAATGGTAATAAGCTTATAGG
GTTTTGGCACACGGATGTAAAACCCCCAATTTGTGTGTTAAAGCGAAATTTCACGCT
TAATGTTAATGCTGATGCATTTTATTTTCATTTTTACCAACATGGTGGTACTTTTTATG
CGTACTATGCGGATAAACCCTCCGCTACTACGTTTTTGTTTAGTGTATATATTGGCGA
TATTTTAACACAGTATTATGTGTTACCTTTCATCTGCAACCCAACAGCTGGTAGCACT
TTTGCTCCGCGCTATTGGGTTACACCTTTGGTTAAGCGCCAATATTTGTTTAATTTCA
ACCAGAAGGGTGTCATTACTAGTGCTGTTGATTGTGCTAGTAGTTATACCAGTGAAA
TAAAATGTAAGACCCAGAGCATGTTACCTAGCACTGGTGTCTATGAGTTATCCGGTT
ATACGGTCCAACCAGTTGGAGTTGTATACCGGCGTGTTGCTAACCTCCCAGCTTGTA
ATATAGAGGAGTGGCTTACTGCTAGGTCAGTCCCCTCCCCTCTCAACTGGGAGCGTA
AGACTTTTCAGAATTGTAATTTTAATTTAAGCAGCCTGTTACGTTATGTTCAGGCTGA
GAGTTTGTTTTGTAATAATATCGATGCTTCCAAAGTGTATGGCAGGTGCTTTGGTAGT
ATTTCAGTTGATAAGTTTGCTGTACCCCGAAGTAGGCAAGTTGATTTACAGCTTGGT
AACTCTGGATTTCTGCAGACTGCTAATTATAAGATTGATACAGCTGCCACTTCGTGTC
AGCTGCATTACACCTTGCCTAAGAATAATGTCACCATAAACAACCATAACCCCTCGT
CTTGGAATAGGAGGTATGGCTTTAATGATGCTGGCGTCTTTGGCAAAAACCAACATG
ACGTTGTTTACGCTCAGCAATGTTTTACTGTAAGATCTAGTTATTGCCCGTGTGCTCA
ACCGGACATAGTTAGCCCTTGCACTACTCAGACTAAGCCTAAGTCTGCTTTTGTTAAT

GTGGGTGACCATTGTGAAGGCTTAGGTGTTTTAGAAGATAATTGTGGCAATGCTGAT
CCACATAAGGGTTGTATCTGTGCCAACAATTCATTTATTGGATGGTCACATGATACCT
GCCTTGTTAATGATCGCTGCCAAATTTTTGCTAATATATTGTTAAATGGCATTAATAG
TGGTACCACATGTTCCACAGATTTGCAGTTGCCTAATACTGAAGTGGTTACTGGCATT
TGTGTCAAATATGACCTCTACGGTATTACTGGACAAGGTGTTTTTAAAGAGGTTAAG
GCTGACTATTATAATAGCTGGCAAACCCTTCTGTATGATGTTAATGGTAATTTGAATG
GTTTTCGTGATCTTACCACTAACAAGACTTATACGATAAGGAGCTGTTATAGTGGCC
GTGTTTCTGCTGCATTTCATAAAGATGCACCCGAACCGGCTCTGCTCTATCGTAATAT
AAATTGTAGCTATGTTTTTAGCAATAATATTTCCCGTGAGGAGAACCCACTTAATTAC
TTTGATAGTTATTTGGGTTGTGTTGTTAATGCTGATAACCGCACGGATGAGGCGCTTC
CTAATTGTGATCTCCGTATGGGTGCTGGCTTATGCGTTGATTATTCAAAATCACGCAG
GGCTGACCGATCAGTTTCTACTGGCTATCGGTTAACTACATTTGAGCCATACACTCCG
ATGTTAGTTAATGATAGTGTCCAATCCGTTGATGGATTATATGAGATGCAAATACCA
ACCAATTTTACTATTGGGCACCATGAGGAGTTCATTCAAACTAGATCTCCAAAGGTG
ACTATAGATTGTGCTGCATTTGTCTGTGGTGATAACACTGCATGCAGGCAGCAGTTG
GTTGAGTATGGCTCTTTCTGTGTTAATGTTAATGCCATTCTTAATGAGGTTAATAACC
TCTTGGATAATATGCAACTACAAGTTGCTAGTGCATTAATGCAGGGTGTTACTATAA
GCTCGAGACTGCCAGACGGCATCTCAGGCCCTATAGATGACATTAATTTTAGTCCTC
TACTTGGATGCATAGGTTCAACATGTGCTGAAGACGGCAATGGACCTAGTGCAATCC
GAGGGCGTTCTGCTATAGAGGATTTGTTATTTGACAAGGTCAAATTATCTGATGTTG
GCTTTGTCGAGGCTTATAATAATTGCACCGGTGGTCAAGAAGTTCGTGACCTCCTTTG
TGTACAATCTTTTAATGGCATCAAAGTATTACCTCCTGTGTTGTCAGAGAGTCAGATC
TCTGGCTACACAACCGGTGCTACTGCGGCAGCTATGTTCCCACCGTGGTCAGCAGCT
GCCGGTGTGCCATTTAGTTTAAGTGTTCAATATAGAATTAATGGTTTAGGTGTCACTA
TGAATGTGCTTAGTGAGAACCAAAAGATGATTGCTAGTGCTTTTAACAATGCGCTGG
GTGCTATCCAGGATGGGTTTGATGCAACCAATTCTGCTTTAGGTAAGATCCAGTCCG
TTGTTAATGCAAATGCTGAAGCACTCAATAACTTACTAAATCAACTTTCTAACAGGT
TTGGTGCTATTAGTGCTTCTTTACAAGAAATTCTAACTCGGCTTGAGGCTGTAGAAGC
AAAAGCCCAGATAGATCGTCTTATTAATGGCAGGTTAACTGCACTTAATGCGTATAT
ATCCAAGCAACTTAGTGATAGTACGCTTATTAAAGTTAGTGCTGCTCAGGCCATAGA
AAAGGTCAATGAGTGCGTTAAGAGCCAAACCACGCGTATTAATTTCTGTGGCAATGG
TAATCATATATTATCTCTTGTCCAGAATGCGCCTTATGGCTTATATTTTATACACTTC
AGCTATGTGCCAATATCCTTTACAACCGCAAATGTGAGTCCTGGACTTTGCATTTCTG
GTGATAGAGGATTAGCACCTAAAGCTGGATATTTTGTTCAAGATGATGGAGAATGGA

AGTTCACAGGCAGTTCATATTACTACCCTGAACCCATTACAGATAAAAACAGTGTCA
TTATGAGTAGTTGCGCAGTAAACTACACAAAGGCACCTGAAGTTTTCTTGAACACTT
CAATACCTAATCCACCCGACTTTAAGGAGGAGTTAGATAAATGGTTTAAGAATCAGA
CGTCTATTGCGCCTGATTTATCTCTCGATTTCGAGAAGTTAAATGTTACTTTGCTGGA
CCTGACGTATGAGATGAACAGGATTCAGGATGCAATTAAGAAGTTAAATGAGAGCT
ACATCAACCTCAAGGAAGTTGGCACATATGAAATGTATGTGAAATGGCCTTGGTATG
TTTGGTTGCTAATTGGATTAGCTGGTGTAGCTGTTTGTGTGTTGTTATTCTTTATATGT
TGCTGCACAGGTTGTGGCTCATGTTGTTTTAAGAAGTGTGGAAATTGTTGTGATGAG
TATGGAGGACACCAGGACAGTATTGTGATACATAATATTTCCTCTCATGAGGATTGA
CTATCACAGCCTCTCCTGGAAAGACAGAAAATCTAAACAATTTATAGCATTCTCATT
GCTACCTGGCCCCGTAAGAGGCAGTCATAGCTATGGCCGTGTTGGTCCTAAGGCTAC
ATTGGCTGCTGTCTTTATTGGTCCATTTATTGTAGCATGTATGCTAGGCATTGGCCTA
GTTTATTTATTGCAATTGCAAGTTCAAATTTTTCATGTTAAGGATACCATACGTGTGA
CTGGCAAGCCAGCCACTGTGTCTTATACTACAAGTACACCAGTAACACCGAGCGCGA
CGACGCTCGATGGTACTACGTATACTTTAATTAGACCCACTAGCTCTTATACAAGAG
TTTATCTTGGTACTCCAAGAGGTTTTGATTATAGTACATTTGGGCCTAAGACCCTAGA
TTATGTTACTAATCTAAACCTCATCTTAATTCTGGTCGTCCATATACTTTTAAGGCAT
TGTCCAGGCATATGAGACCAACAGCCACATGGATTTGGCATGTGAGTGATGCATGGT
TACGCCGCACGCGGGACTTTGGTGTCATTCGCCTAGAAGATTTTTGTTTTCAATTTAA
TTATAGCCAACCCCGAGTTGGTTATTGTAGAGTTCCTTTAAAGGCTTGGTGTAGCAA
CCAGGGTAAATTTGCAGCGCAGTTTACCCTAAAAAGTTGCGAAAAACCAGGTCACG
AAAAATTTATTACTAGCTTCACGGCCTACGGCAGAACTGTCCAACAGGCCGTTAGCA
AGTTAGTAGAAGAAGCTGTTGATTTTATTCTTTTTAGGGCCACGCAGCTCGAAAGAA
ATGTTTAATTTATTCCTTACAGACACAGTATGGTATGTGGGGCAGATTATTTTTATAT
TCGCAGTGTGTTTGATGGTCACCATAATTGTGGTTGCCTTCCTTGCGTCTATCAAACT
TTGTATTCAACTTTGCGGTTTATGTAATACTTTGGTGCTGTCCCCTTCTATTTATTTGT
ATGATAGGAGTAAGCAGCTTTATAAGTATTATAATGAAGAAATGAGACTGCCCCTAT
TAGAGGTGGATGATATCTAATCTAAACATTATGAGTAGTACTACTCAGGCCCCAGAG
CCCGTCTATCAATGGACGGCCGACGAGGCAGTTCAATTCCTTAAGGAATGGAACTTC
TCGTTGGGCATTATACTACTCTTTATTACTATCATACTACAGTTCGGTTACACGAGCC
GTAGCATGTTTATTTATGTTGTGAAAATGATAATCTTGTGGTTAATGTGGCCACTGAC
TATTGTTTTGTGTATTTTCAATTGCGTGTATGCGCTAAATAATGTGTATCTTGGATTTT
CTATAGTGTTTACTATAGTGTCCATTGTAATCTGGATTATGTATTTTGTTAATAGCAT
AAGGTTGTTTATCAGGACTGGTAGCTGGTGGAGCTTCAACCCCGAAACAAACAACCT

TATGTGTATAGATATGAAAGGTACCGTGTATGTTAGACCCATTATTGAGGATTACCA
TACACTAACAGCCACTATTATTCGTGGCCACCTCTACATGCAAGGTGTTAAGCTAGG
CACCGGTTTCTCTTTGTCTGACTTGCCCGCTTATGTTACAGTTGCTAAGGTGTCACAC
CTTTGCACTTATAAGCGCGCATTCTTAGACAAGGTAGACGGTGTTAGCGGTTTTGCT
GTTTATGTGAAGTCCAAGGTCGGAAATTACCGACTGCCCTCAAACAAACCGAGTGGC
GCGGACACCGCATTGTTGAGAATCTAATCTAAACTTTAAGGATGTCTTTTGTTCCTGG
GCAAGAAAATGCCGGTGGCAGAAGCTCCTCTGTAAACCGCGCTGGTAATGGAATCC
TCAAGAAGACCACTTGGGCTGACCAAACCGAGCGTGGACCAAATAATCAAAATAGA
GGCAGAAGGAATCAGCCAAAGCAGACTGCAACTACTCAACCCAACTCCGGGAGTGT
GGTTCCCCATTACTCCTGGTTTTCTGGCATTACCCAGTTCCAAAAGGGAAAGGAGTTT
CAGTTTGCAGAAGGACAAGGAGTGCCTATTGCCAATGGAATCCCCGCTTCAGAGCA
AAAGGGATATTGGTATAGACACAACCGCCGTTCTTTTAAAACACCTGATGGGCAGCA
GAAGCAATTACTGCCCAGATGGTATTTTTACTATCTTGGCACAGGGCCCCATGCTGG
AGCCAGTTATGGAGACAGCATTGAAGGTGTCTTCTGGGTTGCAAACAGCCAAGCGG
ACACCAATACCCGCTCTGATATTGTCGAAAGGGACCCAAGCAGTCATGAGGCTATTC
CTACTAGGTTTGCGCCCGGCACGGTATTGCCTCAGGGCTTTTATGTTGAAGGCTCTGG
AAGGTCTGCACCTGCTAGCCGATCTGGTTCGCGGTCACAATCCCGTGGGCCAAATAA
TCGCGCTAGAAGCAGTTCCAACCAGCGCCAGCCTGCCTCTACTGTAAAACCTGATAT
GGCCGAAGAAATTGCTGCTCTTGTTTTGGCTAAGCTCGGTAAAGATGCCGGCCAGCC
CAAGCAAGTAACGAAGCAAAGTGCCAAAGAAGTCAGGCAGAAAATTTTAAACAAGC
CTCGCCAAAAGAGGACTCCAAACAAGCAGTGCCCAGTGCAGCAGTGTTTTGGAAAG
AGAGGCCCCAATCAGAATTTTGGAGGCTCTGAAATGTTAAAACTTGGAACTAGTGAT
CCACAGTTCCCCATTCTTGCAGAGTTGGCTCCAACAGTTGGTGCCTTCTTCTTTGGAT
CTAAATTAGAATTGGTCAAAAAGAATTCTGGTGGTGCTGATGAACCCACCAAAGATG
TGTATGAGCTGCAATATTCAGGTGCAGTTAGATTTGATAGTACTCTACCTGGTTTTGA
GACTATCATGAAAGTGTTGAATGAGAATTTGAATGCCTACCAGAAGGATGGTGGTGC
AGATGTGGTGAGCCCAAAGCCCCAAAGAAAAGGGCGTAGACAGGCTCAGGAAAAG
AAAGATGAAGTAGATAATGTAAGCGTTGCAAAGCCCAAAAGCTCTGTGCAGCGAAA
TGTAAGTAGAGAATTAACCCCAGAGGATAGAAGTCTGTTGGCTCAGATCCTTGATGA
TGGCGTAGTGCCAGATGGGTTAGAAGATGACTCTAATGTGTAAAGAGAATGAATCCT
ATGTCGGCGCTCGGTGGTAACCCCTCGCGAGAAAGTCGGGATAGGACACTCTCTATC
AGAATGGATGTCTTGCTGTCATAACAGATAGAGAAGGTTGTGGCAGACCCTGTATCA
ATTAGTTGAAAGAGATTGCAAAATAGAGAATGTGTGAGAGAAGTTAGCAAGGTCCT
ACGTCTAACCATAAGAACGGCGATAGGCGCCCCCTGGGAAGAGCTCACATCAGGGT

ACTATTCCTGCAATGCCCTAGTAAATGAATGAAGTTGATCATGGCCAATTGGAAGAA TCACAAAAAAAAAAAAAAAAAAAAAAA (SEQ ID NO.: 34)

***Forward Primer:*** TGAACCCACCAAAGATGTGTATGAG (SEQ ID: No. 35)
***Reverse Primer:*** CCATCCTTCTGGTAGGCATTCAAAT (SEQ ID: No. 36)
***Probe:*** CTGCACCTGAATATTG (SEQ ID: No. 37)

**Mn1Tel**

GGCAGCTGCTGCTCCGAGGCGGTCAAGAGCGCCATGAGCACCATTGACCTGGACTC GCTGATGGCAGAGCACAGCGCTGCCTGGTACATGCCCGCTGACAAGGCCCTGGTGG ACAGCGCGGACGACGACAAGACGTTGGCGCCCTGGGAGAAGGCCAAACCCCAGAAC CCCAACAGCAAAGAAGGCTTGCAGCCAATTTACTGGAGCAGGGATGACGTAGCCCA GTGGCTCAAGTGGGCTGAAAATGAGTTTTCTTTAAGGCCAATTGACAGCAACACGTT TGAAATGAATGGCAAAGCTCTCCTGCTGCTGACCAAAGAGGACTTTCGCTATCGATC TCCTCATTCAGGTGATGTGCTCTATGAACTCCTTCAGCATATTCTGAAGCAGAGGAA ACCTCGGATTCTTTTTCACC (SEQ ID: No. 38)

| | |
|---|---|
| ***Forward Primer:*** | AAACCCCAGAACCCCAACAG (SEQ ID: No. 39) |
| ***Reverse Primer:*** | TCATCCCTGCTCCAGTAAATTGG (SEQ ID: No. 40) |
| ***Probe:*** | CTGCAAGCCTTCTTTG (SEQ ID: No. 41) |

**mutation -** a heritable change in DNA sequence resulting from mutagens. Various types of mutations including frame-shift mutations, missense mutations, and nonsense mutations.

**Neomycin**

CATTGAACAAGATGGATTGCACGCAGGTTCTCCGGCCGCTTGGGTGGAGAGGCTATT CGGCTATGACTGGGCACAACAGACAATCGGCTGCTCTGATGCCGCCGTGTTCCGGCT GTCAGCGCAGGGGCGCCCGGTTCTTTTTGTCAAGACCGACCTGTCCGGTGCCCTGAA TGAACTGCAGGACGAGGCAGCGCGGCTATCGTGGCTGGCCACGACGGGCGTTCCTT GCGCAGCTGTGCTCGACGTTGTCACTGAAGCGGGAAGGGACTGGCTGCTATTGGGCG AAGTGCCGGGGCAGGATCTCCTGTCATCTCACCTTGCTCCTGCCGAGAAAGTATCCA TCATGGCTGATGCAATGCGGCGGCTGCATACGCTTGATCCGGCTACCTGCCCATTCG ACCACCAAGCGAAACATCGCATCGAGCGAGCACGTACTCGGATGGAAGCCGGTCTT

GTCGATCAGGATGATCTGGACGAAGAGCATCAGGGGCTCGCGCCAGCCGAACTGTT CGCCAGGCTCAAGGCGCGCATGCCCGACGGCGAGGATCTCGTCGTGACCCATGGCG ATGCCTGCTTGCCGAATATCATGGTGGAAAATGGCCGCTTTTCTGGATTCATCGACT GTGGCCGGCTGGGTGTGGCGGACCGCTATCAGGACATAGCGTTGGCTACCCGTGATA TTGCTGAAGAGCTTGGCGGCGAATGGGCTGACCGCTTCCTCGTGCTTTACGGTATCG CCGCTCCCGATTCGCAGCGCATCGCCTTCTATCGCCTTCTTGACGAGTTCTTCTG (SEQ ID: No. 42)

| | |
|---|---|
| ***Forward Primer:*** | GGGCGCCCGGTTCTT (SEQ ID: No. 43) |
| ***Reverse Primer:*** | CCTCGTCCTGCAGTTCATTCA (SEQ ID: No. 44) |
| ***Probe:*** | ACCTGTCCGGTGCCC (SEQ ID: No. 45) |

**OPN4ES**

TTAAAGCTCATGCCTAGACCTGATGCTATAGAAGGTGTGCTCCTCGCTTCTCTGCCAATCTTAAGGTGCCCTGGATGGAGCTGGGTGACGTGTTTACCCTTGTAGTCTGTCCTGTCTATATGCATGGATATGCACAGTGCCCTTGACCCAACCCTGCCAACCAGGCACCTGCAGAAGGTGTAGATGACCGTCAGATTGCCCAGCATCCCTGTGAGTCCCACCAGCAGGATCACCGTGCCTAGGGTATAGTGAGCATGGTCTGGGACATCGACTGTGGGGAAGGGGACCCAGGCAGCAGCCNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNNAGCCCATAGAAGAAAGTGCAAGTCTTCCAAAATTTAACCCCACGCCCATATATGTGTGGATACTGAGCTTCTAAGAGGGAGTGAAAGGCTCAGATGGCCTGCTGGAGGTTAACAGGACAAATGCGTGCCTGCAGGACAGAGCACAGCTTGGGTGACCTTAAGGAATGAGTAGAGCCAGGTCCTGGGTACTGCCCTCCCAACGAATGGATACCCCACAGCAAGCCTCCAAGGAGAACTTGCAACCCCTGTNNNNNNNNNNNNNNNNNNNNNNNNNNNNNNNNNNNNNAAACGAGGGAGGAGAACTTTCCACTAGAAAGAGAGTTTAGGTTCCCCCAGGCTGCTGGGAGGCCATTTCCCCCATGAGGTTAGTACACAGGGACTAAGGATAGCTCCCAGGGAGAGGCAGGAGTCTGCCCAATGTCCTGCCCAGCATCCCACTCTGGCCTGTACAAGTCCAGAAGCCTAGGGCATGCCTTTCCCCCTAGGATACTCCCCCAGGGGATNNNNNNNNNNNNNNNNNNNNNNNNNNNNNNNNNNNGAAGAGCAGGTCAGCCCCTGCCTTTCTGGTTCTCCAGTGGTCTCTGCCAACAAAGACATTGCCTGTGCCCTCTTGTCTCAGCCACTGTGTAGAGAAAGCTTAGAGAACTTCAGTGACGCTCAAGGTCCTTCGTCTAAGCTCAGACCTTTTCTATCTCCCTGTTAAAACAAGGGTGGGGACAGGAGTCTCTGTGTACACACATGCTCCCCAAACTTACCGTGGGGCTAACAGAGAGAAGCTGGGCTCTTACGGAGACGTTCTGAGTGCCGTTCCAAATGCCTTGCAGGGCAGGACTGGTTGTGAAGCTGGGATCCTGAGTTAAGCTTGACAAGAC (SEQ ID NO.: 46)

| | |
|---|---|
| ***Forward Primer:*** | TGGGTGACCTTAAGGAATGAGTAGA (SEQ ID: No. 47) |
| ***Reverse Primer:*** | GTTCTCCTTGGAGGCTTGCT (SEQ ID: No. 48) |
| ***Probe:*** | CTGCCCTCCCAACGAA (SEQ ID: No. 49) |

**p16**

GTGATGATGATGGGCAACGTTCACGTAGCAGCTCTTCTGCTCAACTACGGTGCAGAT TCGAACTGCGAGGACCCCACTACCTTCTCCCGCCCGGTGCACGACGCAGCGCGGGAA GGCTTCCTGGACACGCTGGTGGTGCTGCACGGGTCAGGGGCTCGGCTGGATGTGCGC GATGCCTGGGGTCGCCTGCCGCTCGACTTGGCCCAAGAGCGGGGACATCAAGACAT CGTGCGATATTTGCGTTCCGCTGGGTGCTCTTTGTGTTCCGCTGGGTGGTCTTTGTGT ACCGCTGGGAACGTCGCCCAGACCGACGGGCATAGCTTCAGCTCAAGCACGCCCAG (SEQ ID: No. 50)

| | |
|---|---|
| ***Forward Primer:*** | CGAGGACCCCACTACCTTCT (SEQ ID: No. 51) |
| ***Reverse Primer:*** | CCGCTCTTGGGCCAAGT (SEQ ID: No. 52) |
| ***Probe:*** | CAGGCATCGCGCACAT (SEQ ID: No. 53) |

**plate controls** - are wells that include the house-keeping probe without nucleic acid sample.

**Puromycin Sequence**

ATGACCGAGTACAAGCCCACGGTGCGCCTCGCCACCCGCGACGACGTCCCCCGGGC CGTACGCACCCTCGCCGCCGCGTTCGCCGACTACCCCGCCACGCGCCACACCGTCGA CCCGGACCGCCACATCGAGCGGGTCACCGAGCTGCAAGAACTCTTCCTCACGCGCGT CGGGCTCGACATCGGCAAGGTGTGGGTCGCGGACGACGGCGCCGCGGTGGCGGTCT GGACCACGCCGGAGAGCGTCGAAGCGGGGGCGGTGTTCGCCGAGATCGGCCCGCGC ATGGCCGAGTTGAGCGGTTCCCGGCTGGCCGCGCAGCAACAGATGGAAGGCCTCCT GGCGCCGCACCGGCCCAAGGAGCCCGCGTGGTTCCTGGCCACCGTCGGCGTCTCGCC CGACCACCAGGGCAAGGGTCTGGGCAGCGCCGTCGTGCTCCCCGGAGTGGAGGCGG CCGAGCGCGCCGGGGTGCCCGCCTTCCTGGAGACCTCCGCGCCCCGCAACCTCCCCT TCTACGAGCGGCTCGGCTTCACCGTCACCGCCGACGTCGAGTGCCCGAAGGACCGCG CGACCTGGTGCATGACCCGCAAGCCCGGTGCCTGA (SEQ ID: No. 54)

| | |
|---|---|
| ***Forward Primer:*** | GCGGTGTTCGCCGAGAT (SEQ ID NO.: 55) |
| ***Reverse Primer:*** | GAGGCCTTCCATCTGTTGCT (SEQ ID NO.: 56) |
| ***Probe:*** | GCGGTGTTCGCCGAGAT (SEQ ID NO.: 57) |

**RIP7-rtTA**

ATGTCTAGATTAGATAAAAGTAAAGTGATTAACAGCGCATTAGAGCTGCTTAATGAG
GTCGGAATCGAAGGTTTAACAACCCGTAAACTCGCCCAGAAGCTAGGTGTAGAGCA
GCCTACATTGTATTGGCATGTAAAAAATAAGCGGGCTTTGCTCGACGCCTTAGCCAT
TGAGATGTTAGATAGGCACCATACTCACTTTTGCCCTTTAGAAGGGGAAAGCTGGCA
AGATTTTTTACGTAATAACGCTAAAAGTTTTAGATGTGCTTTACTAAGTCATCGCGAT
GGAGCAAAAGTACATTTAGGTACACGGCCTACAGAAAAACAGTATGAAACTCTCGA
AAATCAATTAGCCTTTTTATGCCAACAAGGTTTTTCACTAGAGAATGCATTATATGCA
CTCAGCGCTGTGGGGCATTTTACTTTAGGTTGCGTATTGGAAGATCAAGAGCATCAA
GTCGCTAAAGAAGAAAGGGAAACACCTACTACTGATAGTATGCCGCCATTATTACG
ACAAGCTATCGAATTATTTGATCACCAAGGTGCAGAGCCAGCCTTCTTATTCGGCCT
TGAATTGATCATATGCGGATTAGAAAAACAACTTAAATGTGAAAGTGGGTCCGCGTA
CAGCCGCGCGCGTACGAAAAACAATTACGGGTCTACCATCGAGGGCCTGCTCGATCT
CCCGGACGACGACGCCCCCGAAGAGGCGGGGCTGGCGGCTCCGCGCCTGTCCTTTCT
CCCCGCGGGACACACGCGCAGACTGTCGACGGCCCCCCGACCGATGTCAGCCTGG
GGGACGAGCTCCACTTAGACGGCGAGGACGTGGCGATGGCGCATGCCGACGCGCTA
GACGATTTCGATCTGGACATGTTGGGGGACGGGGATTCCCCGGGTCCGGGATTTACC
CCCCACGACTCCGCCCCCTACGGCGCTCTGGATATGGCCGACTTCGAGTTTGAGCAG
ATGTTTACCGATGCCCTTGGAATTGACGAGTACGGTGGGTAG (SEQ ID NO.: 58)

| | |
|---|---|
| ***Forward Primer:*** | TGCCAACAAGGTTTTTCACTAGAGA (SEQ ID NO.: 59) |
| ***Reverse Primer:*** | CTCTTGATCTTCCAATACGCAACCTA (SEQ ID NO.: 60) |
| ***Probe:*** | CCACAGCGCTGAGTGC (SEQ ID NO.: 61) |

**recombination -** The process by which offspring derive a combination of genes different from that of either parent. In higher organisms, this can occur by crossing over.

recombinant DNA - A combination of DNA molecules of different origin that are joined using recombinant DNA technologies.

**RNA** - on of the two main types of nucleic acid, consisting of a long, unbranched macromolecule formed from ribonucleotides, the 3'-phosphate group of each constituent ribonucleotide (except the last) being joined in 3', 5' -phosphodiester linkage to the 5'-hydroxyl group on each ribose moiety renders these phosphodiester bonds susceptible to hydrolytic attack by alkali, in contrast to those of DNA. The RNA chain has polarity, with one 5' end and on 3' end. Two purines, adenine and guanine, and two pyrimidines, cytosine and uracil, are the major bases usually present. In addition, minor bases may occur; transfer RNA, however, contains unusual bases in relatively large amounts. The sequence of bases carries information, whereas the sugar and phosphate groups play a structural role. RNA is fundamental to protein biosynthesis in all living cells. Oxford Dictionary of Biochemistry and Molecular Biology; p. 577.

**screening reference** - are probes that are run on every sample submitted to screen laboratory. The probe is one that is found in every mouse, mutant or not.

**Six-2 WT**

GGGTGAGGCTGTTGCGACGCCTCTTATTTAAAAAAAAAGGGAGGGGTGTCTCACACT TTTTCTCTTGAAGGCTCCTTCTGTCCCCCTCTTTTCCTTTCCTGAAAGGCACCCCCTTA AACGGTCCTCCGCCTTCCCTTCTACTCCCTTCCTTCCCCACTTCGGTCCTCCTCTTTTC TTCGAGGGCCCCCACCCAGCCCCCTCCTTCGGGGTCCTCCTCCTCCTCTGCTCTTTGG GCGTCCGCCCCGTCAATCACCGCCGTCTCGGGGCCCCAGCCCGGCTCCTCTCCGCCT CCCGGGCTCTGGGAGTGCCTGGGGCTCCCGTCTCGGCCAACCTCCGCTCTGTGCAGA GCCGGGGCGATCTGTCAGCGGAGCTGGCCGAGGGGGGCGGGGGTGGAGCCGCCCG GGCCGCCGGGGCTCGGGTTACCGGTGACTGACAGCGTCTCCATGGCGAATAATTTGA CTCGACTATTGTCTGGCGCGGGCAGGCCCCGGGTCAGATAACCCGACCAATCAGGGC GCGGGCCGCCGCGCCTCATGCCCGCTTAGAATAATATTATTAAAAAAGCTGCAAGCG AGCTAGACGGGAGGGAGAGCGAACGAGCGAGGAGCCGGCGAGCGAGCGGCGGGCG GGCGCGGAGCATGCGGAGCGGCGCCCCGGGCGGCCTCCGGGCTTGGGCGCGGGCGA GGCGCGCGGGCGGCGGGGGCGCGGAGCTGCGCGGGGCCGGCGGCGGGAGCGAGGA CGGATCGTTGTGACTCAGGAGTCGCTCGGGAGCCGGCGCCTGGCCAGGGGGCCCCG CCCGCCTGTCGGCCGGCCGGGGCCGGCGGGGAGGCGCCCATGCGGGGCCGCGAAGC GCGGTGAGGGCGCGCGCGGGCGGGCGGGCGCGCAGCCGCCACCATGTCCATGCTGC CCACCTTCGGCTTCACGCAGGAGCAAGTGGCGTGCGTGTGCGAGGTGCTGCAGCAG GGCGGCAACATCGAGCGGCTGGGTCGCTTCCTGTGGTCGCTGCCCGCCTGCGAGCAC CTCCACAAGAATGAAAGCGTGCTCAAGGCCAAGGCCGTGGTGGCCTTCCACCGGGG CAACTTCCGCGAGCTCTACAAAATCCTGGAGAGCCACCAGTTCTCGCCGCACAACCA CGCCA (SEQ ID NO. 62)

| | |
|---|---|
| ***Forward Primer:*** | GGGTTACCGGTGACTGACA (SEQ ID NO. 63) |
| ***Reverse Primer:*** | CCCGCGCCAGACAATAGT (SEQ ID NO. 64) |
| ***Probe:*** | CCATGGCGAATAATTT (SEQ ID NO. 65) |

**strain** - a group of organisms bred for a genotype (at least one designated genetic sequence).

**strain controls** - are biomatter samples submitted by a remote user 1. Strain controls are controls positive and negative sent to the screen laboratory as the remote user that discloses the genotype.

**TetAKT1**

ATGAACGACGTAGCCATTGTGAAGGAGGGCTGGCTGCACAAACGAGGGGAATATATTAAAACCTGGCGGCCACGCTACTTCCTCCTCAAGAACGATGGCACCTTTATTGGCTACAAGGAACGGCCTCAGGATGTGGATCAGCGAGAGTCCCCACTCAACAACTTCTCAGTGGCACAATGCCAGCTGATGAAGACAGAGCGGCCAAGGCCCAACACCTTTATCATCCGCTGCCTGCAGTGGACCACAGTCATTGAGCGCACCTTCCATGTGGAAACGCCTGAGGAGCGGGAAGAATGGGCCACCGCCATTCAGACTGTGGCCGATGGACTCAAGAGGCAGGAAGAAGAGACGATGGACTTCCGATCAGGCTCACCCAGTGACAACTCAGGGGCTGAAGAGATGGAGGTGTCCCTGGCCAAGCCCAAGCACCGTGTGACCATGAACGAGTTTGAGTACCTGAAACTACTGGGCAAGGGCACCTTTGGGAAAGTGATTCTGGTGAAAGAGAAGGCCACAGGCCGCTACTATGCCATGAAGATCCTCAAGAAGGAGGTCATCGTCGCCAAGGATGAGGTTGCCCACACGCTTACTGAGAACCGTGTCCTGCAGAACTCTAGGCATCCCTTCCTTACGGCCCTCAAGTACTCATTCCAGACCCACGACCGCCTCTGCTTTGTCATGGAGTATGCCAACGGGGGCGAGCTCTTCTTCCACCTGTCTCGAGAGCGCGTGTTCTCCGAGGACCGGGCCCGCTTCTATGGTGCGGAGATTGTGTCTGCCCTGGACTACTTGCACTCCGAGAAGAACGTGGTGTACCGGGACCTGAAGCTGGAGAACCTCATGCTGGACAAGGACGGGCACATCAAGATAACGGACTTCGGGCTGTGCAAGGAGGGGATCAAGGATGGTGCCACTATGAAGACATTCTGCGGAACGCCGGAGTACCTGGCCCCTGAGGTGCTGGAGGACAACGACTACGGCCGTGCAGTGGACTGGTGGGGGCTGGGCGTGGTCATGTATGAGATGATGTGTGGCCGCCTGCCCTTCTACAACCAGGACCACGAGAAGCTGTTCGAGCTGATCCTCATGGAGGAGATCCGCTTCCCGCGCACACTCGGCCCTGAGGCCAAGTCCCTGCTCTCCGGGCTGCTCAAGAAGGACCCTACACAGAGGCTCGGTGGGGGCTCTGAGGATGCCAAGGAGATCATGCAGCACCGGTTCTTTGCCAACATCGTGTGGCAGGATGTGTATGAGAAGAAGCTGAGCCCACCTTTCAAGCCCCAGGTCACCTCTGAGACTGACACCAGGTATTTCGATGAGGAGTTCACAGCTCAGATGATCACCATCACGCCGCCTGATCAAGATGACAGCATGGAGTGTGTGGACAGTGAGCGGAGGCCGCACTTCCCCCAGTTCTCCTACTCAGCCAGTGGCACAGCCTGA (SEQ ID NO.: 66)

***Forward Primer:*** GGAACGCCGGAGTACCT (SEQ ID NO.: 67)
***Reverse Primer:*** ACTGCACGGCCGTAGTC (SEQ ID NO.: 68)
***Probe:*** CTGAGGTGCTGGAGGACA (SEQ ID NO.: 69)

**Tetp27KIP**

CCATGGTGAGCAAGGGCGAGGAGCTGTTCACCGGGGTGGTGCCCATCCTGGTCGAG CTGGACGGCGACGTAAACGGCCACAAGTTCAGCGTGTCCGGCGAGGGCGAGGGCGA TGCCACCCTACGGCAAGCTGACCCTGAAGTTCATCTGCACCACCGGCAAGCTGCCCG TGCCCTGGCCCACCCTCGTGACCACCCTGACCTACGGCGTGCAGTGCTTCAGCCGCT ACCCCGACCACATGAAGCAGCACGACTTCTTCAAGTCCGCCATGCCCGAAGGCTACG TCCAGGAGCGCACCATCTTCTTCAAGGACGACGGCAACTACAAGACCCGCGCCGAG GTGAAGTTCGAGGGCGACACCCTGGTGAACCGCATCGAGCTGAAGGGCATCGACTT CAAGGAGGACGGCAACATCCTGGGGCACAAGCTGGAGTACAACTACAACAGCCACA ACGTCTATATCATGGCCGACAAGCAGAAGAACGGCATCAAGGTGAACTTCAAGATC CGCCACAACATCGAGGACGGCAGCGTGCAGCTCGCCGACCACTACCAGCAGAACAC CCCCATCGGCGACGGCCCCGTGCTGCTGCCCGACAACCACTACCTGAGCACCCAGTC CGCCCTGAGCAAAGACCCCAACGAGAAGCGCGATCACATGGTCCTGCTGGAGTTCG TGACCGCCGCCGGGATCACTCTCGGCATGGACGAGCTGTACAAGTAAAG (SEQ ID NO.: 70)

| | |
|---|---|
| ***Forward Primer:*** | CGTCGTCCTTGAAGAAGATGGT (SEQ ID NO.: 71) |
| ***Reverse Primer:*** | CACATGAAGCAGCACGACTT (SEQ ID NO.: 72) |
| ***Probe:*** | CATGCCCGAAGGCTAC (SEQ ID NO.: 73) |

**transgene -** the foreign gene or DNA.

**transgenic -** this term describes an organism that has had genes from an organism or additional elements of it our sequence put into its genome through recombinant DNA techniques. These organisms are usually made by microinjection of DNA in the pronucleus of fertilized eggs, with the DNA integrating at random.

**transgenic line -** a transgenic mouse or organism strain in which the transgene is stably integrated into the germline and therefore inherited in Mendelian fashion by succeeding generation.

**web site -** a computer system that serves informational content over a network using the standard protocol of the World Wide Web. A web site corresponds to a particular Internet domain name such as TransnetYX.com.

**wild type** - the phenotype that is characteristic of most of the members of a species occurring naturally and contrasting with the phenotype of a mutant.

**zygosity** - This term reflect the genetic makeup of an individual. When identical alleles exist at a loci it is said to be homozygous; when alleles are different the alleles are said to be heterozygous.

2. OVERVIEW OF THE SYSTEMS COMPONENTS AND OPERATIONS:

The present invention provides methods for genotype screening as defined in the claims. More specifically, the present application relates to a method to rapidly screen biological samples for at least one designated genetic sequence. Various aspects of genotype screening involve: sample collection, lysing of the biological sample, isolation of a standard concentration of purified genomic nucleic acid and nucleic acid screening. Additionally, the method operating according to the features described herein can provide screening results to a remote user 1 from the screening laboratory 20 within 24 hours of receiving the biological samples.

In order to screen for a designated genetic sequence, that sequence must first be determined or identified. Only when the designated sequence is known can a test be devised to search for its existence in the biological samples provided by the remote user **1** to the screening laboratory **20.**

There are a variety of ways the designated genetic sequence can be acquired by the remote user **1** or by the screening laboratory **20.** For example, if the sequence of bases that makeup the designated genetic sequence is known by the remote user **1,** the sequence can be directly communicated to the screening laboratory **20** via an electronic link, such as any of the electronic communication links identified herein, and particularly the communication links extending between the remote user's computer and the screening laboratory **20.**

The remote user **1** can indirectly communicate the designated genetic sequence to the screening laboratory **20** by communicating a publication, journal article, a gene name, a sequence name, a line or strain name (if the designated genetic sequence is found in animals of that line or strain), or the name of a mutation having the designated genetic sequence to the screening laboratory **20.** Alternatively, the remote user **1** can communicate to the screening laboratory **20** the sequence of a primer set or probe that corresponds to a target genetic sequence of the designated genetic sequence. These primer sets or probes will have previously been created or defined to indicate the presence of the designated genetic sequence.

The indirect references may provide the entire sequence. Alternatively, the screening laboratory **20** may take the information from the references or from the remote user **1** and use it to search public genetic databases such as The National Center for Biotechnology Information (NCBI), Ensembl, or The Wellcome Trust Sanger Institute database. The screening laboratory **20** can also search proprietary databases, such as the database provided by Celera Bioscience (Rockville, MD).

Another indirect method that may be used to acquire or identify the designated genetic sequence is to use a third party who has specific knowledge of the sequence. For example, the screening laboratory **20** can receive the name of a transgenic animal line or strain from the remote user **1,** then contact the company that engineers that line or strain. The company can then transmit the sequence of bases that constitute the particular genetic sequence corresponding to that line or strain back to the screening laboratory **20.** These companies include such firms as Lexicon Genetics (Woodland, TX) or Charles River Laboratories (Wilmington, Mass.). Even further, individual researchers who have developed the line or strain, or who work with the same line or strain at another laboratory may provide the designated genetic sequence, the primer sets or the probes necessary to identify the designated genetic sequence.

If the designated genetic sequence is not known by the remote user **1** or third party and is not found in any public or private database, the screening laboratory **20** may use scientific methods. If the remote user **1** has a working genotyping assay, and they are performing PCR and separating fragments in a gel, the appropriate bands can be cut from the gel, purified and sequenced to determine the sequence of bases in that band. The company sequencing the bands can directly communicate the base sequence to the screening laboratory **20** or to the remote user **1,** who in turn can communicate the base sequence to the screening laboratory **20.**

Once identity of the designated genetic sequence is acquired by the screening laboratory **20** (and assuming a probe or primer set has yet to be designed), the screening laboratory **20** must then select a target genetic sequence of the designated genetic sequence for which a primer set and/or probe can be constructed. In the preferred embodiment, the sequence of the primer set and probe is determined using software such as Primer Express® (Applied Bio Systems). The target genetic sequence may be directly selected from the designated genetic sequence by the screening laboratory **20.** Once selected, the base sequence corresponding to the target genetic sequence is communicated to an oligonucleotide vendor, who manufactures the probe and primer sets and transmits them to the screening laboratory **20.**

The screening laboratory **20** preferably keeps a supply of probes and primer sets on hand so each future request by the remote user need not require special production of probes and primer sets.

Alternatively, a special probe or primer set may be required. In that situation, the screening laboratory **20** may not select the target genetic sequence itself, but may communicate to a third party specific areas in the designated genetic sequence that are important for mutation detection. The third party is typically an oligonucleotide vendor, who in turn will select the target genetic sequence, manufacture the probes and primer sets, and send the probes and primer sets to the screening laboratory **20.**

This alternative approach is particularly beneficial for zygosity genotyping of nontransgenic samples (as shown in Example 7), which requires such special probes or primer sets. Zygosity testing includes identifying not only the presence of a designated genetic sequence but also whether that designated genetic sequence is located on both (+/+ homozygous), one (+/heterozygous) or neither (-/- wild type) chromosome(s). The results are then determined by evaluating both pieces of information to determine zygosity. If signal is acquired solely from the mutation probe or the endogenous probe then the samples is homozygous for the mutation or homozygous for the endogenous sequence, respectively. If signal is acquired from both primer-probe combinations then the sample is heterozygous. The LIMS will establish three distinct categories to correspond with the three control samples needed (a homozygous, a heterozygous and a wild type sample).

To effectively genotype these nontransgenic samples, additional bioinformatics are needed from the remote user **1.** Specifically, the screening laboratory **20** requests that the remote user **1** provide both the base sequence of the designated genetic sequence of the mutation as well as the DNA sequence of the endogenous location. The endogenous DNA sequence is disrupted if a mutation has occurred. Once the precise sequence data is acquired, two primer-probe sets are designed. The first primer-probe set determines if the sequence of the mutation is present, irrespective of the number of times it is present. The second primer-probe set determines if the endogenous DNA sequence is present. It is these two primer-probe sets that the oligonucleotide vendor designs and transmits to the screening laboratory **20.**

With respect to human genotyping, a remote user **1** can contact the screening laboratory **20** and provide information for a human mutation or suspected endogenous condition of interest. This information may include the remote user's interest in wanting to know if the sample is from a human or a mouse and if it is from a human what gender is the sample. The screening laboratory **20** can acquire primers and probe that can distinguish between humans and mice. This is accomplished by identifying areas of genetic sequence in the mouse genome that are not homologous with the genetic sequence in the *Homo sapiens* genome. With no input from the remote user 1, the screening laboratory **20** can query a database such as Ensembl that would discriminate between the sex chromosomes in humans (X and Y). This query would yield sequence data for the Y chromosome, which is the designated genetic sequence. The screening laboratory **20** can take the designated genetic sequence, or portion thereof, and send it to a vendor indicating where to build the primer set and probe as to be informative for screening. Moreover, where there are a large number of nucleotides that are unique on the human Y chromosome, the screening laboratory **20** may send the sequence of bases to the vendor and have them build primer sets and probe anywhere inside the sequence. The remote user 1's Internet web-based account will have a field populated that represents these reagents with an identifier such as the genetic line identification **84.** The remote user **1** will use the identifier (strain name or profile name) to indicate that these specific reagents are to be used on subsequent samples.

Similarly, if the remote user **1** requires SNP genotyping a remote user **1** can contact the screening laboratory **20** and provide a literature refernce of the mutation which discloses the mutation name. A mutation name query of the Mouse Genome Informatics website yields links to different databases such as Ensenbl and National Center for Biotechnology Information that provides sequence data. This sequence data is the designated genetic sequence. Knowing the endogenous nucleotide and the mutant nucleotide, the screening laboratory **20** can take the designated genetic sequence, or portion thereof, and send it to a vendor indicating specifically where to build the primers and probes as to be informative for screening. For example, if the designated genetic sequence is 500 nucleotides in length, the screening laboratory **20** may indicate to the reagent vendor to build a SNP assay targeting the $239^{th}$ nucleotide. The reagent vendor will then supply to the screening laboratory **20,** the primers and probes to specifically discriminate between a nucleotide change at the $239^{th}$ position of the designated genetic sequence.

The remote user **1**'s Internet web-based account will have a field populated that represents these reagents with an identifier such as a name or number, or what is commonly referred to as the genetic line identification **84.** The remote user **1** will use the genetic line identification **84** to indicate that these specific reagents are to be used on subsequent samples.

The probes and primer sets, if they are new and have not before been tested against a sample containing the designated genetic sequence, must then be tested, preferably by the screening laboratory **20.** To do this, the screening laboratory **20** preferably receives both a positive and a negative strain control samples from the remote user **1** and tests them against the probes and primer sets to confirm that they can be used successfully to determine whether the designated genetic sequence can be detected. These controls include one positive and one negative control for each mutation found in the strain of interest.

If the designated genetic sequence can be detected using the probes and primer sets, the screening laboratory **20** updates the website and the order management software to provide the remote user **1** with a web-based selection for sample testing using those tested probes and primer sets. These selections among which the remote user **1** can select are one of the screening parameter selections identified below.

Alternatively, for example, if the remote user **1** or other third party communicates to the screening laboratory **20** that a particular probe or primer set has already been tested and is known to work, or if the screening laboratory **20** has already designed a probe and primer set for the designated genetic sequence (which is commonly the case for often-used strains or lines of transgenic animals) the screening laboratory **20** can immediately add a selection to the website and does not need to test controls with the probes and primer sets.

The strain controls are used to tell LIMS **24** a signal magnitude that is then associated with a positive or negative sample. In one case, the remote user **1** may send these controls together with the samples to be tested to the screening laboratory **20** in a single shipment. Alternatively, the controls may be sent separately from the samples to be tested.

The screening laboratory **20** tests the strain controls using the process described herein for testing samples. At the end of this testing process, the signal values for the strain controls are recorded into LIMS **24.** The magnitude of the signal provided by the positive control indicates the expected signal level for subsequently tested samples having the designated genetic sequence. The magnitude of the signal provided by the negative control indicating the expected signal level for subsequently tested samples that do not have the designate genetic sequence.

The computer at the screening laboratory **20** is configured to compare the test results (i.e. signal levels) for every sample that it subsequently tests for that designated genetic sequence with these multiple control signal levels and, based on that determination, to decide whether that sample has or does not have the designated genetic sequence. Positive and negative strain controls for a line therefore do not need to be resubmitted for each subsequent order but can be referenced by the screening laboratory **20** computer when later samples are tested for the same designated genetic sequence.

For transgenic zygosity genotyping, additional controls (not just a positive and a negative) are required to indicate each possible variation such as: a homozygous control, a heterozygous control and a wild type control.

Upon receipt of the primers and probe from a vendor, the sample, if available, will be screened using these reagents. Once a determination is made that there is discrimination between different genetic conditions, then the reagents will be placed in the inventory. Additionally, the screening laboratory **20** will populate a data field on the order management system, allowing the remote user **1** to select this primer sets and probe combination(s) for subsequent samples. This data filed will be populated with an indicator such as a mutation name, strain name or genetic line identification that will represent these reagents or combination of reagents that will be used in subsequent samples of this strain. This allows the remote user 1 to select the indicator of the reagents and prevents the need to transfer genetic information with each order.

**FIGS. 1-3** present an overview of certain features of the present invention. The present invention allows a remote user **1** with access to a computer **5** to order genotype screening of samples they submit to screening laboratory **20.** Using the Internet or other communication link **7,** the remote user **1** sends an access request from the remote user's computer **5** to a screening laboratory **20** computer **9** via an electronic communication link **7,** such as the Internet. The screening laboratory **20** website **19** will transmit an access enabling response to the remote user 1 via electronic communication link **7.** This response includes three distinct sections. The three sections are Account Registration **21,** Survey of Work 23 and Sample Identification and Designation 25 (**FIG**. **3).**

Now referring to **FIG**. **2,** a remote user **1** can access screening laboratory **20** website **19** via communication link **7.** The website **19** can be housed by an order manager **22.** An order manager is a software-based order management system. In the preferred embodiment the order manager **22** is an order management system developed by "Big Fish", a software development company in Memphis, TN. The order manager **22** functions to manage the placement of the order. The order received from the remote user **1** is transmitted to website **19,** which reports the order to order manager **22.** Manager **22** is in electronic communication via link **7** with screening laboratory **20** computer **9.** Screening laboratory **20** computer **9** includes LIMS **24,** which is communicatively coupled to a process controller **26.**

LIMS **24** is the generic name for laboratory information management system software. The function of LIMS **24** is to be a repository for data, to control automation of a laboratory, to track samples, to chart work flow, and to provide electronic data capture. LIMS **24** can also, in another embodiment, be in direct communication with the remote user **1** via an electronic communications link **7.** Any standard laboratory information management system software can configured to be used to provide these functions. Alternatively, a standard relational database management system such as Oracle (Oracle Corp., Redwood Shores, CA) or SQL Server (Microsoft Corp., Redmond, WA) either alone or in combination with a standard LIMS system can be used. In the preferred embodiment, the Nautilus@ program (Thermo Lab-Systems, a business of Thermo Electron Corporation, Beverly, MA) is used.

The process controller **26** is communicatively coupled to the workstation **14.** The process controller provides commands to any portions of the workstation **14** that are amenable to automation. For example, process controller **26** directs the delivery of the probes and primers to the Screening Station **95.** The workstation **14** is communicatively linked **28** to LIMS **24.** In this way, the workstation **14** can provide data to LIMS **24** for the formulation of the outcome report **249,** and then, via link **7** to the order manager **22** or remote user **1.** In an alternative embodiment, remote user **1** at remote user computer **5** can be linked **7** to the screening laboratory **20** by a direct phone line, cable or satellite connection.

Now refereing to **FIG**. **4,** the user's Account Registration section 21 begins with logging into the system **30.** A remote user **1** accesses an existing account by entering an account identification **31,** which is, for example, an e-mail address. The user will then enter a password **37.** If a valid password is entered, the user can place a new order **39.** Alternatively, the user can check an order status **41** by providing an order number **43** and can proceed to order tracking **45.** Alternatively, a new account **47** can be opened by providing an institution name, principal investigator, address, phone number, fax number, electronic mail address, billing information, and other authorized user names **49.** The user can enter a password **51,** confirm the password **53** and enter this billing information **55.**

Now referring to **FIGS. 5-6,** once the remote user **1** submits the Survey of Work section **23** the remote user **1** will be presented with the Sample Identification and Designation section **25.** In this section, the user (among other things) identifies where he will place each sample to be tested in an actual (physical) container **2** (**FIG**. **1)** by associating each sample with a corresponding well of a virtual **96** well container displayed on the computer screen of computer **5** as described below. The Sample Identification and Designation section **25** includes **96** well container locations. The remote user **1** designates which sample was or will be placed into each well. If the remote user **1** has more than **96** samples, subsequent **96** source well containers and designations are available. With respect to **FIG**. **6,** a **96** well source well container **2** having a barcode accession number **3** (**FIG**. **1)** will be shown (**FIG**. **6)** oriented in the longitudinal direction having an X axis labeled "A" to "H" (at **80)** and a Y axis labeled "1" to "12" (at **81).** The X and Y axes designate a well position such as "A1".

**FIGS. 5** and **6** together illustrate the Survey of Work section **23** and the Sample Identification and Designation Section **25.** Refereing now to **FIG**. **5,** the remote user **1** is asked to provide: source well container **2** accession number **82,** which the remote user **1** gets from the accession number **3** on the physical source well container **2** at his facility (**FIG**. **1)** that he intends to fill (or has filled) with the samples, number of lines **83,** genetic line identification **84,** number of samples **85,** and well location **88.** The remote user **1** is also asked for any internal sample identification number **91.**

For genotyping (i.e. screening to determine the presence of a designated genetic sequence) the positive strain control and the negative strain control samples are designated and deposited in wells of a microwell container. The remote user **1** indicates that a sample is a control sample at **89.** This assumes, of course, that the strain controls were not earlier provided to the screening laboratory **20** as described above. If a control is deposited in source well container **2,** remote user **1** can also designate the zygosity, mosaic nature and copy number of the sample.

At this point, the remote user has completed the Survey of Work section **23** and the Sample Designation section **25** of **FIGS**. **5-6** and is ready to transmit the screening parameter selections gathered in those sections to website 19 and thence to screening laboratory **20** computer **9.**

Now referring to **FIGS. 1** and **2,** the remote user **1** transmits his or her order including the completed screening parameter selections to the screening laboratory **20** via link **7** such as the Internet or a direct line. The remote user **1** can transmit the selected screening parameter selections to LIMS **24** in screening laboratory **20** via electronic communications link **7.** This link **7** can be direct or indirect. In the indirect route, the screening parameters are first transmitted to web site **19,** wherein order manager **22** receives the order and then provides LIMS **24** with the screening parameter selections.

In a particularly preferred embodiment of the system described in the foregoing paragraphs, remote user **1** at computer **5** transmits a request for a home web page served by screening laboratory **20** web site **19** via the electronic communication link **7.** Web site **19,** in turn, serves a home web page to computer **5** that includes information identifying the source of the web page and including a login button. Remote user **1** at computer **5** clicks on the login button displayed on his computer screen, transmitting a signal to web site **19** requesting access to the web site. This request is transmitted over communications link 7 to web site **19,** which responds with a second web page having fields for the entry of an account identifier (in the preferred embodiment an e-mail address), and a password. Remote user **1** enters the remote user **1** e-mail address and password, and transmits this information to web site **19** to gain access to the web site. Web site **19** receives this access request and compares the account identifier and password against its database of pre-existing accounts in the order manager **22** to determine whether the user is permitted to access the web site **19.** If so, computer order manager **22** serves up a further web page, called an order manager web page, which includes several user selectable choices including an "order status" button for tracking previous orders and results (if any have been received), a "supply request" button for requesting supplies, and an "order" button for ordering additional tests.

To order genetic testing, user **1** clicks on the "order" button displayed on the screen of computer **5.** Computer **5** transmits the user **1** request to web site **19.** Web site **19** receives this request, and transmits a first ordering web page to computer **5.** Computer **5,** in turn, displays several fields on its computer screen, including several data entry widgets. The first of these widgets is list box including two selectable entries for requesting the speed of service. In the preferred embodiment there are two speeds of service: 24-hour service and 72 hour service. The second of these widgets is a list box providing several entries, each entry in the box corresponding to a strain for which the sample is to be tested. The third widget is a text box for entering the number of samples of the selected strain to be tested. The fourth widget is a text box for entering the accession number (typically a bar code number) of the source well container **2** in which the samples are to be placed for shipping to the screening laboratory **20.**

The remote user **1** types in the number of samples to be tested. In this embodiment the samples are taken from transgenic animals, each sample typically corresponding to one animal to be tested. Typically several animals are tested to determine if they received the transgenic gene from their parents. Each strain of animal is defined by one or more designated genetic sequence. Thus, by designating the strain for which the samples are to be tested, the remote user **1** selects the one or more designated genetic sequences associated with that sequence. In the preferred embodiment, the remote user **1** can also select or deselect each individual probe and primer set that is used to screen for the designated sequences in the strain or line of the biological sample.

Once the remote user **1** has entered the number of samples to be tested, he or she then enters the name of the strain that the samples are to be tested for. Again, by selecting a strain the remote user **1** indicates the designated genetic sequence for which the samples are to be tested, since each strain is bred to have that sequence.

Once remote user **1** has selected the speed of service, the strain to be tested, and the number of samples to be tested for that strain, he enters the accession number from the source well container **2** and clicks on a button on the first ordering web page for recording this first group of samples to be tested. Computer **5,** in turn, generates a revised first ordering web page, the revised page including a table entry in a table on the revised web page listing the first group of samples in tabular form, wherein each row in the table corresponds to one group of samples to be tested, identifying that group of samples by the strains for which that group of samples is to be tested, and the number of samples in that group.

This process of creating a new group of samples and identifying them by the strain for which they'll be tested, and the number of the samples, can be continued as many times as necessary until all the samples to be tested are identified in the table.

Once all of the groups of samples have been entered and listed in the table on the revised first ordering web page, the operator then selects a button identified "next" and moves to the next stage in the ordering process. Computer **5** transmits this request to web site **19,** which generates a graphical image of a **96** source well container, appearing on the screen of computer 5 identical to the corresponding **96** source well container **2** that the remote user **1** is filling/has filled with samples, and transmits that image embedded in a second web page back to computer **5** for display. The second web page includes a graphical representation of a **96** well plate, in a top view, showing the two dimensional array of all **96** wells in which the remote user **1** is to place the samples identified previously. Web site **19** calculates the respective positions of each group of samples in the well container **2.** Each group is shown in the graphical representation of the well plate in a different color. All the wells in a group are shaded with the color associated with that group.

Samples of the same color from the same group are grouped together thus producing several different contiguous groups of wells, each group of wells have the same color different from the color of the adjacent groups.

The images of the wells in the web page are displayed on the computer with an initial shading to indicate that they have not been identified to a particular animal from which the sample in each well will be taken. In the preferred embodiment, each well contains a sample, such as a tissue sample, taken from an individual animal. The purpose of the testing performed on the samples in the wells is to determine the genetic characteristics of the animal from which each sample was taken. In order to relate the test results performed on each sample back to the animal from which the sample was taken, the user must make a record of the animal source of each sample (i.e. the animal from which each sample was taken).

To uniquely identify each sample in each well with an associated animal, remote user **1** selects a button on the third ordering web page. This button signals computer **9** to generate an additional web page. This web page lists each well in the well plate that was previously identified as containing a sample. Thus, if the first group of samples were 13 in number, there would be 13 entries listed in the additional web page. The web page itself is arranged as a single column of entries. Each entry in the column of entries includes a well identifier (called well location **88,** above), which is a string of alphanumeric characters that uniquely identifies one well of source well container **2.** A preferred well identifier for the **96** well plate is an alphabetic character followed by a numeric character. A text box is adjacent to each well identifier on the additional web page. To uniquely identify each sample in the source well container **2,** the user enters alphanumeric characters in the text box that are uniquely associated with each sample. This identifier is typically a short string of consecutive alphabet or numeric characters, a practice commonly used by research facilities to identify individual animals used for testing.

Animals in a particular group of animals having (presumed) common genetic characteristics will typically be identified by tattoos, tags, or other permanent means by consecutive or sequential numbers, characters, or combinations of numbers and characters (for example "A1", "A2", "A3", or "101", "102", 103", or "AA", AB", "AC", etc.). In a preferred embodiment, user **1** enters each animal number into the text box as a sample ID **91.** Animals may also be identified by a unique combination of disfigurements such as cutting or cropping toes, tails or ears that can also be approximated to a progressive alphanumeric sequence.

To assist the remote user **1** in entering the sample ID **91** into each of the text boxes in the additional web page, a button is provided to automatically fill several consecutive text boxes based upon the alphanumeric characters typed into a few text boxes from the group. For example, if the user types in "B7" in the first text box of a group, then types in "B8" in the second text box of a group, computer **5** is configured to automatically generate consecutive alphanumeric strings to fill the remaining text boxes of the group based upon these two manually typed-in entries. In this case, computer **5** would automatically generate the alphanumeric strings "B9", "B10", "B11", etc. and insert these characters sequentially into the remaining text boxes of the group in the additional web page. This process can be repeated for each subsequent group shown on the additional web page. Alternatively, the computer can be configured to automatically generate alphanumeric characters for all the groups at once and to fill the text boxes of all the groups all at once. Once the user has finished identifying all of the groups of samples and filling out all of the sample ID's **91** in the text boxes on the screen of computer **5,** he clicks on a button labeled "next". Computer **5** transmits this request to website **19,** which responsively generates another web page in which the user **1** enters shipping and tracking information. This page, called the order confirmation page, includes a text box for entering a character string. This character string provides access to a web-based shipment tracking system of a commercial shipping company. In the preferred embodiment, the character string is a tracking number used by the shipping company to track the samples from the remote user **1** to the screening laboratory **20.** In the preferred embodiment, the tracking number is provided to the user together with the source well container **2** and the packaging materials in which the user places the source well container **2** for shipment to the screening lab **20.**

The order confirmation page also includes an invoice that lists the different tests requested by the operator in the foregoing steps on the screen of computer **5.** Each test or group of tests is displayed on the screen adjacent to the price or prices for those tests. A total price of all the tests is displayed as well.

The order confirmation page has a second text box in which the remote user **1** can type the expected shipping date. The expected shipping date is the date on which remote user **1** intends to give the samples in their packaging materials to the delivery service associated with the tracking number. By providing the anticipated shipping date to the website **19** and then to the screening laboratory **20,** personnel at the screening laboratory **20** can anticipate the arrival of each shipment and prepare for its arrival by pre-ordering reagents, probes and primer sets required for testing the samples in advance.

Once the operator has entered the tracking number and the expected shipping date, he clicks on a button labeled "confirm order", which transmits the completed order, including the tracking number and expected shipping date to website **19** and order manager **22,** and thence to LIMS **24.**

In the preferred embodiment, once the order has been transmitted to the order manager **22,** the order generates two electronic messages, which will be sent to different locations. The first message is cross-referenced in LIMS **24** with a list of stocked probes. If the probe designated by the user is not stocked, an order message is sent to a supplier **11,** such as a contracted probe provider. This request can be transmitted from remote user **1** to screening laboratory **20** via any form of electronic communication, and then via a form of electronic communication **10** to suppliers' computer **8,** or in the alternative, the order message can go from user **1** via any form of electronic communication link **12** to suppliers' computer **8.** The supplier **11** creates the primer sets and probe based on the designated genetic sequence designated by the remote user **1** or the screening laboratory **20.** The made to order probe can be referred to as the target-binding probe. This supplier **11** will then barcode and overnight ship **13** the primer sets and target-binding probes **17** to the screening laboratory **20.** Once the primer sets and target-binding probes for each order for that day's screening are received by screening laboratory **20,** the barcodes on the primer sets and target-binding probes are scanned into LIMS **24.** The LIMS **24** records the date and time the primers and target-binding probes were received along with the quality control data provided from the probe provider.

In the preferred embodiment, the primer sets and target-binding probes are placed in workstation **14** and LIMS **24** will record the barcode of the probe and record its specific location on the deck of the workstation **14,** as will be discussed in more detail with respect to the Screening Station **95.** Additionally, the screening laboratory **20** and the LIMS **24** system correlates which target-binding probes will be used on which samples, as will be discussed in more detail with regard to the Screening Station **95.**

The second message, in the preferred embodiment, that is generated from the order placement of the remote user **1** insures that the remote user **1** has the proper supplies to package and ship their samples. This message, sent via link **12,** will define the barcode number of well container(s), shipping labels tracking number and amount of reagents needed for the user. In response to this message, supplier **11** will package **18** supplies for remote user **1** and ship **14A** the supplies back to remote user **1.**

Once the remote user **1** procures or receives these supplies, the remote user **1** places the appropriate samples into the source well containers **2** previously identified in the order sent to website **19,** order manager **22** and LIMS **24.** In other words, the remote user **1** fills each well of source well container **2** such that each well contains the same sample with the same sample ID **91** that the user previously identified in the order previously sent to website **19.** Alternatively, if the user already had sufficient supplies when the user placed the order the user need not wait for a source well container **2** to be sent by a supplier, but can fill the source well container **2** when the user creates the order, or even before the order is created. What is important is that the contents of the actual **96** source well container **2** that the user fills exactly matches the description of the samples and has the same accession number as the order the user previously sent to website **19.**

The samples can be obtained from prokaryotic or eukaryotic organisms. The samples may be a tissue sample from a mouse **8A,** but can also come from other animals, plants and viruses. In the preferred embodiment, mouse tails or ears are snipped to provide a tissue sample. Source well container **2** is a **96** well plate or the like that receives the sample in each well of the well plate. A sufficient amount of lysis reagent can be added to cover the sample. In one embodiment, the lysis reagent is added prior to transit to the screening laboratory **20.** Although, in the preferred embodiment the lysis reagent is added at the screening laboratory **20** at Lysing Station **92.**

Referring now to **FIG. 1,** source well container **2** has an accession number **3** affixed to the side of the container. The accession number is used by LIMS **24** to track the source of source well container **2.** The remote user **1** places the appropriate samples into the well locations in source well container **2** that they had previously designated while placing their order in **FIG. 6.** Once the samples are in the proper wells in the source well container **2** then the remote user **1** in one embodiment dispenses a predetermined amount of reconstituted lysis reagent **4** to cover the sample into each well using a pipette. The lysis reagent **4** is formulated to lyse the tissue to obtain cellular debris including genomic nucleic acid. A lysis reagent **4** can be formulated to lyse the biological sample while in transit between remote user **1** and the screening laboratory **20.** The transit time is approximately 24 hours as all samples are shipped via an express delivery service, such as FedEx® (Memphis, TN). The remote user **1** will add lysis reagent **4** to each well of the source well container **2.** The lysis reagent **4** should completely cover the samples. Once the samples and lysis reagent **4** are in the source well container **2** the remote user **1** places a seal on the top of the source well container **2** preventing samples from leaking. The remote user **1** then places a plastic lid on the seal for transportation. The remote user **1** then places the source well container **2** into an overnight delivery service package **15.** The remote user **1** will then seal the package and ship **16** to screening laboratory **20,** and apply a barcode shipping label.

A biological sample can be collected in a variety of ways to facilitate rapid screening. In one aspect of the invention, the biological sample is a sample of tissue such as from a mouse biopsy. The sample of tissue can include a portion of a tail, toes and ears. The tissue sample is collected by a remote user **1** and placed in a well of a source well container **2.** The microwell container is transported to the screening laboratory **20.** A multi-well container as shown in **FIG**. **1,** in the preferred embodiment, is a 96 microwell source well container **2** but can include other multi-well containers, such as Strip Racks, 24 well plates, 384 well plates and tube rack holders or the like. As described above with regard to **FIG**. **6,** the remote user **1** operates computer **5** to enter a variety of data regarding the samples placed in the source well container. Once all of the samples in all of the wells have been identified in this manner, the remote user sends the source well container **2** containing a plurality of biological samples to a screening laboratory **20** for screening.

In another embodiment of this invention, the biological sample is a blood sample collected by nicking the animal to be tested and blotting the blood on a filter paper. The blotted filter paper is placed in individual wells of source well container **2** by the remote user **1** and transported to the screening laboratory **20.** In both of these embodiments, the biological sample is disposed on an absorbent carrier.

In another embodiment, the biological sample is embryonic stem cells. A sample is placed or grown in a well of a source well container **2** by the remote user **1** and transmitted to the screening laboratory **20.**

Now referring to **FIG. 7A-D,** the preferred embodiment of the present invention is shown. In **FIG**. 7A, the source well containers **2** arrive **101** at the screening laboratory **20.** The tracking number of the shipping label is read with a barcode reader **103.** If the shipping label is unreadable **105,** the tracking numbers are manually entered **107.** The scanning of the tracking number is received **104** in LIMS **24** and a received message is posted to the user's account as shown in tracking field. The source well container **2** are removed from the package and taken to a clean room **109.** The source well containers **2** contain the raw biological matter and in one embodiment lysis reagent. The source well containers **2** individual barcodes are scanned by the barcode reader **111** and recorded **106** in LIMS **24** as accession numbers. LIMS **24** can send **106** a probe order to supplier **11** through the order manager **22.** If the source well containers **2** individual barcodes are unable to be scanned **113,** the accession numbers are entered manually **115.** If the tracking number, accession number, user order and worklist properly correlate, LIMS **24** will activate (not shown) an active record number for the containers.

The source well containers **2** are loaded **116** into a transportation apparatus in a clean room. A transportation apparatus is any device that holds well containers and that can dock with the workstation. The transportation apparatus, in the preferred embodiment, includes several rigid trays stacked vertically in a housing unit that is mobile. This transportation apparatus can be moved between different automated stations, docked and the rigid trays can be removed in an automated fashion and processed on the deck of a workstation. Each rigid tray consists of nine locations for source well containers **2.** Each of these nine locations per tray has a unique barcode designating its specific location inside the trays of the transportation module.

Source well container **2** accession number **3** is scanned with a barcode reader and the bar-coded source well container **2** location in the transportation apparatus trays is scanned. The barcodes of source well containers **2** are married **117** in LIMS **24** with the unique barcode locations in the transportation apparatus trays for tracking purposes. LIMS **24** records and associates each well container to this location. Once the transportation apparatus is loaded with the source well containers **2,** the transportation apparatus is docked **119** into the laboratory workstation **14.**

LIMS **24** will generate a worksheet for laboratory personnel (not shown). The worksheet outlines the probes and primer sets that the operator will need to prepare or gather in order to test the latest samples. The LIMS 24 worklist will generate a single file. The file format may include, but is not limited to, ASCII, XML or HTML. The file will be written into a specified directory on the network drive. The name of the file will be unique and will correlate to a run number. The extension will be unique for worklist files.

In the configuration described above, a transportation apparatus includes a housing unit provided to support several trays, each tray having nine different locations for nine source well containers 2. In an alternative embodiment, however, the housing unit can be eliminated. Instead, the source well containers **2** can be manually transported throughout the workstation in trays from functional station to functional station. In this system, operator at the laboratory loads source well containers into the trays after the source well containers **2** are received at the screening laboratory **20** and are scanned into LIMS **24** as described above for transportation to workstation **14.** Alternatively, source well containers **2** can be transported individually to workstation **14** and be placed in a tray or trays that are already located at workstation **14.**

We now refer to **FIG.** 8, which depicts one embodiment of the workstation **14.** Standard laboratory stations are logical groupings of laboratory operations. These groupings, however, do not necessarily refer to different physical stations. These logical groupings include: Lysing Station **92,** Automated Accessioning Station 93, Isolation/Purification Station **94,** Screening Station **95** and Detection Station **96,** all of whom comprise workstation **14.** The Screening Station **95** can include other screening processes such as PCR. Lysing Station **92** is an alternative step provided to lyse the samples in containers **2** in the event user **1** does not choose to lyse the samples by adding a lysis reagent before sending them to laboratory **20.** The functions of the various logical stations are described below in connection with the steps shown in **FIGS. 7A**-**D**. The following description provides the preferred embodiment, although one skilled in the art could elect to conduct these methods with varying degrees of automation as required.

As mentioned above, remote user **1** need not add a lysis reagent to the samples before shipping them to screening laboratory **20.** Instead, the samples may be shipped un-lysed (at room temperature) and may be lysed at laboratory **20** by piercing the cover **121** of the container **2** and treating each of the samples with a lysis reagent after docking the tray in the workstation **119** in the lysing station **92.** The samples are incubated **123** to produce a lysate containing cellular debris including at least a portion of intact genomic nucleic acid.

For tissue biopsies, the lysis process in the preferred embodiment includes incubation with the lysis reagent, such as proteinase K and a Nuclei Lysing Solution (NLS) (Promega Corporation, Madison, WI) at 55° C. for three hours. Other lysis reagents such as sodium dodecylsulfate and proteinase K can be used. The lysis reagent is selected to not fragment the genomic nucleic acid. A sufficient amount is an amount in the wells of container **2** sufficient to cover the samples.

With respect to the blood sample collection method, a sufficient amount of a lysis reagent, such as Nuclei Lysing Solution (Promega Corporation, Madison, Wisconsin) is added to each well of source well containers **2** to cover the filter paper. With respect to animal embryonic stem cell Nuclei Lysing Solution (Promega Corporation, Madison, WI) is added to each well containing the tissue/cells. The source well container **2** is treated under conditions to facilitate rapid lysis of the biological sample. In the preferred embodiment, these conditions are heating at 55° C. for three hours. However, samples should not be sonicated for more than 3-5 seconds after lysis to eliminate all intact genomic nucleic acid.

The preferred method of performing the above lysing steps at Lysing Station **92** includes loading source well containers **2** into the tray **9206** and taking the rigid tray to Lysing Station **92** to be lysed. Lysing Station **92** includes a liquid handler **9220,** such as Genesis Tecan (Raleigh Durham, North Carolina) or Multimeck Beckman (Indianapolis, Indiana). An example of a preferred Lysing Station **92** is shown in **FIG. 14.** It includes a frame **9202,** on which a deck **9204** is mounted to provide a horizontal working surface, which supports tray **9206,** which supports and positions up to nine source well containers **2.** A material handler **9214** is fixed to frame **9202** and extends upward and across the top surface of deck **9204.** A computer **9208** is coupled to material handler **9206** to direct the movement and operation of pipettes **9210.** A trough or reservoir **9212** is provided on deck **9204,** from which computer **9208** commands the material handler **9214** to aspirate lysis reagent into pipettes **9210** and to deposit the reagent into wells of container **2.**

The operator first carries a plurality of source well containers **2** and places them on deck **9204** in one of the nine positions on the rigid tray **9206** that support and orient source well containers **2** thereby docking them **119** into the workstation **14.** The operator then enters the number of wells that are filled with samples in each of the source well containers **2** into computer **9208** in combination with the location of that container with respect to tray **9206.**

Knowing the location of each source well container **2** in tray **9206,** and the number of wells that are filled with samples in each of these source well containers **2,** computer **9208** then directs material handler **9214** to move the pipettes **9210** to each source well container **2** in turn, piercing **121** the barrier sealing mechanism and filling each of the wells of source well containers **2** containing a sample with lysis reagent. By providing the location and the number of samples, computer **9208** is configured to fill only the wells containing samples with lysis reagent and to leave the empty wells empty of lysis reagent.

Once each of the sample-containing wells has been filled with lysis reagent, the operator moves the entire tray or trays **9206** containing the samples to an oven **9216 (FIG. 15),** where the samples are incubated **123** by heating for a period of about three hours at a temperature of 55° C (described above). Once the incubation process is complete, the operator moves source well containers **2** supported on the tray or trays **9206** to Automated Accessioning Station **93.**

An Automated Accessioning Station **93** provides a device to remove liquid from the source well container **2** to the primary master well container **6.** The primary master well container **6** is the container in which the nucleic acid is isolated. It is preferably a 384 well plate (Fisher Scientific #NC9134044). Any commercially available automated accessioning device can perform this function such as Genesis® Tecan (Raleigh-Durham, NC) or Multimeck® Beckman (Indianapolis, IN). These devices are referred to as liquid handlers. The source well containers **2** barcode accession numbers **3** are re-scanned **127.** This measurement will be recorded and posted **108** into the LIMS **24** database and reflected in the outcome report **249.** Additionally, LIMS **24** ensures **108** that source well containers **2** are consistent from transportation apparatus to the Automated Accessioning Station **93.** Error codes will be generated if a sufficient amount of raw testing material is not available. The liquid handler utilizes stainless steel, or the like, pipette tips that are washed between each sample transfer. Alternatively, disposable pipette tips may be used.

The nucleic acid lysate is transferred **129** to clean well containers, called primary master well containers **6.** Each of the containers **6** has a scannable accession number, preferably a barcode accession number, called "barcodes" or "accession numbers" below. The barcodes of the primary master well containers **6** are scanned **131** and LIMS **24** marries **102** the barcodes for the primary master well containers **6** to the scanned barcode accession numbers **3** of the source well plates **2.** The automated process accessioning continues until all of the day's pending samples are accessioned into the primary master well containers **6.** The preferred method of performing the above steps at Accessioning Station **93** includes taking the rigid tray **9206** and the source well containers 2 from the incubating oven **9216** back to the same liquid handler **9220** that performs the functions of Lysing Station **92.** This liquid handler **9220** is also preferably configured to function as Accessioning Station **93.**

Referring now to **FIG. 14,** the operator returns tray **9206** to liquid handler **9220** and places tray **9206** back on deck **9204** generally in the same location it was in when the lysis reagent was inserted into each well containing a sample.

Once in that location, the operator commands computer **9208** to fetch the work list from LIMS **24** and electronically stores it in the computer memory of process controller **26.** The work list includes the accession numbers of each source well container **2** that is in tray **9206,** together with the probe type that should be used for each well. The work list uniquely associates the location of the well, the accession number of source well container **2** from which the well is from, the probe type that is to be used with the sample in that source well container **2,** and the quantity of probe to be added to that sample.

Once computer **9208** fetches the work list, computer **9208** directs the operator to electronically scan **127** the accession numbers of all the source well containers **2** that are in rigid tray **9206** on deck **9204** of liquid handler **9220** using scanning device **9218** coupled to computer **9208.** Scanning device **9218** is preferably a glyph scanner, character scanner, bar code scanner, dot matrix scanner, or RFID tag scanner, depending upon the form of the accession identifier (typically a barcode accession number 3) on source well container **2.** Once source well containers **2** have been scanned **127,** computer **9208** transmits **108** the accession numbers **3** to process controller **26** and thence to LIMS **24.** Process controller **26** preferably includes an instrument database to which each of the computers of Lysing Station **92,** Automated Accessioning Station **93,** Isolation/Purification Station **94,** Screening Station **95** and Detection Station 96 transmit their data in order to maintain an ongoing record of the testing process and the location of materials and samples throughout that process. The database is preferably implemented using Microsoft's SQL Server, although any relational database (e.g. Oracle), may be used.

Computer **9208** then commands material handler **9206** to transfer **129** the contents of each well (i.e. lysate) in source well containers **2** to a corresponding well in the primary master well container **6** using pipettes **9210.** Computer **9208** directs the operator to scan **131** the accession numbers on the primary master well container **6.** Like the accession number on source well containers **2,** the accession number on the primary master well container **6** may be any electronically scannable indicia or device. Computer **9208** transmits the accession numbers to process controller **26,** which sends them to LIMS **24.** In this manner, LIMS **24** maintains a record of each sample and its location in each source well container **2** and in each primary master well container **6.** LIMS **24** and process controller **26** correlate the accession number of each primary master well container **6** with the identity of each sample it contains, the strain for which each sample is to be tested, the designated genetic sequence or sequences that identify or indicate that strain, the probes and primer sets necessary to test for those designated genetic sequences and the results of the testing.

The tray of primary master well containers is moved by the transportation apparatus to the Isolation/Purification Station **94.** In this station, the genomic nucleic acid will be isolated and purified using a separation method such as magnetic or paramagnetic particles. Purified genomic nucleic acid, substantially free of protein or chemical contamination is obtained by adding a sufficient amount of magnetic particles to each of the well containers that bind to a predefined quantity of nucleic acid. The term "magnetic" in the present specification means both magnetic and paramagnetic. The magnetic particles can range from 0.1 micron in mean diameter to 100 microns in mean diameter. The magnetic particles can be functionalized as shown by Hawkins, U.S. Patent No. 5,705,628 at col. 3.

In the preferred embodiment, the magnetic particles are purchased from Promega Corporation, a measured amount of magnetically responsive particles are added **133** to the lysate mixture with or without the presence of a chaotropic salt **135.** In the preferred embodiment, 13 μ1 amounts of 1 micron silica magnetic particles with chaotrope 113 μ1 (Promega Corporation, Madison, WI) are added to each well of the microwell container. The fixed volume of particles becomes saturated with nucleic acid and excess nucleic acid is removed. It has been observed that the resulting nucleic acid concentration between samples is very consistent. In a 50 μ1 pathlength read by the Genios (Tecan, Research Triangle Park, NC) a standard $A_{260}$ is 0.2 OD units. A standard concentration range of 0.1 to 0.3 O.D. units is disassociated from the magnetic particles to yield purified genomic nucleic acid.

**Table 1** shows that with increasing amounts of magnetic particles, the nucleic acid concentration also increases.

TABLE 1

| Average | Stdev | Bead Volume per 150 μl of lysate |
|---|---|---|
| 0.7974 | 0.0072 | 27 |
| 0.8750 | 0.040 | 35 |
| 1.2328 | 0.026 | 50 |
| 1.7900 | 0.022 | 75 |

While the nucleic acid concentration is consistent between samples treated with the same protocol, several factors may increase or decrease the resulting standard concentration of genomic nucleic acid. These factors include:

the binding reagent, the number of purification washes, and the solution that is used to elute the nucleic acid. The preferred binding solution for the magnetic particles obtained from Promega (Madison, WI) is a chaotropic salt, such as guadinium isothiocyanate. Alternatively, other binding reagents, such as 20% polyethylene glycol (PEG) 8000, 0.02% sodium azide and 2.5M sodium chloride may be used to nonspecifically bind the genomic nucleic acid to the surface chemistry of the functionalized magnetic particles. If functionalized magnetic particles are used, the preferred binding solution is PEG. The PEG or chaotropic guadinium isothiocyanate allows for the disruption of hydrogen binding of water, which causes binding of the nucleic acid to the particles. The preferred washing procedure to remove contaminants includes two chaotrope washes, after the initial chaotrope binding step, followed by four 95% ethanol washes. Aqueous solutions, or the like, are the best elution solutions. These solutions include water, saline sodium citrate (SSC) and Tris Borate EDTA (ie. 1xTBE).

The preferred device for performing the above functions of the Isolation/Purification Station **94** is a liquid handler **9402** identical in general construction to the liquid handler **9220** identified above for use as the Lysing Station **92** and the Accessioning Station **93** that has been configured to automatically transfer the various reagents and other liquids as well as the magnetic particles in the manner described below.

**FIG. 16** illustrates a preferred embodiment of the liquid handler **9402.** Handler 9402 comprises a frame **9404** on which is mounted a deck **9406,** which is surmounted by material handler **9408,** which supports and positions pipettes **9410** and is coupled to and controlled by computer **9412,** which is in turn coupled to process controller **26** to communicate information to and from LIMS **24.** Liquid handler **9402** includes a syringe pump **9414** that is coupled to and driven by computer **9412** to dispense magnetic particles via a 16x24 array of 384 pipettes **9410** simultaneously into all **384** wells of the primary master well container **6** under the command of computer **9412.** Liquid handler **9402** also includes a second syringe pump **9416** that is configured to dispense a binding buffer into wells of the primary master well container **6** under computer control. The liquid handler also includes a magnet **9418** mounted in deck **9406** as well as a conveyor **9420** that is coupled to and controlled by computer **9412** to move the primary master well container **6** in tray **9206** back and forth between a first position **9422** in which the container is within the magnetic field and a second position **9424** in which the container is outside the magnetic field.

Before the functions of the Isolation and Purification Station **94** can be performed, the operator must first move the primary master well container **6** from Accessioning Station **93** to deck **9406** of liquid handler **9402** and place it in a predetermined location on the deck. Once the operator has placed the primary master well container **6,** the operator starts an isolation/purification program running on computer **9412.** This program drives the operations of liquid handler **9402** causing it to dispense magnetic particles **133** into all the wells of the primary master well container **6** containing lysed samples. Computer **9412** signals syringe pump **9414** to dispense the particles using pipettes **9410** into the primary master well container **6** when container 6 is in position **9424,** away from the magnetic field created by magnet **9418.**

Once the particles have been added, computer **9412** then directs the pipettes **9410** to add a chaotropic salt such as guadinium isothiocyanate to each of the wells to bind the genomic nucleic acid to the magnetic particles at **135.** Once the chaotropic salt has been added, computer **9412** then mixes the contents of the wells by signaling the pipettes **9410** to alternately aspirate and redispense the material in each of the wells. This aspiration/redispensing process is preferably repeated three or four times to mix the contents in each well.

Once the contents of the wells have been mixed, computer **9412** pauses for two minutes to permit the particles, binding reagent, and raw biological material in the wells to incubate at room temperature in position **9424.** When the two minutes have passed, computer **9412** commands the conveyor **9420** to move tray **9206** from position **9424** to position **9422,** directly above magnet **9418** at **137.** In this position the magnet draws the magnetic particles in each of the wells downward to the bottom of the wells of the primary master well container 6. Computer **9412** keeps tray **9206** and the primary master well container **6** over the magnet and within the magnetic field for 2-6 minutes, or until substantially all the magnetic particles are drawn to the bottom of each well and form a small pellet.

The particles drawn to the bottom of each well have genomic nucleic acid attached to their outer surface -- genomic nucleic acid that the particles hold until an elution solution is placed in each well to release the genomic nucleic acid from the particles. With the particles at the bottom of each well and the wells located within the magnetic field, computer 9412 directs the pipettes to aspirate the supernatant **139.**

Once the supernatant is removed, computer **9412** signals the conveyor to move the primary master well container **6** on tray **9206** to the nonmagnetic position **9424.** The foregoing process of adding chaotropic salt, mixing the combination, pausing, drawing the magnetic particles down and aspirating the supernatant is repeated two more times.

Computer **9412** then directs the pipettes to introduce a wash solution (for example 70% ethanol when functionalized beads are used, or 95% ethanol (4x) when silica beads are used) to resuspend the particles **141.** Computer **9412** again mixes the contents of the wells by signaling the pipettes to alternately aspirate and redispense the material in each of the wells. With the wash buffer and particles thoroughly mixed, computer **9412** again moves tray **9206** and the primary master well container **6** back over magnet **9420** in position **9422 143** and draws the magnetic particles back to the bottom of the wells. This wash process **141,143,145** is repeated three times to thoroughly cleanse the magnetic particles, and dilute and remove all supernatant.

Once the particles are thoroughly washed, computer **9412** permits the magnetic particles in each well to air dry **147.** In the preferred embodiment, shown in **FIGURE 17,** the operator moves the primary master well container **6** to a dryer **9426** (an "Ultravap" dryer by Porvair Sciences, UK) having **384** tubules disposed in a 16 x 24 array **9428** that are configured to be simultaneously inserted into each of the wells of the primary master well container **6** and to supply warm, dry air thereto. In an alternative method, computer **9412** causes material handler **9408** to direct compressed dry nitrogen gas into each well of the primary master well container **6,** drying the particles out in place while the container is in the magnetic field. Alternatively the samples can be permitted to air dry. Once the particles are completely dry, the primary master well container **6** can be subsequently moved away from the field of magnet **149.**

Once the particles are almost dry, the operator returns the primary master well container **6** to the liquid handler **9402** and directs the computer **9412** to command the pipettes 9410 to fill the wells with an elution solution **151** and resuspend the particles. This elution solution is formulated to elute the bound genomic nucleic acid from the particles. An example of one such elution solution is 0.01M Tris (pH 7.4), sodium saline citrate (SSC), dimethyl sulfoxide (DMSO), sucrose (20%), 1xTBE, or formamide (100%). In the preferred embodiment, the elution solution is nuclease-free water. Nuclease free water is selected to minimize contamination and produce a standard concentration of purified genomic nucleic acid. In the preferred embodiment, the elution solution temperature is 22°C. A preferred yield is about 20 ng/μL of genomic nucleic acid is obtained.

After resuspending the genomic nucleic acid in a solution for a predetermined period of time, computer **9412** again moves tray **9206** with the primary master well container **6** via conveyor **9420** to position **9422** over magnet **9418** **155.** The magnet, in turn, draws the magnetic particles down to the bottom of each well. This leaves the genomic nucleic acid mixed and suspended in the elution solution. Computer **9412** then directs the pipettes to aspirate a small amount (50 μl of purified genomic nucleic acid and to transfer **159** the small amount from each well into a corresponding well of a clean optical **384-well** container that is also mounted on deck **9406.** The operator scans **161** a barcode accession number on the optical container and computer **9412** transfers the scanned accession number to process controller **26,** which then transfers it to LIMS **24.** The operator takes this optical container to a UV spectrometer (Genios, by Tecan of Raleigh-Durham, NC), and directs the UV spectrometer to optically scan the optical container, by making an $A_{260}$ measurement **163.** This measurement is electronically transferred **112** to LIMS **24** over a data communications link.

If another fully automated system is desired, the magnetic separator can be automated and rise from the bottom of the workstation and make contact with bottoms of all primary well containers simultaneously.

In the preferred embodiment for the biological sample, the genomic nucleic acid is not sonicated after separation from the cellular debris. The genomic nucleic acid includes at least a portion of intact nucleic acid. Unsonicated nucleic acid is recovered in the condition it is found in the lysate. Thus, if the genomic nucleic acid is intact in the lysate, it is intact (i.e., unfragmented) as attached to the particles. The sample contains at least a portion of intact genomic nucleic acid.

In certain types of samples, the genomic nucleic acid is substantially intact. In one embodiment, the genomic nucleic acid can be sonicated before or after separation with the magnetic particles. Sonication can be done by any conventional means such as a fixed horn instrument or plate sonicator. In the one embodiment, the genomic nucleic acid is sonicated for five seconds to produce nucleic acid fragments. Although there is a wide range of fragments from about 100 base pairs to up to 20 kilobases, the average size of the fragment is around about 500 base pairs.

The primary master well container **6** is transported to the deck of the Screening Station **95 (FIG. 18)** where its bar code is scanned **173.** The operator places the container on a magnet, drawing all the magnetic particles to the bottom of the wells. The supernatant contains the purified genomic nucleic acid. LIMS **24** generates a worklist containing barcodes that list the primer/probe combinations that need to be loaded onto the deck of the machine. The primer-probe combinations are contained in barcoded tubes. An operator loads the barcoded tubes randomly into a probe box. The operator then scans the barcodes on the tubes using a Matrix scanner coupled to LIMS **24.** The primer set and probe combinations in the tubes are then loaded into an ABI 384 PCR plate (Applied Biosystems, Forest City, CA). The genomic nucleic acid sample from each well of the primary master well container **6** is added to a corresponding well of the ABI PCR plate that contains the primer-probe combination or combinations appropriate to discern the relevant genotype **187.** The ABI plate is then sealed with sealing tape and taken to the Detection Station **96** and placed in an ABI 7900. In the preferred embodiment the ABI 7900 cycles the ABI PCR plate 40 times between temperatures specified by the manufacturer. The operator can vary the number of cycles and the temperatures as desired to increase the signal provided by the samples.

**FIG. 18** shows a preferred device for performing the Screening Station **95** functions. It comprises a liquid handler **9502** such as Genesis Tecan (Raleigh Durham, North Carolina) or Multimeck Beckman (Indianapolis, Indiana). It includes a frame **9504,** on which a deck **9506** is mounted to provide a horizontal working surface for first tray **9206** and second tray **9206.** The first and second trays (as described above) can support and position nine primary master well containers **6.**

Liquid handler **9502** also includes a material handler **9508** that is fixed to frame 9504 and extends upward and across the top surface of deck **9506.** A computer **9510** is coupled to material handler **9508** to direct the movement and operation of pipettes **9512.** Pipettes **9512** are fluidly coupled to a syringe pump **9514.**

Probe block **9516** is disposed on the surface of deck **9506** and contains several tubes (not shown) each tube containing one or more combined primer sets and probes. The operator bar-codes each tube and enters the data indicative of the tube contents (the particular primer or probe in each tube, its volume and concentration) into LIMS **24,** which stores the data associated with the bar code on the tube for later reference **173.**

The operator places the primary master well containers **6** on deck **9506,** scans the bar code accession number of the primary master well container **6,** and signals computer **9510** to start transferring genomic nucleic acid, probes and primer sets.

Based upon the information provided by the remote user **1,** including the samples, the strains for which the samples are to be tested, and the designated genetic sequences indicated by the strains, as well as the probes and primer sets necessary to detect those designated genetic sequences, as well as the location of each sample in the ABI PCR plate, LIMS **24** calculates a worklist that identifies for the operator which (and how many) tubes containing which probes and which primer sets must be placed in the probe block **9516** to test the samples in the primary master well container **6.**

The operator first prints out this worklist, using it as a guide to identify and select particular tubes containing the proper probes and primers. The operator takes these tubes out of storage, places them in the probe block **9516** and places the probe block **9516** on the Matrix scanner.

The Matrix scanner is coupled to LIMS **24,** and is configured to scan the bar codes on each tube through holes in the bottom of the probe block. The scanner passes this information to LIMS, to which it is coupled, which in turn compares the bar codes of the scanned tubes with the bar codes of the probes identified on the worklist. Only if the operator has loaded the probe block with the appropriate type and number of probes and primer sets will LIMS **24** permit the operator to proceed. In this manner, LIMS is configured to verify that the operator has inserted the appropriate and necessary tubes of probes and primer sets into the probe block.

Once LIMS **24** has verified that the proper tubes of probes and primer sets have been inserted into the probe block, it is configured to indicate to the operator that the probe block is acceptable and that the process steps at Screening Station **95** can begin.

The steps of preparing tubes of probes and primer sets, entering them into LIMS, preparing a worklist, filling a probe block and verifying the probe block, all happen prior to the time the operator takes the primary master well container **6** with its **384** wells to the deck **9506** of liquid handler **9502** and places it in position on deck **9506.**

The operator places the primary master well container 6 in position on first tray **9206** located on deck **9506** of liquid handler **9502.** The operator electronically scans the container with an electronic scanner **9518** coupled to computer **9510** which, in turn, is coupled to process controller **26.** As described above, the scanner may be any of several types of electronic scanner but is preferably a bar code scanner.

If there are several primary master well containers **6,** they are preferably carried from the liquid handler of the Isolation/Purification Station **94** to the liquid handler of the Screening Station **95** in tray **9206,** which can accommodate nine separate primary master well containers **6.**

The operator also places a secondary master well container **27** (preferably an ABI 384 PCR plate) in a predetermined location on the second tray **9206** located on deck **9506** adjacent to the first tray **9206.** The operator electronically scans the secondary master well container **27** with the electronic scanner **9518** and stores the location and identity of the secondary master well container **27** in process controller **26** which transmits the data to LIMS **24.**

If there are several primary master well containers **6** that must be transferred to secondary master well containers **27,** the corresponding secondary master well containers **27** may also be taken to liquid handler **9502** in trays **9206,** rather than the operator carrying each secondary master well container **27** to second tray **9206** individually.

Once the operator places at least one primary master well container **6** in first tray 9506 and at least one secondary master well container **27** in second tray **9506,** the operator signals computer **9510** to begin combining the probes, primer sets, and genomic nucleic acid extracted from the samples.

Generally speaking, computer **9510** commands material handler **9508** to extract probes and primer sets from tubes in probe **box 9516** and deposit them in each secondary master well container **27** in second tray **9206.** Computer **9510** then commands material handler **9508** to extract the genomic nucleic acid from the wells of each primary master well container **6** in first tray **9206** and deposit the samples in wells in a corresponding secondary master well container **27.** When the pipettes **9512** deposit the genomic nucleic acid samples, the probes, and the primer sets in wells in the secondary master well containers **27,** computer **9510** commands material handler **9508** and pipettes **9512** to mix the samples using the aspiration/redispensing methods discussed above.

The secondary master well containers **27** receive a number of aliquots of biological sample in multiple wells of the secondary master well container. In one embodiment, an aliquot of the biological sample of the strain is dispensed into at least four wells of the secondary master well container **27.** To at least two of the four wells at least one probe and primer set (e.g. SEQ ID NO. 23, 24 & 25) corresponding to at least one designated genetic sequence is added. A probe (SEQ ID NO. 21) and primer set (SEQ ID NO. 19 & 20) correspond to a reference sequence (SEQ ID NO. 18) is added to the third and fourth well. Thus, for example, if the genotype screening includes four designated genetic sequences, then four wells of the secondary master well containers **27** receive an aliquot of the biological sample and the corresponding probes and primer sets for each designated genetic sequence. Additionally, four wells receive an aliquot of the biological sample and the corresponding four probe and primer sets. This second set of wells is referred to as the replicants. The function of the replicants is quality control. Additionally, two additional wells receive aliquots of the biological sample and the housekeeping or screening reference probe/primer set.

In a simpler embodiment, the validity of the screening data can be evaluated by dispensing an aliquot of a biological sample of the strain designated by the remote user into at least two wells of a microwell container. In one well at least one probe and primer set is added corresponding to the at least one designated genetic sequence and to the other well at least one probe and primer is added corresponding to the reference sequence (SEQ ID NO. 18). The biological sample is screened and the probe signal values are compared between the probe for the designated genetic sequence and the probe for the referenced sequence.

In other embodiments, multiple probe and primer sets can be multiplexed into a single well. Furthermore, the detection of SNPs involve adding two probes to a well.

Between one and five microliters of nucleic acid and four and fifteen microliters of probes and primer sets are preferred to insure proper mixing of the samples and proper polymerization in the PCR process of the Detection Station **96** that follows.

Once the wells in the secondary master well containers **27** are filled with the appropriate purified genomic nucleic acid samples, primer sets and probes, and these materials are mixed, computer **9510** signals the operator that the screening process is complete. The plate is then sealed with optical sealing tape. The operator then moves the secondary master well containers **27** to Detection Station **96** for further processing.

In the preferred embodiment, the central component of Detection Station **96** is the ABI 7900. The secondary master well containers **27** are placed inside the ABI 7900, where they are thermocycled **189** 40 times and exposed to an excitatory energy source to produce a quantifiable signal **195** from the signal molecule. More particularly, the Detection Station 96 scans the secondary master well container's **27** barcode and reports it **196** to LIMS **24.**

**FIG. 19** illustrates a preferred device for performing the functions of Detection Station **96.** It includes a PCR instrument **9602** (here shown as an ABI 7900), a material handler 9604 (here shown as a ZYmark arm), a computer **9606,** and an electronic scanner **9608** (here shown as a barcode scanner).

Computer **9606** is coupled to PCR instrument **9602,** material handler **9604,** and process controller **26.** It communicates with PCR instrument **9602** to control the insertion and removal of secondary master well containers **27** from PCR **9602** by handler **9604.** Computer 9606 is also coupled to PCR instrument **9602** to process test results from the test performed by PCR instrument **9602** and to transmit those test results to process controller **26** and then to LIMS **24.**

Scanner **9608** is coupled to handler **9604** to scan the accession numbers on the secondary master well containers **27,** and to transmit those accession numbers to LIMS **24.**

Material handler **9604** includes an arm **9610** that is commanded by computer **9606** to move between three positions: an incoming material hopper **9612,** and outgoing material hopper **9614,** and loading/unloading position **9616.** Handler **9604** moves between these positions under the control of computer **9606,** which commands this movement.

The operator first loads incoming material hopper **9612** with one or more secondary master well containers **27.** The operator then operates the computer terminal **9618** of computer **9606,** commanding computer **9606** to load and test the secondary master well containers **27.** In response, computer **9606** commands arm **9610** to move to the incoming material hopper **9612,** grasp the topmost secondary master well container **27,** and to carry that container to the loading/unloading position **9616.** Computer **9606** also commands PCR instrument **9602** to extend a tray (not shown) from an opening **9618** in the side of the ABI 7900, and commands arm **9610** to place the secondary master well container **27** on that tray. Scanner **9608** is configured to scan the barcode accession number on the secondary master well container **27,** thereby making an electronic record of the secondary master well container **27** that is being tested. Scanner **9608** transmits this accession number to computer **9606,** which later correlates the accession number with the test results provided by ABI 7900.

Once the secondary master well container **27** is placed in the tray, computer **9606** commands PCR instrument **9602** to retract the tray, and to begin testing the material in the secondary master well container **27,** which is now inside PCR instrument **9602.** PCR instrument **9602** signals computer **9606** when testing is complete. PCR instrument **9602** also transmits the test results to computer **9606.** Computer **9606,** in turn, commands PCR instrument **9602** to eject the secondary master well container **27** that has just been tested, moving it back to loading/unloading position **9616.** Once the secondary master well container **27** is in this position, computer **9606** commands material handler **9604** to move arm **9610** back to the loading/unloading position **9616** and to retrieve the secondary master well container **27** that has just been tested. Computer **9606** commands arm **9610** to move the just-tested secondary master well container **27** to outgoing material hopper **9614,** where it is deposited, awaiting later removal by the operator of Detection Station **96.**

Now referring to **FIG. 9,** LIMS **24** now prepares the outcome report **249.** Several calculations are performed before they are posted to the outcome report **249.** In the preferred embodiment, such calculations include the evaluation of all replicates per sample. Calculating the relationship between the experimental quantified signal and the quantified signals of designated control may elucidate the copy number, zygosity or mosaic nature of the sample. The ratio for homozygous individuals should be twice the ratio of heterozygous individuals.

A reference sequence (SEQ ID NO. 18) and respective primer set and probe (SEQ ID NO. 19-21) is used to normalize the signal of every other probe used for that sample. The resulting value, called an RCN, is a comparison of the signal of the test probe (i.e. probes for portion of the designated genetic sequences) to the reference sequence. This control serves an additional purpose which is to evaluate the consistency of the nucleic purification system. This control will produce a magnitude of fluorescence directly proportional to the amount of starting nucleic acid, so nucleic acid concentrations can be compared. More specifically, the probe value corresponds to the designated genetic sequence is compared to the probe value of the replicant. Similarly, each value is compared to the probe value for the reference sequence to evaluate the validity of the data obtained.

For each sample, the CT values for the two wells containing the housekeeping gene, cjun, are averaged ($CT_{cjun}$). The RCN values are calculated by comparing the test probe (i.e. Neo or Cre) signal to the housekeeping gene signals or each of the two test probe wells ($T_1$ and $T_2$), the following equation is applied:

<u>Table 2</u> - Example of RCN Calculation

$$\boldsymbol{RCN_1 = 2^{-(CT1CTcjun)}}$$

$$RCN_2 = 2^{-(CT2-,CTcjun)}$$

| Well | Sample Name | Detector | Task | CT | Average c-jun | RCN |
|---|---|---|---|---|---|---|
| C1 | Neomycin KO1 | c-jun | Unknown | 25.37 | 25.27 | |
| D1 | Neomycin KO1 | c-jun | Unknown | 25.17 | | |
| E1 | Neomycin KO1 | Neo A | Unknown | 33.27 | | 0.00 |
| F1 | Neomycin KO1 | Neo A | Unknown | 34.24 | | 0.00 |

Now referring to **FIG.** 9, the sample outcome report **249** may include account registration **250,** well plate container 2 barcode number(s) (i.e. accession numbers) **252,** control sample locations 252 and genetic characterization of the designated control **252.** Additionally, the outcome report **249** may include well location **254,** sample identification **256,** nucleic acid concentration **260,** signal quantification **266,** qualitative results **268,** zygosity/copy number **270,** quantitative analysis via comparison to designated control signal strengths allowing for copy number estimation, zygosity or mosaic nature **270.** The outcome report **249** may also include a picture file (email) or pictorial representations of results **272** as shown in **FIG. 10.** Additionally, information gathered at the request of the remote user **1** from optimization and sequence confirmation quality control data and error messages may be included in the outcome report **249.** The remote user **1** may choose to have this file electronically sent or choose to be electronically notified. Additionally, remote user **1** has the option to have a hard copy sent via the postal service or facsimile.

Once the LIMS **24** has compiled all the data for the outcome report **249,** the outcome report will be sent **7** to the remote user **1**. In the preferred embodiment, LIMS **24** will send the report via a remote link **7** to either the remote user **1** or the order manager **22,** which can post the results on the web site **16** or via an electronic link **7.** The LIMS **24** will keep results available for six months and then the results will be recorded onto a long-term storage disk and archived.

The following examples are provided by way of examples and are not intended to limit the scope of the invention.

8. Examples

**Example 1 - Mouse Tail Genotyping** A biological sample in the form of a mouse tail biopsy is submitted via FedEx (Memphis, Tennessee) overnight delivery to the screening laboratory **20** from the remote user **1.** Each sample occupies one well of a 96-well source well container **2.**

The remote user **1** provides the genetic line identification **84.** A line includes at least one designated genetic sequence. In the genetic line identification **84** provided by the remote user **1.** The remote user **1** selects a designated genetic sequence. The genetic line identification **84** has been previously associated with the designated genetic sequence CRE (SEQ ID NO. 22); Mn1Tel (SEQ ID NO. 38) and p16 (SEQ ID NO. 50).

A lysis reagent (made of 2.5 μl of proteinase K (VWR EM-24568-3) and 147.5 μl of Nuclei Lysing Solution (Promega Corporation, Madison, Wisconsin A7943) per sample) is gently mixed and poured into a 25 ml trough or reservoir and is placed on the deck of a Tecan Genesis Workstation (Research Triangle Park, NC). The liquid handler dispenses 150 μl of the lysis reagent in to each sample well of the source well container **2.** The well plate is then placed in a 55°C oven for three hours. The well plate is then placed back on the deck of the Tecan Genesis Workstation (Research Triangle Park, NC). The liquid handler aspirates 50 μl of each sample and dispenses it in to a 384 well primary master well container (Fisher Scientific #NC9134044). Once all of the samples are transferred, the primary master well container is moved to the deck of the Isolation Station Purification Station **94.**

One-hundred and twelve microliters of SV Lysis reagent (Promega Corporation, Madison WI, # Z305X) a chaotropic salt are added to each sample. Next, 13 μl of magnetic particles (Promega Corporation, #A220X) are added and the well components are mixed. The well plate is then moved into the magnetic field of a magnet where the magnetic particles are drawn to the bottom of each well. The supernatant is then aspirated and discarded. The well plate is moved out of the magnetic field and 95 μl of SV Lysis reagent is added to each well and mixed. The well plate is then moved into the magnetic field and the supernatant is drawn off and discarded. This washing process is repeated two additional times. Next, the samples are washed four times in 130 μl of 95% ethanol as described above. After the fourth ethanol wash, the microwell container are placed on a 384 tip dryer for 11 minutes. Then the microwell container are moved back to the deck of the Isolation Station Purification Station **94** and 155 μl of Ambion's (Houston, TX) nuclease free water (catalog #B9934) is added to each well at room temperature. The plate is then moved into the magnetic field and 50 μl of DNA elution is transferred to a 384 well optical storage plate (Fisher Scientific, #08-772136) for optical density analysis. An $A_{260}$ reading of the storage plate read is performed with a Tecan Genios Spectrometer (Research Triangle Park, NC). This reading shows nucleic acid is present at the desired concentration of 0.2 O.D. units, but a range of 0.1 to 0.5 OD units is acceptable.

The primary master wellplate with the isolated DNA is moved to the deck of a Tecan Freedom Workstation. The TaqMan Universal Master Mix, real time PCR primer mixture and Ambion water are placed on the deck as well. The final PCR mixture is made of 1x TaqMan Universal Master Mix (catalog # 4326708), 1x real time PCR primer set/probe mix for a designated genetic sequence (Applied Biosystems Assays-by-Design(SM) Service 4331348) and 25% isolated DNA. In this example, the primer set as set out in SEQ ID NO. 23 and 24 and probe as set out in SEQ ID NO. 25 correspond to the designated genetic sequence CRE (SEQ ID NO. 22). Additionally, the primer set as set out in SEQ ID NO. 35 and 36 and probe as set out in SEQ ID NO. 37 correspond to the designated genetic sequence Mn1Tel (SEQ ID NO. 38). Additionally, the primer set as set out in SEQ ID NO. 51 and 52 and probe as set out in SEQ ID NO. 53 correspond to the designated genetic sequence for p16 (SEQ ID NO. 50). The Tecan Genesis added the reagents together in the ABI 7900 384 Well Optical Plate. The plate is then sealed with optical sealing tape (ABI, #4311971).

The samples are then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Tables 3 and 4. On average, these results are transmitted to the remote user **1** within twenty-four hours of receiving the biological sample at the screening laboratory **20.**

TABLE 3

| Well | Sample Named | Designated Genetic Sequence | CT | RCN |
|---|---|---|---|---|
| 25 | 51 | Cjun | 25.722 | - |
| 49 | 51 | Cjun | 25.927 | - |
| 121 | 51 | CRE | 21.937 | 14.799 |
| 145 | 51 | CRE | 21.939 | 14.779 |
| 73 | 51 | Mn1Tel | 22.24 | 12.879 |
| 97 | 51 | Mn1Tel | 21.816 | 17.278 |
| 169 | 51 | p16 | 27.945 | 0.247 |
| 193 | 51 | p16 | 28.076 | 0.225 |
| 217 | 52 | Cjun | 26.26 | - |
| 241 | 52 | Cjun | 26.188 | - |
| 313 | 52 | CRE | 22.475 | 13.451 |
| 337 | 52 | CRE | 22.441 | 13.767 |
| 265 | 52 | Mn1Tel | 22.747 | 11.134 |
| 289 | 52 | Mn1Tel | 23.62 | 6.081 |
| 2 | 52 | p16 | 28.884 | 0.158 |
| 361 | 52 | p16 | 28.612 | 0.191 |
| 26 | 53 | Cjun | 25.919 | - |
| 50 | 53 | Cjun | 25.919 | - |
| 122 | 53 | CRE | 31.432 | 0.022 |
| 146 | 53 | CRE | 31.553 | 0.02 |
| 74 | 53 | Mn1Tel | 22.122 | 13.898 |
| 98 | 53 | Mn1Tel | 21.968 | 15.467 |
| 170 | 53 | p16 | 27.722 | 0.286 |

(continued)

| Well | Sample Named | Designated Genetic Sequence | CT | RCN |
|---|---|---|---|---|
| 194 | 53 | p16 | 27.717 | 0.287 |
| 218 | 54 | Cjun | 25.909 | - |
| 242 | 54 | Cjun | 25.915 | - |
| 314 | 54 | CRE | 21.745 | 17.96 |
| 338 | 54 | CRE | 21.669 | 18.937 |
| 266 | 54 | Mn1Tel | 22.15 | 13.567 |
| 290 | 54 | Mn1Tel | 24.116 | 3.472 |
| 3 | 54 | p16 | 28.029 | 0.231 |
| 362 | 54 | p16 | 27.703 | 0.289 |
| 27 | 55 | Cjun | 26.729 | - |
| 51 | 55 | Cjun | 26.836 | - |
| 123 | 55 | CRE | 22.146 | 24.865 |
| 147 | 55 | CRE | 22.028 | 26.993 |
| 75 | 55 | Mn1Tel | 22.602 | 18.134 |
| 99 | 55 | Mn1Tel | 28.724 | 0.26 |
| 171 | 55 | p16 | 28.258 | 0.36 |
| 195 | 55 | p16 | 28.501 | 0.304 |
| 219 | 56 | Cjun | 27.348 | - |
| 243 | 56 | Cjun | 27.839 | - |
| 315 | 56 | CRE | 35.193 | 0.005 |
| 339 | 56 | CRE | 35.477 | 0.004 |
| 267 | 56 | Mn1Tel | 33.428 | 0.018 |
| 291 | 56 | Mn1Tel | 33.316 | 0.019 |
| 4 | 56 | p16 | 28.411 | 0.568 |
| 363 | 56 | p16 | 28.226 | 0.645 |
| 28 | 57 | Cjun | 25.569 | - |
| 52 | 57 | Cjun | 25.476 | - |
| 124 | 57 | CRE | 20.724 | 27.822 |
| 148 | 57 | CRE | 20.582 | 30.705 |
| 76 | 57 | Mn1Tel | 21.283 | 18.893 |
| 100 | 57 | Mn1Tel | 21.123 | 21.105 |
| 172 | 57 | p16 | 26.215 | 0.619 |
| 196 | 57 | p16 | 26.147 | 0.649 |
| 220 | 58 | Cjun | 25.541 | - |
| 244 | 58 | Cjun | 25.49 | - |
| 316 | 58 | CRE | 36.935 | 0 |
| 340 | 58 | CRE | 36.228 | 0.001 |
| 268 | 58 | Mn1Tel | 34.213 | 0.002 |
| 292 | 58 | Mn1Tel | 34.939 | 0.001 |
| 5 | 58 | p16 | 26.481 | 0.512 |
| 364 | 58 | p16 | 26.304 | 0.579 |
| 29 | 59 | Cjun | 25.794 | - |
| 53 | 59 | Cjun | 25.694 | - |
| 125 | 59 | CRE | 33.834 | 0.004 |
| 149 | 59 | CRE | 33.354 | 0.005 |
| 77 | 59 | Mn1Tel | 36.546 | 0.001 |
| 101 | 59 | Mn1Tel | 33.896 | 0.004 |
| 173 | 59 | p16 | 26.414 | 0.628 |
| 197 | 59 | p16 | 26.442 | 0.616 |
| 221 | 60 | Cjun | 25.998 | - |

(continued)

| Well | Sample Named | Designated Genetic Sequence | CT | RCN |
|---|---|---|---|---|
| 245 | 60 | Cjun | 26.105 | - |
| 317 | 60 | CRE | 21.593 | 21.981 |
| 341 | 60 | CRE | 21.442 | 24.421 |
| 269 | 60 | Mn1Tel | 21.864 | 18.223 |
| 293 | 60 | Mn1Tel | 21.74 | 19.858 |
| 6 | 60 | p16 | 26.954 | 0.535 |
| 365 | 60 | p16 | 26.499 | 0.733 |
| 30 | 61 | Cjun | 24.083 | - |
| 54 | 61 | Cjun | 24.067 | - |
| 126 | 61 | CRE | 34.69 | 0.001 |
| 150 | 61 | CRE | 35.396 | 0 |
| 78 | 61 | Mn1Tel | 40 | 0 |
| 102 | 61 | Mn1Tel | 35.961 | 0 |
| 174 | 61 | p16 | 26.1 | 0.246 |
| 198 | 61 | p16 | 26.164 | 0.235 |

TABLE 4

| Sample # | Cre | | | Mn1tel | | | p16 | | |
|---|---|---|---|---|---|---|---|---|---|
| 51 | 15.89 | 15.87 | + | 12.88 | 17.28 | + | 0.25 | 0.23 | + |
| 52 | 13.45 | 13.77 | + | 11.13 | 6.08 | + | 0.16 | 0.19 | + |
| 53 | 0.02 | 0.02 | - | 13.90 | 15.47 | + | 0.29 | 0.29 | + |
| 54 | 17.96 | 18.94 | + | 13.57 | 3.47 | + | 0.23 | 0.29 | + |
| 55 | 24.87 | 26.99 | + | 18.13 | 0.26 | + | 0.36 | 0.30 | + |
| 56 | 0.01 | 0.00 | - | 0.02 | 0.02 | - | 0.57 | 0.65 | + |
| 57 | 27.82 | 30.71 | + | 18.89 | 21.11 | + | 0.62 | 0.65 | + |
| 58 | 0.00 | 0.00 | - | 0.00 | 0.00 | - | 0.51 | 0.58 | + |
| 59 | 0.00 | 0.01 | - | 0.00 | 0.00 | - | 0.63 | 0.62 | + |
| 60 | 21.98 | 24.42 | + | 18.22 | 19.86 | + | 0.54 | 0.73 | + |
| 61 | 0.00 | 0.00 | - | 0.00 | 0.00 | - | 0.25 | 0.24 | + |

**Example 2 Blood Sample Collection Method:** Mouse tails are nicked with a razor blade and the resulting blood droplets are blotted on to filter paper (V&P Scientific Lint Free Blotting Media (114 mm long, 74 mm wide) #VP540D). The samples are placed in individual wells of a Nunc **96**-well plate (Fisher Scientific 12-565-368). The well locations are labeled and the plates are transported shipped to the screening laboratory **20.**

The remote user 1 provides the genetic line identification **84.** The genetic line in this example has been previously associated by the remote user **1** with the designated genetic sequence for Mn1Tel (SEQ ID NO. 38), CRE (SEQ ID NO. 22) and MHV (SEQ ID NO. 34).

The number of samples are counted and lysis reagent is made (2.5 μl of proteinase K (VWR EM-24568-3) and 147.5 μl of Nuclei Lysing Solution (Promega Corporation, Madison WI, A7943) per sample. The solution is gently mixed and poured into a 25 ml trough or reservoir and placed on the deck of a Tecan Genesis Workstation (Research Triangle Park, NC). The liquid handler dispenses 150 μl of the solution into each sample well. The well plate was then placed in a 55°C oven for three hours.

The well plate is then placed back on the deck of the Tecan Genesis Workstation. The liquid handler aspirates 50 μl of each sample and dispenses it in to a 384 primary master well container (Fisher Scientific #NC9134044). Once all of the samples are transferred, the primary master well container is moved to the deck of the Isolation Station Purification Station 94.

One-hundred and twelve microliters of SV Lysis reagent (Promega Corporation, # Z305X) are added to each sample. Next, 13 μl of magnetic particles (Promega Corporation # A220X) are added and the well components are mixed. The well plate is then moved into the magnetic field of a magnet where the magnetic particles are drawn to the bottom of each well. The supernatant is then aspirated and discarded. The well plate is moved out of the magnetic field

and 95 μl of SV Lysis reagent is added to each well and mixed. The well plate is then moved into the magnetic field and the supernatant is drawn off and discarded. This washing process is repeated two additional times. Next, the samples are washed four times in 130 μl of 95% ethanol as described above. After the last ethanol wash, the well plate is placed on a 384 tip dryer for 11 minutes. Then the well plate is moved back to the deck of the Isolation Station and 155 μl of Ambion's (Houston, TX) nuclease free water (catalog #B9934) is added to each well. The elution solution is heated to 95°. The plate is then moved into the magnetic field and 50 μl of DNA elution is transferred to a 384 well optical storage plate (Fisher Scientific, #08-772136) for optical density analysis.

An $A_{260}$ reading of the storage plate read is performed with a Tecan Genios Spectrometer. This reading shows nucleic acid was present at the desired concentration of 0.2 O.D. units, but, a range of 0.1 to 0.5 O.D. units is acceptable.

The amount of DNA isolated from the blood is less than the DNA yield recovered from tissue. The tissue lysate has enough DNA content to saturate the binding ability of the fixed volume of beads. However, the blood lysate does not have enough DNA to saturate the binding ability of the fixed amount of beads. This is evidence by the CT (cycle threshold) values for the housekeeping probe. The housekeeping (cjun) CT values for tissue isolations are approximately 26 whereas the approximate CT for housekeeping (cjun) for the blood isolations are approximately 35. This nine cycle difference represents approximately a 512 (2^9) fold difference in the amount DNA present. This non-saturated DNA yield does not present a problem for results because the housekeeping probe normalizes the results. For each sample, the CT values for the wells containing the housekeeping probe, cjun, are averaged ($CT_{cjun}$). The RCN ($RCN_1$ and $RCN_2$) values are calculated by comparing the test probe (i.e. Cre or MN1TEL) signal to the housekeeping gene signal average for each of the two test probe wells ($CT_1$ and $CT_2$), the following equation is applied:

$$RCN_1 = 2^{-(CT_1 - CT_{cjun})}$$

$$RCN_2 = 2^{-(CT_2 - CT_{cjun})}$$

The plate with the isolated DNA is moved to the deck of a Tecan Freedom Workstation; TaqMan Universal Master Mix, real time PCR primer mixture and Ambion water are placed on the deck as well. The final PCR mixture is made of 1x TaqMan Universal Master Mix (catalog # 4326708), 1x real time PCR primer mix for a designated genetic sequence (Applied Biosystems Assays-by-Design(SM) Service 4331348) and 25% isolated genomic DNA. The Tecan Genesis adds the reagents together in the ABI 7900 384 Well Optical Plate (Foster City, CA) catalog #4309849). The 384 well plate is then sealed with optical sealing tape (ABI, #4311971).

The samples are then placed in an Applied Biosystems SDS HT7900 (Foster City, CA). A standard real time PCR protocol is followed by heating the samples to 50°C for two minutes then incubated at 95°C for 10 minutes, followed by thermally cycling the samples 40 times between 95°C for 15 seconds and 60°C for one minute.

TABLE 5

Blood Samples Taken from Double KO mice Whatman Filter Paper used to capture samples

| Well | Sample Name | Designated Genetic Sequence | CT | Std. Dev. CT |
|---|---|---|---|---|
| A1 | WATER | Cjun | Undetermined | |
| A2 | Blood 2 | Cjun | 35.31 | 0.587 |
| A3 | Blood 3 | MN1TEL | 33.51 | 0.061 |
| A4 | Blood 4 | CRE | 34.72 | 0.27 |
| A5 | Blood 6 | Cjun | 35.78 | 0.175 |
| A6 | Blood 7 | MN1TEL | 33.24 | 0.325 |
| A7 | Blood 8 | CRE | Undetermined | |
| A8 | Blood 10 | Cjun | 35.44 | 0.023 |
| A9 | Blood 11 | MN1TEL | 35.25 | 0.004 |
| A10 | AF 2 | Cjun | 37.25 | 0.786 |
| A11 | AF 4 | Cjun | 35.17 | 0.165 |
| B1 | WATER | Cjun | Undetermined | |
| B2 | Blood 2 | Cjun | 34.48 | 0.587 |
| B3 | Blood 3 | MN1TEL | 33.42 | 0.061 |
| B4 | Blood 4 | CRE | 34.34 | 0.27 |

(continued)

| Blood Samples Taken from Double KO mice Whatman Filter Paper used to capture samples | | | | |
|---|---|---|---|---|
| Well | Sample Name | Designated Genetic Sequence | CT | Std. Dev. CT |
| B5 | Blood 6 | Cjun | 36.03 | 0.175 |
| B6 | Blood 7 | MN1TEL | 33.7 | 0.325 |
| B7 | Blood 8 | CRE | Undetermined | |
| B8 | Blood 10 | Cjun | 35.47 | 0.023 |
| B9 | Blood 11 | MN1TEL | 35.25 | 0.004 |
| B10 | AF 2 | Cjun | 36.14 | 0.786 |
| B11 | AF 4 | Cjun | 34.94 | 0.165 |
| C1 | Blood 1 | Cjun | 35.39 | 0.218 |
| C2 | Blood 2 | MN1TEL | 34.37 | 0.281 |
| C3 | Blood 3 | CRE | Undetermined | |
| C4 | Blood 5 | Cjun | 36.35 | 0.172 |
| C5 | Blood 6 | MN1TEL | 34.96 | 0.634 |
| C6 | Blood 7 | CRE | 37.76 | 0.556 |
| C7 | Blood 9 | Cjun | 33.61 | 0.069 |
| C8 | Blood 10 | MN1TEL | 34.3 | 0.734 |
| C9 | Blood 11 | CRE | 32.9 | 0.6 |
| C10 | AF 2 | MHV | Undetermined | |
| C11 | AF 4 | MHV | Undetermined | |
| D1 | Blood 1 | Cjun | 35.08 | 0.218 |
| D2 | Blood 2 | MN1TEL | 34.77 | 0.281 |
| D3 | Blood 3 | CRE | 39.09 | |
| D4 | Blood 5 | Cjun | 36.6 | 0.172 |
| D5 | Blood 6 | MN1TEL | 34.06 | 0.634 |
| D6 | Blood 7 | CRE | 38.55 | 0.556 |
| D7 | Blood 9 | Cjun | 33.71 | 0.069 |
| D8 | Blood 10 | MN1TEL | 33.26 | 0.734 |
| D9 | Blood 11 | CRE | 33.74 | 0.6 |
| D10 | AF 2 | MHV | Undetermined | |
| D11 | AF 4 | MHV | Undetermined | |
| E1 | Blood 1 | MN1TEL | 33.7 | 0.131 |
| E2 | Blood 2 | CRE | Undetermined | |
| E3 | Blood 4 | Cjun | 37.7 | 0.252 |
| E4 | Blood 5 | MN1TEL | 35.48 | 1.053 |
| E5 | Blood 6 | CRE | 31.84 | 0.03 |
| E6 | Blood 8 | Cjun | 34.57 | 0.13 |
| E7 | Blood 9 | MN1TEL | 32.45 | 0.111 |
| E8 | Blood 10 | CRE | Undetermined | |
| E9 | AF 1 | Cjun | 39.35 | 0.278 |
| E10 | AF 3 | Cjun | 33.75 | 0.213 |
| E11 | BF 1 | Cjun | 28.14 | 0.048 |
| F1 | Blood 1 | MN1TEL | 33.52 | 0.131 |
| F2 | Blood 2 | CRE | Undetermined | |
| F3 | Blood 4 | Cjun | 38.06 | 0.252 |
| F4 | Blood 5 | MN1TEL | 36.97 | 1.053 |
| F5 | Blood 6 | CRE | 31.88 | 0.03 |
| F6 | Blood 8 | Cjun | 34.75 | 0.13 |
| F7 | Blood 9 | MN1TEL | 32.29 | 0.111 |
| F8 | Blood 10 | CRE | Undetermined | |
| F9 | AF 1 | Cjun | 38.96 | 0.278 |

(continued)

Blood Samples Taken from Double KO mice Whatman Filter Paper used to capture samples

| Well | Sample Name | Designated Genetic Sequence | CT | Std. Dev. CT |
|---|---|---|---|---|
| F10 | AF 3 | Cjun | 34.05 | 0.213 |
| F11 | BF 1 | Cjun | 28.21 | 0.048 |
| G1 | Blood 1 | CRE | Undetermined | |
| G2 | Blood 3 | Cjun | 34.52 | 0.041 |
| G3 | Blood 4 | MN1TEL | 36.02 | 0.284 |
| G4 | Blood 5 | CRE | 38.12 | 0.071 |
| G5 | Blood 7 | Cjun | 34.69 | 0.387 |
| G6 | Blood 8 | MN1TEL | 33.29 | 0.302 |
| G7 | Blood 9 | CRE | 37.75 | |
| G8 | Blood 11 | Cjun | 36.57 | 0.057 |
| G9 | AF 1 | MHV | Undetermined | |
| G10 | AF 3 | MHV | Undetermined | |
| G11 | BF 1 | MHV | Undetermined | |
| H1 | Blood 1 | CRE | Undetermined | |
| H2 | Blood 3 | Cjun | 34.46 | 0.041 |
| H3 | Blood 4 | MN1TEL | 35.62 | 0.284 |
| H4 | Blood 5 | CRE | 38.02 | 0.071 |
| H5 | Blood 7 | Cjun | 35.24 | 0.387 |
| H6 | Blood 8 | MN1TEL | 33.72 | 0.302 |
| H7 | Blood 9 | CRE | Undetermined | |
| H8 | Blood 11 | Cjun | 36.65 | 0.057 |
| H9 | AF 1 | MHV | Undetermined | |
| H10 | AF 3 | MHV | Undetermined | |
| H11 | BF 1 | MHV | Undetermined | |

**Example 3 Mouse Embryonic Genotyping Protocol:** Mouse embryonic tissue is submitted via FedEx (Memphis, TN) overnight delivery. Each sample occupies one well of a **96**-well microwell container **2.** The remote user **1** provides the genetic line identification **84.** The genetic line in this example has been previously associated with the designated genetic sequence Neomycin (SEQ ID NO. 42) and Six 2 WT (SEQ. ID NO. 62).

A lysis reagent is made of (2.5 μl of proteinase K (VWR EM-24568-3) and 147.5 μl of Nuclei Lysing Solution (Promega Corporation A7943) per sample). The lysis reagent is gently mixed and poured into a 25 ml trough or reservoir and placed on the deck of a Tecan (Research Triangle Park, NC) Genesis Workstation. The liquid handler dispensed 150 μl of solution in to each sample well in the well plate. The well plate is then placed in a 55°C oven for three hours. Samples are sonicated with a fixed horn sonicator for 3-5 seconds, to yield a sample having at least a portion of intact genomic nucleic acids and at least a portion of nucleic acid fragments. Samples are then allowed to settle at room temperature for five minutes prior to accessioning.

The well plate is then placed back on the deck of the Tecan Genesis Workstation. The liquid handler aspirates 50 μl of each sample and dispenses it in to a 384 destination well plate (Fisher Scientific #NC9134044). Once all of the samples are transferred, the well plate is moved to the deck of the Isolation/Purification Station **94.**

One-hundred and twelve microliters of SV Lysis reagent (Promega Corporation, Madison WI, # Z305X) is added to each sample. Next, 13 μl of magnetic particles (Promega Corporation, #A220X) are added and the well components are mixed. The well plate is then moved into the magnetic field of a magnet where the magnetic particles are drawn to the bottom of each well. The supernatant is then aspirated and discarded. The well plate is moved out of the magnetic field and 95 μl of SV Lysis reagent is added to each well and mixed. The well plate is them moved into the magnetic field and the supernatant is drawn off and discarded. This washing process is repeated two additional times. Next, the sample is washed four times in 130 μl of 95% ethanol as described above. After the fourth ethanol wash, the destination plate is placed on a 384 tip dryer for 11 minutes. Then the well plate is moved back to the deck of the Isolation/Purification Station **94** and 155 μl of Ambion's (Houston, TX) nuclease free water (catalog #B9934) is added to each well of the well plate. The elution solution is heated to 95°. The well plate is then moved into the magnetic field and 50 μl of DNA elution is transferred to a 384 well optical storage plate (Fisher Scientific, #08-772136) for optical density analysis.

An $A_{260}$ reading of the storage plate read is performed with a Tecan Genios Spectrometer. This reading shows that nucleic acid is present at the desired concentration of 0.2 O.D units, but a range of 0.1 to 0.5 O.D. units is acceptable.

The plate with the isolated DNA is moved to the deck of a Tecan Freedom Workstation. The final PCR mixture is made of 1x TaqMan Universal Master Mix (catalog # 4326708), 1x real time PCR probe and primer mix for a designated genetic sequence (Applied Biosystems Assays-by-Design(SM) Service 4331348) free water and 25% isolated DNA to an ABI 7900 384 Well Optical Plate (Foster City, CA) catalog #4309849). The well plate is then sealed with optical sealing tape (ABI, #4311971).

The samples are then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Table 5 and 6. The designated genetic sequence is Neomycin (SEQ ID NO. 42)

Table 6

| Well | Sample Name | Designated Genetic Sequence | CT | RCN |
|---|---|---|---|---|
| 49 | 161016 | Cjun | 25.691 | - |
| 73 | 161016 | Cjun | 25.45 | - |
| 97 | 161016 | Neomycin | 22.873 | 6.488 |
| 121 | 161016 | Neomycin | 22.387 | 9.083 |
| 145 | 161016 | Six2 WT #1 | 25.063 | 1.422 |
| 169 | 161016 | Six2 WT #1 | 25.034 | 1.451 |
| 193 | 161017 | Cjun | 25.73 | - |
| 217 | 161017 | Cjun | 25.705 | - |
| 241 | 161017 | Neomycin | 33.269 | 0.005 |
| 265 | 161017 | Neomycin | 32.837 | 0.007 |
| 289 | 161017 | Six2 WT #1 | 25.403 | 1.244 |
| 313 | 161017 | Six2 WT #1 | 25.347 | 1.293 |
| 337 | 161018 | Cjun | 25.4 | - |
| 361 | 161018 | Cjun | 25.136 | - |
| 2 | 161018 | Neomycin | 22.706 | 5.908 |
| 26 | 161018 | Neomycin | 22.59 | 6.401 |
| 50 | 161018 | Six2 WT #1 | 25.34 | 0.951 |
| 74 | 161018 | Six2 WT #1 | 25.138 | 1.094 |
| 98 | 161019 | Cjun | 25.903 | - |
| 122 | 161019 | Cjun | 25.681 | - |
| 146 | 161019 | Neomycin | 21.993 | 13.921 |
| 170 | 161019 | Neomycin | 21.81 | 15.797 |
| 194 | 161019 | Six2 WT #1 | 36.329 | 0.001 |
| 218 | 161019 | Six2 WT #1 | 36.057 | 0.001 |
| 242 | 161020 | Cjun | 25.354 | - |
| 266 | 161020 | Cjun | 25.068 | - |
| 290 | 161020 | Neomycin | 21.654 | 11.767 |
| 314 | 161020 | Neomycin | 21.519 | 12.926 |
| 338 | 161020 | Six2 WT #1 | 34.738 | 0.001 |
| 362 | 161020 | Six2 WT #1 | 35.638 | 0.001 |
| 3 | 161021 | Cjun | 26.136 | - |
| 27 | 161021 | Cjun | 26.029 | - |
| 51 | 161021 | Neomycin | 34.416 | 0.003 |
| 75 | 161021 | Neomycin | 35.935 | 0.001 |
| 99 | 161021 | Six2 WT #1 | 25.762 | 1.249 |
| 123 | 161021 | Six2 WT #1 | 25.807 | 1.21 |
| 147 | 161022 | Cjun | 25.825 | - |
| 171 | 161022 | Cjun | 25.669 | - |
| 195 | 161022 | Neomycin | 22.954 | 6.931 |
| 219 | 161022 | Neomycin | 22.88 | 7.295 |

(continued)

| Well | Sample Name | Designated Genetic Sequence | CT | RCN |
|---|---|---|---|---|
| 243 | 161022 | Six2 WT #1 | 26.04 | 0.816 |
| 267 | 161022 | Six2 WT #1 | 25.735 | 1.008 |
| 291 | 161023 | Cjun | 25.09 | - |
| 315 | 161023 | Cjun | 25.304 | - |
| 339 | 161023 | Neomycin | 21.543 | 12.592 |
| 363 | 161023 | Neomycin | 21.422 | 13.688 |
| 4 | 161023 | Six2 WT #1 | 40 | 0 |
| 28 | 161023 | Six2 WT #1 | 34.991 | 0.001 |
| 52 | 161024 | Cjun | 25.461 | - |
| 76 | 161024 | Cjun | 25.062 | - |
| 100 | 161024 | Neomycin | 34.749 | 0.001 |
| 124 | 161024 | Neomycin | 35.415 | 0.001 |
| 148 | 161024 | Six2 WT #1 | 24.991 | 1.206 |
| 172 | 161024 | Six2 WT #1 | 24.676 | 1.501 |
| 196 | 161025 | Cjun | 26.426 | - |
| 220 | 161025 | Cjun | 26.073 | - |
| 244 | 161025 | Neomycin | 23.711 | 5.81 |
| 268 | 161025 | Neomycin | 23.539 | 6.544 |
| 292 | 161025 | Six2 WT #1 | 27.013 | 0.589 |
| 316 | 161025 | Six2 WT #1 | 26.959 | 0.612 |
| 340 | 161026 | Cjun | 25.343 | - |
| 364 | 161026 | Cjun | 25.111 | - |
| 5 | 161026 | Neomycin | 32.086 | 0.009 |
| 29 | 161026 | Neomycin | 31.224 | 0.016 |
| 53 | 161026 | Six2 WT #1 | 24.779 | 1.364 |
| 77 | 161026 | Six2 WT #1 | 24.526 | 1.626 |
| 101 | 161027 | Cjun | 25.955 | - |
| 125 | 161027 | Cjun | 25.668 | - |
| 149 | 161027 | Neomycin | 23.1 | 6.549 |
| 173 | 161027 | Neomycin | 23.125 | 6.434 |
| 197 | 161027 | Six2 WT #1 | 26.701 | 0.54 |
| 221 | 161027 | Six2 WT #1 | 26.203 | 0.762 |
| 245 | 161028 | Cjun | 25.232 | - |
| 269 | 161028 | Cjun | 25.151 | - |
| 293 | 161028 | Neomycin | 22.614 | 5.966 |
| 317 | 161028 | Neomycin | 22.635 | 5.881 |
| 341 | 161028 | Six2 WT #1 | 25.977 | 0.58 |
| 365 | 161028 | Six2 WT #1 | 25.709 | 0.698 |

**Example 4 Embryonic Stem Cell Genotyping Protocol:** Mouse embryonic stem cells were grown to confluence in a 96 well source well container **2** such as a cell culture plate and was submitted via FedEx (Memphis, TN) overnight delivery to the screening laboratory **20.** The remote user **1** provides the genetic line identification **84.** The genetic line in this example has been previously associated with the designated genetic sequence for OPN4 ES (SEQ ID NO. 46). The samples are counted and a lysis reagent is made of (2.5 μl of proteinase K (VWR EM-24568-3) and 147.5 μl of Nuclei Lysing Solution (Promega Corporation A7943) per sample). The solution is gently mixed and poured into a 25 ml trough or reservoir and placed on the deck of a Tecan (Research Triangle Park, NC) Genesis Workstation. The liquid handler dispenses 150 μl of solution in to each source well container **2.** The samples are then incubated at room temperature for ten minutes before being transferred to a polypropylene 96 well plate. The well plate is then covered and placed in a 55°C oven for three hours.

The source well container **2** is then placed back on the deck of the Tecan Genesis Workstation. The liquid handler aspirates 50 μl of each sample and dispenses it in to a 384 primary master well container (Fisher Scientific #NC9134044). Once all of the samples are transferred, the primary master well container **6** is moved to the deck of the Isolation/Purification Station **94.**

One-hundred and twelve microliters of SV Lysis reagent (Promega Corporation, Madison WI, # Z305X) are added to each sample. Next, 13 μl of magnetic particles (Promega Corporation, #A220X) are added and the well components were mixed. The well plate is then moved into the magnetic field of a magnet where the magnetic particles are drawn to the bottom of each well. The supernatant is then aspirated and discarded. The well plate is moved out of the magnetic field; 95 μl of SV Lysis reagent are added to each well and mixed. The well plate is them moved into the magnetic field and the supernatant is drawn off and discarded. This washing process is repeated two additional times. Next, the sample is washed four times in 130 μl of 95% ethanol as described above. After the fourth ethanol wash, the plate is placed on a 384 tip dryer for eleven minutes. Then the plate is moved back to the deck of the Isolation/Purification Station **94** and 155 μl of Ambion's (Houston, TX) nuclease free water (catalog #B9934) is added to each well. The elution solution is heated to 95°. The plate is then moved into the magnetic field and 50 μl of DNA elution was transferred to a 384 well optical storage plate (Fisher Scientific, #08-772136) for optical density analysis.

An $A_{260}$ reading of the storage plate read is performed with a Tecan Genios Spectrometer. This reading should nucleic acid be present at the desired 0.2 O.D. a range of 0.1 to 0.5 O.D. units is acceptable.

The primary master wellplate with the isolated DNA is moved to the deck of a Tecan Freedom Workstation. The TaqMan Universal Master Mix, real time PCR primer mixture and Ambion water are placed on the deck as well. The final PCR mixture is made of 1x TaqMan Universal Master Mix (catalog # 4326708), 1x real time PCR primer sets/probe mix for a designated genetic sequence (Applied Biosystems Assays-by-Design(SM) Service 4331348) and 25% isolated DNA. The Tecan Genesis adds the reagents together in the ABI 7900 384 Well Optical Plate. The plate is then sealed with optical sealing tape (ABI, #4311971).

The samples were then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Table 7.

TABLE 7

| Well | Sample Name | Designated Genetic Sequence | Reporter | Ct |
|---|---|---|---|---|
| 49 | 1 | Cjun | VIC | 31.46 |
| 73 | 1 | Cjun | VIC | 31.41 |
| 97 | 1 | OPN4ES | FAM | 29.54 |
| 121 | 1 | OPN4ES | FAM | 29.54 |
| 145 | 2 | Cjun | VIC | 30.39 |
| 169 | 2 | Cjun | VIC | 30.32 |
| 193 | 2 | OPN4ES | FAM | 28.61 |
| 217 | 2 | OPN4ES | FAM | 29.09 |
| 241 | 3 | Cjun | VIC | 31.13 |
| 265 | 3 | Cjun | VIC | 31.05 |
| 289 | 3 | OPN4ES | FAM | 29.62 |
| 313 | 3 | OPN4ES | FAM | 29.63 |
| 337 | 4 | Cjun | VIC | 31.01 |
| 361 | 4 | Cjun | VIC | 31.64 |
| 2 | 4 | OPN4ES | FAM | 29.57 |
| 26 | 4 | OPN4ES | FAM | 29.66 |
| 50 | 5 | Cjun | VIC | 31.76 |
| 74 | 5 | Cjun | VIC | 31.19 |
| 98 | 5 | OPN4ES | FAM | 30.36 |
| 122 | 5 | OPN4ES | FAM | 30.08 |
| 146 | 6 | Cjun | VIC | 30.79 |
| 170 | 6 | Cjun | VIC | 30.90 |
| 194 | 6 | OPN4ES | FAM | 29.47 |
| 218 | 6 | OPN4ES | FAM | 29.57 |
| 242 | 7 | Cjun | VIC | 33.59 |
| 266 | 7 | Cjun | VIC | 33.58 |
| 290 | 7 | OPN4ES | FAM | 32.06 |

(continued)

| Well | Sample Name | Designated Genetic Sequence | Reporter | Ct |
|---|---|---|---|---|
| 314 | 7 | OPN4ES | FAM | 32.11 |
| 338 | 8 | Cjun | VIC | 32.82 |
| 362 | 8 | Cjun | VIC | 33.25 |
| 3 | 8 | OPN4ES | FAM | 31.68 |
| 27 | 8 | OPN4ES | FAM | 31.44 |
| 51 | 9 | Cjun | VIC | 32.69 |
| 75 | 9 | Cjun | VIC | 32.96 |
| 99 | 9 | OPN4ES | FAM | 31.82 |
| 123 | 9 | OPN4ES | FAM | 31.33 |
| 147 | 10 | Cjun | VIC | 32.89 |
| 171 | 10 | Cjun | VIC | 32.80 |
| 195 | 10 | OPN4ES | FAM | 31.71 |
| 219 | 10 | OPN4ES | FAM | 31.46 |
| 243 | 11 | Cjun | VIC | 33.39 |
| 267 | 11 | Cjun | VIC | 32.98 |
| 291 | 11 | OPN4ES | FAM | 31.77 |
| 315 | 11 | OPN4ES | FAM | 31.69 |
| 339 | 12 | Cjun | VIC | 33.20 |
| 363 | 12 | Cjun | VIC | 33.81 |
| 4 | 12 | OPN4ES | FAM | 31.96 |
| 28 | 12 | OPN4ES | FAM | 31.90 |
| 52 | 13 | Cjun | VIC | 32.73 |
| 76 | 13 | Cjun | VIC | 32.87 |
| 100 | 13 | OPN4ES | FAM | 31.16 |
| 124 | 13 | OPN4ES | FAM | 31.52 |
| 148 | 14 | Cjun | VIC | 32.86 |
| 172 | 14 | Cjun | VIC | 32.30 |
| 196 | 14 | OPN4ES | FAM | 30.82 |
| 220 | 14 | OPN4ES | FAM | 30.64 |
| 244 | 15 | Cjun | VIC | 33.15 |
| 268 | 15 | Cjun | VIC | 33.16 |
| 292 | 15 | OPN4ES | FAM | 31.25 |
| 316 | 15 | OPN4ES | FAM | 31.81 |
| 340 | 16 | Cjun | VIC | 32.41 |
| 364 | 16 | Cjun | VIC | 32.51 |
| 5 | 16 | OPN4ES | FAM | 30.70 |
| 29 | 16 | OPN4ES | FAM | 30.89 |
| 53 | 17 | Cjun | VIC | 33.07 |
| 77 | 17 | Cjun | VIC | 33.44 |
| 101 | 17 | OPN4ES | FAM | 31.67 |
| 125 | 17 | OPN4ES | FAM | 31.94 |
| 149 | 18 | Cjun | VIC | 32.63 |
| 173 | 18 | Cjun | VIC | 32.48 |
| 197 | 18 | OPN4ES | FAM | 30.97 |
| 221 | 18 | OPN4ES | FAM | 31.10 |
| 245 | 19 | Cjun | VIC | 34.61 |
| 269 | 19 | Cjun | VIC | 34.18 |
| 293 | 19 | OPN4ES | FAM | 32.90 |
| 317 | 19 | OPN4ES | FAM | 32.93 |
| 341 | 20 | Cjun | VIC | 34.11 |

(continued)

| Well | Sample Name | Designated Genetic Sequence | Reporter | Ct |
|---|---|---|---|---|
| 365 | 20 | Cjun | VIC | 34.53 |
| 6 | 20 | OPN4ES | FAM | 32.25 |
| 30 | 20 | OPN4ES | FAM | 32.64 |
| 54 | 21 | Cjun | VIC | 33.76 |
| 78 | 21 | Cjun | VIC | 33.80 |
| 102 | 21 | OPN4ES | FAM | 31.93 |
| 126 | 21 | OPN4ES | FAM | 32.36 |
| 150 | 22 | Cjun | VIC | 33.59 |
| 174 | 22 | Cjun | VIC | 33.78 |
| 198 | 22 | OPN4ES | FAM | 32.64 |
| 222 | 22 | OPN4ES | FAM | 31.98 |
| 246 | 23 | Cjun | VIC | 34.32 |
| 270 | 23 | Cjun | VIC | 34.24 |
| 294 | 23 | OPN4ES | FAM | 32.87 |
| 318 | 23 | OPN4ES | FAM | 33.08 |
| 342 | 24 | Cjun | VIC | 34.14 |
| 366 | 24 | Cjun | VIC | 34.72 |
| 7 | 24 | OPN4ES | FAM | 33.08 |
| 31 | 24 | OPN4ES | FAM | 33.46 |
| 1 | NTC | Cjun | VIC | 36.07 |
| 25 | NTC | Cjun | VIC | 36.93 |

**Example 5 MHV (RNA Virus) Screening:** Biomatter in the form of fecal samples from mice is submitted via FedEx® (Memphis, TN) overnight delivery. Each sample occupies one well of a **96** source well container **2.** The remote user **1** provides the genetic line identification **84.** The genetic line in this example has been previously associated by the remote user 1 with the designated genetic sequence for MHV (SEQ ID NO. 34). Samples are counted and 250 µl of SV Lysis reagent (Promega Corporation, Madison WI, # Z305X) is added to each sample well of the source well container **2.** The source well container **2** is then vortexed to homogenize the samples. Next, the source well container **2** two is spun in a centrifuge for one minute.

The source well container **2** is then placed back on the deck of the Tecan Genesis Workstation® (Research Triangle Park, NC). Once all of the samples are transferred to the primary master well plate, the well plate is moved to the deck of the Isolation/Purification Station **94.**

One hundred and twelve microliters of lysis reagent (Promega Corporation #Z305X) are added to each sample. Thirty microliters of magnetic particles (Promega Corporation A220X) are added to the wells of a 384 destination well plate (Fisher Scientific #NC9134044). The well plate is moved into a magnetic field and the packing oil supernatant is aspirated off the particle bed. The liquid handler aspirates 100 µl of each sample liquid fecal biomatter sample and dispenses it into the 384 primary master well container, mixing the samples and particles. The particles are allowed to incubate at room temperature for three minutes with a sufficient amount of chaotropic salt to cover the particles. The primary master well container is then moved into the magnetic field of a magnet where the magnetic particles are drawn to the bottom of each well. The supernatant are then aspirated and discarded. The primary master well container is then moved out of the magnetic field. Next, 150 µl of 95% ethanol is added. The primary master well container is moved into the magnetic field and the ethanol supernatant is aspirated off the bead bed. Then, the primary master well container is placed on a 384 tip dryer for one minute. Then the primary master well container is moved back to the deck of the Isolation/Purification Station **94** and 50 µl of DNase solution (Promega Corporation, Yellow Core Buffer #Z317D, $MnCl_2$ # Z318D and DNase # Z358A) is prepared according to Promega Technical Bulletin 328 and added to each sample and incubated at room temperature for 15 minutes. Next, 100 µl of stop buffer (Promega Corporation, DNase Stop #Z312D) is added and incubated for two minutes at room temperature. Two ethanol washes are done as described above. The primary master well container is then placed back on the dryer for two minutes. Finally, 60 µl Ambion's (Houston, TX) nuclease free water (catalog #B9934) is added to each well of the primary master well container. The elution solution was heated to 95°C. The primary master well container is then moved into the magnetic field and 50 µl of DNA was transferred to a 384 well optical storage plate (Fisher Scientific, #08-772136) for optical density analysis.

An $A_{260}$ reading of the storage plate read was performed with a Tecan Genios Spectrometer. This reading showed nucleic acid is present at the desired standard concentration of 0.2 O.D. units, but a range of 0.1 to 0.5 O.D.

units is acceptable.

The plate with the isolated RNA was moved to the deck of a Tecan Freedom Workstation; reverse transcriptase-PCR mixture and Ambion water was placed on the deck as well as a 384 optical well plate (Applied Biosystems (Foster City, CA) catalog #4309849)). The reverse transcriptase-PCR mixture was made with TAQ-Man® EZ RT-PCR Kit (Applied Biosystems, catalog #N808-0236). The Tecan Genesis adds the reagents together in the ABI 7900 384 Well Optical Plate. The plate is then sealed with optical sealing tape (ABI, #4311971). The samples were incubated for two minutes at 50°C, thirty minutes at 60°C and five minutes at 95°C. The plate was then thermocycled for twenty seconds at 94°C and one minute at 62°C, for forty cycles. The results are shown in Table 8.

TABLE 8

| Well | Sample Name | Designated Genetic Sequence | CT | Std. Dev. CT |
|---|---|---|---|---|
| A1 | 1 + Full | MHV | 27.15 | 0.408 |
| A2 | 1 + 3/4 | MHV | 27.64 | 0.474 |
| A3 | 1+ 1/2 | MHV | 28.41 | 0.226 |
| A4 | 1+ 1/4 | MHV | 32.5 | 1.917 |
| A5 | Water Full | MHV | Undetermined | |
| B1 | 1 + Full | MHV | 26.57 | 0.408 |
| B2 | 1 + 3/4 | MHV | 26.97 | 0.474 |
| B3 | 1+ 1/2 | MHV | 28.09 | 0.226 |
| B4 | 1+ 1/4 | MHV | 29.79 | 1.917 |
| B5 | Water Full | MHV | Undetermined | |
| C1 | 2 + Full | MHV | 24.03 | 0.033 |
| C2 | 2+3/4 | MHV | 24.41 | 0.385 |
| C3 | 2 + 1/2 | MHV | 24.86 | 0.252 |
| C4 | 2 + 1/4 | MHV | 26.21 | 0.273 |
| C5 | Water 3/4 | MHV | Undetermined | |
| D1 | 2 + Full | MHV | 23.98 | 0.033 |
| D2 | 2+3/4 | MHV | 23.87 | 0.385 |
| D3 | 2+ 1/2 | MHV | 24.51 | 0.252 |
| D4 | 2+ 1/4 | MHV | 25.83 | 0.273 |

**Example 6 Zygosity Genotyping of Nontransgenic Samples (Targeted Mutation):** Specifically, a remote user **1** can contact the screening laboratory **20** and provide a description of the mutation. This description may include information such as the endogenous gene Bgal (also known as Glb1) was disrupted with the deletion of a particular exon with a Neomycin cassette. The gene name may be used to query databases to yield literature specific for this mutation by the screening laboratory **20.** The Mouse Genome Informatics (MGI) J:38620, PubMed 9063740, or Medline 97217779 databases with their respective journal numbers, yield the following literature reference: Hahn CN; del Pilar Martin M; Schroder M; Vanier MT; Hara Y; Suzuki K; Suzuki K; d'Azzo A, Generalized CNS disease and massive GM1-ganglioside accumulation in mice defective in lysosomal acid beta-galactosidase., Hum Mol Genet 1997 Feb; 6(2):205-11.

This reference discloses that a Neomycin cassette was inserted into exon six of the Bgal at a AatII restriction site. The screening laboratory **20** would then query a database such as Ensembl. The Ensembl gene identification number is ENSMUSG00000042315. The genomic sequence with the exons and restriction sites is identified.

The screening laboratory **20** queries a database such as Ensembl. This query yields sequence data, which is the designated genetic sequence. By knowing the endogenous bases that have been deleted, the screening laboratory **20** can take the designated genetic sequence, or portion thereof, and send it to a vendor indicating where to build the primers and probes as to be informative for screening. Moreover, if there are a large number of bases that have been deleted, the screening laboratory **20** may only send the sequence of bases that will be deleted if the mutation has occurred to the vendor and have them build primers and probe anywhere inside the sequence.

The Neomycin coding sequence, or mutation sequence, does not naturally occur in mice. The same mechanism of identifying the designated genetic sequence using the National Center for Biotechnology Information database and having a vendor build anywhere inside the sequence is used.

A biological sample in the form of a mouse tail biopsy is submitted via FedEx (Memphis, TN) overnight delivery to the screening laboratory **20** from the remote user **1.** Each sample occupies one well of a 96-well source well container. A lysis reagent (made of 2.5 μl of proteinase K (VWR EM-24568-3) and 147.5 μl of Nuclei Lysing Solution (Promega Corporation, Madison, Wisconsin A7943) per sample)) is gently mixed and poured into a 25 ml trough or reservoir and is placed on the deck of a Tecan Genesis Workstation (Research Triangle Park, NC). The liquid handler dispenses 150 μl of the lysis reagent in to each sample well of the source well container **2.** The well plate is then placed in a 55°C oven for three hours. The well plate is then placed back on the deck of the Tecan Genesis Workstation (Research Triangle Park, NC). The liquid handler aspirates 50 μl of each sample and dispenses it into a 384 well primary master well container (Fisher Scientific #NC9134044). Once all of the samples are transferred, the primary master well container is moved to the deck of the Isolation Station Purification Station **94.**

One hundred and twelve microliters of SV Lysis reagent (Promega Corporation, Madison WI, #Z305X) a chaotropic salt are added to each sample. Next, 13 μl of magnetic particles (Promega Corporation, #A220X) are added and the well components are mixed. The well plate is then moved into the magnetic field of a magnet where the magnetic particles are drawn to the bottom of each well. The supernatant is then aspirated and discarded. The well plate is moved out of the magnetic field and 95 μl of SV Lysis reagent is added to each well and mixed. The well plate is then moved into the magnetic field and the supernatant is drawn off and discarded. This washing process is repeated two additional times. Next, the samples are washed four times in 130 μl of 95% ethanol as described above. After the fourth ethanol wash, the microwell container are placed on a 384 tip dryer for 11 minutes. Then the microwell container are moved back to the deck of the Isolation Station Purification Station **94** and 155 μl of Ambion's (Houston, TX) nuclease free water (catalog #B9934) is added to each well at room temperature. The plate is then moved into the magnetic field and 50 μl of DNA elution is transferred to a 384 well optical storage plate (Fisher Scientific, #08-772136) for optical density analysis. An $A_{260}$ reading of the storage plate read is performed with a Tecan Genios Spectrometer (Research Triangle Park, NC). This reading shows nucleic acid is present at the desired concentration of 0.2 O.D. units, but a range of 0.1 to 0.5 OD units is acceptable.

The primary master wellplate with the isolated DNA is moved to the deck of a Tecan Freedom Workstation. The TaqMan Universal Master Mix, real time PCR primer mixture and Ambion water are placed on the deck as well. The final PCR mixture is made of 1x TaqMan Universal Master Mix (catalog #4326708), 1x real time PCR primer set/probe mix for the designated genetic sequence (Applied Biosystems Assays-by-Design (SM) Service 4331348) and 25% isolated DNA. The Tecan Genesis added the reagents together in the ABI 7900 384 Well Optical Plate. The plate is then sealed with optical sealing tape (ABI, #4311971).

The samples are then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Table 9. On average, these results are transmitted to the remote user **1** within twenty-four hours of receiving the biological sample at the screening laboratory **20.**

Table 9

| Sample Name | Bgal WT | RCN | Bgal WT Result | NEO RCN | | NEO Result | Interpretation |
|---|---|---|---|---|---|---|---|
| 147711 | 0.005 | 0.014 | - | 28.465 | 33.608 | + | Sample is Homozygous |
| 147712 | 0.004 | 0.004 | - | 27.832 | 25.023 | + | Sample is Homozygous |
| 147713 | 0.011 | 0.011 | - | 29.842 | 22.576 | + | Sample is Homozygous |
| 147714 | 0.008 | 0.006 | - | 24.467 | 22.744 | + | Sample is Homozygous |
| 147715 | 0.001 | 0.001 | - | 23.767 | 25.853 | + | Sample is Homozygous |
| 147716 | 0.024 | 0.006 | - | 23.403 | 31.924 | + | Sample is Homozygous |
| 147717 | 0.011 | 0.012 | - | 30.323 | 27.709 | + | Sample is Homozygous |
| 147718 | 0.011 | 0.013 | - | 22.351 | 24.558 | + | Sample is Homozygous |
| 147719 | 0.009 | 0.017 | - | 26.118 | 29.585 | + | Sample is Homozygous |
| 147720 | 0.009 | 0.006 | - | 25.341 | 27.121 | + | Sample is Homozygous |
| 147721 | 0.002 | 0.002 | - | 20.551 | 23.563 | + | Sample is Homozygous |
| 147722 | 0.002 | 0.005 | - | 27.756 | 29.563 | + | Sample is Homozygous |
| 147723 | 0.005 | 0.002 | - | 24.062 | 24.874 | + | Sample is Homozygous |
| 147724 | 0.01 | 0.016 | - | 24.854 | 26.924 | + | Sample is Homozygous |
| 147725 | 0.003 | 0.004 | - | 25.518 | 27.715 | + | Sample is Homozygous |
| 147726 | 0.004 | 0.003 | - | 21.355 | 22.03 | + | Sample is Homozygous |
| 147727 | 0.004 | 0.002 | - | 21.928 | 29.168 | + | Sample is Homozygous |
| 147728 | 2.39 | 2.774 | + | 12.544 | 12.556 | + | Sample is Heterozygous |
| 147729 | 2.311 | 2.242 | + | 12.77 | 12.486 | + | Sample is Heterozygous |

(continued)

| Sample Name | Bgal WT | RCN | Bgal WT Result | NEO RCN | | NEO Result | Interpretation |
|---|---|---|---|---|---|---|---|
| 147730 | 2.529 | 2.531 | + | 14.622 | 14.119 | + | Sample is Heterozygous |
| 147731 | 5.064 | 4.727 | + | 0.009 | 0.007 | - | Sample is Wild Type |
| 147732 | 4.934 | 5.245 | + | 0.008 | 0.007 | - | Sample is Wild Type |
| 147733 | 0.015 | 0.009 | - | 32.759 | 31.868 | + | Sample is Homozygous |
| 147734 | 4.72 | 5.425 | + | 0.003 | 0.037 | - | Sample is Wild Type |
| 147735 | 4.604 | 5.268 | + | 0.008 | 0.02 | - | Sample is Wild Type |
| 147736 | 3.338 | 3.141 | + | 18.119 | 17.679 | + | Sample is Heterozygous |
| 147737 | 4.858 | 5.23 | + | 0.01 | 0.022 | - | Sample is Wild Type |
| 147738 | 6.477 | 6.364 | + | 0.013 | 0.026 | - | Sample is Wild Type |
| 147739 | 3.898 | 3.195 | + | 16.335 | 17.008 | + | Sample is Heterozygous |
| 147740 | 5.975 | 7.19 | + | 0.018 | 0.006 | - | Sample is Wild Type |
| 147741 | 0.014 | 0.003 | - | 32.369 | 38.082 | + | Sample is Homozygous |
| 147742 | 7.463 | 7.069 | + | 0.007 | 0.006 | - | Sample is Wild Type |
| 147743 | 6.464 | 6.393 | + | 0.008 | 0.004 | - | Sample is Wild Type |
| 147744 | 6.043 | 5.761 | + | 0.001 | 0.008 | - | Sample is Wild Type |
| 147745 | 4.726 | 6.105 | + | 0.007 | 0.021 | - | Sample is Wild Type |
| 147746 | 5.739 | 5.811 | + | 0.001 | 0.051 | - | Sample is Wild Type |
| 147747 | 6.254 | 6.476 | + | 0.001 | 0.006 | - | Sample is Wild Type |
| 147748 | 3.91 | 4.671 | + | 0.005 | 0 | - | Sample is Wild Type |
| 147749 | 4.805 | 4.112 | + | 0.011 | 0 | - | Sample is Wild Type |
| 147750 | 2.608 | 2.361 | + | 14.852 | 14.503 | + | Sample is Heterozygous |
| 147751 | 2.474 | 2.25 | + | 13.137 | 14.106 | + | Sample is Heterozygous |
| 147752 | 3.951 | 4.665 | + | 0.005 | 0.004 | - | Sample is Wild Type |
| 147753 | 1.649 | 1.997 | + | 9.593 | 12.709 | + | Sample is Heterozygous |
| 147754 | 2.018 | 2.075 | + | 11.349 | 13.382 | + | Sample is Heterozygous |
| 147755 | 4.045 | 4.373 | + | 0.004 | 0.003 | - | Sample is Wild Type |
| 147756 | 4.749 | 5.414 | + | 0.001 | 0 | - | Sample is Wild Type |

**Example 7 Transgenic Zygosity Genotyping:** A plurality of tissue samples of RIP7-rtTA strain of mice are deposited in wells of a microwell container **2** by a remote user **1** and transmitted to the screening laboratory **20.** The screening laboratory **20** has received instruction that transgenic zygosity genotyping of the strain is required. The remote user **1** correlates the source well container **2** well location with the sample identification number on a web page provided by the screening laboratory **20,** e.g. www.transnetyx.com. Additionally, the remote user **1** indicates the transgene sequence information (i.e. designated genetic sequence) or a genetic line identification **4** in the survey of work section. Once the transgene sequence information (SEQ ID NO. 58) is acquired, the primer set/probe combination is created, (SEQ ID NO. 59-61) or may have been created previously for a remote user **1.** The probe/primer set combination can be created for a transgene sequence using software, such as Primer Express ® (Applied Biosystems, Forest City, CA). The tissue samples are screened using the primer set and probe for the designated genetic sequence. The magnitude of the signal for each sample, is captured and reported to the remote user **1**. A remote user **1** interprets higher magnitude signal with a transgene on more chromosomes than the initial transgenic strain. Typically a remote user **1** will keep breeding individuals together with the highest magnitude. This breeding and genotyping continues until the remote user **1** is satisfied that the transgene is present in the 'homozygous' condition.

Now referring to **FIGS. 11-12,** the plurality of samples have been treated as described in Example 1 to obtain screening results which are shown as a graph of signal magnitude for the designated genetic sequence. The remote user **1** is provided with the graphs as shown in **FIGS. 11-12** and asked to select a signal magnitude for the homozygous, heterozygous and wild type strains. In **FIG. 11,** the top 1/3 data points are considered homozygous samples, the middle 1/3 data points are considered heterozygous samples and the bottom 1/3 data points are considered wild type samples. The remote user **1** transmits their signal magnitude designation corresponding to the sample types to the screening laboratory **20.** Then as additional RIP7-rtTA samples are received from the remote user **1** at the screening laboratory **20** in designated microwell containers and at the request of the remote user **1** for transgenic zygosity genotyping then the plurality of samples are screened according to the method described in Example 1. The remote user **1** then receives screening results as an electronic image which shows whether a sample, as designated by its well plate location and sample identification number is homozygotic (+ +); heterozygotic (+ -) or homozygotic (- -).

TABLE 10

| Well plate Location | Strain | Sample ID | rtTA | | AKT | | Tep Op | |
|---|---|---|---|---|---|---|---|---|
| **A1** | RIP7-rtTA | 1 | + | - | | | | |
| **B1** | RIP7-rtTA | 2 | + | - | | | | |
| **C1** | RIP7-rtTA | 3 | + | + | | | | |
| **D1** | RIP7-rtTA | 4 | - | - | | | | |
| **E1** | RIP7-rtTA | 5 | + | - | | | | |
| **F1** | RIP7-rtTA | 6 | - | - | | | | |
| **G1** | RIP7-rtTA | 7 | + | + | | | | |
| **H1** | RIP7-rtTA | 8 | + | + | | | | |
| **A2** | RIP7-rtTA | 9 | + | - | | | | |
| **B2** | RIP7-rtTA | 10 | + | - | | | | |
| **C2** | RIP7-rtTA | 11 | - | - | | | | |
| **D2** | RIP7-rtTA | 12 | + | - | | | | |
| **E2** | RIP7-rtTA | 13 | - | - | | | | |
| **F2** | RIP7-rtTA | 14 | + | - | | | | |
| **G2** | RIP7-rtTA | 15 | - | - | | | | |
| **H2** | RIP7-rtTA | 16 | - | - | | | | |
| **A3** | TetAKT1 | 1 | | | + | - | | |
| **B3** | TetAKT1 | 2 | | | + | - | | |
| **C3** | TetAKT1 | 3 | | | + | - | | |
| **D3** | TetAKT1 | 4 | | | - | - | | |
| **E3** | TetAKT1 | 5 | | | + | + | | |
| **F3** | TetAKT1 | 6 | | | - | - | | |
| **G3** | TetAKT1 | 7 | | | - | - | | |
| **H3** | TetAKT1 | 8 | | | + | + | | |
| **A4** | TetAKT1 | 9 | | | + | - | | |
| **B4** | TetAKT1 | 10 | | | - | - | | |
| **C4** | TetAKT1 | 11 | | | + | - | | |
| **D4** | TetAKT1 | 12 | | | + | + | | |
| **E4** | TetAKT1 | 13 | | | + | + | | |
| **F5** | TetAKT1 | 14 | | | - | - | | |
| **G4** | TetAKT1 | 15 | | | - | - | | |
| **H4** | TetAKT1 | 16 | | | + | - | | |
| **A5** | TetAKT1 | 17 | | | + | - | | |
| **B5** | TetAKT1 | 18 | | | - | - | | |
| **C5** | Tetp27KIP | 1 | | | | | - | - |
| **D5** | Tetp27KIP | 2 | | | | | + | - |
| **E5** | Tetp27KIP | 3 | | | | | + | - |
| **F5** | Tetp27KIP | 4 | | | | | - | - |
| **G5** | Tetp27KIP | 5 | | | | | + | + |
| **H5** | Tetp27KIP | 6 | | | | | + | + |
| **A6** | Tetp27KIP | 7 | | | | | - | - |
| **B6** | Tetp27KIP | 8 | | | | | - | - |

(continued)

| Well plate Location | Strain | Sample ID | rtTA | AKT | Tep Op | |
|---|---|---|---|---|---|---|
| **C6** | Tetp27KIP | 9 | | | + | - |
| **D6** | Tetp27KIP | 10 | | | + | + |
| **E6** | Tetp27KIP | 11 | | | - | - |
| **F6** | Tetp27KIP | 12 | | | + | + |
| **Well plate Location** | **Strain** | **Sample ID** | **rtTA** | **AKT** | **TepOp** | |
| **G6** | Tetp27KIP | 13 | | | + | + |
| **H6** | Tetp27KIP | 14 | | | - | - |

**Example 9 Single Nucleotide Polymorphism Genotyping:** A single nucleotide polymorphism (SNP) is a mutation that affects only one base in the genetic sequence. These mutations occur naturally or can be engineered into a subject. Although, SNPs occur in both humans and mice the tissue source for this experiment was a mouse tail biopsies. Once the bioinformatics and SNP sequence information is acquired, two primers and two probes are created. The forward and reverse primers will hybridize to the genomic sequence flanking each side of the point mutation during the annealing step of the PCR reaction. Moreover, the wild type probe and the mutant probe will compete to hybridize to the DNA. The wild type probe, being perfectly homologous to the wild type genetic condition, will out compete the mutant probe on wild type DNA. Conversely, the mutant probe will out compete the wild type probe on mutated DNA that has the SNP. The two probes multiplexed with two primers discern the correct genotype in this reaction. The first probe determines if the sequence of the mutant is present by the probe being perfectly homologous to the mutant condition. The second probe determines if the endogenous DNA sequence is present. The second probe is perfectly homogolous to the endogenous sequence. The two primers and probes are run on the individual samples at the same time. The probes compete for the DNA and a genotype is discernable. The results are then determined by evaluating both pieces of information to determine mutants from nonmutant individuals. Mutations that differ at two or more bases can also be genotyped using this method.

Specifically, a remote user **1** contacts the screening laboratory **20** and provides a mouse vendor stock number. The screening laboratory **20** can then use this number to query a vendor's database, which yields a description. This particular description states that the mutant *Apc$^{Min}$* allele has a T to A transversion at nucleotide 2549. This point mutation changes codon 850 to a pre-mature stop codon. The Ensembl database is then queried for the transcript sequence which has an Ensembl Transcript Identification number of ENSMUST00000079362. This is the designated genetic sequence. The 850th codon is identified.

GAGCTTCGGGCGAAGGCCCGGGAGCAGCGGACCGAGGCTGGCGCGAT
GCTGTTCCCGGGGAGCGCAGTCGGCTACCGTTGAGGAAGGTGGAGTGAGGAGTGGC

CCTTCCAGCGCCCCCTATGTACGCCTTCCTGCGCTCGGGGCCGGTCGCCGCGTTGCCC
GCCTCCGTACCGCCCGTGACTCTCGGGGCCCGGAGCTCCGGCGGCGGCCGGGGTCGA
GTCCCGGGGGAGGGGAGGCGCCCGGGCGGCGCCCGAGCTTGCGGCCGCGGAGCGAG
CGTCTGGCAGGTCCAAGGGTAGCCAAGGATGGCTGCAGCTTCATATGATCAGTTGTT
AAAGCAAGTTGAGGCACTGAAGATGGAGAACTCAAATCTTCGACAAGAGCTAGAAG
ATAATTCCAATCATCTTACAAAACTGGAAACTGAGGCATCTAATATGAAGGAAGTAC
TTAAGCAGCTACAGGGAAGTATTGAAGATGAGACTATGACTTCTGGACAGATTGACT
TACTAGAGCGTCTTAAAGAATTTAACTTAGATAGTAATTTCCCCGGAGTGAAACTAC
GCTCAAAAATGTCCCTTCGCTCCTACGGAAGTCGGGAAGGATCTGTATCCAGCCGTT
CAGGAGAATGCAGTCCTGTCCCCATGGGGTCATTCCCAAGAAGAACATTTGTAAATG
GAAGCAGAGAGAGTACTGGGTATCTAGAAGAGCTTGAAAAAGAAAGATCATTACTC
CTTGCTGATCTTGACAAAGAAGAGAAGGAAAAGGACTGGTATTATGCTCAACTTCAG
AACCTCACAAAAAGAATAGATAGCCTGCCTTTAACTGAAAATTTTTCCTTACAGACA
GACATGACAAGACGGCAGCTGGAGTATGAAGCAAGGCAGATCAGGGCTGCAATGGA
GGAGCAGCTTGGCACCTGCCAGGACATGGAGAAGCGTGCACAGCGAAGAATAGCCA
GGATCCAGCAAATAGAAAAGGACATACTGCGCGTGCGCCAGCTTTTACAGTCCCAG
GCGGCGGAAGCGGAGAGGTCATCTCAGAGCAGGCATGATGCTGCCTCCCATGAAGC
TGGCCGGCAGCACGAAGGCCACGGAGTGGCAGAAAGCAACACCGCAGCCTCCAGTA
GTGGTCAGAGTCCAGCTACACGTGTGGATCACGAAACAGCCAGTGTTTTGAGTTCTA
GCGGCACGCACTCTGCTCCTCGAAGGTTGACAAGTCATCTGGGGACAAAGGTGGAA
ATGGTGTATTCCTTGTTGTCAATGCTTGGTACTCATGATAAGGACGATATGTCACGA
ACTTTGCTAGCTATGTCCAGCTCCCAAGACAGCTGTATATCCATGCGGCAGTCTGGA
TGTCTTCCTCTCCTCATCCAGCTTTTACATGGCAATGACAAAGACTCTGTATTGTTGG
GAAATTCCCGGGGCAGTAAAGAGGCTCGGGCCAGGGCCAGTGCAGCACTCCACAAC
ATCATTCACTCACAGCCTGATGACAAGAGAGGCAGGCGTGAAATCCGAGTCCTTCAT
CTTTTGGAACAGATACGAGCTTACTGTGAAACCTGTTGGGAGTGGCAGGAAGCCCAC
GAACAAGGCATGGACCAGGACAAAAACCCAATGCCAGCTCCTGTTGAGCATCAGAT
CTGTCCTGCTGTGTGTGTTCTAATGAAGCTTTCATTTGATGAAGAGCATAGGCATGCA
ATGAATGAACTTGGGGGACTGCAGGCCATTGCAGAGTTATTGCAGGTGGACTGTGA
GATGTATGGGCTTACTAATGACCACTACAGTGTTACTTTAAGACGGTATGCTGGAAT
GGCTTTGACAAACTTGACCTTTGGAGATGTTGCCAACAAGGCTACGCTGTGTTCTAT
GAAAGGCTGCATGAGAGCACTTGTGGCCCAGTTAAAATCTGAGAGTGAAGACTTAC
AGCAGGTTATTGCAAGTGTTTTGAGGAATTTGTCTTGGCGAGCAGATGTAAATAGCA
AAAAGACGTTGAGAGAAGTTGGAAGTGTGAAAGCATTGATGGAATGTGCTTTGGAA

GTTAAAAAGGAATCAACCCTCAAAAGCGTTTTGAGTGCCTTATGGAACCTGTCTGCA
CACTGCACTGAGAATAAGGCTGACATCTGTGCTGTGGATGGAGCACTGGCATTTCTG
GTTGGCACCCTCACTTACCGGAGCCAGACAAATACTTTAGCCATTATTGAAAGTGGA
GGTGGGATATTACGGAATGTGTCCAGCTTGATAGCTACAAACGAAGACCACAGGCA
AATCCTAAGAGAGAACAATTGCCTACAAACTTTATTACAGCACTTGAAATCTCACAG
CTTGACAATAGTCAGTAATGCATGTGGAACTTTGTGGAATCTCTCAGCAAGAAATCC
TAAAGACCAGGAAGCCTTGTGGGACATGGGGGCAGTGAGCATGCTCAAGAACCTCA
TTCATTCCAAGCACAAAATGATTGCCATGGGAAGTGCAGCAGCTTTAAGGAATCTCA
TGGCAAACAGACCTGCAAAGTATAAGGATGCCAATATCATGTCTCCCGGCTCAAGTC
TGCCATCCCTTCACGTTAGGAAACAGAAAGCTCTAGAAGCTGAGCTAGATGCTCAGC
ATTTATCAGAAACCTTCGACAACATTGACAACCTAAGTCCCAAGGCCTCTCACCGGA
GTAAGCAGAGACACAAGCAGAATCTTTATGGTGACTATGCTTTTGACGCCAATCGAC
ATGATGATAGTAGGTCAGACAATTTCAATACTGGAAACATGACTGTTCTTTCACCAT
ATTTAAATACTACGGTATTGCCCAGCTCTTCTTCCTCAAGGGGAAGTTTAGACAGTTC
TCGTTCTGAGAAAGACAGAAGTT**T**GGAGAGAGAGCGAGGTATTGGCCTCAGTGCTT
ACCATCCAACAACAGAAAATGCAGGAACCTCATCAAAACGAGGTCTGCAGATCACT
ACCACTGCAGCCCAGATAGCCAAAGTTATGGAAGAAGTATCAGCCATTCATACCTCC
CAGGACGACAGAAGTTCTGCTTCTACCACCGAGTTCCATTGTGTGGCAGACGACAGG
AGTGCGGCACGAAGAAGCTCTGCCTCCCACACACACTCAAACACATACAACTTCACT
AAGTCGGAAAATTCAAATAGGACATGCTCTATGCCTTATGCCAAAGTGGAATATAAA
CGATCTTCAAATGACAGTTTAAATAGTGTCACTAGTAGTGATGGATATGGTAAAAGA
GGCCAAATGAAACCCTCAGTTGAATCCTATTCTGAAGATGATGAAAGTAAATTTTGC
AGTTATGGTCAGTATCCAGCTGACCTAGCCCATAAGATACACAGTGCAAATCATATG
GATGATAATGATGGAGAACTGGATACACCAATAAATTACAGTCTTAAATATTCAGAT
GAGCAGTTGAACTCAGGAAGGCAGAGTCCCTCACAGAATGAAAGGTGGGCAAGACC
AAAGCATGTGATAGAAGATGAAATAAAGCAAAACGAGCAAAGACAAGCAAGAAGC
CAGAACACCAGTTATCCTGTCTATTCTGAGAATACCGATGACAAACACCTCAAATTC
CAACCACATTTTGGACAACAAGAATGTGTTTCCCCATATAGGTCAAGGGGAACCAGT
GGTTCAGAAACAAATCGAATGGGTTCTAGTCATGCAATTAATCAAAATGTAAACCAG
TCTCTGTGTCAGGAAGATGATTATGAAGATGATAAACCTACCAACTACAGTGAACGT
TATTCTGAGGAAGAACAACATGAAGAAGAAGAAGAGAGACCGACAAATTATAGCAT
AAAATATAATGAAGAGAAACATCATGTGGATCAGCCTATTGATTATAGTTTAAAATA
TGCCACTGACATTTCTTCCTCACAAAAACCATCATTTTCATTCTCAAAGAATTCATCA
GCACAAAGCACTAAACCTGAACATCTCTCTCCAAGCAGCGAGAATACAGCTGTACCT

CCATCTAATGCCAAAAGGCAGAATCAGCTGCGTCCAAGTTCAGCACAAAGAAATGG
CCAGACTCAAAAAGGCACTACTTGCAAAGTCCCCTCCATCAACCAAGAAACAATAC
AGACTTACTGCGTAGAAGACACCCCAATATGTTTTCAAGGTGCAGTTCATTATCAT
CACTGTCATCAGCTGACGATGAAATAGGATGTGATCAGACAACACAGGAAGCAGAT
TCTGCTAATACTCTGCAGACAGCAGAAGTAAAAGAGAATGATGTAACTCGGTCAGCT
GAAGATCCTGCAACTGAAGTTCCAGCAGTGTCCCAGAATGCTAGAGCCAAACCCAG
CCGACTCCAGGCTTCTGGCTTATCTTCAGAATCAACCAGGCATAATAAAGCTGTTGA
GTTTTCTTCAGGAGCCAAGTCTCCCTCCAAAAGTGGTGCTCAGACACCCAAAAGTCC
CCCAGAACACTATGTCCAGGAGACTCCGCTCGTATTCAGCAGGTGTACTTCTGTCAG
CTCCCTTGACAGTTTTGAGAGTCGCTCCATTGCCAGCTCTGTTCAGAGTGAGCCATGT
AGTGGAATGGTGAGTGGCATCATAAGCCCCAGTGACCTTCCAGATAGTCCTGGGCAG
ACCATGCCACCAAGCAGAAGCAAAACCCCTCCACCTCCTCCACAGACAGTGCAGGC
CAAGAGAGAGGTGCCAAAAAGTAAAGTCCCTGCTGCTGAGAAGAGAGAGAGTGGG
CCTAAGCAGACTGCTGTAAATGCTGCCGTGCAGAGGGTGCAGGTCCTTCCAGACGTG
GATACTTTGTTACACTTCGCCACAGAAAGTACTCCAGACGGGTTTTCTTGTTCCTCCA
GCCTAAGTGCTCTGAGCCTGGATGAGCCATTTATACAGAAAGATGTAGAATTAAGAA
TCATGCCTCCAGTTCAGGAAAACGACAATGGGAATGAAACTGAATCAGAACAGCCT
GAGGAATCAAATGAAAACCAGGATAAAGAGGTAGAAAAGCCTGACTCTGAAAAAG
ACTTATTAGATGATTCTGATGACGATGATATTGAAATATTAGAAGAATGTATTATTTC
AGCCATGCCAACAAAGTCATCACGCAAAGCCAAAAAACTAGCCCAGACTGCTTCAA
AATTACCTCCACCTGTGGCAAGGAAACCAAGTCAGCTACCTGTGTATAAACTTCTGC
CAGCACAGAATAGGCTGCAGGCACAAAAACATGTTAGCTTTACACCAGGGGATGAT
GTGCCCCGGGTGTACTGTGTAGAAGGGACACCTATAAACTTTTCCACAGCAACGTCT
CTAAGTGATCTGACAATAGAGTCCCCTCCAAATGAATTGGCTACTGGAGATGGGGTC
AGAGCGGGTATACAGTCAGGTGAATTTGAAAAACGAGATACCATTCCTACAGAAGG
CAGAAGTACAGATGATGCTCAGCGAGGAAAAATCTCATCTATAGTTACACCAGACCT
GGATGACAACAAAGCAGAGGAAGGAGATATTCTTGCAGAATGTATCAATTCTGCTA
TGCCCAAAGGAAAAAGCCACAAGCCTTTCCGAGTGAAAAAGATAATGGACCAAGTC
CAACAAGCATCCTCGACTTCATCTGGAGCTAACAAAAATCAAGTAGACACTAAGAA
AAAGAAGCCTACTTCACCAGTAAAGCCCATGCCACAAAATACTGAATATAGAACGC
GTGTGAGAAAGAATACAGACTCAAAAGTTAATGTAAATACTGAAGAAACTTTCTCA
GACAACAAAGACTCAAAGAAACCAAGCTTACAAACCAATGCCAAGGCCTTCAATGA
AAAGCTACCTAACAATGAAGACAGAGTGCGGGGAGCTTCGCCTTGGACTCACCGC
ATCACTACACCCCTATTGAGGGGACGCCGTACTGCTTTTCCCGAAATGACTCCTTGA

GTTCTCTGGATTTTGATGATGACGATGTTGACCTTTCCAGGGAAAAGGCCGAGTTAA
GAAAGGGCAAAGAAAGCAAGGATTCCGAAGCCAAAGTTACCTGCCGCCCAGAACCA
AACTCAAGCCAGCAGGCAGCTAGTAAGTCACAAGCCAGTATAAAACATCCAGCAAA
CAGAGCACAGTCCAAACCAGTGCTGCAGAAACAGCCCACTTTCCCCCAGTCCTCCAA
AGACGGACCAGATAGAGGGGCAGCAACTGACGAAAAACTGCAGAATTTTGCTATTG
AAAATACTCCAGTTTGCTTTTCTCGAAATTCCTCTCTGAGTTCCCTTAGTGACATTGA
CCAGGAAAACAACAATAACAAAGAAAGTGAACCAATCAAAGAAGCTGAACCTGCC
AACTCACAAGGAGAGCCCAGTAAGCCTCAGGCATCCGGGTATGCTCCCAAGTCCTTC
CACGTCGAAGACACCCCTGTCTGTTTCTCAAGAAACAGCTCTCTCAGTTCTCTTAGCA
TTGACTCTGAGGACGACCTGTTACAGGAGTGTATAAGTTCTGCCATGCCAAAAAAGA
AAAGGCCTTCAAGACTCAAGAGTGAGAGCGAAAAGCAGAGCCCTAGAAAAGTGGGT
GGCATATTAGCTGAAGACCTGACGCTTGATTTGAAAGATCTACAGAGGCCAGATTCA
GAACACGCTTTCTCCCCCGACTCAGAAAATTTTGACTGGAAAGCTATTCAGGAAGGC
GCAAACTCCATAGTAAGTAGTTTGCACCAAGCTGCTGCAGCCGCCGCGTGCTTATCT
AGACAAGCGTCATCCGACTCAGATTCCATTCTGTCACTAAAGTCCGGCATTTCTCTG
GGATCGCCTTTTCATCTTACACCTGATCAAGAGGAAAAGCCATTCACAAGCAATAAA
GGCCCAAGAATTCTCAAACCTGGAGAGAAAAGCACATTAGAAGCAAAAAAAATAGA
ATCTGAAAACAAAGGAATCAAAGGCGGAAAAAGGTTTATAAAAGCTTGATTACGG
GAAAGATTCGCTCCAATTCAGAAATTTCCAGCCAAATGAAACAACCCCTCCCGACAA
ACATGCCTTCAATCTCAAGAGGCAGGACGATGATTCACATCCCAGGGCTTCGGAATA
GCTCCTCTAGTACAAGCCCTGTCTCTAAGAAAGGCCCACCCCTCAAGACTCCAGCCT
CTAAAAGCCCCAGTGAAGGGCCGGGAGCTACCACTTCTCCTCGAGGAACTAAGCCA
GCAGGAAAGTCAGAGCTTAGCCCTATCACCAGGCAAACTTCCCAAATCAGTGGGTC
AAATAAGGGGTCTTCTAGATCAGGATCTAGAGACTCCACTCCCTCAAGACCTACACA
GCAACCATTAAGTAGGCCAATGCAGTCTCCAGGGCGAAACTCAATTTCCCCTGGTAG
AAATGGAATAAGCCCTCCTAACAAACTGTCTCAGCTGCCCAGAACATCATCTCCCAG
TACTGCTTCAACTAAGTCCTCCGGTTCTGGGAAAATGTCATATACATCCCCAGGTAG
ACAGCTGAGCCAACAAAATCTTACCAAACAAGCAAGTTTATCCAAGAATGCCAGCA
GTATCCCCAGAAGTGAGTCGGCATCTAAAGGACTGAATCAGATGAGTAACGGCAAT
GGGTCAAATAAAAAGGTAGAACTTTCTAGAATGTCTTCAACTAAATCAAGTGGAAGT
GAATCAGACAGATCAGAAAGGCCTGCATTAGTACGCCAGTCTACTTTCATCAAAGAA
GCCCCAAGCCCAACCCTGAGGAGGAAACTGGAGGAATCTGCCTCATTTGAATCCCTT
TCTCCATCTTCTAGACCAGATTCTCCCACCAGGTCGCAGGCACAGACCCCAGTTTTA
AGCCCTTCCCTTCCTGATATGTCTCTGTCCACACATCCATCTGTTCAGGCAGGTGGGT

GGCGAAAGCTCCCGCCTAATCTCAGCCCCACTATCGAGTATAATGACGGAAGGCCCA
CAAAACGGCATGATATTGCACGCTCCCATTCTGAAAGTCCTTCCAGACTACCAATCA
ACCGGGCGGGAACCTGGAAGCGTGAACACAGCAAACATTCCTCGTCCCTTCCTCGAG
TGAGTACTTGGAGAAGAACTGGAAGCTCATCTTCTATTCTTTCTGCTTCATCAGAGTC
CAGTGAAAAAGCAAAAAGTGAGGATGAAAGGCATGTGAGCTCCATGCCAGCACCCA
GACAGATGAAGGAAAACCAGGTGCCCACCAAAGGAACATGGAGGAAAATCAAGGA
AAGTGACATTTCTCCCACAGGCATGGCTTCTCAGAGCGCTTCCTCAGGTGCTGCCAG
TGGTGCTGAATCCAAGCCTCTGATCTATCAGATGGCACCTCCTGTCTCTAAAACAGA
GGATGTTTGGGTGAGAATTGAGGACTGCCCCATTAACAACCCTAGATCTGGACGGTC
CCCCACAGGCAACACCCCCCCAGTGATTGACAGTGTTTCAGAGAAGGGAAGTTCAA
GCATTAAAGATTCAAAAGACACCCATGGGAAACAGAGTGTGGGCAGTGGCAGTCCT
GTGCAAACCGTGGGTCTGGAAACCCGCCTCAACTCCTTTGTTCAGGTAGAGGCCCCA
GAACAGAAAGGAACTGAGGCAAAACCAGGACAGAGTAACCCAGTCTCTATAGCAGA
GACTGCTGAGACGTGTATAGCAGAGCGTACCCCTTTCAGTTCCAGTAGCTCCAGCAA
GCACAGCTCACCTAGCGGGACTGTTGCTGCCAGAGTGACACCTTTTAATTACAACCC
TAGCCCTAGGAAGAGCAGCGCAGACAGCACTTCAGCCCGGCCGTCTCAGATCCCTAC
GCCAGTGAGCACCAACACGAAGAAGAGAGATTCGAAGACTGACAGCACAGAATCCA
GTGGAGCCCAAAGTCCTAAACGCCATTCCGGGTCTTACCTCGTGACGTCTGTTTAA
(SEQ ID NO. 74)

This large designated genetic sequence can be truncated for easier data handling. The smaller designated genetic sequence is a subset of nucleotides of the larger designated genetic sequence. The smaller designated genetic sequence contains the informative locations and nucleotides for the assay to be designed. The smaller designated genetic sequence is:

TATCATGTCTCCCGGCTCAAGTCTGCCATCCCTTCACGTTAGGAAACAG
AAAGCTCTAGAAGCTGAGCTAGATGCTCAGCATTTATCAGAAACCTTCGACAACATT
GACAACCTAAGTCCCAAGGCCTCTCACCGGAGTAAGCAGAGACACAAGCAGAATCT
TTATGGTGACTATGCTTTTGACGCCAATCGACATGATGATAGTAGGTCAGACAATTT
CAATACTGGAAACATGACTGTTCTTTCACCATATTTAAATACTACGGTATTGCCCAGC
TCTTCTTCCTCAAGGGGAAGTTTAGACAGTTCTCGTTCTGAGAAAGACAGAAGTT**T**G
GAGAGAGAGCGAGGTATTGGCCTCAGTGCTTACCATCCAACAACAGAAAATGCAGG
AACCTCATCAAAACGAGGTCTGCAGATCACTACCACTGCAGCCCAGATAGCCAAAG
TTATGGAAGAAGTATCAGCCATTCATACCTCCCAGGACGACAGAAGTTCTGCTTCTA

CCACCGAGTTCCATTGTGTGGCAGACGACAGGAGTGCGGCACGAAGAAGCTCTGCC
TNNNNNNNNNNNNNNNNNNNNNNNNNCTTCACTAAGTCGGAAAATTCAAATAGGA
CATGCTCTATGCCTTATGCCAAAGTGGAATATAAACGATCTTCAAATGACAGTTTAA
ATA GTGTCACTAGTA (SEQ ID NO. 9)

Upon identification of the designated genetic sequence two other software programs are utilized. The first of these programs is a blast program that identifies homologies between the designated genetic sequence and the endogenous genome of the mouse, as well as other species. The blast software can be found at http://www.ncbi.nlm.nih.gov/BLAST/.

The second of these programs is repeat masking program, such as Repeat Master Web Server found at http://www.repeatmasker.org/cgi-bin/WEBRepeatMasker. This program identifies areas in the designated genetic sequence that are highly repetitive, making them less than ideal locations to build a primer or probe. If such areas are found in the designated genetic sequence they are masked by replacing the normal nucleotide designation A,C,G or T with the letter N or X.

Applied Biosystem's FileBuilder software program is then utilized to generate a SNP assay. The FileBuilder software allows the screening laboratory **20** to identify the location inside the designated genetic sequence that is informative. The transversion, of T to an A in the mutant condition is targeted. In this designated genetic sequence this would correspond to a target location of the 333rd nucleotide. The FileBuilder software file with the 333rd nucleotide designated as the target, is electronically transmitted to Applied Biosystems to generate an Assays-by-Design order. Applied Biosystems will use a software program, such as Primer Express® or Taq Pipe, to identify primer and probe sequences that will detect this genetic condition. The software generates the following primers and probe.

| | |
|---|---|
| ***Forward Primer:*** | GGGAAGTTTAGACAGTTCTCGTTCT (SEQ ID NO. 10) |
| ***Reverse Primer:*** | GTAAGCACTGAGGCCAATACCT (SEQ ID NO. 11) |
| ***Probe 1:*** | CTCTCTCCAAACTTC (SEQ ID NO. 12) |
| ***Probe 2:*** | TCTCTCTCCTAACTTC (SEQ ID NO. 13) |

The primers and probes will hybridized or anneal the following areas in the designated genetic sequence.

TATCATGTCTCCCGGCTCAAGTCTGCCATCCCTTCACGTTAGGAAACAG

AAAGCTCTAGAAGCTGAGCTAGATGCTCAGCATTTATCAGAAACCTTCGACAACATT
GACAACCTAAGTCCCAAGGCCTCTCACCGGAGTAAGCAGAGACACAAGCAGAATCT
TTATGGTGACTATGCTTTTGACGCCAATCGACATGATGATAGTAGGTCAGACAATTT
CAATACTGGAAACATGACTGTTCTTTCACCATATTTAAATACTACGGTATTGCCCAGC
TCTTCTTCCTCAAG**GGGAAGTTTAGACAGTTCTCGTTCT**GAGAAAGACA**GAAGTT**
**TGGAGAGAGA**GCG**AGGTATTGGCCTCAGTGCTTAC**CATCCAACAACAGAAAATG
CAGGAACCTCATCAAAACGAGGTCTGCAGATCACTACCACTGCAGCCCAGATAGCC
AAAGTTATGGAAGAAGTATCAGCCATTCATACCTCCCAGGACGACAGAAGTTCTGCT
TCTACCACCGAGTTCCATTGTGTGGCAGACGACAGGAGTGCGGCACGAAGAAGCTCT
GCCTNNNNNNNNNNNNNNNNNNNNNNNNNCTTCACTAAGTCGGAAAATTCAAATA
GGACATGCTCTATGCCTTATGCCAAAGTGGAATATAAACGATCTTCAAATGACAGTT
TAAATA GTGTCACTAGTA (SEQ ID NO. 9)

The genomic DNA nucleotides from the forward primer to the end of the reverse primer and all the bases in between, whether they hybridized to primer probe are not, are known as the target genetic sequence. For *Apc*$^{Min}$ the target genetic sequence is:

TATCATGTCTCCCGGCTCAAGTCTGCCATCCCTTCACGTTAGGAAACAG
AAAGCTCTAGAAGCTGAGCTAGATGCTCAGCATTTATCAGAAACCTTCGACAACATT
GACAACCTAAGTCCCAAGGCCTCTCACCGGAGTAAGCAGAGACACAAGCAGAATCT
TTATGGTGACTATGCTTTTGACGCCAATCGACATGATGATAGTAGGTCAGACAATTT
CAATACTGGAAACATGACTGTTCTTTCACCATATTTAAATACTACGGTATTGCCCAGC
TCTTCTTCCTCAAG**GGGAAGTTTAGACAGTTCTCGTTCTGAGAAAGACAGAAGTT**
**TGGAGAGAGAGCGAGGTATTGGCCTCAGTGCTTAC**CATCCAACAACAGAAAATG
CAGGAACCTCATCAAAACGAGGTCTGCAGATCACTACCACTGCAGCCCAGATAGCC
AAAGTTATGGAAGAAGTATCAGCCATTCATACCTCCCAGGACGACAGAAGTTCTGCT
TCTACCACCGAGTTCCATTGTGTGGCAGACGACAGGAGTGCGGCACGAAGAAGCTCT
GCCTNNNNNNNNNNNNNNNNNNNNNNNNNNNNCTTCACTAAGTCGGAAAATTCAAATA
GGACATGCTCTATGCCTTATGCCAAAGTGGAATATAAACGATCTTCAAATGACAGTT
TAAATA GTGTCACTAGTA (SEQ ID NO. 9)

A vendor, such as Applied Biosystems, will synthesize these Real-Time primer and probe sequences and send them to the screening laboratory **20.** One fluorescent probe will be perfectly homologous to the endogenous condition, while the other probe labeled with a different fluorescence will be perfectly homologous to the mutant condition.

A biological sample in the form of a mouse tail biopsy is submitted via FedEx (Memphis, TN) overnight delivery to the screening laboratory **20** from the remote user **1.** Each sample occupies one well of a **96** well source well container. A lysis reagent (made of 2.5 µl of proteinase K (VWR EM-24568-3) and 147.5 µl of Nuclei Lysing Solution (Promega Corporation, Madison, Wisconsin A 7943) per sample)) is gently mixed and poured into a 25 ml trough or reservoir and is placed on the deck of a Tecan Genesis Workstation (Research Triangle Park, NC). The liquid handler dispenses 150 µl of the lysis reagent in to each sample well of the source well container **2.** The well plate is then placed in a 55°C oven for three hours. The well plate is then placed back on the deck of the Tecan Genesis Workstation (Research Triangle Park, NC). The liquid handler aspirates 50 µl of each sample and dispenses it in to a 384 well primary master well container (Fisher Scientific #NC9134044). Once all of the samples are transferred, the primary master well container is moved to the deck of the Isolation Purification Station **94.**

One hundred and twelve microliters of SV Lysis reagent (Promega Corporation, Madison WI, #Z305X) a chaotropic salt are added to each sample. Next, 13 µl of magnetic particles (Promega Corporation, #A220X) are added and the well components are mixed. The well plate is then moved into the magnetic field of a magnet where the magnetic particles are drawn to the bottom of each well. The supernatant is then aspirated and discarded. The well plate is moved out of the magnetic field and 95 µl of SV Lysis reagent is added to each well and mixed. The well plate is then moved into the magnetic field and the supernatant is drawn off and discarded. This washing process is repeated two additional times. Next, the samples are washed four times in 130 µl of 95% ethanol as described above. After the fourth ethanol wash, the microwell container are placed on a 384 tip dryer for 11 minutes. Then the microwell container are moved back to the deck of the Isolation Station Purification Station 94 and 155 µl of Ambion's (Houston, TX) nuclease free water (catalog #B9934) is added to each well at room temperature. The plate is then moved into the magnetic field and 50 µl of DNA elution is transferred to a 384 well optical stoage plate (Fisher Scientific, #08-772136) for optical density analysis. An $A_{260}$ reading of the storage plate read is performed with a Tecan Genios Spectrometer (Research Triangle Park, NC). This reading shows nucleic acid is present at the desired concentration of 0.2 O.D. units, but a range of 0.1 to 0.5 units is acceptable.

The primary master wellplate with the isolated DNA is moved to the deck of a Tecan Freedom Workstation. The TaqMan Universal Master Mix, real time PCR primer mixture and Ambion water are placed on the deck as well. The final PCR mixture is made of 1x TaqMan Universal Master Mix (catalog #4326708), 1x real time PCR primer set/probe mix for the designated genetic sequence (Applied Biosystems Assays-by-Design (SM) Service 4331348) and 25% isolated DNA. The Tecan Genesis added the reagents together in the ABI 7900 384 Well Optical Plate. The plate is then sealed with optical sealing tape (ABI, #4311971). The samples are then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Table 11. On average, these results are transmitted to the remote user **1** within twenty-four hours of receiving the biological sample at the screening laboratory **20.**

TABLE 11

| **APC MIN** | | | | | | |
|---|---|---|---|---|---|---|
| | **APC MIN Positives for the mutation** | | | **APC MIN Negatives for the mutation** | | |
| | **MIN** | 1.0 | | **MIN** | 0.0 | |
| | **MAX** | 7.5 | | **MAX** | 0.7 | |
| **T5009** | 5.9 | 6.6 | + | 0.6 | 0.4 | - |
| | 5.7 | 6.0 | + | 0.5 | 0.4 | - |
| | 5.7 | 6.1 | + | 0.4 | 0.4 | - |
| | 6.2 | 5.9 | + | 0.0 | 0.0 | - |
| | 5.8 | 6.1 | + | 0.0 | 0.0 | - |
| | 5.5 | 6.0 | + | 0.5 | 0.4 | - |
| | 7.0 | 6.1 | + | 0.4 | 0.5 | - |
| | 6.1 | 6.3 | + | 0.5 | 0.5 | - |
| | 6.9 | 5.4 | + | 0.4 | 0.4 | - |
| | 7.0 | 6.1 | + | 0.5 | 0.6 | - |
| | | | | 0.5 | 0.6 | - |
| | | | | 0.4 | 0.5 | - |

In this example the relative signal for a positive individual with the mutant probe verses the endogenous probe fall in the range of 1.0 and 7.5.

**Example 10 Human Genotyping of Endogenous Sequences**

The remote user **1** provides the genetic line identification **84.** The genetic line in this example has been previously associated by the remote user **1** with the designated genetic sequence for Human TTTY8 (SEQ ID NO. 26).

A biological sample in the form of human tissue and mouse tissue is submitted via FedEx (Memphis, TN) overnight delivery to the screening laboratory **20** from the remote user **1.** Each sample occupies one well of a 96 well source well container. A lysis reagent (made of 2.5 µl of proteinase K (VWR EM-24568-3) and 147.5 µl of Nuclei Lysing Solution (Promega Corporation, Madison, Wisconsin A7943) per sample)) is gently mixed and poured into a 25 ml trough or reservoir and is placed on the deck of a Tecan Genesis Workstation (Research Triangle Park, NC). The liquid handler dispenses 150 µl of the lysis reagent in to each sample well of the source well container **2.** The well plate is then placed in a 55°C oven for three hours. The well plate is then placed back on the deck of the Tecan Genesis Workstation (Research Triangle Park, NC). The liquid handler aspirated 50 µl of each sample and dispenses it into a 384 well primary master well container (Fisher Scientific #NC9134044). Once all of the samples are transferred, the primary master well container is moved to the deck of the Isolation Station Purification Station **94.**

One hundred and twelve microliters of SV Lysis reagent (Promega Corporation, Madison WI, #Z305X) a chaotropic salt are added to each sample. Next, 13 µl of magnetic particles (Promega Corporation, #A220X) are added and the well components are mixed. The well plate is then moved into the magnetic field of a magnet where the magnetic particles are drawn to the bottom of each well. The supernatant is then aspirated and discarded. The well plate is moved out of the magnetic field and 95 µl of SV Lysis reagent is added to each well and mixed. The well plate is then moved into the magnetic field and the supernatant is drawn off and is discarded. This washing process is repeated two additional times. Next, the samples are washed four times in 130 µl of 95% ethanol as described above. After the fourth ethanol wash, the microwell container is placed on a 384 tip dryer for 11 minutes. Then the microwell container is moved back to the deck of the Isolation Station Purification Station **94** and 155 µl of Ambion's (Houston, TX) nuclease free water (catalog #B9934) is added to each well at room temperature. The plate is then moved into the magnetic field and 50 µl of DNA elution is transferred to a 384 well optical storage plate (Fisher Scientific, #08-772136) for optical density analysis.

An $A_{260}$ reading of the storage plate read is performed with a Tecan Genios Spectrometer (Research Triangle Park, NC). This reading shows nucleic acid is present at the desired concentration of 0.2 O.D. units, but a range of 0.1 to 0.5 O.D. units is acceptable.

The primary master well plate with the isolated DNA is moved to the deck of a Tecan Freedom Workstation. The TaqMan Universal Master Mix, real time PCR primer mixture and Ambion water are placed on the deck as well. The final PCR mixture is made of 1x TaqMan Universal Master Mix (catalog #4326708), 1x real time PCR primer set/probe mix for the designated genetic sequence (Applied Biosystems Assays-by-Design (SM) Service 4331348) and 25% isolated DNA. The Tecan Genesis added the reagents together in the ABI 7900 384 Well Optical Plate. The plate is then sealed with optical sealing tape (ABI, #4311971).

The samples are then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Table 12. On average, these results are transmitted to the remote user 1 within twenty-four hours of receiving the biological sample at the screening laboratory **20.**

TABLE 12

| Sample Name | HumanTTTY8 RCN | | | | HumanTTTY8 Result | Interpretation |
|---|---|---|---|---|---|---|
| Human - Not Sonicated | 1.26 | 1.22 | 1.36 | 1.39 | + | Sample is Positive |
| Human- Sonicated | 1.33 | 1.52 | 1.37 | 1.32 | + | Sample is Positive |
| Mouse DNA | N/A | N/A | N/A | N/A | - | Sample is Negative for both HumanTTTY8 and HS0277190 |

**Example 11 Genotyping of Mouse Bone Marrow:** Specifically, a remote user **1** can contacted the screening laboratory **20** and provide the Jackson Laboratory stock number, PCR genotyping protocol and the Alox-5 mutation description. The description disclosed that a pgk- neomycin cassette was inserted into the mutant sequence. However, this particular mutant model contains more than one pgk-neomycin mutation therefore a specific junction site must be targeted in order to discriminate this neomycin mutation from other neomycin mutations. Unfortunately, none of these pieces of information yielded the specific location (junction site) and nucleotide sequence of the mutation. A third party source was identified that had a working PCR fragment analysis genotyping protocol. The mutant band and the wild type band were cut from the gel. The pieces of gel were sent to a sequencing company to be purified and sequenced. Subsequently, the third party sent the remote user **1** the sequence data, who in turn forwarded it to the screening laboratory **20.** The sequence data that was provided is the designated genetic sequence for the mutant.

TGCCCAGCGGTCCTATCTAGAGGTCATTCTCTCCACAGAGCGAGTCAA

GAACCACTGGCAGGAAGACCTCATGTTTGGCTACCAGTTCCTGAATGGCTGCAACCC
AGTAATTCTACCGGGTAGGGGAGGCGCTTTTCCCAAGGCAGTCTGGAGCATGCGCTT
TAGCAGCCCCGCTGGCACTTGGCGCTACACAAGTGGCCTCTGGCCTCGCACACATTC
CACATCCACCGGTAGCGCCAACCGGCTCCGTTCTTTGGTGGCCCCTTCGCGCCACCTT
CTACTCCTCCCCTAGTCAGGAAGTTCCCCCCCGCCCCGCAGCTCGCGTCGTGCAGGA
CGTGACAAATGGAAGTAGCACGTCTCACTAGTCTCGTGCAGATGGACAGCACCGCTG
AGCAATGGAAGCGGGTAGGCCTTTGGGGCAGCGGCCAATAGCAGCTTTGCTCCTTCG
CTTTCTGGGCTCAGAGGCTGGGAAGGGGTGGGTCCGGGGGCGGGCTCAGGG (SEQ ID NO. 1)

Knowing the gene name, Alox-5, from the mutation description provided from the remote user, the screening laboratory **20** can query the Ensembl database to provide the endogenous DNA sequence. The Ensembl gene identification number is ENSMUSG00000025701. This query yields sequence data, which is the designated genetic sequence for the endogenous condition.

GTTCCAGACAGTCCCACAGGTGCAGATTAGGAGTCGCCCACTCGGGCC
ACTACTTTCTAAGGCTGGTTCCCAGTACCACTAACCATTTCCCCCAAGTTTGCTCCCA
CCCCGCGCCTCCCAGTACCTTGCCCAGAGAGAGAAGGTTTACTCATTTTGTGAAGAA
ACCAACATTCAAGTTTCCTTGGGGTCCACCGTGGAGCTACAGGTACTCTCCTTGTGG
GCTTCAGACCCCCTGCTCTAAGTGTAACTATTGCATACGCTGTGTGCTGCAACTGAAT
GAGGACACAGGTAGTTCTTGAGCTGACAGTGAGGGACACTGAAACAGGCAAGAAGC
CATAAAGATGGAAGAAATAAGGGATTAATTGACTAATGAAAACAAAAATGCATGAG
GGATAAAAGCTAGGTAGAGATGGGGCAGAGGAACAAGGGCTTCAGCCTATCAGAGA
TCACTGTCCCTCCAGTGCCACAGGAGAGAAGGATGCGTTGGAAGGTGGGGTCCTGG
GACTGGCCAGAGACAGGGGCGGAGCCAGCGCCTGAAGGCAGGGCCGGTCGCAGGG
TGGAGCCAGACCCAAGCGCAAGGCTGGCCCGCTGCTGGCCACTGTGGCAGGGAGCT
GCCGCGAGTGACAGGGTCAAGAAGTTGGTGGGCTGCCACGCCGAGCTTCGCGGGCT
CCTGCTCCCACACCAGCAGCACTCACTTGCCCGGAGTCATGCCCTCCTACACGGTCA
CCGTGGCCACCGGCAGCCAGTGGTTCGCGGGCACCGACGACTACATCTACCTCAGCC
TCATTGGCTCTGCGGGCTGTAGCGAGAAGCATCTGCTGGACAAGGCATTCTACAATG
ACTTCGAACGGGGCGCGGTGAGAATGCGCGCTTGGGACCGACGGCTGGCAGCAAAG
AGCGGGAGGGCGGCGGGGCAGGACAGGCAGGCACCTGAGAACTGTCTGTCCCAGCG
CGCTCGTGACCCTATGTGAGCGCATCTGGGGATCTGATGCAGCGCCAGAGTCGGTGC
ATGCCAGGGCAAGCGAGGCACCCTAGACTTCCTGATGACTCGGGTATCTTAAGGGAC

AAATGACTTCCAATGTGGGGGACTGATGTGGCTGGCCTCTTATGTGAGAGATGGCAC
AAACTTTCACCAACAGGACCCAGAATTTGTGAGAGGCCCCTTCACCTCGGGAGTTCT
GAGGTCCTAGTGCCCCAGAGTCCAGTACCACTGCAAAGCATAGAAAGCCCTGTTCGA
CAGCTAGCCATTTTGTGTAGACAGTGTAAGTCCAGGGTAAGTCAGAGATCCAGGATC
GGGAGTCAACTTGGGCATAATCACTCTTCTATCCCTACTGTGGACCTTCGTTTACCAA
ACTAAAAATTGGTTAGGTTTAATCTTACCCATGAGTCATGAGGGAGCCCAGTCCCAT
TTGGGGGCTAGGAATGAGTCCAGGGATTGCCCCCCACACTTAGGTACCCTACATTTC
CTGCACTCAGTCCCAGTGGAGGAATGTAAAGGAAGGAAGGTCTGGCCCGAGCAGCC
TTTGGAAAGACTGTCAGCAGGGTGCGTGCAAGAGGACACTTCCTCCCTGAGTATTAG
CTTCTGAAGGAAAAAAGGAAGAAAGATTTCCTTTCTTCCCTCCTAATACAGACTTTG
GAGTCTGGCAGCCCCACAGGCACTGTGGGAGGCCATTCCGTTTGGATGCTGCTGGTG
TGTAAAGTCTAGCCCCATGACTTTCTCTTAACACAGGCAGGTCATGGTTTTCAGTGAC
CCACTAAGTGCCTAGTACATCAGACTTGCTCAGTAAGTGAGTCTGTAATAGACAGGC
ACGCTGCAGACCCTTGGGGTGGGGGTGGGGGGTTCCTCTTCCTTCTACTACCTCCAG
GTCTAAACTAGGCTTAGGTTTGATTTTACCAGGCTGCCTTCTTATCATTTAGTTTACT
ATTGAGTCGACCCACATGAACTTGCTTAGAATTAACCATTTGTGATAAGCACAATGA
CAATTTCATATGCTTCCACTAGATAGATCTGTTCAGCCTAAACCAAGGAAATGATAG
TTAAGCCCTTTTCTGGGTCCCAAACTCTAAGCACGACGCTTACATTCCTATCCTGGGA
CCTGGTACTTTGCCCTGATTAGCAAGCTTCTAACCAGGCTTGAACAAGCAAGCGTGG
GTGTTGACCTCAGAATGGCATCTTTTGCTCAGTTTCACCAGAGCTGGCCAGAGAATG
GTGCTGCCAAAGACCAAGCATGGCATCCGTTTGGGATGCTGACACCCTGTGCAACCT
CCAAAGCACTTGTGTTTATTTTCAGTAACTGGGGCTTCTCCCAGTATACAGGGGGAG
GAAGAGAGGACAGAATGCTTCCTCTTTATAAATGGACTACAGGGGGCCTTCTCCACA
AATCTAGCTATCAGTGGGTTCAGTCTAGGTGCAGCACAGGACACCTTATGTGTCATT
TCCTCCAGGAGGAGAATGGCAATGTGGCCATCATAAATGACTGGACAGGAAGTAAG
AGGCCTGTCCTGTTCATCATTTGCCTTTCTGTCCTGCCTCCCAACCTGAAAAGTCATT
CAGGTGACATTAATTTAACACCTTAGCAAGAACCCCAGAGGCAAATTTCAGGAGAG
ATTTGCATACATATTTCCATTGTGGGGAGGGAACCACAGCTCAAGTGAACTGCTGTC
TTCTGGCTGGATGCAAAAAGAGCCTTAAAAAAAGAAAAAGAAAACAGCCTTTGAGA
AAGTTCCTGTTGATGCAAAGTTACTCCATACTTTGTCTTGCACAGTCCAGAGCCACTT
CCTCATTCTGTGGCCAGTGTATCTTTAAAGTCCAGATGTCCCTTTTGGGTAAAGGTAG
AAAAGAAACCTTAAGGGAAGTGTTAGCTAAGAAGATAAGGTTATCCCACTGTTCTTA
GAAAAGTGCCACATTGTTTCCATGAATAGCAGCCACCGTGAAGGCCGGCCAGCCACT
TCCAGCACCCCTTCCTAATGTACGACCGAATAATGGTCAGTGCTAACCTGTTTTAATA

CATTCCACTATTGCCCATCCCCCATGATTCCCACATGAGTTCTGTGTAGCGATTCAGG TCAGGAGCCTTTGCAGATGGGATTCTCCTGAGTGTCAGGTGTCAGGCATCAGGGTTT CCCTGTTGAGAACCCAGACGGAACAGAATGGCTTTGTTCCATAAGCTAGTCCTACGT GGCACAGCTCTAACAAACCTTGAAATCTTGAGTGCACATTAGTTGGCTACTTTAACT GCTCAACAGTACTTTGAAAAGCTAAGGATAGGCTGTCCTGCTGAGTATATGGTTCTT GAACATATTCAGTACATGTAAATTCATCTTACAGGTAATACGCTTCTTAAATACATCT AACAAATATTCTTATATTTATAGAGAGTAAATTCTATAGTACCTCTTCATGAGGAAG AGAATACTGATCGGGAACAAACATTTCTTTCTTCATCAGCCTTCATATACTAGTTCTC TCTCACTATCCATCTCCCCTACCTCTGCCCTTCGTCCTCCTCTTCTTCCTTCCTTTATCC CCCAATGGTCAATTTCTCTCCTGCTGCTCACCCCCCAATCTCTCCTTCCTTTTCTACTT TAATGCCCCTTACCTCTCTCCTTCAGCTTCTCCTAACTGCAACCTCACTTTCCCACTTC GGTCCCTACATCCTCTTGACTACTGCTCCCAGTGTTCTCATTCTCTGCAACTTTTGCTC TTCTTATTGCTGCCTTTGGTCTCTCATTCCCTCTGCCCCAGTCTCTCTTTTGCCCTCCA GCCTCCCACCCCCTTCTCTGCTCTACAGCCTCTTCCTCCTCCCACCAGTTTCTCATACC CCGTCCAAGATGGACAGCCAAGTTGGCCATGCGGTCGCTCCAGAAGAGATCCATTTT CTCTTCCACCCATCCACAGAGAGGTGATATAACTGAAGGTGGACAATGCTTGAGGAA TATAGAGTTGTCCTCTGGCAGAGATGGAAGTGTAAATGAATAGTTGAGTGTTTATAA AATGGTGATAGAGAGCTCAGAGGCTGATCAACACCACTTTGCTGAGATATGCATGTA CCACATGATGATGTTTGTGTCACAACAGTCCACGTATATGATGGTGTCCTTGTAAGA TTATGACAGAGCTGAAATTTCATTTTTCCTGGAATATTCAGTATGTTTAAACACATAA ATACTTATTATATGTTACCTAATGTATTCAATACAGCAATAGTCAGTACATATATATT ACTTAGGAGCAATTGGCTATACCATGTAGCCTGGGTGTGCAGTATGATATATATCTA TGTTCATGTAAATATACACTATGACAGAATTGCCTTATACTTCATTTTCTAGAATGTA CCCCTGTCACTGATGTATGATTGTGTTTGGATACTTTAAAACTAAATATACAACAGA GTTGTCCTGACTTGGGCTAATGAACTACATTCTTCTTGTGTCTCTGACCAATGCAGTG GCATCTCTGTCTTGAGAGCTGTGTTCTAGCCATGTCCTCCTCTGTGTGGATTCCCTAA ATTGAGAATATTGTTCTGCCAACTCCCTGTATCTGAAATCTCCTGCCCACTCCTCTCT GCTCTCTGTACCTCTGAGGTGGTCAGTCACTCACTAACACATCGATACCTGTTCTCTG AGTCTCAGATACACCTGTAAAATGGAGTGACTGTCCACATCAACATTTACATGTGCA GCATTCCCTGAGTACCTGGATCAAGCCCCCTTTATAGTACCCACCATAAAGAACTCA GTATGTCAAGGACATAGACAACAAATGAAGTGTCGTCCCAACACACTCAACCATAA ACAAGGACAGAAAAGGTCAGCACATTTGGTGGCTCCACATGCCCTTCTGAGGCCTTC TTTTTGTGAGACACCACCTATCACCCTGTATTTTCCTAGAGGGAAGCCTCTCTCTGCT TTCTGTGATCTTGTTCTAAATACACTCTGGAACTCTAGACCCACAAACAACATACTTT

ATCTCTAAGACTTCCGGGGTTCTAACTGAACCCTTCAACTCTTGTTTATCTCCTGTCA
CATCCAGGCCTGAGGGCAGAGCAGCAAGGTCTCTGGGTTGACAAGAGATAAACCAG
ACTACAGGCCTGACCTCCATCTTCTTTGGAAGATCCTACTGATTAAGTCCCTCTAAGA
TTGCACAATGGTCTATTTCAGTGCAGTTCTGAGGCCCCCAGCCAGGTGGAGTTAAGG
ACCCATTTCCTGTGACTATACTATAGGTGTGCTAAAGGGTCAGCCTCCCCTCCCCCAG
AAAAGCTACTGTTTTTGTATGTCTGTCTTTTGTTTGTTTTGTTTGTTTGCTTGCTTGCTT
GCTTGCTTGCTTGCTTTGTTTGGTTTGGTTTAATTTAGTTTGGTTGGATTTTTTTGAGA
CAGGATTTCTCTGTCTAGCCCTGGCTGTCTTAGAATTACAGCTCTGTAGACCATGCCG
CCCTCACACTCACAGAGATTCATCTGTCTCTGCCTCCCAAGTGCTGGGATTCAAGGC
ATGCACCACCACCACCCAGCTGCTCAGTGTCTTTCTTACAGGTCACCAGTTGTAGCTG
CATAGATGGCCTCAAAGTACATGGCTTTCTCCTCGGATCTTTCTCATTACTTTAGAAT
GGGTGTTTTCTCTTCATTCACTCCTGATGTCATCTTTCCAACCCTCAAGACCGTCCTCT
CTAGTCTCTCAATGCCACAGGAAAACCACTTCCTCTGAAGCAGCTGCTCTGGGGCTG
GGTCCAGGCATTCTTGGTGGCTGCCCTTTGGTCTTGACCACAGGCTGACTTCCCTCTC
CTCTGCATGCACTATAAGTCCCACCCACTGTCCCTTGTCCTTACAAGAGTTCTCCATC
CACCCAGTCCAGGGCTTAAACACATAGAGTGTTTCTCTCACTCCACGGGCACTTGGA
GCTATGAATGAATTGCAGTGCAAAGGGTTGGCTCAGGCTGGTCGTAGCTAGTTCTAA
TATATATATATATACCTAGTGGACTATGCCCTTGGAGAAGGTAGCTACTCAAGTGTC
CCGCCACTCTGATTTCTCAATGATGCTGCAAGCCAGCTGTTATCTCTTCTTAAAAGAT
GAAGAAACACAGCTGGGGTGATACTCAGGCAGTCAAGTGTTCACCATGCAAGCACA
AGGACCCAAGTTTGATCCTTAGACCCCATATTTTTAAAAGCCAGTAATCCTGGGGC
TACATTATAATTCTAGTCCTGAGACAAAGGAGACAGGTGGATCTCTGAGGTTTTCTG
GCTAGCCAGCCTAGCCTAGTTAGTCAGTTCTAGGCCAGTAAGAGACATTGATTTGGT
GTAGGGAGTGATAGTGCCTGAGGAATGACATCCAAGGTTGCCCACTGTCCTACACAC
ACACACACACACACACACACACACACACACACACACACACATCTCCTCATATGTACA
TGTGTGAGACTGAACAATATTCCATTGCATTCATATACACTATATTCTCTGTACTCAT
TCATATGAATACTCATTCATATATCTTTAGCCAAGATTCTTAAGGAATCTCTTCATTA
ATTTAGGTAGACTCACCTCCTGCAAGACCCTGGACCCTAACTTTACACAGATGCGGC
TCCAGTGACACAGGAATGTCTTCAGCTCAAAAACTGATCACATAGAAATAGAGACA
AGAGTCAGTCAGTAGCATCCAGGAAGGAGTAGGGGTGGTGGTGTTGAGAGGAGGCA
CCAAAATAAAGTTAGATAGGAGACATATGCTTTAATTTTTCATGGCACAGTACAGTG
ACAATAACTCATAATGATTTTTGTATATTTCAAAGTCTGAAGAGAAAATTTTTAATGT
TTCTTAGATGAGTATTTGAGGTAACAGAAATGTCAATTACCCTGGTTTGCCAGTGTTT
TTTCTATACTTATATTGAGAGGTCATATTGTACTTCACAAACTTATGATTATCATCAG

CTATAAAAATTAATTATATAGAAAGAAAAACTCCCTTGTCCCATTTTAGATAAGTCA
AGCTTAGGAATTAAATTAGCAGAAGCCAAAAATAATAGGAGAAAAGTGGACAAATT
TGCCTAATGCAGCAATGTGTGTCATATGTTACTTAAGACCAAAAGAAGACTCACAAA
GGCAGTGGGAGTCCACAGCTCACACACTAAATTGTATGTAAAACATGTGATAAAGA
GAGTTGGGCTAGGGAAGTCAGATGGAGTGACTGTGGCTGTTTGAGATGGCCAACCG
GGTCTAACTGTACAGGTTTCTGCTTTCCTTCTAAGGAAGACCTCTTCACACGAAGGC
AGCCATTAGAATGATCTTGAACTGCCATTTTCTATCACTTAACTCAAGTATCTGCTGT
GCTAGAGCAGCACGTTGAGGAAGGAGGGTTGTCCACAGCATCAGTATTCAGGCCCA
GGAGGCAGTGGTGGTTGATTGTCCTGTTTTCCTGCCCAGGTGGACTCCTACGATGTC
ACCGTGGATGAGGAGCTGGGCGAGATCTACCTAGTCAAAATTGAGAAGCGCAAATA
CTGGCTCCATGATGACTGGTACCTGAAGTACATCACACTGAAGACACCCCACGGGGA
CTACATCGAGTTCCCATGTTACCGCTGGATCACAGGCGAGGGCGAGATTGTCCTGAG
GGATGGACGTGGTAAGCTGCTCGGACCCCTGACACTCGTAGGCTTCCTGGAAACTAG
AAAGTCTCCCCTTCTCAGAGAGCTCTATTTCTGGGCGTGAGAATTCCCTCTTGGGTAG
AGCACACCTCTGTATCTTGTTCCCTCATGGGAGATGAGATCTTCCTCTGCTCTAACGA
GCTCTAGAGTGGAGCTAATAGCCTGAGGAGTCATACCAACTATGGCTTCCCTCTGCA
CACTGTCCTGGAAAGGTGTGGAAACAGGCCACTAGGTAGTGAGGGCCTCTTAACAG
GCACCCGTGACAAGAATCCTGCAAATGGGGAGCGCTGCTAGTTAGCACATGACCTCT
TAAGCCAAATTCTCTGGCTGTCCTCTCTAAGCCAGGGGAGTGGGGAGGTGTTCAGGG
CCATAGGACCCCTCCTGAAGGCCTGGACGCTAAAGGTTTGGACTAAGATTGCCAATA
TAATGCCAGCTCTAGGGGCCAGAGGCAAGCTGGAGTTCACTACCCCCTGTTTTACTT
GTTTGCTTTGAAACAAAGCCTCAGTTTCACTACATAGCTCAGGCTGGCCTTGAACTTT
CAGTTCTGCTGATTTAGCCTCCCAAGATCCTAGATATGCAGGGTCCAACTTGGTACTT
CCATAAGAGGCCATTCTTTTTCAGACCCCACTCCCACAGAGGGTCAGAGAAGGAAG
ATGTCAAAGATCAGCTGTTTGTTGTTTGCACTCCCCCCTCACCTCCCTCTGAGTGACA
CCCCTCACCCCCTCTGAGTAACACCATTTGCACACTAGCTGCTTATATGAATGGGTCA
AACTAAGCCTTGGCAAACTTCTATCTTGTAGTCGTAACCCTTGACTTCGCTTTCAGTC
TGCCGACAGCCACTGCAGTGAGGATGACTGCTGATGCTTATGACAGCAATGAGCATC
TCTCAGATAAGGATCTTCTGCCGTTGTTCATCTTCACAGTTGGGAGGGGCACACAAG
ATGAGATTGATAATTAAACCACCACCATTTTGATAGAAAAATAAGACAGAGGGGAA
GAAGAAGGGGAAGAAGAGGAAGAAAGAGGAGGAGAAAGAAGGAGGAAGAAGGAG
GAGGAAGGAGGAGGAAGGAGGAGGAAGGAGGAGGAAGGAGGAGGAAGGAGGAGA
AGGAGGAGGAGGAGGAAGAGGAAGAAGAAGAAGAAGAAGAAGAAGAAGAAGAAG
AAGAAGAAGAAGAAGAAGAAGAAGAAGAAGAAGAAGAAGAAGGAAGAAGAAGAA

GAAGAAGAAGAAGAAGAAGAAGAAGAAGAAGAAGAAGAAGAAGAAGAAGGA
AGAAGAAGAAGAAGGAAGAAGAAGGAAGAAGAAGAAGAAGAAGAAGAAGAA
GAAGAAGAAGAAGAAGAAGAAGAAGAAGAAGAAGAAGAAGAAGAAGAAGGA
AGAGGAAGAGGAAGAGGAAGAGGAAGAGGAGGAAGAAGAAGAAGAAGAAGA
AGAAGAAGAAGAAGAAGAAGAAGAAGAAGAAGAAGAAGAAGAAGAAGAAGA
AGAAGAAGAAGAAGAAGAAGAAGAAGAAGAAGAAGAAGAAGAAGACCCTCT
AGGGTCCTTTGGCTCACTGTCCTTGATGTTTAACTTGACTTACACCAAATTCTGGGAA
ACCTTTTGACTTGGATACTTGGGTTTAGAAAGATTTTTATCACCCCTCCCCCAAGCTC
AGTGGGAACCCCCGCCTCCACTCACCCACTCACCCACAGATCATTTAAGTCATCCTG
TGGCTAGAACAAGGAAGGGTGCTTCTCTTAACCCAGGGTTTAGCAGGCCTTAGAATC
TATTTTCTGTCTGCTCCACCCAAGATCTCAGTGGGGAAATCTTTCCTGCCTACAGAGT
CTGTAGCTTTGCACAGCAGCATGCTTGATAACGCAGGCAGATTGGAATGATTTGAGG
AGGTGTTGAGAGAGGGACATCAAGAAGGTGTGAAAATGTCCAGAGGGGCCACTTCT
GTATGTAGGGTCGGCAGCTTGGCCAATCCTGATGAGCTGGGAGAGAAGAAGAGTTT
GAGAACAATGTAGTAAGTGGCATTTTAAAGATAACCTCTATCTTATGAGTCAATGCA
GAGCTGTGACCTCAGTAGAGAAGTGGACATCCCTGGAGATGAGGAAGAGCAGAATG
GAGAAGACATAACAGCCCCACCCTCACCACACACACCTCCCCTCTGCTCTGTCCCCA
GAGCTAAATCTAATGGGAAGTCCATTGATGATATCTATTCTTATCAGCTTTATTGACA
AGATAAAATGGAAGGGGGGTCTTCAGAGACACAATCTGGCTGACAACTTCCATTAA
CCATGGCTGGTAACCGACCATCATAGCTTTAATCAGTCATTTAATTTGGCTAGTGATC
ATGTAGGTCTAGAACCTTGGAAGGGATCTGCCAAGTACTTGGTCTGTGTCCTGTGTG
CCATTAACTCAATGGACTGCAAGCTTCACCTTGAAGATGGTTCTTCTTCATGGCCTTC
ATCTCTGCATAACACCCCATTTTTAAGGAGTCTCACAACATGAGGGACTCAGGGTAG
CCTCATTTCTTACATAGTAGCTCACTTCTTTTTTCCTTCTTCTCTTTTTATTAGATATTT
TCTTTATTTACATTTCAAATGCTATCCCAAAAGTCCCCTATACTCTCCCCCCCCCCGC
CCTGCTCCCCTACCCACCCACTCCCACTTCTTGGCGCTGGCGTTCCCCTGTACTGAGG
CATATAAAGTTTGCAAGACCAAGGGGCCTCTCCTCCCAATGATGACCAGCTAGGCCA
TCTTCTGCTACATATGCAGCTAGAGACACGAGCTTTGGCGGTATTGGTTAGTTCATAT
TGTTGTTCCTCCTATAGGGTTGCAGACCCCTTGGTAGCTCACTTCTAAGAGCAAATAT
TCCAAGAAACAGAGAATAGATGCTGCCAGGATCCAGGCCTGTGCCTGGAACAAGTA
TAGTGTAACTCCACCACCCAGCCTTGAGGGGAAGAACACCAAAATATCTCAATTTAA
AGAATGCCAGAGGATTTGGGACCATAATTAACCTACATGGCACAACAACCAACGCC
TTGAACAATGTGGCAAGAAGAGGGCCCATTCCACAAGTGACCCCATTTCCCACCACT
GCTGGGGGCCTGCTTAGCCTTAGAGGAGTGAGCTGAGGTGGGACACAGAGTTGGAT

GAAGGCCAAAGACTGATGATCTCTGGCCTTGAAACCCTCTCTCTTGAAACTCAGTAC
AGTTAGTACTATCAATAACAAGTTCAAAGCAGTATTTCATATTCATTATAAGTTCAA
AGCAGCAGTTTTTACATGGCCTGGTCTTATCATAGTGCACACCTGTGGCTTCATCCTT
GGACCTGAATGTATTTCCTTGAACACAAATTTGTCTCAAGCCTTATTTCTCTTTTTAG
TGTAGTTATGAAAGCTCACTGTATGTCTTATTATGCAGCCTTTTCTTGCTATTATGAG
GAGTGGGTACATTCTATTCTTGATTCTAACTTTATTATATCTTTCTGGCAAAACAACT
TTCTTGGAACCTTCTTCCTATAATTATTTAAGTTGAATCATGAGTTCTATATAAAATA
TAAAATCATTAATTCATTTAAACAGCATTAATTCATAAAAGTCCATCTTCATGTTGAT
CTGCAGGAAATTTGCCCAATAGCATGCACTGATGCCCAGAAGTTGTTAGACTGTATA
ATAGTGATATGAGTGACATATGATTAGCAAGAAGCCATTCTGGGATGAGTCTTTTGG
GACTTCACCTCCCAGAGCTCACCCATCGCAAACCATGCAAAAAAACCAGTGAGCCAT
CTCCAGGAAAGTCATCCAATAGCCTTAGCCCCTCCTAGATATCTTCTGACAAGCCAC
ATGATGGCCTACATCTCATATTTTACCCCTTCACCTCAGTAATCATAAAAGTGATAGG
ACTATCAATGTGGTCCCAGAAGCTATAGCAGATGTGTGTACAAATGGTTCATTCCAG
AGCTCATGCGAGCCCCTGGGGATGGGTTCTGCCATACTCAGTTCAGAAGCAGCTGAA
TCGGTCATCCTGCAGCCTGGGAAGCCCCATCCATTGTAAGCCGGAGTCTCATAACTG
GGGAGACTGAGTCAGTCTCTTGAGTCAACTTGTTTTTAGTTCCTTGGAACTCTATTCA
TACCAAACGCAGGAAAAAAAAAAAAAGAATCGTCTTAGAGTCAAGATGTCCAAGGA
ATATTCTGACCATCTAAGGCATTTAGCAAAGAAAATGTCAAATGTTCACAACAGTGT
CTAGCACAGTCAGTGACAACAGGCATTGCCACAGTTTAGACTCACCTCAACCTCGGG
GCTATTGATCCTGCTCTGTTGTCTTGATCCTCCCCTTTGGGATCACTTCTATACTCGAG
GCTGGACATACAGTGACCTAGAGAGCTCTGGGGTTTTCTGAGAACTGAGGGGAATG
GATCTGCTGTAAGGGAAACGAGAACTGAGAGTTGGTGCCTAAGAACCATTCACGGC
GCCTTGAAACAATCATCTTGTGGTAGCTAAGCCTAATGAAATAAAAAAAATGACTTG
TAATCTTTCATGTTTTTTATTTAACTTTTCCATCAATGTTAATATGTTGTTCAAAATAT
AAATGTTTAGTGCAATTAAATATTTAACTCAGGTAGCTAGGGAGTATAGTATACTAC
AGTACACTGCAGTACAGAATAATACAGTATATATTATAATCACCTTTTCAGATTCTTA
CTTGCGACCCTGTTTGCTGAATTTACCTTAGATTACAGTATAGCTGATTACCATTTCA
TCTGTGCTGAATGCTCTGAGAAAGGGCCTTTAGCAACTTGTCAAAGTTCCTGAAATG
GCAGATGGCATGACAGGCCAAGTGACAAAATGCTGTGACTCTTAGGCCTGCAGGCA
AGCAGGAAACCCTTGAGGTTATGGGTTACCCATTATGGGGGTAAGACATAATAACC
ATTATTCTCAAATATTAGACACATAAGAAGACCAATGCTTTGTAGAATGCTTTACAA
TGTTTTTACATTAACATTCTCCTAAGATCCCAAGAAGTAGGCGTTATCATCTATATTT
TATATGAGGCAGTCAAGGTAGCTTGCTCTGGGTCACATGACTTTAAACCACAGATCC

CAAGTCCATGCCTGCTGTATCTCTTTTACATACCCCCACAGCCTAGATGAGCACATTT
GTTTTAGAGTAGAGCCTGTCTTCTGTGTAAATAGCACAAGGGACTGGACAGTGTCTT
GCATATTGGGACTTACAGCATGCATTTCTCCATGAAGCAACCAAATGGACTTAAAAG
CATGAGCCATGAGTCCTGCTGTTTTCTCTGGCGGTATGAAAGTCAGCTGCTCTTTCAC
CTCAATATCTCAAAATAACAGTTATTGGATCACTCTAAAGTGGCTTATTAATATCCAC
CAAAGCCTGTCATGAGGTGTATACTTATAATCCCAGCACTTGGAAGGCAGAAGTAAG
AAAATCGGGAGTTCACAGCCAGCTTTGGGTAAATAGAAAGTTCCAGGTCAGCCGAG
TTATGTGAGATCTTGTCTCAAACAAACAAACAAAAAACAAAAAATCTAAACAAAAC
AAGGGTATCTAATAAACCCCACAAAATACCTCTCAGCTTGTCTTGGCAGACAGGTCT
AGACCAAGAGCCCCAGGATTCCTGCATAAGCAGCATGCTGAAGGCTAATGGAAAAG
GCTGGGAAAACCTGACCACGGGGCATTGTGTGGACATTGTTGCTCTTCCTCAGGGTG
CTCTCATATTTGTCCTTCCTCTGCAGCAAAATTGGCCCGAGATGACCAAATTCACATC
CTCAAGCAGCACAGACGTAAAGAACTGGAGGCACGGCAAAAACAGTATCGGTGAGT
TGTGGCATGGACCAAGTGGCCGTGAGTAGCCCCTTCTGGAGGAGGACCATGGTAGG
AGACAAGCTTGCCAATGTCACATGGACCATATTCTTACCTCCGCCTTCAGGCAGTCA
GAGTGAAGGGGGTCAAAGAAAAGACTCCTTGGGGAGAATGGGTCCAAGAGAGTGA
GTCGCCTTTCCCATTATTGTATGGGCTGCCTCAACTATGTATGTCTTCATGTAATATTT
TAACTATAAAAGTGATACATAATCTACAACAGCATCAAAAAGTCACTAGACGTGATC
AAGAAAAAAGGGTCCAAATTATTCAAATTAAACATAAGGATGTAATCCTAGCACTA
CTAGAAAGATGGTGGTGGTGACAAAGAGCAGCCAGCCTGGAATACAGAGCCCAGCA
GTAGAAACAACCCCAAAAGGTGAAAAGCCATTACCAGCTCCCAAAAGTTGACCTCT
GACCTCCACACACAGTGTTGTGTGTGCATGCCTGAACATAGATGCACACAGACACCC
ACATACATACCACAAACACAATACAATATTTTTAAGAAAATAGAGAATCTGAATAA
GCCAATCCCATGCAATATAATTTAATTAACAATTTTCAGATGTCCTATAAATAAAAG
CTTGGATAGCTTCATGAGTAAATTCTAGCAAACATTAAGGAATTAATATCAATCTTA
TATATATATTCCCACAAATAGGTGAAAAGGCAATATTTTCCAACATATCGTATGGTA
CCAATGTTGTACAAAAATTAGGCAAAGACATCATTAAAAAATAAAGCAATACACAA
ATACCTCACAAAAATACAACTGCAATTATTTTCTACAAAATATTAGCAAGCCAAGTT
CATATGGTATTAACAAATAAGATTTTTCCCTAGGAATACAGTGCTTTTGAACATTCA
AAACTTTGTTAATCTAATATATATGTAAAAAATGAACCAAAGTACTTATACACATAT
ATAAAATATATAATATAGATACACAAAAAGGAATTTGAGAAAACTCAACATTCTTTC
ATAATGAAACATTCAAAATACTGGAATTGAAGAGAATGTCCTCAACACAATACAGA
ACGTCTATGGAAAACTCCCAAGACAAAGTGCTTTTCCCCAAGATCTAGAAATGATAG
GACACCCACTTGTGCTGCTTCTGTTCGATATTTCACTGAAGGGTCTATTTGGTCAGCT

AGGAAAGAAGAAGAAAATGTTTTACACAATCACCTTTCATAGATGACACAGTCTTTT ATATAACTAATAAATTCACCAAAATTTAATTATTTATGAAAAACTAATAAGTTAGCA AGGTTATGAGATAGAAACTAAATCAAAAAATTGATATATTATAACAAAAGAAGAAA AAAATCTACTTACAGTAAGTTCAAAATCAATAGAATATTTATTAGGATGTTTAACAA AAGCATTGTAAGACTAGTGACAAAACCCTAGAACACAGTTGAAAAGAATAAAGAAA ATCTAAGTAAGTCGAAAATATCTCATGTCCCCTTAGCTTTGCTAAAGTGGCAATCCTT CTCCTGGGAATGACGGATGATGGAGATCCAATGCAATCGCTGTTAAAATCTCAGCTA GTGTTTTTTAAAGCAAGATTGATAAGATGGTCCTCAAATCCATATGGAAATGTATGG AAAGGCATACAAAGTTACCAGACCAATCTTGAAAATAGGAAAACTAAGTTAGAGAA GCTATATTTCCCAACATCCAAATTTCTGCATAATCATAGTATTCGGACGAAATGTAG CCGTATCATGAGCAATGACATAGACTTCCAAGTCCAGGAAAAAAAATCCCTTATACT TACAGTGAATTGAATGTGGTGGGATCAGCAAGACAAATCTGTATGTAAAGAATAAC CTTTTAAATAAATGGAGCCAAGGAAACTAGATAGGCAAAGTACAAACAACCATGAC ATTGTGCACACATGTACAAAGTACATGATGTTGGATCCTTACCTAACACCAAATTGA TTACCTAAAAGGTGGAATTAAAACCATATAATCCTGTGAAAATACAAAAGTAAACA TCCATGGCTTTGAGTTGGCAAAGGATTCTGAGACAGAACACCACATCACACATAGCA AGAGAAAAACGGATGACTTGGCATCACCAAAGTTTCAGGCTTTTATGTTTCAAAGAA CCCCATGAAGAAAATGAAAGACTGTTGGGGAAGTGTTTGCCTAATGAGCAGAAAGA GCTGAGTCCAACCCCAGAATCCACATTTAAAAAAAAAAAAAACAAAAAACAGAAAA CTGTGGGAGCTGCAGAGACCACCCAGCAAATAAAATGTCTGCCACACAAGATGGAG GACCTAAGTTTCAATCTTTGGCATCCACTTAAAAGCCCAGAGCTACAGTGCATGTCT ATCACCCCAGCACTGTTGGAGTAGAGACAGGTGGAATATCAGGGCTCACTGGTCAG CCAGTCTAGCTAAATCAGTGAATCCAGGTTCAGTAAAGAGATCCAGTCTGAAGAAAT AAAGTGGAGAGTGACTGAAGACACCCAACATCAGCCTCTGTCCTCTACATTCACACA CACACACACACACACACACACACACACACACATGCACACGCACACACATACACAAT CAGACATAGTAATGTATATGTGTGATCATAGTGCTGAGGATGAAGAGACAGGTAGA TCCCTGAGGCTCTCTAACCAGCCATCCTAGCCTACTTGGTGAGCACCTATCTAGAGA GAAAGCATATTTCAAAAACCAGGTGGATGGCTCCTAAAATATGACAGCTGAGTTGTC CTCTGGCCTCCGCATGAATGGATACACTTGTGCACACAAACATACCTACACACAAAG AGCCCACAGAATGAGAAAGAATATCTGCAGAACAGTTATGTCCAAACTATATAAAG AATTCTTAAAACTCCAGAAAGAAATAATACAATTTTATTTTTTATGAAGTTATTTACT TACTTTACATCCCAATCACATCTTCCCCTCCCTTTTCTCCTCCCAGTTCCTCTCTCTCA TCTTCTCTCCCCACCCCTCTCTCCTACTCCCCTTCTTCTCAGAAAAGGGCAGGCCTCC AATGGAAATTAATCAGCTTTGGCATATCAAGTTGCAATAAGATTAAACCCAACTTCT

CCTATAGAGGCTAGACAAGGCAGCCCAGTTAGGGGAAAGGGATCCAAAGGCAGGCA
GTGTAGTCAGAGACAGCCAATGCTCCCACTTTAGTAGTCTCATATGAAGACCCAGCT
GCGCAACTATTACATGTGTGCAGAGGGCCTACATCTATCCTATGTGTGCTCTCTGGTT
GGCATTTCAGTCTCTATGAGTCCTTATGGGCCCAGGTTAGTTGAGTCTATGGGTTTTG
TTGTAGTGTCCTTAACTCTTTAGGCTCCTACAATCCTTCCTTCCCATCTTCTGCAGGAT
TTCCCAAGCTCCACTTAATGTTTGTCTGTGGGTCTCTGTATCAGTGTCAATCAGTTGC
TGGGTGAACTCCCTCTGATGACAGTTATGTAGGATCCTGTCTAAAAGTATATCAGAA
TATCATGAATAGTGTTGGGGGGGTCCCTCTCATGGCATGGGTCTCAAGTGGGCCAAT
CATTGGTTGGCTCTTCCTTCAAATTCTGCTCAAAAATACAATTTTAAAAGGGGTAAG
AACTCAAAGTCCAAAGAATGGACATTTCTCCAAGAAAAACATATAACTGGGTAAAC
ACATAAAAAGTCAGGTGCTACATTAGTTGTCTGGGAAATGCTAATTCAAGCCCAGTA
CAGCAGCAAGAATCAAAAAGTCATATTATTACAAATTTTGGTGAAATTACAACACTC
ATATACCACTGTTGAGAATGTTAAATGGTGCATCTGCTTTGGAAAATGGCTTAGTCA
TTCCCCAAACTAACAAATAGAGTAATTATGTTGCCCAGCCACCGCACTCTTGATTAT
GTACCTATTAAAAAGGAAGTCATATGTTCTGATGGTTACTGCTCACTGCCATCTAAA
CTGGAGAAGCACATGCAGGTCAGTGTGCCTGCAAGCTCCAGGGAGGATTAAGTGAG
TGAGAAAGACCCAACTTGAATGTGAGTGGCTCCATCCTACAACCCTAGAGTCCTAGA
TCTAACATAGGGAGAAAGGGGGAAAGCAAGGACCTCTGGCACTCCCCTTTCTCCACT
TCGTGGACCACCATGATGTGAATGGCTCTATTACACCTCACCCATCCCACCAGAATG
GACAAGATCTCTCAATCTGAACAAAACATAACTTTCTACCCTGAAGTTGTATTGAAG
TCACAGTGACAAAAAAGACACCTAATACATATGTCTACTCTAAAATTTCTATCTGCA
TGATTGCTGTTCTAATAACCGCACTAACACAAAGATGCGTACAACCTAAATGTCTAT
TAATGGACGAATGGATAAAACGTCATATATCTGTGTAATTTGATGTGGATTCCAGGG
GTCAGACGCCACTCATCAGGCTTGGTGGTGGCACCTTTACCTGATGGCCATCTTGTC
AGTCCTACTATATAGTCTTTAAAAGCTTTTGAAGTTTGTTAGTGGGAGGAACATATA
AAAGTTTGAATCTTTGGGCTAGAGAAACCCAATAATGCTTCAAACAGAGCTTACTGC
ACCATCTTGGTGGGGCTTCAGAAGGCTGAGGTGCCAAGAGAAGCACAGAGAGTGAA
GACCTGCTTCATGAGCTTCAGTGAGGAATCAGGACTCTGCTGGTATTGGGCTGTAGG
CCATCATGTGGTATTCTGGCATGAGATCTGGCTTCACTCTACCCGTGGCCTAAGAACT
CGAATGAGGCTTAATTAGTGAATAATAGATTAATATGTTTGGCAGCAAAAATGTGAA
GACACGATAGCACCTAGCATGTGCTACAGCTACTGTCTGCTGCTCTTACCCAGGTGT
CCAATGAGAGAGATTCCTTGAGTCTTAGAAGAGGCTTTGGAGTTTTGAATGAGCATT
CCAAAGACTCTGGAACTTTTAAAGCTTTGAGGGCTTTTAGAGATGAACAAAACTTAT
TTTCCATTATAAGATTGGCATGAAGCTACAGAGAATAGGATGGGAGTTTATGGCTTA

AATTTGTTTGAATGTCAAACTGACAAGGATGCTGGACTTGTGATGGCTAATCTTGTC
AACTTGACAGGATGTAGAATTATCTAGGAGACAAATCTCTGGGTGTGTCTGTGAGGG
AGATTTCAGATCGGGTGAATTAAGGTGGTAATTCACCCTCTGTGTGTTTCCCCACCA
AGTGAACCACAGTAAACCTTCTACTAGGAAGTTGCTTTTATCAAATGCTGTCACAAT
AAAGATAGAAATAAATAATACCTAAACTAGCATAGCAGCCAAACATAGACTAGTGT
CTTTAAAGATTACCCTTATCTGAACATTCCTCATAAAATGAATGATACAATTAATGA
CATGACATTTATGACTGGCTTGTTTTACTTAACATGATAGTTTCTAGGCTCATGCATG
TTGTAGCAGCTATTAGTATTTCATTCACTATCTGTAGAAAGAAAAGACTCCCAAAAA
TTGTCCTCTGACCTTCACACACATGTCATAACAGGTGCACAGAGAGAGAGAGAGAG
AGAGAGAGAATTCATATAGAGGAAAAGAACCACATGATCAGCATTGCCATGGCGAT
ACCTTACTTCCCAATCTTAGATATTATCTATTGTTCTCATCACTGTGACAAAATGCTT
GAAAACAGCAACTAAAATGAGATTGTTTCACCTTGGACTTTGGTTTGTTTGCTGTTTT
GTATTTTTGTACAGTCTGAGAGAAGACATAGAGGCAAAGTAGGAGGAGCTGATGAC
ATTGAGCCCATAGTCGGGGAACAGAGAGAGATGAAGTCTGGTACTCTACTTGCTTTC
TCCTTTGTATTTAGTCTAGGACCCCATCTCATGGAACGGTACTATTTACATTTGGGGT
GGGTCTTCCCATGTCAGTTAATGGCACTTTAGCAACTACCTCACAAGCATGCTGGAG
GTTTATCACCCAAATGATTCTAGGTCCCATCAAGCTAACTGTCAACATTAGCCACCA
TTGTTAGCTTTTTCTTAGATTGAAAAGTACCACCGGGCCTGGTGGCGCATGCCTTTAA
TCCCAGTACTCAGGAGGCAGAGGCAGGCAGATTTCTGAGTTCGAGGCCAGCCTGGTC
TACAAAGTGAGTTCCAGGACAGCCATGGCTATACAGAGAAACCCTGTCTCGAAAAA
ACCAAACCAAACCAAAACAAACAAAAAACAAAAAAACAAACAAACAAACAAAAAC
CAGAAAAGTATCTGAGTTAAAATAACTTATAGGAAATGTTTATTTTAGATCATTGTTT
TGGAAGATTGGGTTTACAGCCAGGAGGACCTATGGTTTTGCCTTGTGTCAAAGAAGC
ACACCATTTACAGTCATTGTGGTAGAACACAAAGCTACTTAATCTCACAGCAGCTGG
AAGACAGACAAAAAGGCCCATGGTCCAACATTCCTTCAATGTGTGTCAAGTGACTGA
ACTTCTGGCTACCATTTCTCAGGAAGACCATTAGCAGGAGTCAAGCTTTGCCTTTATT
ATCCAAAGTATAGATGTTGCCTAGGGGCAGAGGGGCCAGTAATAGCAATGATGACT
AAAGATATGTGTTCCTTTGGGGAAATCATAAAAATGTTCAAATATTGACTATGGAGA
TGGTTACCCCTATGTGATAAGGGTAAAAAAGACTTTAATTATTGACTATCATTGGGT
AAATTTTATGGTATAAAAATTATATGCCTATAAATCTGTAAAAATGTTTAATTAATTC
AAATTGTAATATAGACTTCAACATAGCAACAAAATCTATACAACATATAGAGAACA
GAAGAGCAAATCATGATAACCAGGCTCAGCAAAACCTCTTAGGTATGACACCAAGC
ACACACAGCACAAGGACAATCGCCACACTGGACTTTGTCAAAACATAAATCTTCTAT
ACTTCACTGAACTCCACCAAAACAGTAAAATGATAAAATGGGCCAACGTATGCAGA

CTGCATCCAGCAAGAGGCTTAAATTCAAAATATATAGAGAACACTAACAAAAGGTA
AAAAGACAGGTTATAACAAATGCTGCCAATGAGGGATGGAGGTCAAACCCCAGCAC
TCTGGTAAGGTCAAAAAGCCACAGCCATTCCGGGAGACATATCAGTTCTTCCTCAGA
ATTATAGATAGTGTCACCTCGTGACCCAGCAGCACTTCTTATCACAAGTGTTTCCAA
AAATAATACAACCAGAATTAGGGGTGTAGCTCATGGCAGAGCACTTACCTAGTATAT
AGAAGAGCCAGGGACTCAGTGCACTCAGTGCACACCCCTGTACTGCAATATTAACAT
TAATAACTAAGCCATGTGTCTACACCAAATACTTGTATGCAAATATTCGTGGCAACC
GTCTTCCTTGGGGTTTCTATTGGTGTAATAAGACACCATGACCAAACGAAACTTGGG
AAGGGAAGGGTTTATTTCACTTCATACTGCCAGGTAATATCTACCAGTGAGCCACTG
AGACCAATATAGTTGATCATACAATGAAGGCAAGATGCCAGAATCGCATGCAGCTG
TCCCATAACTCTGGATTCTGTTGCTAAGGGAAACATGTGGCAGTGGAGCTATGACCA
ATAACAAAGAAGTGACATCACATCCCAGGGGTGGCTGGGCTTGGTGCCGTGCCTATT
GTCCTAGATGATTTGGGAGGCTAAACAGAAGAATCATTTAGTCCCCGATGTTCAAGA
CCAACCTATTTCAAAAAGAAAATTCAGTATGTCAATGCTAAGAGTAAGGGTTGAGG
GTACTCTCACTGATGTGATAGATAGCTAGAGAGATGTTTAGGGTCTGAAAGGGAGCA
GGGAAGAAAGAGAGAGAGGGAGAAAGAGAAAAACAGGCAGAAAGACAGACAGGT
TGAGAGAGATAGAGACAAATGCATATGTCTACATACCCTAGTTCACAGTTTAAAAGT
TAAAAACTCTTGCGCCATCCCTGCATGGTGTAAGAGCATAACCTGATTATACTGTAG
TCAGGCCAAAAATTCAAAAATTCAATGTGTATGCAAGCCTTTCCGCTCAGCACCCTA
TGATGTAGTTTGGAACTTCTGTACTTTTACAGGGCATAGATCATTGTCCCAGAGTCTG
CCTTCCCTTCCATCCGGCTGAGGAAATCTTCAGCTCTCTTCTCCATTTCAACTCCCTGT
GACAAAGCCCTTTATAGAGTTCTACAGGACTTCTAGTCTGTGGAGTGGGTTAGGACA
AAACTCCAGAGTGAGTCAATTTGTTGTCAGCCACTATTATCTTAGTGTGGTTTGGTGC
ACACCTAATTAGACTTCTTACCCATTACACCTAAGAGATCACAATCTAGCCTGATCT
GGAAGCCGTTTACTTCATCACAACCTGGGTCTTGTGAAGAGGATGAGAAGGGCACTG
TGAAATATCTTGTCCCTTTCTTAAGCCCTATTTTACTCCCTCCTTCCAAGCCGCTGCTC
TCTTCAGCAGCCCGCCTCCCACCCATGCCCCATTTCCGGTCAGAAAACACTGAACTTT
GCACTGTCTGCACAATTGTGAAGTTCCCCTTTCACATACACTTTCAGGCTTCCAGATT
ATGGAAGCTGAGCAAGAGAATACTTCCAGGAATGCTGCTGGCTTGACTCAGTAGCCC
ATCTGGGAAAGGGCACAGAAAGGGCCCAGGTCAGAGCTCCGTGGCCCCTGGCCCTC
ACTCGCTGCACTGTCAGTGAGTATGACACAGTATACTGTGGTCTGAGGGCTCTCACA
TCCCTGACCTAATACGTCAGTATCTTCAGTTTTCAATCATCATTATGCTCTGAAGACC
AGCCATTGACACAACTCGACAGCCTTTCATTTTAATTCCTGCATAGTTAAATGCATCA
CACATTTTTGGTTTTGTGTTCTGAAAACTGAGCGCAGGACCTATTAAATGGTAAGCA

AGCAAACACTCTATCCCCAGCTCCGGAGGCATCATTCTTGGGTACCACTTTGCTATAT TCATTTGGCTACCTGCTGATGAACACATGGGTACCTCCTAGTGCTTTCAGACTGAGCC ACAGTGAGCTTTCTGCTAACGTGAGAGAATTACTCTGGGGAACATGTCTAGATGAAG ATCTGTGAGATGACCAAGTGTGTGCGTCATCTGACTTCTCTAGATCTTGGTAATTGTT TGCCCAGCTGTCCAGACACTTTCCACCCTTAGCTGGTGTTGCTGTAAGATGCCATTCC CCTGCATCACCAACCCATTCGACATGAGACATTTTTGTTCTCGACTGTCTAATGGGTA GGATTGTGTCTCATTTCTTCGTCATAAGATGCTACATATTCATGGATTACACATAAGC ACTACAGGCTGAGCCAATGACTCCCCTACTGGTGAACATTCAGCTCGATTCCCTACT TCTGTTTGTTTTGAATATTACAGAGAAGCAACTTGTATGCATGATATATAAGTCATTT AATCATTGGCAGATCTGTCTAGAGAACATTAATCACTTGTCTCAAAGCAGCTCTCAT CTCACATAAAATGAGATAATGTATTGTGACACAAATATGAATGTCCAGGCCTGGCTG GCTTGGGTGTTCTGCATGCTGAATTTCCACACAGAAGCAATTGCATAAACATCCATA CCCACAGGATAAGAGAGAGCATTAAACCAAAGCATTTTTTCCAGCACATCAGTGGG AACAAGAGTTGCATGAGCTACATCCAATTGGGGAAAATCTGCTGCAGGTGTGAGAG TTACCTACCTGGTGGTAGTCTTAGCTTTGGGGTGAGAGTTACCTGGTGGTAGTCTCAG CTTTGGGGTTAGTAAGGAAGCTGCTGGTGTGTTTATACGGTCCAAAGATTTCACCTG GTATTTGGAAGGATAAAAAGTCAGACATAGAGGCAGACAATGATTGGCTATTAAAG AGACAAAAGGTGACCCAAGATAACTTTTCTCTAGATCTGCACACCAACTCTTTATAA TAACAAAGTTCCCATAAGCTGATCAACCTTGTAAAACCTTTAGAGCAAGTGCAGCTT TCTTTTCTAAGAACATTATTAGTTCACAGTTGTGTCGGGCAAGTGTCACATTTTGCGC GTGCAGAAATGTTGTTTTATGTCCTCTGATGCTAAGAAGGCTCATTCTGAGGAACTG ATAAACAGGAGGCGGAGCAGCCAGGAGAGCAGTCTCCAGAAGAGAGAAGAGGGTA AAGAAGAGGATGTCTTTCAGAAGACATCCTTTATTTATAGCAGTTTTACTGGCTATC ATGATCTCAATATGACCTTTCTTTCTCCCTCTGCATCTGACAAAACATATACATATAA CATAAAATGGTAATATTATCCCAAAAGTTAAAACAATTAAACACAGAGCCTAAAGG AGAAAAAGGTAATGGGTGAAGGAAGCAGGCAGCTAGCCAGCCAAAAAGTCTTTCTA AAAAAAAAAAATATGTATAATTCTATTTGTCAATTAAATGAATGAAAGCATTAAAAT AATTAAATTAAAATAACTATTTTAATCTTTTCATAATTTAATTAAAATATGATTATAT CATTTCTCTCCTCTCCTAACTCCTCCCCTTCCAATGTCTACACATCCCCCAATTCACCG CCTCCTCTTTTATCTAACCCTTGTTGGCAAATATACATAAAAACCAGTTAACATACAA ATACAACCTGCTGAGCCCCTGTCCCCTAAGTGTTGCCTGCATGTGTATGTTTCTAGGG CTTGGGATTAGATGACAAACCAAGCCAGGCATCTCTGGGTGAGACTGACTCTCCTCT CCACAGCTGTTAACTGCTTGTAGCTCTTCAACTAGGAGTGGGACCCCTGAGATTTTTC CCCAGTCTGTGTTGGAATGACAATTATTTTGGAATTATTTGGGTTCTTTTTTGGCAGC

CATGTTGTGGGCGTAGCTTTTCTGTCATAGCTAGAAGAGACTATCACACAGCAGACC
TTCTAATTTTCTAGTTCTTATGATCTTTCTGACTCCTCTTCTTCGATGCTTTCTGAGAC
TCAGGTGCTGGAGTTGTGTGGTAGATACATCCCCTGGGTACCACACAACCATTTCTT
CTCTTCATTTTGACTAGATGTGGCTTTCTGTAACGGTCTCCTTCTGCTGCAAAAAGAA
GTTCCTTTGATGAGGGTTGAAAGTTACACTTGCTTGTGGGTGTCTGGATAAGTATTCA
GAATTCCATTAAGAATTATTCTGGTTAGAGAAATTGGCAGTAGTACAATCTATGACC
TCCCTAGCCAAAAGCAGTTGGCTAAGTTTCCAGGGCCAAGAAGGATGTCTTTCCTGT
TAAGCAGGCTTTGAGTCCAAGTAGACAGTTGTTGGTTACTGCCAAGATATAGGCACC
ACTACTGCCCTGTAGGGATATCTTGCCATGATGGTCATTGTGGTTTATATTCAATGAC
AGCTGGCTAAGACTATTGACTCTTTCCTTCCCTGCATATGAAACACAGCTTATCACTT
TCTGGTCCTATGAAAGCTGGTCCTCGAGGGACTCTTTTGGTTCCTTTCCAGCTCAAAT
CCTCCACATTTTATGTGTGGAGTGCATGGTGTCCTCAGCAATAAGGACTTGCTTTTAA
CTTCTGGGAGGCAATCAAGGACAAGCAATAGCCTATATTGTTTCAAGAGCTCTCTTG
GATTTCCCTGATCAAAAGCTCAAAGGGGGCTTGTCATGCATGGTACCGAACTTTTTG
TTAGCTACGCTTGGCTCTTGTTGGAGCGAATCATCCCTCCATATGTTGTAATTTTTTA
TATTGCATGTTTTCTCTTGTTTTTGAGAAATTGAGAAATTTCCCACAGGGTATTAATA
TATTATTATATTGGCATTACATATAGTGATTCACTGTGAGGCTTCCATGGTCATAAAG
ACTGCACACTATTCAACATTACGTATATGATGTTGGCTGAAGAGAGAAGGTACTATT
CTGCATTTGTAATTTAGAAGGTTGGATTCTCTGGATGATTTTTTTCTTTTTTTTTAAGC
CTAAGTATACTTTAATTTGGTACTGATATAGTAGTTCATAAAGATGATACCACTATG
GTGTAGAACTCAAAGTTATGATATTAAGTTCAGGAAGCGAATGTATGTAATTCATTG
CAAATTTGCAATGCTTGATTACACATGTTCAAGATTCAACACGGAGAGGCTTCTGAT
ACTACGGTGGCACCAGCAGGGAAGTCACAAGTTCAGACTGTCTGAGACATGCAATA
AGGTTCTTTCTTAAAGAAAACAAAATGTGGAAAAACAGTCTTGAAATTTTTCACAGC
TCTTTCTTCCTTTCAATTTTCTTGTGCAGAAATTTGTGTGATGATAAGAATCAGAATG
TTGCTCTGCATTATCTAGTAATACTAACTATCTTGTGGAGTGATCCTGTGCATTCACC
AGAATGCAAATACTGCTTCCTTACTAAATCACTCGGAGCCACTGATTCCCATCAGCT
AGAAACAAACCTGAGCAACAGAGTCAGTGGGCAGAACACCATAATCCTCCTAAGAG
CATGGAGTTAGTACTCTTCGCATACATCCCTTTATTTATTATTTATGTATTTATTTTCG
GGGAAAGGGATAGCATTTTATTTTTTAATCTTCTTATATCTGTTGAGGCCCGTTTTGT
GACCAATTATATGGTCAATTTTGGAGAAGGTACCATGAGGTGCTGAAAAGAAGGTA
TATTCTTTTGTTTTAGGATGAAGTAGTCTATAAATATCTGTTAAGTTCATTTGGTTCAT
AACTTCTGTTAGTTTCACTGTGTCTCTGTTTGGTTTCTGTTTCTATGATCTGTCCATTG
ATGAGAGTGGGGTGGCGAAGTCTCCCACTACTATTGTGTGCAGTACAATGTGTACTT

TGAGTTTTACTAACGTTTCTTTTATGAATGTGGATGCCCTTGCATTTCGAGAATAGAT
GTTCAGAATTGAGAGTTTATCTTGGTAGATTTTTCCTTTGATGTGTATGAAGTGTCCT
TCCTTATCTTTTTAAATAACTTTTGGTTGAAAGTGAATTTTATTCAATATTAGAATGG
CTACTCCAGCTTGTTTCTTGGGACCATTTGCTTGGAAAATTGTTTTCCAGCCCTTTACT
CTGAGGTAATGTTTTAGCCATTGAAGTGCATTTCCTGTAGGCAGCAAAATTCTGGGT
TCTGTTTATGTATCCAGTCTGTTATTCTATGTCTTTTTATTTGGGAATCTGGTCCATCA
ATGTTAAGATATTGAGGAATAATGATTGTTACTTCCTGTTATTTTTGTTGTTAGAGGT
AGAATTATGATTGTGTGACTATCTTCTATTGGGTTTGTTGAAAGAAGATTACTTTCTT
GCTTTTTCTATGGTATAGTTTTCCTCCTTGTTTTGGTGTTTTCCATCTATTATCCTTTGT
AGGGCTGGATTTGCGAAAAGATATTGTGTAAATTTGGTTTTGTCATGGAATATCTCG
TTTTCTCCATCTATGATAATTGAGAGTTTTGCTGGGTATAGTAGCCTGGGCTGGCATT
TGTGTTCTCTTAGGGTCTGTATGACATGTGCCCAGGATCTTCTAGCTTTCATAGTCTC
TGGTGAGAAGTCTGGTATAATTTTGATAAGTCTGCCTTTATATGTTACTTGACCTTTT
TCCATTACTGCTTTTAATATTCTTTCTTTGTTTAGTGCATTTGGTGTTTTGATTATTAT
GTGATGGGAGGAATTTCTTTTCTGGTCCCCTCTATTTGGAGCTCTGTAGGCTTCTTGT
ATGTTCATGGGGATCTCTCTCTTTAGGTTAGGGAAGTTTTCTTCTATAATTTTGTTGA
AGATATTTACTGGCCCTTTAAGTTGAAAATCTTCACTCTCTTCTATACCTGTCATCCTT
TGGTTTGGTCTTCTCATTGTGTCCTGGATTTCCTGGATGTTTTTGGTTAGGAGCTTTTT
GCTTTTTGCATTTTCTTTGCGTGTTGTGTCAATGTTTTCTATGGTATCTTCTGCACCTG
AGATTCTCTCTTCTATCTCTTGTATTCTGTTGGTGATGCTTGCATCTATGACTCCTGAT
ATCTTTCCAATGTTTTCTAACTTCAGGGTTGTCTCCCTTTGTGATTTCTTTATTGTTTCT
AGTTCCATTTTTAGATCCTGGGTGGTTTTGTTCATTCCCTTCACCTGTTTGATTGTGTT
TTCCCATAATTCTTTAAGGGATTTTTGTGTTTCTTCTTTAAGGGCTTCTACTTGTTTAC
CCATGTTCTCCTGTATTTTTTAAGGGAGTTATTTACGTCCTTCTTAAAGTCCTCTATCA
TCATCATGAGAAGTGATTTAAGGTCCAAATCTTTCTTTTCCAGTGTGATGGTGTATCC
AGGACTTGCTATGTTGGAGCAGTGGGTTCTGATTATGCTAAATAACCTTGGTTTCTGT
TGCTTACGTTCTTATGCTTGCCTCTTGCTATCTGATTATCTCTAGAGCTACATGCCCTC
ACTGTATCTGACTGGAGCCTGTCTTTCCTATGATCCTGGTTGTGTCAAAACTCCTTAG
AGTTTGGCTGTCTCTGTGGTCCTGTGATTCTGGGATCTTGTGATCCTGAGATCCTGGA
TGTGTCAGAGCTCCTGGGAGTCAAGCTGCCTCTGGGACCCTGAAGATCCTGGTGTGA
CCAAGCTCCTGAGATTCTGTGATCCTGTGATCCTAGGCATGTTAGAGAGCCTGGGAG
TTGAGCTTTCTCTGGGTGTTGTGGGGCTGGCTCTCATGTTTTCTCTTATATGTAGATAT
TAGATTTTAAGCTTTCCATATGTGTGTTTCATTTAAAATAGCCACAGAGGTTGGGTAG
CTTTTATGAGACCAGAGAGAGGAAGGAAACTCCCCCCAAAAGAGACATAGAATATA

GTGTTATGGGTAATCGTAAGGGAAACTAAAATGGGAGGTTTAAATGGGGAGAGGAT
GGGAATGTGTTACAAAGGAGAAATTATGGGAGGGACAACTAATACTAAAAGCCTTT
TGACTAGCCATATGGAAACTCACAACTCTCAAAGCTTCCTAAAATGTATAAAATGGA
ATTACCGTATAATGGAAGAGACAATGCCCCAACTAGACATCATATGCTAGCAAGTA
AAACTGCCAGTATCAGGAATGGGTTACATCTTGTTGTGTCCCTGATCAAAGGTGACC
CACAGACAGTCCCCCATAAACAATATAGGCTATTGTCTATTATTAGTTATCCTTCAGA
ACCTGACAGAAAATGAGTGTTGTGTCCGGCCAGCAGACCACAACCTGGGTTCTAGCC
TGGAAAGGCATTTTGGAAACCTGGAAGAGAAGAGGGGCTAGGTGGCGAGAGAAAA
GAATGTAGCCAAGACAGTTACTCTGATCAAGGCTCAAATTTTATTGTTGCGACACTA
GTTATGAAGGAAGGGGGAGGGGACCCGATTCCCGCCGAATAATCTCTGGTCCAGTA
GAAAGGTGCACGTGTGTGGCTCCGCAGGTTCCAGCAGTGGGCGTGGCAGAACGAAT
GAGCAGGAAGCTCCACCCCTGAGCAAGCAGGTTTCAGGCTAGGGGAGGGGAGACTA
CAAATGAGGACATCACTTACTTATGTCATCAAACATGAGAAAATTAACCTGGTGCCC
AACCCGAAGCTTTAGCCATACTGACAAGCACTCACAATGATGAAAGGTGCTATATAT
ACATGTGACCAGATGAGCAACAGCATCATCATTCTTTCCCAGCTACAAACCCTGAGA
CCTATAACTGTGTCCTGCCTTGCAAGATATACTGGTACAATAATGGCACAAACGTTA
TGGAAAAACCAACCACTTCCTAAAAATCACATTTAAAGCTTATTCCATGAGATGAAA
CCCATATCCAGAAACTATTAATGAGGCCACAAACTTGAAACAAGATGAGGCATGGA
CCCTAGGGGAACAACTACTACTGCTATCCTGCTAACGGAGCATGCCACGCCTAAAGA
CATACTGCCATACCCATAAGCTAATATGTATGACAAGCCCCATCAGAGAAGTTTCTT
CTTGGAGTAGATGAGAATTAGCACAGAAACACACACACTCCCAAATGGAGGATGGG
CAGACAATGTCAGTTCTTGAAATGCTCAATCCTAAATGGAGTGTCTTTGTCAAACCC
CTTTCCTCCAGCCTTGGGGATCTAGGCAGACGTGGCAGAAAGAGCTGAAGGTGGTG
GATGACTCCGAAAATACAGTGTCTTTCAGACACAAAGGGGTTGATGGACATTGAAA
CTCACAGAGACATTAACACTATGTACAAGACTTTGCAAATTCAAGCTAGATAAATCT
CAGTACCGAGAGAAGTGGGCACAAGTCCCACCCTCAACCAGGATGCTATTTAAAATT
GATACCTGCTCAGAGAGGGAAAATCCATTTTCTCCAATGGAGTGACACTGGGTATAT
ATCAACTGTACTGCAGGGCAGGGCTCATGCTCAAGGGTAGTTGGTCAACACAACATG
GGCTCCGTAGTTCTCTTTGGGGGAGGGTGTGTCTCTTTTTGTATTGTTTATATTTTTCT
GAGAGAGAGAGAGTAAAAGTGTGGATTTTGGGTGGGTAGGAAGGTATAGAAAACTT
AGGAGTCGAGGAAGTGAAAGATATGGTGAAAATATGTTGTATGAAAATTTCAAATA
ATTTTTAAAATGTAATAAAAAGAAAACAGCCTTATTCCAAAAGAAAAAAAAATCCA
TAAACCCTTTACAGTGTGTAACACTTTAAGCCTGAGAAATTTATTAAATTTTCTGATT
TGGCATTCAAGCATCTAGAGAGTTTTCAACCATAGCTCTTCTGCATTTCCTTTGGAGC

TTTACTGCCCACTTGTGTTACATTGCCTCTGGATTGCAGAAAGTCAAGAGCACTTCAT GGACCAAAGATGGCACTTAGATAGTTCATTATGGAATGAATACACTTTGTCCTTTAA GAGCCTGATCAGTTCTCATGCTGATTGTAATCTTCTCCTATTTCTTTGTTGAAGTCAT ACTTTATCAATCATGTCTTTATGAGGTCAGATATCTTTTTTACAGTAAATTGCACCAT CTACACCTCCTTAAGCTCTCTCCTTCCAAACCAGATGTCTTTGCTGCCTCTTCCTTATT TCTGTGTAGTTGATCCTTGTGGCCTTCCTCCATGGAGTGTGAGGTTTTTGTTCCCCCA CCCCCACCCCCACCACCTGGCCTATTTATGTCTGTGGGTCTGGAGGCCTTTGGCTTCT GTGGGTTGACTTGTCTGTATGTGTCATTCAGTGGAAGGGACATGCCAGTAGCAAGCT AGCTGGCAAACTGATCCACTCTCATCTGCAAGGCTCTCATCTGCAAAGGAGGTAACG GAGAGCCCCTTGGTGTCATATCTTGAGAAAGTTCTTTCTGAGCTGACAAAACAATTT CTAAACTCTGCAGAAAATTTGTATGGACATTTGGGGCAGGTGAAATGGCTCAGCTAG TAAAAGCACTCGCCGTGCAAGCCAATGACCTGAGTCTTTCCTCTACTTTCCTGACTTG CTGCAGGAGAGAACCAACTCCCAAAGCAGTCCTCACAGGGAGGTCCTGTCTGATCA AGAAGCTGCTTTAGGAACCTGTTTCTTAGTTTTAAGAAACTAAGCAGCCTGGAGAGC TGCAAGTGCACCTGCTGAAACCGTGCCTGAGGTACTGACAGCACAGTGCCTCGGGTA CTGACAGCACAGTGCCTCAGGGACTGACAGCACAGTGCCTCAGGGACTGACAGCAC AGTGCCTCAGGGACTGACAGCACAGTGCCTCAGGGACTGACAGCACAGTGCCTCAG GGACTGACAGCACAGTGCCTCGGATACTGACAACACAGTGCCTCAGGTACTGACAG CACAGTGCCTCAGGTACTGACAGCACAGTGCCTCAGGTACTGGCAGCACAGTGCCTC AGGTACTGGCAGCACAGTGCCTCAGGTACTGGCAGCACAGTGCCTCAGGGACTGAC AGCACAGTGCCTCGGATACTGACAAAGGTGCTTTCTCTCTTCCGGCTGCAAACAAGC CCAGTCTTCCTACTAACTCCCAAAGGTCAGGGACACTTCGAATTGTGGCCCACTTCTT ATCAACTCTTGCCTGAATTCTGGATCAAAGTACCTCATATAGCACCTAGCCCTTCCGG GAAAACACATAAGTAAAGACTCCTCTTTGTTCCTCATTCTTCCTTCAACAGCAATCTG GGATTGAGTCCCCACTATGGTGGTCAGCAATATTGAAGGATCTGTTTACCTGAAACA CATCTCAATGTTGTTTTTGTGTTATTTTTGTTTTCTTTTGTTTCTGAGATAGAGTCTCA CTGTAGAGCCTTAGCTGGCCTGGAAGTAGCTCTGTAGAGCAGGCTGGCCTGAGACTC ACAGAGACCCGTCTGGCTCTGCCTCTTGAGTGCTGGGATTAAAGTGCACCACTTGGA TGGCTCTGCCTATCTATTTTTAAACCAGAACTCAAAGCTATGCACACAACTACACTC ACATGTCCTCACACATATCATGTACACACAAGTTAACAATAAATATAATTTTAAAAA TAAAATAATAAAATGTGAATGCTATTGTTGGAAACTAAGAAAGGGGGGCCTGGGGA AGAGGGGGGAAAGGGAAAACCCACACCCCACCAGAGTTTTGCCTATTCTCTGGTCTG TCAGGCGTGGGAGAGCTGCTATCTACCTTCCACTCATCCCTGGGTGGGCATTCAAGC CTCTGACCCGCTCTTCGAGGGGGCTGCAAGGGGCAGCTCTACCTGGGATTCCCGGAG

CTANNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNCCAATTCCCACA
TGCTATCCCTGGTACACATGTTGCCTGGTACACTGGATCAATGAAAACTTTAGCTCC
ACTCAACCCTTTGAAGGCATTGCATCCTTTGAACTGCCAAGCATGAAGAGAAGAACA
TTTATGTCATTGCTGATAGTTGACTGGTGGGTGGTCATACAGTGGAGTCCCTCAATA
ACTGGCTTGCTCGTGGATGTGTATTGGAGACCCCTCTATTCATTTTTCCAAGTAAACA
ACATTGTACACTATTCTTATTTTTATGCTTATTAATGTACTTGTAAAGGTCACAGAAA
CAATAAAAATGGAAGGTTTTCATAAAGCTCGCTGAGGAGGGTCTAGAAGTTAGACG
GATCTCTGATAGACAGTGGGACCACAAATAGAATTTTTCAATAAAAGGCAAACATCC
AGCTTTTTCCTCACACTCTTGACCTGAAAAAGCCATAAAGGCCTGGAGAGTATTTCT
CCCACCAGTAGGGCAGAAGGGCCCCCTGCTGGTGGACTGGCCAACAAATCGAAAGC
CTGCTCAGGATATTTACCCTGAGACAAGGACCTATCAATCAGCCCTCTACTCTTTGTT
CAAACTGATGAACCTTAAACTGGGGGAAGCACGTTCCTCAAAGACCCAGAAACCCC
CAATTATGGAGGAGAGACTTGATTTCCGACAATGCTCTTGGACCTATAATATTTCAA
ACTCCACAAACTCATCAAAGTTTCAATCAGTCCATCAGTCCCATTTGTACACTCAGTT
TCTTTCCAGGGATGTTCATTTCTAAGAGGGTCCCTGGGCTAGCCAGACACCAAGGAG
AAGACGAGGAGTCTGCACCAGTGGGAGCACAAGGTCATGTTAAAGTGCTGAAGACT
TCTTGATGCCCAAAACCTGCATCCCTAAAGGGAGCTTTAATGGCAAGGAAAATCGGG

AATATCCATGAAAACTGAGCTCAGGGTGAATATTTCAGTGTTTAAGCAAAACTGATT
TATTTTCTCTGTCCCAAATGAATGAACATGATTTTGTTTTCATTAGTTTGTTCTCAGAG
AATGTTCAGTAAGTGTTCAATGCATGAAGGCTGTAAGAATTTAAACGTGCTCACTGG
AGTTGGTGTCTGCTTCTCTCCAGAGGGAACAACTTTGGTTGTGGCTACCTCTAACCGG
GCTGGAGGCCAGATGTTATAGGGGCATTCCCTCAGTGGTCTGCACCCAATGTTCTTT
CTCACTTCCTTTAATGCCCTCTGATTCCACCGTGAAAACTATCTCCTTCCCCAACCAC
CACACACAGAATCAGGATGTGCATTGACCAAGCTGGCAGGACATTTGACACTGGCT
GAGTGTGGCATAGGAGCCTTAAGCCCTCTTCCTTTGACTGTCTAGACAGACCCCTCTT
GGGGGCTGGGGCTGTTCAAAGTCACCTGTATTAAGTGAGGTCTCTAGGATCTCCCTC
GCTCCTTTCTATTCATGAGTGCACATCTGTTCCACTCACTAACTCTTTGGTATAAGCC
AGTCTCATAGCTCTACTGTTGTGATCATGCCATGGCACAAATGTTTGCCATTCAGCAT
TGGAAAGATTCCTGGCACTTCCACTTATAGTGCATTGAGCTTATAATGGGAAATAGA
CCACCATATACAACTTTGTCTGCAACATGGCAGCCTCAGCTCAGGGGATAGCCAAAA
GGGCCACCTCTAACATAGTGTTTTCACTTTTCTCATGCTAGATGGATGGAGTGGAAC
CCCGGCTTCCCTTTGAGTATTGATGCCAAATGCCACAAGGATCTGCCCCGAGATATC
CAGTTTGATAGTGAAAAAGGAGTGGACTTTGTTCTGAACTACTCAAAAGCGTAAGTT
TTAAGAACTTCAGGGTATGAGTGGCTCATCTGATAGCTGGGGATGGGAGAGTGTTAG
GACTTCAGGACCACAAAGAAAGAGCACACCATTCCACCTCAAGTGTCTGGACCAGG
AACAGCTGTACTCTTGATCAGAAGGGCAGCCATACACCAAGACAGACATTGTATTTG
CTTATTCTAACTCAGGTAGTGTCTGTATTCTCCCCATTGACTGGGATCCAATTCACGG
CCTTAATGAGCCAACCTGAACAGAATTGGTACACAAGAAATGAAGGAAGGGGAAAA
GGGTCACTGATCCAGGTCATCAAATGCCTAGAATACAGCAATGGACCTTGGTGTAAA
TAAAGATTTCGTTGGGGAAGGGGAAATTAAAGCATTTCCATAGAAGTCCAAGGTTAT
TTGGGGAGGGATAAAAAATGAGTTTATTCCTTGTCTGAAGGCAAGTTGTAGAGCATC
TGATAGAAAAGACTCGTATTTGTTCTTTCTTATAGTAGGAAAGGCCTTTTTTATAGGA
CTTGGGCTGGGGGAGCAGTTCAGCTGAACACCATTCCCCATGCGTTTGCTCTACCCA
GGCCAGGAGGTAGTCTCTCATCGAACAGTTGCAAGATGACTTATATCTAAACAGCCT
TGACCCAAAAGTCTTCTAGCACCTACAGCCCCGTGCTTTCAGCTAAAAGCAGAGGCT
GTTCTTACTTACAACACTCCATCTCCACTTCCAGTTCACTCCTGTGGCCAGTCCATCA
GAAGATGGAGTCCAGACTGATGCCAAGAGGCAGGTTCCACAGTAACAGTGACTCAG
ATGACACTGTTGGTCCCATGACTTAGATTCTTGTTCATTTGTCTTAAGGACCCCATGA
AGTAGGCATATGTGTTTATCACTGTTCTGGTGCTGTGAAGAGACACTAAGACCAAAG
CAATTCTTATAAAAGAAAGCATTTAAGTGGGAGCTTGCTCACAGTTCCAGAGGTTAA
GTTCATTATCATCCTGGTGGGGAGCACGGTGGCACACACGATGCTGGAGAAGTAGCT

CAGAACTTTACATGCTGAACCACAGGCAGACACAGAGAAAGACCTGGATCAGGTGT
AGGCTTTTGAAACCTCAAAGCCCAACCCCAGTTTCCTCCAACAAGGACACATCTATT
CCAACAAGGCAACATCTCCTAATCCTTTTAATCCTTTCAAATAGGCCCATTCCCTGAT
GACTAAGCATTCAAATATAGGAGCCTATGGGGGCCATCCAAGCACCATAGGACATA
TTGTCCTCTCTGCAGTTGAGGAACCGAGAAGGAATAAGAAGGTTGAGGACCTTGCCT
AAAGTCCTTTGCTAATAAACAACAGAGACACTCAACTCTGGGTTACATGTCTGCACT
AGAGTTGCTGCAGGATGCCAGCACAACCATGGATACTCTGTCAAAGAGTAGACTCTT
TCAGCACCAGACCAAGGTTAGCAAGGACACCTTTGATGTTATACTTTGACTCTTAGA
GAACTTTTATACTCATACAAACCTAGAAAACCCTAAACTCTTGAAGCCCAACTGAGC
CTTATACTCCCCCAGGAACTTTTAGCACTTCCCAGCACACAGCACATCCACAGACAA
GTCTCAGAGAGAAGGACTTGGCTACCTACACACCCAACAGGCAGCCAGGGCTGAGG
ACCACAAATCAGTCTGCTCCATCTGGGTGTTTTCACAGCTATTGTTGGTCATTTCTAG
GTAAGGAAATCAAGAGTCCAGTGGGTTAAGATAAAGTAATTTACATATATATATTCC
CATACATAAATAGATGATTTATATATAAACTAACTCTGGTGACCTGAATGAGAAATG
ACCCTGTAAGCTTGAGTATATGAACACTTAGTACCCAGTTGGTGCTGGCTTTAGAGG
CATGAAGGATGCAAGAATGAAGGGATTGTGGAGTCTTCCCCCATGGTTTTAGAAAGC
CACTAAGGCAAGGCACTGTGTGGTACGGATGTCCCTGTGTGAAGACCACAAGAGCA
AGAGGCTCTGAGAGGCTATTGATCAAGCTATACAAGTGTGGCCTTGGCTGCAGTGGA
GACACCAAGAGATTGGAGGTGTCAGAGCTGAGAGATACATACATAGGAGAGCTGCT
CACATAGATTGGAATCAGCCAAAGAGAGAAATATATGTTGTAGTCAGCAAAGTTGG
AAGGGAAGAGCCATTTAAGCCCTTTGACAATAGGCATAGGGCTAAAGGATTTGGAG
TGTGCCCTGCTGGGTTTCAGTCTTGCTTTCATCCAGTATTTCCTCTCTATTCCTGCTTT
CCTCTCTTGTGGAACGGTAATGTATATTCTTTGTGGAAGTATGTAGTTTTTTTTTTTAA
ATGGGGGTTATAATTAAGAGATTGCCTAGAGTCTCAGAAGAGACTTTAACACAGAA
CTGAGCTTGCAAGACTCTGGGGACATCTGCAGTTGGACTAAAAGATTTTGAATTATG
ATATGGCACAAACCTACAGGGAACCAGGAGTTGAGTGTGGTTTAAGTGAGAAATGT
CTATAGAACTGGCGTAGGTAATATTTGGTGCTCTATTTGGGAAAGTTTAGGACGGGC
AGCCTTGCTGCAGAAAGAATGAACTAGGGGCAGGTTTTTAAAGTTTAAAGTCTTGCC
CCATTTATCATTTGTGCTCTGCAAAGGAATGAATGAACACATGCAGAGGTGGGATTC
AGAGACATTCCAGAGGGCATAGGCCTGGTCTCTTACACAGCTGGGGTGCAGTAGGA
AGAGCGTCTGCCACAGGATAAGTTCTATCCTGGTCCCACAAGGCCAGCACCTACCTC
TTGGAACTGCCTGGGCTATGAGGAGGTTCCCAAGTATGAAGTAAAAACACTCTGCGA
TGGCATAGTGGGTGGGCAAGGTGACCTGTCCCCTGGTTTGCAGGATGGAGAACCTGT
TCATCAACCGCTTCATGCACATGTTCCAGTCTTCCTGGCACGACTTTGCTGACTTTGA

GAAAATCTTCGTCAAAATCAGCAACACTATATCTGGTAAGAGGGTTCTGTGGACGGG AGAGTTTATTTTTCTGTCTGAGACTTGCAAACATCCAACCCATGCACCCCCTCGACAT CATCTCACCAGAAGTGTCAAGGCCAATGGGACTTTTTCTTTACATACATAAATCATTT GTGGCTCTTCTGCTCAGACCCGAGGCAGGACTGGGCTACATCAACCTAGCATTGGAG GCTGAAATGATGGCTGAGCAATTCTGCTCTTGCAAAAGACTCAAGTGCAGTTTCAAA CATCCACACTGGGTGGCTCACAACCACCACTAACTTCAGCTACAGGGCATCCAAAGT CTCTGGTCTCTGGAGACACATGTGCATNNNNNNNNNNNNNNNNNNNNNNNNNNN NNNNNNNNNNNNNNNNNNNNNNNNNNNNNNNNNNNNNNNNNNNNNNNNNNNNNN NNNNNNNNNNNNNNNNNNNTTACACACACACACACACACACACACACACACACACA CTTAATAGTAGTAAAAACAAAATTTACAGCAATGGGGTCCTTAAGACCAAAGTTCAT TTAGTTGGAGCCCTGGCACTGTCCTGGACAGTAATAGATGGAGAGTCATGACAGCCA TCACTCTTCCAAGAGGCAGGAATGGTTAAAGATGCTGACCTCAGAACAAGGGATCA ACATTCTCCCCTCTCTTTTCATGCTGGGCGGAAGGACCGGAAACCTTGAGAAGAGCA AGTGCCTGGAAATCTTACTGTTCACTAAGGCATAGCTCTAGGAAAACTAGCTCTATA GCCTGACTCAGCAATGAGTAGGGACACAGCAAGTAGGGACATGGCAAGTGAGAAGA TCCCGATACCTACAAAGATTTGTCCTCCTGCAGGATCGTGGGATCCTGCCCAGCGGT CCTATCTAGAGGTCATTCTCTCCACAGAGCGAGTCAAGAACCACTGGCAGGAAGACC TCATGTTTGGCTACCAGTTCCTGAATGGCTGCAACCCAGTACTCATCAAGCGCTGCA CAGCGTTGCCCCCGAAGCTCCCAGTGACCACAGAGATGGTGGAGTGCAGCCTAGAG CGGCAGCTCAGTTTAGAACAGGAAGTACAGGTAGGCCAGCTTGCTGAAGACCTAGT CTTTCCACAGTAGCCACTTCCTGCTGTCCAATGCTTGCTGGTCCCCAGACTTTTACTG GCTTGTCTGGTTGATTGATTGTGTGAAATCAGTAGTTTCTTCATTTTTCTGGCTGCAG CATCCAGGAGGATCTTGGGCACTGTGCAAGCCACATGCAGTCACAGGAGGGGCTGC ATCTACACAATCCTCACATCTGCAAACACAGTGTCAGTGTTGTGTTCAATGTCCATAT CAATAATTACTTGACATTCCCTGTGATAGGTGTGTTCTACACATAGTCACCCAACTGT CATCTGCTTTGCAAAGGTGAGGAAACTAGAGAAAGAAGTAGACTTTGTGTAGGTAG AACTTACTGCTAATTAGAAACATAGGCACTACCTGTCCCTCACACACTTTCCCAAGC CAGTCTTTCCTGACTTTCACTTGCAGGCTCAAGCCCTCATCCCCAGTTCCAAGGCTGA TCTTGTCTTGAGTCGGATAGAGTTTAAACCAAATCGGCTTGCATCTGATGGAATCTTC CAGGCATTACCGTGCTAGATTTTATTTTGCTTGCTGAGAGTCCCAACAACCCTCAGCA TACTCCCATCTCCCCATTGCAGCTAGCACCACTGGGCCTCCATCTCCAGCTGTTCATG TAACCTCCATTGCCTCTGTTCACCCTGTCTAGGTACCCAGCCCACATGGAGACTCCAA ACACATACCATTCAGGCCTAACTTCCTGCCTACATGACTTTGGGGGTTACTTAACCA ACTGAAGTTGGGGTCCTGATCCACATGGAAAGTCCTTCCCCACTCATCAGGACCTTC

ATGGGATCTGACATATTTCTAGGCATGCAGACAGCCAGCAACAACAACTCCTTACAC
CCACCCCATGCACAGAAATAAGCATGTCTTAGAACTAAGAGGGACCTAAACCTCTGC
AATTGCCCCTAGCTACCCAGAAGTAGGACCTTGCCTGTTCTCCAAGACAAGCCCCCT
ACCAACTCCAGGGGAGGTGGCTTATGGGTTATCTTCACTTTGAGCTGACCCATCAAA
CACATGATAGTGATGATGTTCTGCTGTGGTTTAGATCATGAGGCAATAATCTAACAA
TCTAACAGCCACTCCTGATTAGACTCAGACTGACTACCTTGTCTCTTATACTGTCACC
AACTAACACACCAGAACAGTTCACAGGGCCCTCCATCATTCTTGACACCTTTCTTTG
ACAGCCTCAGTGACATGTTAAACACCAAGCCCACGAGTTGCTTTGTTTACAGTATTG
AGAAGTGGCCCCAGAGCCTCACATGTCTTAGAAAGCCCTCTTCCACTGAGCTATATT
ACCAACCCCTAAATATTTTTAAAAATATTTTCCAGTGAAATTTGGTTTTGGAACATG
ATTGGAAGAACCTTTCAAGGACCCTTTTCCCCCCATATATGCAGAGAGTAGCCCCCC
CCCCCCATCTCTTCCTTTCCAGGAGAACCCTGTCCTGGATGACAATGGCTAGTTTGCC
CAGTGTTGAACTTGATCTAAATGAAGTCCTCCAGCTGCATCTGCATGTCTGACTTCTC
CTCTCTCACTCTCAGAGTGGTCTTTCCGCTTCTTTCCTGAGCATTAAGCAGCTTTCAG
GCACAATGAACCTGAGTCAGTTGTGGCCAGTCTTTCATTTTAGCTTTGAAAATAATTA
GAAAGCAGAAAATAAATATGTAAGCCAGCTTTGGAGCTGGAGAACCAGACCCATGC
ATTCTGAATTCCATTTGTACCCAAAGCTTTAGGGTAAAAGCCTGGGTTCCCGAGGCC
CAGATAGACTAGGTTTGGCCACCCAGCCCCACACACAAACAAAAGAGATAGGTAGA
AAGGGAAAAAACAGAGATTTAATCAATATGGACACACAGGGGGAAGAGATAATGA
GGTCCAGTGACCCCCTAAATCCATCTTGGGGCACTGTCATGAGTTTTAAGTTTAAAT
AGAAGAAGTTCTGGCCTAGGGGACAAGATGTGGCTAAATAAGAAGTCCCAGTCTTTT
TGACCTATTTTTTGATACTGCAAGTATCAAACAATAGCAAGTCTTCATTACTGATTGA
ACATACGGATCTAGTTTCCACAAGATTACTTCTGCCCCAGGCTCTGTAGTATTGCTAT
CCTGGTACAAGTGTGTGTGTGTGTGTGTGTGTGTATCTGTCTGTCTGTCCTGGTAGGT
GTTACCTGTTTACCTGTTTCTGGGACACAAGACTGTGACATATTTTAAATTTTTGCAG
CAAATATTTTGGTTCAAATAAACCTCTGAACCATGAAGGAGGAAGCTCAGAACTTCT
GTTCTATATGATACATGAATGGATCACTGCAGAAACATTCCTGTGTTGAATGTAAAT
GTAAAATGTATTATGTTAATATACTGCAGGAGAGAATGAGGGCTTACCTTTGTACCC
TTGGCCTTGAAGAGAGGAAAGTAGGTTAGACAGACTCTTCCAGTGACCAGCAGGCT
GACTTGCAGAGTCTGCAGGGGTGTGACAAAGGCAGGAGTAGCAGGCTGTCGTCCTG
CATTTAGTTACTACGTGGTCACATGAAGAATCGTCCACTCTTACTAAAACAGTTTCTA
GTGTTTGTTACACACCAAGTTACAGGGAAGGTGCGTGCATGTGGGTGTGGTGCACAT
GTCCTGAAGACCGTCAGTCACATCCCACCCCTGGCCTGGCTCCCGGGTCCGTTTCTGC
TTCAAGTAGCTTGAATATATTTGGCTGCCTCCTGCAGGAAGGAACATTTTCATCGTT

GATTACGAGCTACTGGATGGCATCGATGCTAACAAAACTGACCCCTGTACACACCAG
TTCCTGGCTGCCCCCATCTGCCTGCTATATAAGAACCTAGCCAACAAGATTGTTCCCA
TTGCCATCCAGGTAGGTGGCTGCTCAGCCCCAGCCCTTCTGTACGACTTGTACCTCTA
GGGCTATGTGTGTGTGTGTGTGTGGTGGGGGTTCTGGGGCAGCACCATCCACTCCAC
TGAGAGGCACCAGAAGCTCTCACTCCAGCTTTCCCTGGGGTGGGGGAGAAAAATGA
GCTACTGGGGGTCGCTGCTGCCATGAGAAGGCTGTCCACACCACGGCAGACAGTGC
CTTTCCCAGGTACCTTTGAGACATTCCCTACTGAGGCTAAGCACTGGGGAGGGCTTG
TCCTGCAGTGACCTGCTTTTCCTCCTACCACCTCCTGACGACTGTTTCCAAATTGACA
TGCCCACACAAGCCAGATGGGTGAAGCTAGGGGTGAAGTTGCAAACAGAAGCCACT
GAGCTGTGTCGGAGACCTCACCAGTCCTGGAACATTGTCTTCCATACAAGTAGATTC
TTGCAGCCCCGAGGACAGTGGAGGGCCACGGGAGCAGTCTCATGGGGATGGAAAGT
CTGTGACACTGGGTAATGGGAATAATATCAGTGATATGCTGGTGTCTACACAGTCTA
AAAGCCTTTGCCTGGGAAAGCTTAGAAATCATGAAAACTGCTGTATGTTAAGTGCCT
AAGAACAGACACTATGCTTGAGCTTGGCCAAAAGACCAAGAAGCGGTAAAACTGTG
ACACATTTTAAATTTTCCAGCAACTGTATTAGTTTCAAATAGACCTCTGAACCAGAG
GAGAAGTAGTTTAGAGCTTTTGTTCTGTTCAATATGATTCCCGAATGGGTCACTGCA
GAAACATCTCTGTGTTGACTGTAGATATAAAATACCTTCTGTTGTCTCAGACCTGGA
GGAGGGACTTGGCACATAAAGAAGCGTGTATATCGCTGGCATCCTAAATATTCTGGA
TCCTGGAGCCTATGATCATTGGGTCTCACACAGCATCTGTCTCCATTTGTGTAACTGT
CACCTTACTGCTCTCAACCATCTTACCCATGCAAGGAGACTAAGGTACAGGTGCTCA
TAGTTAGCCAGCCACGGAGCTGAAATTCAAGCCAGCTGTCTCAAGCCTGAAGTTTAA
CTTATGTTTATAATCCCAGGCTGTCGGTTCAAGGACAGCCTGGGCTACAGAGTGGTG
CCCAGTGTCAATTTGTTTAACTAATTAATCAAATCAGTTAGCTGCATATCTATACCCC
CACCCCTCTGCACTCCAGGAGTCCTGAGCCTATTATCAGAGAAAGCTCTATGTGTAA
GAAGCTGAAGCATCAAATGAAGGCACAGGGGATGTGGGAAATCACCACTGAAAGAT
GCCAGGAGAGGGATGGATTACCACTGACAGGTGTTCTGAAATAGCATGTCCCAGAC
ATGTATTTATTAGAGCCAGAGGCCTGGCTCAAATCTCAGCTCTTTCCATTGTTAGCAG
GAATTAGGCATTGGACAAGCCACAGGACATGGTTAACCCTTTCTCTATAGCTGCCCA
GGAGAATTAATGCACCCCAGGGCAGTGGTAGCTGCAGTTACTGGGACAACCCTTGA
ACAGGGCCCACAGCTGCTGTAACAGATGGATTCTAGGCTGGGCTGTAAGAAGGCCC
ATGGGTCTCCGTGTTGAGGCTGGCAAGGCCAGGACAATTGAAAGCAGTGCTGAGAG
GTTCTCTGCCAAGACTGAACTGAACCAGCCTTTTTGAGGATCAACAGAGCAACATGC
TAGAGGATGGAGATCATGGTTCTGGCTTGGGTGTGAGGCTGCAGACAGACAGAACA
TAGGAGAGGTTGAGGGGATGGGACCGTGTCCAAAGCAGGAAACACTACAGCACTGT

ACACCTTCAAAGAGGACTGCATCTGCTCTCAGCTGACTTGGCCCTTCCCAGGGCTAG
TACCAAAGCAGAAAAGCAAAACATGGAATATGGATGATATCAGCTCATTTATGCAA
AAATGAATGCAAGAACACAGCATGCCACAAACCATATGCTTTACATGGGTAGACAT
TGGTGTTGGCTTGGTGAGACACAGGACACAGGGTCACCACGCTGCATACAGAACAG
GTTGGAGATAGTAAGGATAGGTCTAAGTTCCTCATAATAAGGTTCTGTTTATGAAAA
GTAAACACAAATACACACATATGCAAAGACCTAGGGAAGCAGCGACCTCTTGTGGC
TGGACAAGAGGTACATTCTCACTAGTTCTTGGACTCTTCTGTAAATGCTGGAGTAGTT
TACAATAATAATCATATATTATTTCTACTCAGAAACAAAAGATGGCCTCCTTCAAA
AGCATTTAAAATAATGCAAGCACGTTGTCCTGGGCATATTCTAGAAGAGCTGGGACA
AGGACAAGTCTGGAGGGGTGGGAGGAGGTGTGGCATGCTCTGTATAAAAGCTCACC
ATGGGAGCCCCTGTGTGGCATGGTCAGACAGAGAGGAAGGAACTGCAGGGTGATGG
AGTTACAGCAGGCAAAGCAACAGAGCATCCCTACTTAGCTCCATCCAGACCTCTGTG
TGGCCTGGAGAGAGGCTGAGTCACTCAGAACCTCACACTTTATCTCTTTGCAAAATA
GGAAGGATGAGGAAGAACTGTTGGTCAGGCTGTGGTGAGGATTTTGTGATGACACA
TACGTCAAACCTTACGACACAGCAGAAAGTCTCTGGACTCAGTGCCTGAGGATACCT
GAGTAGGACAGGTGGCCCGTGTGCTCTAGGAAGCAGGCCACAGGGTGGCTGTCTGT
GGGCCACACAGCTATGGAAACAGAGCGATCACTGTGAGGAGCAGTCCTCTGAGAAC
CTTGCTCACTAGGACCCTCTGGAAACGGCAAGCCTGTTGTCCTCTGAGGACACATGT
GCTGACTTCTGACTTAAACCTGATCACTTAAGGAATCTGAAATTCTCTCCTAAAGGT
AGAGGACAAATCCATTAGCGAACAAGTGGGAAGGATACTGTGGGGGTCAGGAGTGT
CTCCCTGGCCTCCTTGCTCATCCTCAGGCGGTAGGGGAGAGCTGCTTCCCTAGTAGTC
CTGCCACCTCCCCCACAGCACAGTGGCTTAGGAAGCTTAGGACGCAGTACTCCTTGC
TCTGGTGTTCTCGAGACGCACTTAATCCAAACCAAGACCCGGAGTAAATTCTTAGTG
TCCATAACCCAAAGACTTAGAAGAAGAGTAAAAGAATGAAACCTGCCAGGACTATT
TCAGAATGGTTTTGTGTTGTAGAAAAGTGGGTGAGGCAAGGAGCTAAAGGGGTCTG
CAACCCTATAGGTGGAACAACAATATGAACTAACCAGTACCCCCGGAGCTCGTGTCT
CTAGCTGCATATGTAGCAGAAGACGGCCTAGTCGGCCATCATTGGGAAGAGAGGCC
CCTTGGTCTTGCAAACTTTATATGCCTCAGTACAGGGGAATGCCAGGGCCAAGAAGT
GGGAGTGGGGGGGGGAGTGGGGGGAGGGTCTGGGGGACTTTTGGGATAGCATTTGA
AATGTAAATGAAGAAAATACCTAATTAAAAAATAAATAAATAAAATTCTCAAAGAT
TAAAAAAGAAAGAAAGAAAGAAAAGTAAAGAAAAGAAAAGTGGGTGAGGTAAATA
TTTTACCCTGGGGTTACGTATTTCTTTTTTCTTTTAAAAAATATGGCTGACTAAAAAA
AAAAAAATGTAATTTAAAAAAAGGTGGCCTCCTAGAAATTTGAATTTGTGCTGCTTG
CATTATATTTCTATTGGCTAGTGCTACTTTGGATGGAGAGACAAATCAGAAAGGTGC

TTATAACCTCTCTAATGAAAACAACTTAAGGAAGGGAGAGTTATTTGGTGCTCAGT
TTAAGGGAATACAGTCCATCGCAGAGGGTGAGGCATGGCTGTAGAAATAGGAAGTG
GTTGGTTCCATTCATAATCAGGAAGCAAATAGACAGGAAGTGGGGCCAGTATGTGG
AACCTCCAAGCCCAAGTCTCCTGCTCCTAAAAGTTCCAAATCTTCCAAAACAGCACT
GCCAGCTAGCGATCAAGTGTTCAAACACACGAGCCTACCGGGACATCACATTTAAAC
CGCAAAACCATCTTCCAGATTAGTTAGGGGGGTGCAGCTAACTCTGCTCAGGGGCCT
TATCTTTCTGGCTTTAAAGAAATGAGTTTTTAAAGAACAGATAACAAATTTCTTTACA
TTTTAGAGAAAAATCACATGACCCAAGCAAGGAAACTGGAAATGCAAACGCAAGCT
GGGCCCCTGGGTACCTGGTTTCAGAGTTAGGGCTCAGAGCAGGGCTCCAGCAGCAA
ATCCTGATTTGCAGCTGGAGCAAAAGGGAACTGGGCAGCATGCAGCCCCAGCCTTG
GTCCAGAAGGAGCACGGCACTCAAGGTCAGGGTGTCTGTCAGGGAGGAGGATGGAG
GGCACTGCTGAGCAGAAGTGGTAAGCTGGAGGATGCCAGGCTCTGGGGAGGGCACA
GGTCACCGACCCTTGCAGCTGCTCGCTCCTTCCCCGAGGTCTGTACCACTGTCCTCAA
GTTACAGACAAGGAACCTACAGCTCACAAACAGGTCAAAAGACATGTCACTAGGGC
CCTCCACCACTCCCCACGTGCTTTTTGTGATCTGAAAGTCATGGCCGGTTGATGTTTG
CTTTCTTGTGGAACTTCCCCAGAGTTCTCCCACAGCTCCCAATGAGAAATAAAGTGG
AGCGTTCTCTTTCTCTAATGCACCAGCTCAACCAAACCCCTGGAGAGAGTAACCCAA
TCTTCCTCCCTACGGATTCAAAGTACGACTGGCTTTTGGCCAAAATCTGGGTGCGTTC
CAGTGACTTCCACGTCCATCAAACGATCACCCACCTTCTGCGCACGCATCTGGTGTCT
GAGGTGTTTGGTATCGCCATGTACCGCCAGCTGCCTGCTGTGCATCCCCTTTTCAAGG
TACAACCAGCCAGGGCTCCACCTACAAGGAAAGATTATCTAGGAGAGTAGCTGGCA
TCCCAGGGTGTGTGCGAGTGGAGTGGTGATAGCTAGGAGTAGGTTGCAAAGAGGGG
CCTAGGACAGACATTCAAGGGCCAAGTCACAGGAGCCTGTTACAACCCTGCTGTCCA
GAAAGCAGAGTTCAAAAGGGCAGTTAAGTCATTTTCCTTCTCTTCCCAAATGCTGCC
AGCACTTGATTGTTGGGTCAACTACAGGCTAAAAAAAATTACTGCTGCTAAGCCAGG
TGGTGGTGGTGGCAGCACTTGTCTTTAATGCCAGTACTTGGGAGGCAGAAGCAGGCA
GATCTCTGAGTTCAAGGCTAGCCTGGTCTACAGAGTGAGTTCTGGTACAGCCAGGGC
TATATACCTAGAGGAACTCTTTGTCTAGAAAAAAAAATAAAAATAAAAAAAAATAA
AACAAAAAAGAAGAGAGCTGGTGCTTTGGTCTCTTCGTTGTAAATTAGTTCAAAGCA
GGACGTCTGCCTTAGAGACTGATAACCAGGATCTGTTCCTAGTGTAGTAGGAGACAA
GCGCCAGGGCCCTTACTCCTCAGCATAACAGTGGTGGCCACAGGCCTCCTATAGCTA
CAGGGCAGGAAGGTCTACACAGAGCCCTTTCACTTCCTCTCTGGGGCCAGAGTGACC
CCCGCACTGTGGGCTGGCCTGGCCTGGGCTGGGAGTGCTGAGTGAGCATGGAGACC
CTCAGTGAGATTTCTCCTCCATCCAGCTGCTGGTAGCCCATGTGAGGTTCACCATTGC

TATCAACACTAAGGCCCGGGAACAGCTTATCTGCGAGTATGGCCTTTTTGACAAGGT
GAGTGCCCTCTCTTCATGTGGAGCCTGGACAAGCTCTGCCTTGTGGCTCCATCCCTAT
CTGAGGTGTGAAAAGTGTGGGAAACTCTGGTCCTTCAACCAACACCACAGCCAGCTC
TCCCACTCTGTGTTCTCTGTCACCTTTTTATGTATCCTGTCCAGTTTCAGTGTCTGAGC
CTCCTCCACACAAGCCACTAACCTCTACCTTACAAAACATCTGTTATTTCTCCCATAC
TACCATATCCCTGTCACCCTGCCTGTGGCCTTCGAGGTATTTGAACAGAACTGCCCA
ATAATTGCCTACCCATGTCCTTGATGTTCCCAGTTCTTCAAAGCTGATTGGATGCCCA
AAGCTATGTTCTGCACAAAATCCTGCTCTCTCCGTTTGCAGCTATACAGTGTCCGGTG
CACGTCACCTCATCGTGGGAGTGGGAGCGTGGGCCTACTTAAAGCTGAACAGCTCTC
CCACTCCACCTCACAGGACAGACTGGCCTCTCTATACCCAGCCACCCACTGGCCTCC
AAGTCCCTCACAGATGAATCTACAGTATGGATATGGACAGCTTTTGGGGAAAGCAAC
CAAACTCAGGCTCTCTGCTCTCTTACCAGGCCAATGCCACCGGGGGTGGAGGGCACG
TGCAGATGGTGCAGAGGGCTGTCCAGGATCTGACCTATTCCTCCCTGTGTTTCCCGG
AGGCCATCAAGGCCCGGGGCATGGACAGCACGGAAGACATCCCCTTCTACTTCTATC
GTGATGATGGACTGCTCGTGTGGGAGGCTATCCAGTCGTGAGTGTGACTGGGTTCTG
TGGGAAGGGGAAACCCTAAAGAAGAGTATGATGAGGCAAGCTTGCTCCTGGGTTGG
CTGCTGATGGAGGGAAGGGGCAGAGTCCGACATTGAGAACTGATGGGACTGGGAGA
GGGACCTGCTGGATACGCACTCCTGATAGCCCCCTGACCCAGGTTCACAATGGAGGT
GGTGAGCATCTACTATGAGAACGACCAGGTGGTGGAGGAGGACCAGGAACTGCAGG
ACTTCGTGAAGGATGTTTACGTGTACGGCATGCGGGGCAAAAAGGCCTCAGGTAGG
CTACAGGGCAAGTGTGCATCTCCAGGTCATGAGGAACAGAAGGCAGGTGACTCTGC
TCTCGGGTACCCACCAGTCTCAGAGCGTCCCTCGGAGCATCCAGCCTCCCTTTCTCTG
AGCATCTTGCTAAGTGTGTGTGGGGAGCTAAGATAGAGGCAGAGGTGGGTGTCCCTC
ACAGAGATGAGCTCACGGTCGGTGGTCGGTGGCTCTGTCTTAGGTTTCCCCAAGTCC
ATCAAGAGCAGGGAGAAGCTGTCCGAGTACCTGACGGTGGTGATCTTCACGGCCTCT
GCCCAGCATGCAGCTGTAAACTTCGGCCAGGTAGGCCAGGCTAGCCCTTTCTGGGGG
AGAGTCTTAGGTACCTCACAGTGGGAACCACCTCCAATTCCACACCCCTGGCCCAGG
CTCTTGCCCTACTGGTCCTCCCATCTCCATGCAGCCTCTGAGCCTGGAACCAGCAGCA
GCAGGCATGAGGCACCCACCAGGCTGGGGCAGTTGGAAGTTCTGGAAAAGGAGGGA
AAGGGTCTGAGGAGGGAGGTCTGGGGACCCTGGAAGAAAGGCAAGGTAGGTAACA
AAGGAGGGGGACACTAGTGGTATGGTGACATGTCAAAGGTGTGGCCTGGGAAACCA
GGAGAGGAGAGGGCCAAGACAGGTGCAGTGGGGACATCAGAGAGGTGGATGGTGT
CCACAGGGCGGGGTGGAGCCTGCTGAGCTCCCTATGAACCAAGGAAAAGCAGGCCT
GTGGATCCGAGGTGGGCAGCCACTGGGCTTCTTGGGCACCCACGCTGTTGATGTTGA

TGTTGCTCTCACAGTATGACTGGTGCTCCTGGATCCCCAACGCTCCTCCAACTATGCG GGCCCCACCACCCACGGCCAAGGGTGTGGTCACCATCGAGCAGATCGTGGATACTCT ACCAGACCGTGGCCGATCATGTTGGCATCTAGGTGCAGTGTGGGCCTTGAGCCAGTT TCAAGAAAATGAGGTGAGACCAGGCACTGTTGGAAACACCGTAGATCACTCTAGTTT TAACCCATTCTCCAGCGCACGGCTTTGGGGCTCTGACTCAAGCTAGAAACCTGCAGT CAGAAATCCTGATTTCTAAGGTGGAGCTATTAGGAGTTGGGGGTGGGGTTACTCCAC CCCAGGTCCTCAGCGTGGGTCTGAGCCCTGGGCAGGATGGCAGTGGGAGGCACAGG CTCTGCTCGGAGTCACCAGAGGGGTGTGTGTGTGTGTGTGTGTGTGTGTGTGTGT GTGTGTGTGTGTGCTATCACCCTGTACCCCATACTCAGTTGATAAATCTATTCCACAT GGTTTCCTGACTCCCCACAAGGAGAATTAGAGCTTCCTAGTTTCACTTAGGTATCCTT CACAGCTATGCTAAGTGCAGGTCTTGGTGGAATCAGCCCTTTGCAATGTCTCAGCGC TGTTCCTATCAAAGTACCTTCAGCTTGTCCCAGACCTGCTAATGTGCTGGCTCTATGC TCAGTAGGACCAGAGGAGTTGTTCCTAGGAGACTAATTTCCTGGTCAAAGGATGTCA AGTCTGACTTAGCTTCTCCAAACTACACCCCAGATCTCCCACCCCCTTCCAGCCACTG AACACTTTGCAGACATTGGTGTAAGCACATTCCCCTGCAGGATGGCCACCTCTCCAT GCCCAGGACCCCTGCCGGTCTCACGCCTTAAAAGGAAGATGAGAAAGACAAAGGGC AGGCCAGCAGGACCCTGTGGCCACAGAACTTCAGAGCTGGGAGCTTCCAGGTTCCCT TGCACCTTACCTTGTCTAAAAAAAAACACCTGGCAACAAGGAAGTAACTTCCTTTAA GATCTCCAGCCCTTAGCTTTTCATAGGGCAAAGGAGGTAGCATTTGCATAATCTAAG TTTTAAAAAAGAAACAAACTTAATTTGCATATTTATGAGATCTAAGTGTGATATGTG TATGCATTGGTGCTGCTCAGTTATGGCATCTGATCTATCACCTCAAACAAGTTACTTC CTTGTGTCCTTGTGTAACATTCAGACTCTTCTAGTTTAGAGATATACTACAGCATTAA CTATTTACCCTACCATGCTGTAGAACACTGCTTAGGCCTCCTTTGAATATACTAATTT GCCATTTTTCCTCCTCTGTGGTAACTTCTTTGAGATGACCAGGCAGGACTGCCTTACT GTTTTAGATAGGGTCTCACTATGTAGCCCTGGCTGGCCTGGAACTCTCAGAGACCTG CTAGCGTTTGCCTCCCAAATGCTGAGATTAAAGACATGTATGTGCCACTGCACCTGT TGAGACAGCAGTTTTTAGTATCCTGTATATGGTATTTGCATCTGCTTCTTTCCCAGTT CATCTAAGTTCCAAAGACAGGATTTTGTATGGCCACGTAGTGTTCCATCGAGCATTC TCAGTGGGTAGCAAAGGTCAAAGGTGATAAGAGTAGGTCCTCCTCCTTCCCCCCCCC TCCCCCCAAAGGAAGCCTCTTAGAGTTAGACAAGGCCTATCAGTCTATAAGGTACCC TCTTAAACTCTTTCCATGTCTGTCGCAGCTGTTTCTAGGCATGTACCCAGAGGAGCAT TTCATTGAGAAGCCAGTGAAGGAAGCCATGATCCGATTCCGCAAGAACCTGGAGGC CATCGTCAGCGTGATCGCCGAGCGCAATAAGAACAAAAAGCTCCCCTACTACTACCT GTCACCAGACAGGATTCCCAACAGCGTAGCCATCTAAGGCCTTGCCTCCCTACCCAG

CAGCTCTCTGGGAAGGCCAGTGGCTTTATTAGCCAGATCCCAGCTTGCCTGGCAGGC
TCTGGGTCGATCTTCCTGCAGCTGGTGCCTCTTCCAAGCTCGAAGTGCTGCTCTTGGG
CCTAGGTGGTCTGGTTGAAACTGAAGGCTGTTGTAGGATGGGGAGACATCACAGAG
CCTCAGCATGTGCTACTTCTTCAGTGGACACAGTTGAGGAACCTCCCAGGCAGGGCA
GAGATGTGCAGCTGTGTCCCCCAGCCCAGCTCAGTGCCTCGTCACTCGGTAGCATCA
GAATAAGTGACAACTGTTCTGGCTGGGTCAGGGGTACTTTATTCTATTTATGCTTCCT
CCAATTGCTTGCATAGAGTAGGTGCTTAGAGAAAGTTCTTGGATTAAGAGTTTGTTA
TAAAATAAACTTCATTTAAAACAGGTGTCATACCACATGCTGAGGTCCAGTCACCCC
CACTCCCACCCCCACCAAAATCACTGTTCTCTTTCGATCAACAATAAAAAAGCTGGT
CTACTACCTCCTCCAACTGACAAGGTCTTTGCCCCCCACTCCATACCAGTGGCCCTTT
CTGCTTTTGTAGACAATACAGGTATTCTAAATTAAACACACAAATCTAAAGATCTGA
ATTTATGCTATGATTCATATATGAGAACACAT (SEQ ID NO. 75)

The screening laboratory **20** having both the endogenous DNA sequence, and the mutation DNA sequence can compare these elements to reveal the junction site in the mutant DNA sequence. These sequences are compared using a software program, such as Fasta Two Sequence Compare found at http://fasta.bioch.virginia.edu/fasta_www/cgi/search_frm2.cgi. These alignment show the screening laboratory **20** that the junction site where the mutation is inserted occurs at eight 108th nucleotide of the mutant designated genetic sequence.

Upon identification of the mutant designated genetic sequence and the junction site, two other software programs are utilized. The first of these programs is a blast program that identifies homologies between the designated genetic sequence and the endogenous genome of the mouse, as well as other species. The blast software can be found at http://www.ncbi.nlm.nih.gov/BLAST/.

The second of these programs is repeat masking program, such as Repeat Master Web Server found at http://www.repeatmasker.org/cgi-bin/WEBRepeatMasker. This program identifies areas in the designated genetic sequence that are highly repetitive, making them less than ideal locations to build a primer probe. If such areas are found in the designated genetic sequence they are masked by replacing the normal nucleotide designation A,C,G or T with the letter N or X.

Applied Biosystem's FileBuilder software program is then utilized to generate a gene expression assay. The FileBuilder software allows the screening laboratory **20** to identify the location inside the designated genetic sequence that is informative. The insertion of the pgk-neomycin cassette is at the $108^{th}$ nucleotide of the mutant designated genetic sequence. The FileBuilder software file with the 108th nucleotide designated as the target, is electronically transmitted to Applied Biosystems to generate Assays-by-Design order. Applied Biosystems will use a software program to identify primer and probe sequences that will detect this genetic condition. The software generates the following primers and probe.

| | |
|---|---|
| ***Forward Primer Seq.:*** | TTGGCTACCAGTTCCTGAATGG (SEQ ID NO. 2) |
| ***Reverse Primer Seq.:*** | CAGACTGCCTTGGGAAAAGC (SEQ ID NO. 3) |
| ***Probe:*** | CTGCAACCCAGTAATTC (SEQ ID NO. 4) |

The primers and probes will hybridized or anneal the following areas in the designated genetic sequence.

TGCCCAGCGGTCCTATCTAGAGGTCATTCTCTCCACAGAGCGAGTCAA GAACCACTGGCAGGAAGACCTCATGT**TTGGCTACCAGTTCCTGAATGGCTGCAAC CCAGTAATTC**TACCGGGTAGGGGAGGC**GCTTTTCCCAAGGCAGTCTG**GAGCATGC GCTTTAGCAGCCCCGCTGGCACTTGGCGCTACACAAGTGGCCTCTGGCCTCGCACAC ATTCCACATCCACCGGTAGCGCCAACCGGCTCCGTTCTTTGGTGGCCCCTTCGCGCCA CCTTCTACTCCTCCCCTAGTCAGGAAGTTCCCCCCCGCCCCGCAGCTCGCGTCGTGCA GGACGTGACAAATGGAAGTAGCACGTCTCACTAGTCTCGTGCAGATGGACAGCACC GCTGAGCAATGGAAGCGGGTAGGCCTTTGGGGCAGCGGCCAATAGCAGCTTTGCTC CTTCGCTTTCTGGGCTCAGAGGCTGGGAAGGGGTGGGTCCGGGGGCGGGCTCAGGG (SEQ ID NO. 1)

The genomic DNA nucleotides from the forward primer to the end of the reverse primer and all the bases in between, whether they hybridized to primer probe are not, are known as the target genetic sequence.

TGCCCAGCGGTCCTATCTAGAGGTCATTCTCTCCACAGAGCGAGTCAA GAACCACTGGCAGGAAGACCTCATGT**TTGGCTACCAGTTCCTGAATGGCTGCAAC CCAGTAATTCTACCGGGTAGGGGAGGCGCTTTTCCCAAGGCAGTCTG**GAGCATG CGCTTTAGCAGCCCCGCTGGCACTTGGCGCTACACAAGTGGCCTCTGGCCTCGCACA CATTCCACATCCACCGGTAGCGCCAACCGGCTCCGTTCTTTGGTGGCCCCTTCGCGCC ACCTTCTACTCCTCCCCTAGTCAGGAAGTTCCCCCCCGCCCCGCAGCTCGCGTCGTGC AGGACGTGACAAATGGAAGTAGCACGTCTCACTAGTCTCGTGCAGATGGACAGCAC CGCTGAGCAATGGAAGCGGGTAGGCCTTTGGGGCAGCGGCCAATAGCAGCTTTGCT CCTTCGCTTTCTGGGCTCAGAGGCTGGGAAGGGGTGGGTCCGGGGGCGGGCTCAGG

G (SEQ ID NO. 1)

A vendor, such as Applied Biosystems, will synthesize these Real-Time primer and probe sequences and send them to the screening laboratory **20.**

This large endogenous designated genetic sequence can be truncated for easier data handling. The smaller designated genetic sequence is a subset of nucleotides of the larger designated genetic sequence. The smaller designated genetic sequence contains the informative locations and nucleotides for the assay to be designed. The smaller designated genetic sequence contains the site where the endogenous DNA is disrupted by the pgk-neomycin insert. The 62nd nucleotide is where the disruption occurs in the endogenous DNA.

AAGAACCACTGGCAGGAAGACCTCATGTTTGGCTACCAGTTCCTGAAT GGCTGCAACCCAGTACTCATCAAGCGCTGCACAGCGTTGCCCCCGAAGCTCCCAGTG ACCACAGAGATGGTGGAGTGCAGCCTAGAGCGGCAGCTCAGTTTAGAACA (SEQ ID NO. 5)

Upon identification of the designated genetic sequence and junction site, two other software programs are utilized. The first of these programs is a blast program that identifies homologies between the designated genetic sequence and the endogenous genome of the mouse, as well as other species. The blast software can be found at

http://www.ncbi.nlm.nih.gov/BLAST/.

The second of these programs is repeat masking program, such as Repeat Master Web Server found at http://www.repeatmasker.org/cgi-bin/WEBRepeatMasker. This program identifies areas in the designated genetic sequence that are highly repetitive, making them less than ideal locations to build a primer probe. If such areas are found in the designated genetic sequence they are masked by replacing the normal nucleotide designation A,C,G or T with the letter N or X.

Applied Biosystem's FileBuilder software program is then utilized to generate a gene expression assay. The FileBuilder software allows the screening laboratory **20** to identify the location inside the designated genetic sequence that is informative. The insertion of the neomycin cassette in the designated genetic sequence would correspond to a target location of the 62nd nucleotide. The FileBuilder software file with the 62nd nucleotide designated as the target, is electronically transmitted to Applied Biosystems to generate Assays-by-Design order. Applied Biosystems will use a software program to identify primer and probe sequences that will detect this genetic condition. The software generates the following primers and probe.

| | |
|---|---|
| ***Forward Primer Seq.:*** | TTGGCTACCAGTTCCTGAATGG (SEQ ID NO. 6) |
| ***Reverse Primer Seq.:*** | CTGTGGTCACTGGGAGCTT (SEQ ID NO. 7) |
| ***Probe:*** | CTGCAACCCAGTACTCAT (SEQ ID NO. 8) |

The primers and probes will hybridized or anneal the following areas in the designated genetic sequence.

AAGAACCACTGGCAGGAAGACCTCATGT**TTGGCTACCAGTTCCTGAA**
**TGGCTGCAACCCAGTACTCAT**CAAGCGCTGCACAGCGTTGCCCCCG**AAGCTCCCA**
**GTGACCACAG**AGATGGTGGAGTGCAGCCTAGAGCGGCAGCTCAGTTTAGAACA
(SEQ ID NO. 5)

The genomic DNA nucleotides from the forward primer to the end of the reverse primer and all the bases in between, whether they hybridized to primer probe are not, are known as the target genetic sequence.

AAGAACCACTGGCAGGAAGACCTCATGT**TTGGCTACCAGTTCCTGAA**
**TGGCTGCAACCCAGTACTCATCAAGCGCTGCACAGCGTTGCCCCCGAAGCTCC**
**CAGTGACCACAG**AGATGGTGGAGTGCAGCCTAGAGCGGCAGCTCAGTTTAGAACA
(SEQ ID NO. 5)

A vendor, such as Applied Biosystems, will synthesize these Real-Time primer and probe sequences and send them to the screening laboratory **20.**

A biological sample in the form of a mouse bone marrow is submitted via FedEx (Memphis, TN) overnight delivery to the screening laboratory **20** from the remote user **1.** Each sample occupies one well of a **96** well source well container. A lysis reagent (made of 2.5 μl of Nuclei Lysing Solution (Promega Corporation, Madison, Wisconsin A7943) per sample)) is gently mixed and poured into a 25 ml trough or reservoir and is placed on the deck of a Tecan Genesis Workstation (Research Triangle Park, NC). The liquid handler dispenses 150 μl of the lysis reagent into each sample well of the source well container **2.** The well plate is then placed in a 55°C oven for three hours. The well plate is then placed back on the deck of the Tecan Genesis Workstation (Research Triangle Park, NC). The liquid handler aspirates 50 μl of each sample and dispenses it into a 384 well primary master well container (Fisher Scientific #NC9134044). Once all of the samples are transferred, the primary master well container is moved to the deck of the Isolation Purification Station **94.**

One hundred and twelve microliters of SV Lysis reagent (Promega Corporation, Madison WI, #Z305X) a chaotropic salt are added to each sample. Next, 13 μl of magnetic particles (Promegal Corporation, #A220X) are added and the well components are mixed. The well plate is then moved into the magnetic field of a magnet where the magnetic particles are drawn to the bottom of each well. The supernatant is then aspirated and discarded. The well plate is moved out of the magnetic field and 95 μl of SV Lysis reagent is added to each well and mixed. The well plate is then moved into the magnetic field and the supernatant is drawn off and discarded. This washing process is repeated two additional times. Next, the samples are washed four times in 130 μl of 95% ethanol as described above. After the fourth ethanol wash, the microwell container is placed on a 384 tip dryer for 11 minutes. Then the microwell container is moved back to the deck of the Isolation Purification Station **94** and 155 μl of Ambion's (Houston, TX) nuclease free water (catalog #B9934) is added to each well at room temperature. The plate is then moved into the magnetic field and 50 μl of DNA elution is transferred to a 384 well optical storage plate (Fisher Scientific, #08-772136) for optical density analysis. An $A_{260}$ reading of the storage plate read is performed with a Tecan Genios Spectrometer (Research Triangle Park, NC). This reading shows nucleic acid is present at the desired concentration of 0.2 O.D. units, but a range of 0.1 to 0.5 O.D. units is acceptable.

The primary master well plate with the isolated DNA is moved to the deck of a Tecan Freedom Workstation. The TaqMan Universal Master Mix, real time PCR primer mixture and Ambion water are placed on the deck as well. The final PCR mixture is made of 1x TaqMan Universal Master Mix (catalog #4326708), 1x real time PCR primer set/probe mix for the designated genetic sequence (Applied Biosystems Assays-by-Design (SM) Service 4331348) and 25% isolated DNA. The Tecan Genesis added the reagents together in the ABI 7900 384 Well Optical Plate. The plate is then sealed with optical sealing tape (ABI, #4311971).

The samples are then placed in an Applied Biosystems SDS HT7900. A standard real time PCR protocol is followed by heating the samples to 50°C for two minutes then incubated at 95°C for 10 minutes, followed by thermally cycling the samples 40 times between 95°C for 15 seconds and 60°C for one minute. The results are shown in Tables 2 and 3. On average, these results are transmitted to the remote user **1** within twenty-four hours of receiving the biological sample at the screening laboratory **20.**

TABLE 13

| Sample Name | Alox5 KO | RCN | Alox5 KO Result | Alox5 WT | RCN | Alox5 WT Result | Interpretation |
|---|---|---|---|---|---|---|---|
| 149198 | 0.032 | 0.018 | - | 9.397 | 9.634 | + | Sample is Wild Type |
| 149199 | 0.072 | 0.03 | - | 9.912 | 8.196 | + | Sample is Wild Type |
| 149200 | 0.015 | 0.025 | - | 7.513 | 9.054 | + | Sample is Wild Type |
| 149201 | 0.041 | 0.09 | - | 11.946 | 15.737 | + | Sample is Wild Type |
| 149202 | 0.03 | 0.037 | - | 7.897 | 6.941 | + | Sample is Wild Type |
| 149203 | 0.044 | 0.013 | - | 10.855 | 13.577 | + | Sample is Wild Type |
| 149204 | 0.032 | 0.131 | - | 9.29 | 10.966 | + | Sample is Wild Type |
| 149205 | 2.693 | 2.901 | + | 0.028 | 0.155 | - | Sample is Homozygous |
| 149206 | 2.753 | 2.702 | + | 0.011 | 0.376 | - | Sample is Homozygous |
| 149207 | 3.027 | 3.424 | + | 0.303 | 0.254 | - | Sample is Homozygous |

SEQUENCE LISTING

<110> TransnetYX, Inc. Hodge, Timothy A.

<120> METHODS FOR GENOTYPE SCREENING

<130> 023131.41500

<150> 11/166,990
<151> 2005-06-24

<160> 75

<170> PatentIn version 3.3

<210> 1
<211> 499
<212> DNA
<213> Mus sp.

<400> 1

```
tgcccagcgg tcctatctag aggtcattct ctccacagag cgagtcaaga accactggca      60
ggaagacctc atgtttggct accagttcct gaatggctgc aacccagtaa ttctaccggg     120
taggggaggc gcttttccca aggcagtctg gagcatgcgc tttagcagcc ccgctggcac     180
ttggcgctac acaagtggcc tctggcctcg cacacattcc acatccaccg gtagcgccaa     240
ccggctccgt tctttggtgg ccccttcgcg ccaccttcta ctcctcccct agtcaggaag     300
ttcccccccg ccccgcagct cgcgtcgtgc aggacgtgac aaatggaagt agcacgtctc     360
actagtctcg tgcagatgga cagcaccgct gagcaatgga agcgggtagg cctttggggc     420
agcggccaat agcagctttg ctccttcgct ttctgggctc agaggctggg aaggggtggg     480
tccgggggcg ggctcaggg                                                  499
```

<210> 2
<211> 22
<212> DNA
<213> Mus sp.

<220>
<221> misc_feature
<222> (1)..(22)
<223> Forward Primer

<400> 2
ttggctacca gttcctgaat gg 22

<210> 3
<211> 20
<212> DNA
<213> Mus sp.

<220>
<221> misc_feature
<222> (1)..(20)
<223> Reverse Primer

<400> 3
cagactgcct tgggaaaagc 20

<210> 4
<211> 17
<212> DNA
<213> Mus sp.

<220>
<221> misc_feature
<222> (1)..(17)
<223> Probe

<400> 4
ctgcaaccca gtaattc 17

<210> 5
<211> 155
<212> DNA
<213> Mus sp.

<400> 5

aagaaccact ggcaggaaga cctcatgttt ggctaccagt tcctgaatgg ctgcaaccca 60
gtactcatca agcgctgcac agcgttgccc ccgaagctcc cagtgaccac agagatggtg 120
gagtgcagcc tagagcggca gctcagttta gaaca 155

<210> 6
<211> 22
<212> DNA
<213> Mus sp.

<220>
<221> misc_feature
<222> (1)..(22)
<223> Forward Primer

<400> 6
ttggctacca gttcctgaat gg 22

<210> 7
<211> 19
<212> DNA
<213> Mus sp.

<220>
<221> misc_feature
<222> (1)..(19)
<223> Reverse Primer

<400> 7
ctgtggtcac tgggagctt 19

<210> 8
<211> 18
<212> DNA
<213> Mus sp.

<220>
<221> misc_feature
<222> (1)..(18)
<223> Probe

<400> 8
ctgcaaccca gtactcat 18

<210> 9
<211> 686
<212> DNA
<213> Mus sp.

<220>
<221> misc_feature
<222> (561)..(585)
<223> n is a, c, g, or t

<400> 9

tatcatgtct cccggctcaa gtctgccatc ccttcacgtt aggaaacaga aagctctaga 60
agctgagcta gatgctcagc atttatcaga aaccttcgac aacattgaca acctaagtcc 120
caaggcctct caccggagta agcagagaca caagcagaat ctttatggtg actatgcttt 180
tgacgccaat cgacatgatg atagtaggtc agacaatttc aatactggaa acatgactgt 240
tctttcacca tatttaaata ctacggtatt gcccagctct tcttcctcaa ggggaagttt 300
agacagttct cgttctgaga aagacagaag ttaggagaga gagcgaggta ttggcctcag 360
tgcttaccat ccaacaacag aaaatgcagg aacctcatca aaacgaggtc tgcagatcac 420
taccactgca gcccagatag ccaaagttat ggaagaagta tcagccattc atacctccca 480
ggacgacaga agttctgctt ctaccaccga gttccattgt gtggcagacg acaggagtgc 540
ggcacgaaga agctctgcct nnnnnnnnnn nnnnnnnnnn nnnnncttca ctaagtcgga 600
aaattcaaat aggacatgct ctatgcctta tgccaaagtg gaatataaac gatcttcaaa 660
tgacagttta aatagtgtca ctagta 686

<210> 10
<211> 25
<212> DNA
<213> Mus sp.

<220>
<221> misc_feature
<222> (1)..(25)
<223> Forward Primer

<400> 10
gggaagttta gacagttctc gttct 25

<210> 11
<211> 22
<212> DNA
<213> Mus sp.

<220>
<221> misc_feature
<222> (1)..(22)

<223> Reverse Primer

<400> 11
gtaagcactg aggccaatac ct 22

<210> 12
<211> 15
<212> DNA
<213> Mus sp.

<220>
<221> misc_feature
<222> (1)..(15)
<223> Probe 1

<400> 12
ctctctccaa acttc 15

<210> 13
<211> 16
<212> DNA
<213> Mus sp.

<220>
<221> misc_feature
<222> (1)..(16)
<223> Probe 2

<400> 13
tctctctcct aacttc 16

<210> 14
<211> 181
<212> DNA
<213> Mus sp.

<400> 14

```
gttgagaatg agtacgggtc ctactttgcc tgcgattacg actacctacg cttcctggtg     60
caccgcttcc gctaccatct gggtaatgac gtcattctct tcaccaccga cggagcaagt    120
gaaaaaatgc tgaagtgtgg gaccctgcag gacctgtacg ccacagtgga ttttggaaca    180
g                                                                    181
```

<210> 15
<211> 18
<212> DNA
<213> Mus sp.

<220>
<221> misc_feature
<222> (1)..(18)
<223> Forward primer

<400> 15
caccgcttcc gctaccat 18

<210> 16
<211> 18
<212> DNA
<213> Mus sp.

<220>
<221> misc_feature
<222> (1)..(18)
<223> Reverse Primer

<400> 16
gctccgtcgg tggtgaag 18

<210> 17
<211> 20
<212> DNA
<213> Mus sp.

<220>
<221> misc_feature
<222> (1)..(20)
<223> Probe

<400> 17
ctgggtaatg acgtcattct 20

<210> 18
<211> 2205
<212> DNA
<213> Mus sp.

<400> 18

```
gaccggtaac aagtggccgg gagcgaactt ttgcaaatct cttctgcgcc ttaaggctgc      60
caccgagact gtaaagaaaa gggagaagag gaacctatac tcataccagt tcgcacaggc     120
ggctgaagtt gggcgagcgc tagccgcggc tgcctagcgt ccccctcccc ctcacagcgg     180
aggaggggac agttgtcgga ggccgggcgg cagagcccga tcgcgggctt ccaccgagaa     240
ttccgtgacg actggtcagc accgccggag agccgctgtt gctgggactg gtctgcgggc     300
tccaaggaac cgctgctccc cgagagcgct ccgtgagtga ccgcgacttt tcaaagctcg     360
gcatcgcgcg ggagcctacc aacgtgagtg ctagcggagt cttaaccctg cgctccctgg     420
agcgaactgg ggaggagggc tcagggggaa gcactgccgt ctggagcgca cgctcctaaa     480
caaactttgt tacagaagcg gggacgcgcg ggtatccccc cgcttcccgg cgcgctgttg     540
cggccccgaa acttctgcgc acagcccagg ctaaccccgc gtgaagtgac ggaccgttct     600
atgactgcaa agatggaaac gaccttctac gacgatgccc tcaacgcctc gttcctccag     660
tccgagagcg gtgcctacgg ctacagtaac cctaagatcc taaaacagag catgaccttg     720
aacctggccg acccggtggg cagtctgaag ccgcacctcc gcgccaagaa ctcggacctt     780
ctcacgtcgc ccgacgtcgg gctgctcaag ctggcgtcgc cggagctgga gcgcctgatc     840
```

atccagtcca gcaatgggca catcaccact acaccgaccc ccacccagtt cttgtgcccc 900

aagaacgtga ccgacgagca ggagggcttc gccgagggct tcgtgcgcgc cctggctgaa 960

ctgcatagcc agaacacgct tcccagtgtc acctccgcgg cacagccggt cagcggggcg 1020

ggcatggtgg ctcccgcggt ggcctcagta gcaggcgctg gcggcggtgg tggctacagc 1080

gccagcctgc acagtgagcc tccggtctac gccaacctca gcaacttcaa cccgggtgcg 1140

ctgagcagcg gcggtggggc gccctcctat ggcgcggccg ggctggcctt tccctcgcag 1200

ccgcagcagc agcagcagcc gcctcagccg ccgcaccact tgccccaaca gatcccggtg 1260

cagcacccgc ggctgcaagc cctgaaggaa gagccgcaga ccgtgccgga gatgccggga 1320

gagacgccgc ccctgtcccc tatcgacatg gagtctcagg agcggatcaa ggcagagagg 1380

aagcgcatga ggaaccgcat tgccgcctcc aagtgccgga aaaggaagct ggagcggatc 1440

gctcggctag aggaaaaagt gaaaaccttg aaagcgcaaa actccgagct ggcatccacg 1500

gccaacatgc tcagggaaca ggtggcacag cttaagcaga aagtcatgaa ccacgttaac 1560

agtgggtgcc aactcatgct aacgcagcag ttgcaaacgt tttgagaaca gactgtcagg 1620

gctgaggggc aatggaagaa aaaaaataac agagacaaac ttgagaactt gactggttgc 1680

gacagagaaa aaaaaagtgt ccgagtactg aagccaaggg tacacaagat ggactgggtt 1740

gcgacctgac ggcgccccca gtgtgctgga gtgggaagga cgtggcgcgc ctggctttgg 1800

cgtggagcca gagagcagcg gcctattggc cggcagactt tgcggacggg ctgtgcccgc 1860

gcgcgaccag aacgatggac ttttcgttaa cattgaccaa gaactgcatg gacctaacat 1920

tcgatctcat tcagtattaa agggggtgg gaggggttac aaactgcaat agagactgta 1980

gattgcttct gtagtgctcc ttaacacaaa gcagggaggg ctgggaaggg gggggaggct 2040

tgtaagtgcc aggctagact gcagatgaac tcccctggcc tgcctctctc aactgtgtat 2100

gtacatatat attttttttt aatttgatga aagctgatta ctgtcaataa acagcttcct 2160

gcctttgtaa gttattccat gtttgtttgt ttgggtgtcc tgccc 2205

<210> 19
<211> 22
<212> DNA
<213> Mus sp.

<220>
<221> misc_feature
<222> (1)..(22)
<223> Forward Primer

<400> 19
gagtgctagc ggagtcttaa cc 22

<210> 20
<211> 18
<212> DNA
<213> Mus sp.

<220>
<221> misc_feature
<222> (1)..(18)
<223> Reverse Primer

<400> 20
ctccagacgg cagtgctt 18

<210> 21
<211> 18
<212> DNA
<213> Mus sp.

<220>
<221> misc_feature
<222> (1)..(18)
<223> Probe

<400> 21
aagcactgcc gtctggag 18

<210> 22
<211> 1091
<212> DNA
<213> Mus sp.

<400> 22

atgcccaaga agaagaggaa ggtgtccaat ttactgaccg tacaccaaaa tttgcctgca 60
ttaccggtcg atgcaacgag tgatgaggtt cgcaagaacc tgatggacat gttcagggat 120
cgccaggcgt tttctgagca tacctggaaa atgcttctgt ccgtttgccg gtcgtgggcg 180
gcatggtgca agttgaataa ccggaaatgg tttcccgcag aacctgaaga tgttcgcgat 240
tatcttctat atcttcaggc gcgcggtctg gcagtaaaaa ctatccagca acatttgggc 300
cagctaaaca tgcttcatcg tcggtccggg ctgccacgac caagtgacag caatgctgtt 360
tcactggtta tgcggcggat ccgaaaagaa aacgttgatg ccggtgaacg tgcaaaacag 420
gctctagcgt tcgaacgcac tgatttcgac caggttcgtt cactcatgga aaatagcgat 480
cgctgccagg atatacgtaa tctggcattt ctggggattg cttataacac cctgttacgt 540
atagccgaaa ttgccaggat cagggttaaa gatatctcac gtactgacgg tgggagaatg 600
ttaatccata ttggcagaac gaaaacgctg gttagcaccg caggtgtaga gaaggcactt 660
agcctggggg taactaaact ggtcgagcga tggatttccg tctctggtgt agctgatgat 720
ccgaataact acctgttttg ccgggtcaga aaaaatggtg ttgccgcgcc atctgccacc 780
agccagctat caactcgcgc cctggaaggg atttttgaag caactcatcg attgatttac 840
ggcgctaagg atgactctgg tcagagatac ctggcctggt ctggacacag tgcccgtgtc 900
ggagccgcgc gagatatggc ccgcgctgga gtttcaatac cggagatcat gcaagctggt 960
ggctggacca atgtaaatat tgtcatgaac tatatccgta acctggatag tgaaacaggg 1020
gcaatggtgc gcctgctgga agatggcgat tagccattaa cgcgtaaatg attgctataa 1080

ttatttgata t 1091

<210> 23
<211> 27
<212> DNA
<213> Mus sp.

<220>
<221> misc_feature
<222> (1)..(27)
<223> Forward Primer

<400> 23
ttaatccata ttggcagaac gaaaacg 27

<210> 24
<211> 22
<212> DNA
<213> Mus sp.

<220>
<221> misc_feature
<222> (1)..(22)
<223> Reverse Primer

<400> 24
caggctaagt gccttctcta ca 22

<210> 25
<211> 15
<212> DNA
<213> Mus sp.

<220>
<221> misc_feature
<222> (1)..(15)
<223> Probe

<400> 25
cctgcggtgc taacc 15

<210> 26
<211> 205
<212> DNA
<213> Homo sapiens

<400> 26

aaagaagagc agcacgtcat acccaagacc aacatctctc agtgtttcac gctaacccaa 60
ggagagacac tagcagtctt ctctgcagga ccccttgaat ttacattgaa ttccatcccc 120
agccgagcag gtgcttaaag tcaacagggg acactccatt ttcttggaat ttcattctgg 180
caaagagggt gtgagcagca ataag 205

<210> 27
<211> 25
<212> DNA
<213> Homo sapiens

<220>
<221> misc_feature
<222> (1)..(25)
<223> Forward Primer

<400> 27
gcaggacccc ttgaatttac attga 25

<210> 28
<211> 20
<212> DNA
<213> Homo sapiens

<220>
<221> misc_feature
<222> (1)..(20)
<223> Reverse Primer

<400> 28
tggagtgtcc cctgttgact 20

<210> 29
<211> 16
<212> DNA
<213> Homo sapiens

<220>
<221> misc_feature
<222> (1)..(16)
<223> Probe

<400> 29
ccgagcaggt gcttaa 16

<210> 30
<211> 1026
<212> DNA
<213> Mus sp.

<400> 30

atgaaaaagc ctgaactcac cgcgacgtct gtcgagaagt ttctgatcga aaagttcgac 60
agcgtctccg acctgatgca gctctcggag ggcgaagaat ctcgtgcttt cagcttcgat 120
gtaggagggc gtggatatgt cctgcgggta aatagctgcg ccgatggttt ctacaaagat 180
cgttatgttt atcggcactt tgcatcggcc gcgctcccga ttccggaagt gcttgacatt 240
ggggaattca gcgagagcct gacctattgc atctcccgcc gtgcacaggg tgtcacgttg 300
caagacctgc ctgaaaccga actgcccgct gttctgcagc cggtcgcgga ggccatggat 360
gcgatcgctg cggccgatct tagccagacg agcgggttcg gcccattcgg accgcaagga 420
atcggtcaat acactacatg gcgtgatttc atatgcgcga ttgctgatcc ccatgtgtat 480
cactggcaaa ctgtgatgga cgacaccgtc agtgcgtccg tcgcgcaggc tctcgatgag 540
ctgatgcttt gggccgagga ctgccccgaa gtccggcacc tcgtgcacgc ggatttcggc 600
tccaacaatg tcctgacgga caatggccgc ataacagcgg tcattgactg gagcgaggcg 660
atgttcgggg attcccaata cgaggtcgcc aacatcttct tctggaggcc gtggttggct 720
tgtatggagc agcagacgcg ctacttcgag cggaggcatc cggagcttgc aggatcgccg 780
cggctccggg cgtatatgct ccgcattggt cttgaccaac tctatcagag cttggttgac 840
ggcaatttcg atgatgcagc ttgggcgcag ggtcgatgcg acgcaatcgt ccgatccgga 900
gccgggactg tcgggcgtac acaaatcgcc cgcagaagcg cggccgtctg gaccgatggc 960
tgtgtagaag tactcgccga tagtggaaac cgacgcccca gcactcgtcc gagggcaaag 1020
gaatag 1026

<210> 31
<211> 24
<212> DNA
<213> Mus sp.

<220>
<221> misc_feature
<222> (1)..(24)
<223> Forward Promer

<400> 31
cgcaaggaat cggtcaatac acta 24

<210> 32
<211> 24
<212> DNA
<213> Mus sp.

<220>
<221> misc_feature
<222> (1)..(24)
<223> Reverse Primer

<400> 32
cacagtttgc cagtgataca catg 24

<210> 33
<211> 16
<212> DNA
<213> Mus sp.

<220>
<221> misc_feature
<222> (1)..(16)
<223> Probe

<400> 33
catggcgtga tttcat 16

<210> 34
<211> 31357
<212> DNA
<213> Mus sp.

<400> 34

```
tataagagtg attggcgtcc gtacgtaccc tctcaactct aaaactcttg tagtttaaat    60
ctaatctaaa ctttataaac ggcacttcct gcgtgtccat gcccgcgggc ctggtcttgt   120
catagtgctg acatttgtag ttccttgact ttcgttctct gccagtgacg tgtccattcg   180
gcgccagcag cccacccata ggttgcataa tggcaaagat gggcaaatac ggtctcggct   240
tcaaatgggc cccagaattt ccatggatgc ttccgaacgc atcggagaag ttgggtaacc   300
ctgagaggtc agaggaggat gggttttgcc cctctgctgc gcaagaaccg aaagttaaag   360
gaaaaacttt ggttaatcac gtgagggtga attgtagccg gcttccagct ttggaatgct   420
gtgttcagtc tgccataatc cgtgatattt ttgtagatga ggatccccag aaggtggagg   480
cctcaactat gatggcattg cagttcggta gtgccgtctt ggttaagcca tccaagcgct   540
tgtctattca ggcatggact aatttgggtg tgcttcccaa aacagctgcc atggggttgt   600
tcaagcgcgt ctgcctgtgt aacaccaggg agtgctcttg tgacgcccac gtggcctttc   660
acctttttac ggtccaaccc gatggtgtat gcctgggtaa tggccgtttt ataggctggt   720
tcgttccagt cacagccata ccggagtatg cgaagcagtg gttgcaaccc tggtccatcc   780
ttcttcgtaa gggtggtaac aaagggtctg tgacatccgg ccacttccgc cgcgctgtta   840
ccatgcctgt gtatgacttt aatgtagagg atgcttgtga ggaggttcat cttaacccga   900
agggtaagta ctcctgcaag gcgtatgctc ttcttaaggg ctatcgcggt gttaagccca   960
tcctgtttgt ggaccagtat ggttgcgact atactggatg tctcgccaag ggtcttgagg  1020
actatggcga tctcaccttg agtgagatga aggagttgtt ccctgtgtgg cgtgactcct  1080
tggatagtga agtccttgtg gcttggcacg ttgatcgaga tcctcgggct gctatgcgtc  1140
tgcagactct tgctactgta cgttgcattg attatgtggg ccaaccgacc gaggatgtgg  1200
tggatggaga tgtggtagtg cgtgagcctg ctcatcttct cgcagccaat gccattgtta  1260
aaagactccc ccgtttggtg gagactatgc tgtatacgga ttcgtccgtt acagaattct  1320
gttataaaac caagctgtgt gaatgcggtt ttatcacgca gtttggctat gtggattgtt  1380
gtggtgacac ctgcgatttt cgtgggtggg ttgccggcaa tatgatggat ggctttccat  1440
gtccagggtg taccaaaaat tatatgccct gggaattgga ggcccagtca tcaggtgtta  1500
taccagaagg aggtgttcta ttcactcaga gcactgatac agtgaatcgt gagtccttta  1560
agctctacgg tcatgctgtt gtgccttttg gttctgctgt gtattggagc ccttgcccag  1620
gtatgtggct tccagtaatt tggtcttctg ttaagtcata ctctggtttg acttatacag  1680
gagtagttgg ttgtaaggca attgttcaag agacagacgc tatatgtcgt tctctgtata  1740
tggattatgt ccagcacaag tgtggcaatc tcgagcagag agctatcctt ggattggacg  1800
atgtctatca tagacagttg cttgtgaata ggggtgacta tagtctcctc cttgagaatg  1860
tggatttgtt tgttaagcgg cgcgctgaat ttgcttgcaa attcgccacc tgtggagatg  1920
gtcttgtacc cctcctacta gatggtttag tgccccgcag ttattatttg attaagagtg  1980
```

gtcaagcttt cacctctatg atggttaatt ttagccatga ggtgactgac atgtgtatgg 2040
acatggcttt attgttcatg catgatgtta aagtggccac taagtatgtt aagaaggtta 2100
ctggcaaact ggccgtgcgc tttaaagcgt tgggtgtagc cgttgtcaga aaaattactg 2160
aatggtttga tttagccgtg gacattgctg ctagtgccgc tggatggctt tgctaccagc 2220
tggtaaatgg cttatttgca gtggccaatg gtgttataac ctttgtacag gaggtgcctg 2280
agcttgtcaa gaattttgtt gacaagttca aggcattttt caaggttttg atcgactcta 2340
tgtcggtttc tatcttgtct ggacttactg ttgtcaagac tgcctcaaat agggtgtgtc 2400
ttgctggcag taaggtttat gaagttgtgc agaaatcttt gtctgcatat gttatgcctg 2460
tgggttgcag tgaagccact tgtttggtgg gtgagattga acctgcagtt tttgaagatg 2520
atgttgttga tgtggttaaa gccccattaa catatcaagg ctgttgtaag ccacccactt 2580
ctttcgagaa gatttgtatt gtggataaat tgtatatggc caagtgtggt gatcaatttt 2640
accctgtggt tgttgataac gacactgttg gcgtgttaga tcagtgctgg aggtttccct 2700
gtgcgggcaa gaaagtcgag tttaacgaca agcccaaagt caggaagata ccctccaccc 2760
gtaagattaa gatcaccttc gcactggatg cgacctttga tagtgttctt tcgaaggcgt 2820
gttcagagtt tgaagttgat aaagatgtta cattggatga gctgcttgat gttgtgcttg 2880
acgcagttga gagtacgctc agcccttgta aggagcatga tgtgataggc acaaaagttt 2940
gtgctttact tgataggttg gcaggagatt atgtctatct ttttgatgag ggaggcgatg 3000
aagtgatcgc cccgaggatg tattgttcct tttctgctcc tgatgatgaa gactgcgttg 3060
cagcggatgt tgtagatgca gatgaaaacc aagatgatga tgctgaagac tcagcagtcc 3120
ttgtcgctga tacccaagaa gaggacggcg ttgccaaggg gcaggttgag gcggattcgg 3180
aaatttgcgt tgcgcatact ggtagtcaag aagaattggc tgagcctgat gctgtcggat 3240
ctcaaactcc catcgcctct gctgaggaaa ccgaagtcgg agaggcaagc gacagggaag 3300
ggattgctga ggcgaaggca actgtgtgtg ctgatgctgt agatgcctgc cccgatcaag 3360
tggaggcatt tgaaattgaa aaggttgaag actctatctt ggatgagctt caaactgaac 3420
ttaatgcgcc agcggacaag acctatgagg atgtcttggc attcgatgcc gtatgctcag 3480
aggcgttgtc tgcattctat gctgtgccga gtgatgagac gcactttaaa gtgtgtggat 3540
tctattcgcc tgctatagag cgcactaatt gttggctgcg ttctactttg atagtaatgc 3600
agagtctacc tttggaattt aaagacttgg agatgcaaaa gctctggttg tcttacaagg 3660
ccggctatga ccaatgcttt gtggacaaac tagttaagag cgtgcccaag tctattatcc 3720
ttccacaagg tggttatgtg gcagattttg cctatttctt tctaagccag tgtagcttta 3780
aagcttatgc taactggcgt tgtttagagt gtgacatgga gttaaagctt caaggcttgg 3840
acgccatgtt tttctatggg gacgttgtgt ctcatatgtg caagtgtggt aatagcatga 3900
ccttgttgtc tgcagatata ccctacactt tgcattttgg agtgcgagat gataagtttt 3960
gcgcttttta cacgccaaga aaggtcttta gggctgcttg tgcggtagat gttaatgatt 4020

gtcactctat ggctgtagta gagggcaagc aaattgatgg taaagtggtt accaaattta 4080
ttggtgacaa atttgatttt atggtgggtt acgggatgac atttagtatg tctccttttg 4140
aactcgccca gttatatggt tcatgtataa caccaaatgt ttgttttgtt aaaggagatg 4200
ttataaaggt tgttcgctta gttaatgctg aagtcattgt taaccctgct aatgggcgta 4260
tggctcatgg tgcaggtgtt gcaggtgcta tagctgaaaa ggcgggcagt gcttttatta 4320
aagaaacctc cgatatggtg aaggctcagg gcgtttgcca ggttggtgaa tgctatgaat 4380
ctgccggtgg taagttatgt aaaaaggtgc ttaacattgt agggccagat gcgcgagggc 4440
atggcaagca atgctattca cttttagagc gtgcttatca gcatattaat aagtgtgaca 4500
atgttgtcac tactttaatt tcggctggta tatttagtgt gcctactgat gtctccctaa 4560
cttacttact tggtgtagtg acaaagaatg tcattcttgt cagtaacaac caggatgatt 4620
ttgatgtgat agagaagtgt caggtgacct ccgttgctgg taccaaagcg ctatcacttc 4680
aattggccaa aaatttgtgc cgtgatgtaa agtttgtgac gaatgcatgt agttcgcttt 4740
ttagtgaatc ttgctttgtc tcaagctatg atgtgttgca ggaagttgaa gcgctgcgac 4800
atgatataca attggatgat gatgctcgtg tctttgtgca ggctaatatg gactgtctgc 4860
ccacagactg gcgtctcgtt aacaaatttg atagtgttga tggtgttaga accattaagt 4920
attttgaatg cccgggcggg atttttgtat ccagccaggg caaaaagttt ggttatgttc 4980
agaatggttc atttaaggag gcgagtgtta gccaaataag ggctttactc gctaataagg 5040
ttgatgtctt gtgtactgtt gatggtgtta acttccgctc ctgctgcgta gcagagggtg 5100
aagtttttgg caagacatta ggttcagtct tttgtgatgg cataaatgtc accaaagtta 5160
ggtgtagtgc catttacaag ggtaaggttt tctttcagta cagtgatttg tccgaggcag 5220
atcttgtggc tgttaaagat gcctttggtt ttgatgaacc acaactgctg aagtactaca 5280
ctatgcttgg catgtgtaag tggtcagtag ttgtttgtgg caattatttt gctttcaagc 5340
agtcaaataa taattgctat ataaatgtgg catgtttaat gctgcaacac ttgagtttaa 5400
agtttcctaa gtggcaatgg caagaggctt ggaacgagtt ccgctctggt aaaccactaa 5460
ggtttgtgtc cttggtatta gcaaagggca gctttaaatt taatgaacct tctgattcta 5520
tcgattttat gcgtgtggtg ctacgtgaag cagatttgag tggtgccacg tgcaatttgg 5580
aatttgtttg taaatgtggt gtgaagcaag agcagcgcaa aggtgttgac gctgttatgc 5640
attttggtac gttggataaa ggtgatcttg tcaggggtta taatatcgca tgtacgtgcg 5700
gtagtaaact tgtgcattgc acccaattta acgtaccatt tttaatttgc tccaacacac 5760
cagagggtag gaaactgccc gacgatgttg ttgcagctaa tatttttact ggtggtagtg 5820
tgggccatta cacgcatgtg aaatgtaaac ccaagtacca gctttatgat gcttgtaatg 5880
ttaataaggt ttcggaggct aagggtaatt ttaccgattg cctctacctt aaaaatttaa 5940
agcaaacttt ttcgtctgtg ctgacgactt tttatttaga tgatgtaaag tgtgtggagt 6000

ataagccaga tttatcgcag tattactgtg agtctggtaa atattataca aaacccatta 6060
ttaaggccca atttagaaca tttgagaagg ttgatggtgt ctataccaac tttaaattgg 6120
tgggacatag tattgctgaa aaactcaatg ctaagctggg atttgattgt aattctccct 6180
ttgtggagta taaaattaca gagtggccaa cagctactgg agatgtggtg ttggctagtg 6240
atgatttgta tgtaagtcgg tactcaagcg ggtgcattac ttttggtaaa ccggttgtct 6300
ggcttggcca tgaggaagca tcgctgaaat ctctcacata ttttaataga cctagtgtcg 6360
tttgtgaaaa taaatttaat gtgttgcccg ttgatgtcag tgaacccacg gacaaggggc 6420
ctgtgcctgc tgcagtcctt gttaccggcg tccctggagc tgatgcgtca gctggtgccg 6480
gtattgccaa ggagcaaaaa gcctgtgctt ctgctagtgt ggaggatcag gttgttacgg 6540
aggttcgtca agagccatct gtttcagctg ctgatgtcaa agaggttaaa ttgaatggtg 6600
ttaaaaagcc tgttaaggtg gaaggtagtg tggttgttaa tgatcccact agcgaaacca 6660
aagttgttaa aagtttgtct attgttgatg tctatgatat gttcctgaca gggtgtaagt 6720
atgtggtttg gactgctaat gagttgtctc gactagtaaa ttcaccgact gttagggagt 6780
atgtgaagtg gggtaaggga aagattgtaa cacccgctaa gttgttgttg ttaagagatg 6840
agaagcaaga gttcgtagcg ccaaaagtag tcaaggcgaa agctattgcc tgctattgtg 6900
ctgtgaagtg gtttctcctc tattgtttta gttggataaa gtttaatact gataataagg 6960
ttatatacac cacagaagta gcttcaaagc ttactttcaa gttgtgctgt ttggccttta 7020
agaatgcctt acagacgttt aattggagcg ttgtgtctag ggctttttc ctagttgcaa 7080
cggtcttttt attatggttt aacttttgt atgctaatgt tattttgagt gacttctatt 7140
tgcctaatat tgggcctctc cctacgtttg tgggacagat agttgcgtgg tttaagacta 7200
catttggtgt gtcaaccatc tgtgatttct accaggtgac ggatttgggc tatagaagtt 7260
cgttttgtaa tggaagtatg gtatgtgaac tatgcttctc aggttttgat atgctggaca 7320
actatgatgc tataaatgtt gttcaacacg ttgtagatag gcgtttgtcc tttgactata 7380
ttagcctatt taaattagta gttgagcttg taatcggcta ctctctttat actgtgtgct 7440
tctacccact gtttgtcctt attggaatgc agttgttgac cacatggttg cctgaattct 7500
ttatgctgga gactatgcat tggagtgctc gtttgtttgt gtttgttgcc aatatgcttc 7560
cagcttttac gttactgcga ttttacatcg tggtgacagc tatgtataag gtctattgtc 7620
tttgtagaca tgttatgtat ggatgtagta agcctggttg cttgttttgt tataagagaa 7680
accgtagtgt ccgtgttaag tgtagcaccg ttgttggtgg ttcactacgc tattacgatg 7740
taatggctaa cggcggcaca ggtttctgta caaagcacca gtggaactgt cttaattgca 7800
attcctggaa accaggcaat acattcataa ctcatgaagc agcggcggac ctctctaagg 7860
agttgaaacg ccctgtgaat ccaacagatt ctgcttatta ctcggtcaca gaggttaagc 7920
aggttggttg ttccatgcgt ttgttctacg agagagatgg acagcgtgtt tatgatgatg 7980
ttaatgctag tttgtttgtg gacatgaatg gtctgctgca ttctaaagtt aaaggtgtgc 8040

ctgaaacgca tgttgtggtt gttgagaatg aagctgataa agctggtttt ctcggcgccg 8100
cagtgtttta tgcacaatcg ctctacagac ctatgttgat ggtggaaaag aaattaataa 8160
ctaccgccaa cactggtttg tctgttagtc gaactatgtt tgacctttat gtagattcat 8220
tgctgaacgt cctcgacgtg gatcgcaaga gtctaacaag ttttgtaaat gctgcgcaca 8280
actctctaaa ggagggtgtt cagcttgaac aagttatgga tacctttatt ggctgtgccc 8340
gacgtaagtg tgctatagat tctgatgttg aaaccaagtc tattaccaag tccgtcatgt 8400
cggcagtaaa tgctggcgtt gattttacgg atgagagttg taataacttg gtgcctacct 8460
atgttaaaag tgacactatc gttgcagccg atttgggtgt tcttattcag aataatgcta 8520
agcatgtaca ggctaatgtt gctaaagccg ctaatgtggc ttgcatttgg tctgtggatg 8580
cttttaacca gctatctgct gacttacagc ataggctgcg aaaagcatgt tcaaaaactg 8640
gcttgaagat taagcttact tataataagc aggaggcaaa tgttcctatt ttaactacac 8700
cgttctctct taaagggggc gctgttttta gtagaatgtt acaatggttg tttgttgcta 8760
atttgatttg tttcattgtg ttgtgggccc ttatgccaac atatgcagtg cacaaatcgg 8820
atatgcagtt gcctttatat gccagtttta aagttataga taatggtgtg ctaagggatg 8880
tgtctgttac tgacgcatgc ttcgcaaaca aatttaatca atttgatcaa tggtatgagt 8940
ctacttttgg tcttgcttat taccgcaact ctaaggcttg tcctgttgtg gttgctgtaa 9000
tagatcaaga cattggccat accttattta atgttcctac cacagtttta agatatggat 9060
ttcatgtgtt gcattttata acccatgcat ttgctactga tagcgtgcag tgttacacgc 9120
cacatatgca aatcccctat gataatttct atgctagtgg ttgcgtgttg tcatccctct 9180
gtactatgct tgcgcatgca gatggaaccc cgcatcctta ttgttataca gggggtgtta 9240
tgcacaatgc ctctctgtat agttctttgg ctcctcatgt ccgttataac ctggctagtt 9300
caaatggtta tatacgtttt cccgaagtgg ttagtgaagg cattgtgcgt gttgtgcgca 9360
ctcgctctat gacctactgc agggttggtt tatgtgagga ggccgaggag ggtatctgct 9420
ttaattttaa tcgttcatgg gtattgaaca acccgtatta tagggccatg cctggaactt 9480
tttgtggtag gaatgctttt gatttaatac atcaagtttt aggaggatta gtgcggccta 9540
ttgatttctt tgccttaacg gcgagttcag tggctggtgc tatccttgca attattgtcg 9600
ttttggcttt ctattattta ataaagctta aacgtgcctt tggtgactac actagtgttg 9660
tggttatcaa tgtaattgtg tggtgtataa attttctgat gctttttgtg tttcaggttt 9720
atcccacatt gtcttgttta tatgcttgtt tttatttcta cacaacgctt tatttccctt 9780
cggagataag tgttgttatg catttgcaat ggcttgtcat gtatggtgct attatgccct 9840
tgtggttttg cattatttac gtggcagtcg ttgtttcaaa ccatgcattg tggttgttct 9900
cttactgccg caaaattggt accgaggttc gtagtgacgg cacatttgag gaaatggccc 9960
ttactacctt tatgattact aaagaatctt attgtaagtt gaaaaattct gtttctgatg 10020

ttgcttttaa caggtacttg agtctttata acaagtatcg ttattttagt ggcaaaatgg 10080
atactgccgc ttatagagag gctgcctgtt cacaactggc aaaggcaatg gaaacattta 10140
accataataa tggtaatgat gttctctatc agcctccaac cgcctctgtt actacatcat 10200
ttttacagtc tggtatagtg aagatggtgt cgcccacctc taaagtggag ccttgtattg 10260
ttagtgttac ttatggtaac atgacactta atgggttgtg gttggatgat aaagtttatt 10320
gcccaagaca tgttatctgt tcttcagctg acatgacaga ccctgattat cctaatttgc 10380
tttgtagagt gacatcaagt gatttttgtg ttatgtctgg tcgtatgagc cttactgtaa 10440
tgtcttatca aatgcagggc tgccaacttg ttttgactgt tacactgcaa aatcctaaca 10500
cgcctaagta ttccttcggt gttgttaagc ctggtgagac atttactgta ctggctgcat 10560
acaatggcag acctcaagga gccttccatg ttacgcttcg tagtagccat accataaagg 10620
gctcctttct atgtggatcc tgcggttctg taggatatgt tttaactggc gatagtgtac 10680
gatttgttta tatgcatcag ctagagttga gtactggttg tcataccggt actgacttta 10740
gtgggaactt ttatggtccc tatagagatg cgcaagttgt acaattgcct gttcaggatt 10800
atacgcagac tgttaatgtt gtagcttggc tttatgctgc tatttttaac agatgcaact 10860
ggtttgtgca aagtgatagt tgttccctgg aggagtttaa tgtttgggct atgaccaatg 10920
gttttagctc aatcaaagcc gatcttgtct tggatgcgct tgcttctatg acaggcgtta 10980
cagttgaaca ggtgttggcc gctattaaga ggctgcattc tggattccag ggcaaacaaa 11040
ttttaggtag ttgtgtgctt gaagatgagc tgacaccaag tgatgtttat caacaactag 11100
ctggtgtcaa gctacagtca aagcgcacaa gagttataaa aggtacatgt tgctggatat 11160
tggcttcaac gtttttgttc tgtagcatta tctcagcatt tgtaaaatgg actatgttta 11220
tgtatgttac tacccatatg ttgggagtga cattgtgtgc actttgtttt gtaagctttg 11280
ctatgttgtt gatcaagcat aagcatttgt atttaactat gtatattatg cctgtgttat 11340
gcacactgtt ttacaccaac tatttggttg tgtacaaaca gagttttaga ggtctagctt 11400
atgcttggct ttcacacttt gtccctgctg tagattatac atatatggat gaagttttat 11460
atggtgttgt gttgctagta gctatggtgt ttgttaccat gcgtagcata aaccacgacg 11520
tctttttctat tatgttcttg gttggtagac ttgtcagcct ggtatccatg tggtattttg 11580
gagccaattt agaggaagag gtactattgt tcctcacatc cctatttggc acgtacacat 11640
ggactactat gttgtcattg gctaccgcta aggttattgc taaatggttg gctgtgaatg 11700
tcttgtactt cacagacgta ccgcaaatta aattagttct tttgagctac ttgtgtattg 11760
gttatgtgtg ttgttgttat tggggaatct tgtcactcct taatagcatt tttaggatgc 11820
cattgggcgt ctacaattat aaaatctccg ttcaggagtt acgttatatg aatgctaatg 11880
gcttgcgccc acctagaaat agttttgagg ccctgatgct taattttaag ctgttgggaa 11940
ttggtggtgt gccagtcatt gaagtatctc aaattcaatc aagattgacg gatgttaaat 12000
gtgctaatgt tgtgttgctt aattgcctcc agcacttgca tattgcatct aattctaagt 12060

tgtggcagta ttgtagtact ttgcacaatg aaatactggc tacatctgat ttgagcgtgg 12120
ccttcgataa gttggctcag ctcttagttg ttttatttgc taatccagca gcagtggata 12180
gcaagtgcct tgcaagtatt gaagaagtga gcgatgatta cgttcgcgac aatactgtct 12240
tgcaagcctt acagagtgaa tttgttaata tggctagctt cgttgagtat gaacttgcta 12300
agaagaatct agatgaggct aaggctagcg gctctgccaa tcaacagcag attaagcagc 12360
tagagaaggc gtgtaatatt gctaagtcag catatgagcg cgacagagct gttgctcgta 12420
agctggaacg tatggctgat ttagctctta caaacatgta taaagaagct agaattaatg 12480
ataagaagag taaggtagtg tctgcattgc aaaccatgct ctttagtatg gtgcgtaagc 12540
tagataacca agctcttaat tctattttag ataatgcagt taagggttgt gtacctttga 12600
atgcaatacc atcattgact tcgaacactc tgactataat agtgccagat aagcaggttt 12660
ttgatcaggt tgtggataat gtgtatgtca cctatgctgg gaatgtatgg catatacagt 12720
ttattcaaga tgctgatggt gctgttaaac aattgaatga gatagatgtt aattcaacct 12780
ggcctctagt cattgctgca aataggcata atgaagtgtc tactgttgtt ttgcagaaca 12840
atgagttgat gcctcagaag ttgagaactc aggttgtcaa tagtggctca gatatgaatt 12900
gtaatactcc tacccagtgt tactataata ctactggcac gggtaagatt gtgtatgcta 12960
tacttagtga ctgtgatggt ctcaagtaca ctaagatagt aaaagaagat ggaaattgtg 13020
ttgttttgga attggatcct ccctgtaagt tttctgttca ggatgtgaag ggccttaaaa 13080
ttaagtacct ttactttgtg aaggggtgta atacactggc tagaggctgg gttgtaggca 13140
ccttatcctc gacagtgaga ttgcaggcgg gtacggcaac tgagtatgcc tccaactctg 13200
caatactgtc gctgtgtgcg ttttctgtag atcctaagaa aacgtacttg gattatataa 13260
aacagggtgg agttcccgtt actaattgtg ttaagatgtt atgtgaccat gctggcactg 13320
gtatggccat tactattaag ccggaggcaa ccactaatca ggattcttat ggtggtgctt 13380
ccgtttgtat atattgccgc tcgcgtgttg aacatccaga tgttgatgga ttgtgcaaat 13440
tacgcggcaa gtttgtccaa gtgcccttag gcataaaaga tcctgtgtca tatgtgttga 13500
cgcatgatgt ttgtcaggtt tgtggctttt ggcgagatgg tagctgttcc tgtgtaggca 13560
caggctccca gtttcagtca aaagacacga actttttaaa cgggttcggg gtacaagtgt 13620
aaatgcccgt cttgtaccct gtgccagtgg cttggacact gatgttcaat taagggcatt 13680
tgacatttgt aatgctaatc gagctggcat tggtttgtat tataaagtga attgctgccg 13740
cttccagcgt gtagatgagg acggcaacaa gttggataag ttctttgttg ttaaaagaac 13800
taatttagaa gtgtataata aggagaaaga atgctatgag ttgacaaaag aatgcggtgt 13860
tgtggctgaa cacgagttct tcacatttga tgtggaggga agtcgggtac cacacatagt 13920
ccgtaaagat ctttcaaagt ttactatgtt agatctttgc tatgcattgc gtcattttga 13980
ccgcaatgat tgttcaactc ttaaggaaat tctccttaca tatgctgagt gtgaagagtc 14040

ctacttccaa aagaaggact ggtatgattt tgttgagaat cctgatataa ttaatgtgta 14100
taaaaagctt ggtcctatat ttaatagagc cctgcttaac actgccaagt ttgcagacgc 14160
attagtggag gcaggcttag taggtgtttt aacacttgat aatcaagatt tatatggtca 14220
atggtatgac tttggagatt ttgtcaagac agtacctggt tgtggtgttg ccgtggcaga 14280
ctcttattat tcatatatga tgccaatgct gactatgtgt catgcgttgg atagtgagtt 14340
gtttgttaat ggtacttata gggagtttga ccttgttcag tatgatttta ctgatttcaa 14400
gctagagctc ttcactaagt attttaagca ttggagtatg acctaccacc cgaacacctg 14460
tgagtgcgag gatgacaggt gcattattca ttgcgccaat tttaatatac tttttagtat 14520
ggtcttacct aagacctgtt ttgggcctct tgttaggcag atatttgtgg atggtgttcc 14580
tttcgttgtg tcgatcggtt accattataa agaattaggt gttgttatga atatggatgt 14640
ggatacacat cgttatcgct tgtctcttaa ggacttgctt ttgtatgctg cagaccctgc 14700
ccttcatgtg gcgtctgcta gtgcactgct tgatttgcgc acatgttgtt ttagcgttgc 14760
agctattaca agtggcgtaa aatttcaaac agttaaacct ggaaatttta atcaggattt 14820
ttatgagttt attttgagta aaggcctgct taaagagggg agctccgttg atttgaagca 14880
cttcttcttt acgcaggatg gtaatgctgc tattactgat tataattatt acaagtataa 14940
tctacccacc atggtggata ttaagcagtt gttgtttgtt ttagaagttg ttaataagta 15000
ttttgagatc tatgagggtg ggtgtatacc cgcaacacag gtcattgtta ataattatga 15060
taagagtgct ggctatccat ttaataaatt tggaaaggcc aggctctatt atgaggcatt 15120
atcatttgag gagcaggatg aaatttatgc gtataccaaa cgcaatgtcc tgccgaccct 15180
aactcaaatg aatcttaaat atgctattag tgctaagaat agggcccgca ccgttgctgg 15240
tgtctctatt ctcagtacta tgactggcag aatgtttcat caaaagtgtc taaagagtat 15300
agcagctact cgcggtgttc ctgtagttat aggcaccacg aagttctatg gcggttggga 15360
tgatatgtta cgccgcctta ttaaagatgt tgatagtcct gtactcatgg gttgggacta 15420
tcctaaatgt gatcgtgcta tgccaaacat actgcgtatt gttagtagtt tggtgctagc 15480
ccgtaaacat gattcgtgct gttcgcatac ggatagattc tatcgtcttg cgaacgagtg 15540
cgcccaagtt ttgagtgaaa ttgttatgtg tggtggttgt tattatgtta aaccaggtgg 15600
cactagtagt ggggatgcaa ccactgcttt tgctaattct gtgtttaaca tttgtcaagc 15660
tgtttccgcc aatgtatgct cgcttatggc atgcaatgga cacaaaattg aagatttgag 15720
tatacgcgag ttacaaaagc gcctatactc taatgtctat cgtgcggacc atgttgaccc 15780
cgcatttgtt agtgagtatt atgagttttt aaataagcat tttagtatga tgattttgag 15840
tgatgatggt gttgtgtgtt ataattcaga gtttgcgtcc aagggttata ttgctaatat 15900
aagtgccttt caacaggtat tatattatca aaataatgtg tttatgtctg aggccaaatg 15960
ttgggtagaa acagacatcg aaaagggacc gcatgaattt tgttctcaac atacaatgct 16020
agtcaagatg gatggtgatg aagtctacct tccataccct gatccttcga gaatcttagg 16080

agcaggctgt tttgttgatg atttattaaa gactgatagc gttctcttga tagagcgttt 16140
cgtaagtctt gcaattgatg cttatccttt agtataccat gagaacccag agtatcaaaa 16200
tgtgttccgg gtatatttag aatatataaa gaagctgtac aatgatctcg gtaatcagat 16260
cctggacagc tacagtgtta ttttaagtac ttgtgatggt caaaagttta ctgatgagac 16320
cttttacaag aacatgtatt taagaagtgc agtgctgcaa agcgttggtg cctgcgttgt 16380
ctgtagttct caaacatcat tacgttgtgg cagttgcata cgcaagcctt tgctgtgttg 16440
caaatgcgcc tatgatcatg ttatgtccac tgatcataaa tatgtcctga gtgtgtcacc 16500
atatgtgtgt aattcaccgg gatgtgatgt aaatgatgtt accaaattgt atttaggtgg 16560
tatgtcatat tattgtgagg accataaacc acagtattca ttcaaattgg tgatgaatgg 16620
tatggttttt ggtttatata aacaatcttg tactggttcg ccctacatag aggattttaa 16680
taaaatagct agttgcaaat ggacagaagt cgatgattat gtgctagcta atgaatgcac 16740
cgaacgcctt aaattgtttg ccgcagaaac gcagaaggcc acagaagagg cctttaagca 16800
atgttatgcg tcagcaacga tccgtgagat cgtgagcgat cgggagttaa ttttatcttg 16860
ggaaattggt aaagtgagac caccacttaa taaaaattat gtttttactg gctaccattt 16920
tactaataat ggtaagacag ttttaggtga gtatgttttt gataagagtg agttgactaa 16980
tggtgtgtac tatcgcgcca caaccactta taagttatct gtaggtgatg tgttcatttt 17040
aacatcacac gcagtgtcta gtttaagtgc tcctacatta gtaccgcagg agaattatac 17100
tagcattcgt tttgctagtg tttatagtgt gcctgagacg tttcagaata atgtgcctaa 17160
ttatcagcac attggaatga agcgctattg tactgtacag ggaccgcctg gtactggtaa 17220
gtcccatcta gccattgggc tagctgttta ttattgtaca gcgcgcgtgg tgtataccgc 17280
tgctagccat gctgcagttg acgcgctgtg tgaaaaggca cataaatttt taaatattaa 17340
tgactgcacg cgtattgttc ctgcaaaggt gcgtgtagat tgttatgata aatttaaggt 17400
caatgacacc actcgcaagt atgtgtttac tacaataaat gcattacctg agttggtgac 17460
tgacattatt gtcgttgatg aagttagtat gcttaccaac tatgagctgt ctgttattaa 17520
cagtcgtgtt agtgctaagc attatgtgta tattggagac cctgcgcagt tacctgcacc 17580
acgtgtgcta ctgaataagg gaactctaga acctagatat tttaattccg ttaccaagct 17640
aatgtgttgt ttgggtccag atattttctt gggcacctgt tatagatgcc ctaaggagat 17700
tgtggatacg gtgtcagcct tggtttataa taataagctg aaggctaaaa atgataatag 17760
ctccatgtgc tttaaggttt attataaggg ccagactaca catgagagtt ctagtgctgt 17820
taatatgcag caaatacatt taattagtaa gtttttaaag gcaaacccca gttggagtaa 17880
cgccgtattt attagtcctt ataatagtca gaactatgtt gctaagagag tcttgggatt 17940
acaaacccag acagtagact cagcgcaggg ttctgaatat gattttgtta tttattcaca 18000
gactgcggaa acagcgcatt ctgtcaatgt aaatagattc aatgttgcta ttacacgtgc 18060

taagaagggt attctctgtg tcatgagtag tatgcaatta tttgagtctc ttaattttac 18120
tacactgacg ttggataaga ttaacaatcc acgattacag tgtactacaa atttgtttaa 18180
ggattgtagc aggagctatg taggatatca cccagcccat gcaccatcct ttttggcagt 18240
tgatgacaaa tataaggtag gcggtgattt agccgtttgc cttaatgttg ctgattctgc 18300
tgtcacttat tcgcggctta tatcactcat gggattcaag cttgacttga cccttgatgg 18360
ttattgtaag ctgtttataa ctagagatga agctatcaaa cgtgttagag cctgggttgg 18420
cttcgatgca gaaggtgccc atgcgatacg tgatagcatt gggacaaatt tcccattaca 18480
attaggcttt tcgactggaa ttgattttgt tgtcgaagcc actggaatgt ttgctgagag 18540
agatggttat gtctttaaaa aggcagccgc acgagctcct cctggcgaac aatttaaaca 18600
ccttatccca cttatgtcaa gagggcagaa atgggatgtg gttcgaatta gaatagtaca 18660
aatgttgtca gaccacctag cggatttggc agacagtgtt gtacttgtga cgtgggctgc 18720
cagctttgag ctcacatgtt tgcgatattt cgctaaagtt ggaagagaag ttgtgtgtag 18780
tgtctgcacc aagcgtgcga catgttttaa ttctagaact ggatactatg gatgctggcg 18840
acatagttat tcctgtgatt acctgtacaa cccactaata gttgacattc aacagtgggg 18900
atatacagga tctttaacta gcaatcatga tcctatttgc agcgtgcata agggtgctca 18960
tgttgcatca tctgatgcta tcatgacccg gtgtctagct gttcatgatt gcttttgtaa 19020
gtctgttaat tggaatttag aataccccat tatttcaaat gaggtcagtg ttaatacctc 19080
ctgcaggtta ttgcagcgcg taatgtttag ggctgcgatg ctatgcaata ggtatgatgt 19140
gtgttatgac attggcaacc ctaaaggtct tgcctgtgtc aaaggatatg attttaagtt 19200
ttatgatgcc tcccctgttg ttaagtctgt taaacagttt gtttataaat acgaggcaca 19260
taaagatcaa tttttagatg gtttgtgtat gttttggaac tgcaatgtgg ataagtatcc 19320
agcgaatgca gttgtgtgta ggtttgacac gcgtgtgttg aacaaattaa atctccctgg 19380
ctgtaatggt ggcagtttgt atgttaacaa acatgcattc cacaccagtc cctttacccg 19440
ggctgccttc gagaatttga agcctatgcc tttcttttat tattcagata cgccctgtgt 19500
gtatatggaa ggcatggaat ctaagcaggt cgattatgtc ccattgagaa gcgctacatg 19560
catcacaaga tgcaatttag gtggcgctgt ttgtttaaaa catgctgagg agtatcgtga 19620
gtaccttgag tcttacaata cggcaaccac agcgggtttt actttttggg tctataagac 19680
ttttgatttt tataaccttt ggaatacttt tactaggctc caaagtttag aaaatgtagt 19740
gtataatttg gtcaatgctg gacactttga tggccgggcg ggtgaactgc cttgtgctgt 19800
tataggtgag aaagtcattg ccaagattca aaatgaggat gtcgtggtct ttaaaaataa 19860
cacgccattc cccactaatg tggctgtcga attatttgct aagcgcagta ttcggcccca 19920
ccccgagctt aagctcttta gaaatttgaa tattgacgtg tgctggagtc acgtcctttg 19980
ggattatgct aaggatagtg tgttttgcag ttcgacgtat aaggtctgca aatacacaga 20040
tttacagtgc attgaaagct tgaatgtact tttgatggt cgtgataatg gtgctcttga 20100

agcttttaag aagtgccgga atggcgtcta cattaacacg acaaaaatta aaagtctgtc 20160
gatgattaaa ggcccacaac gtgccgattt gaatggcgta gttgtggaga aagttggaga 20220
ttctgatgtg gaattttggt ttgctgtgcg taaagacggt gacgatgtta tcttcagccg 20280
tacagggagc cttgaaccga gccattaccg gagcccacaa ggtaatccgg gtggtaatcg 20340
cgtgggtgat ctcagcggta atgaagctct agcgcgtggc actatcttta ctcaaagcag 20400
attattatct tctttcacac ctcgatcaga gatggagaaa gattttatgg atttagatga 20460
tgatgtgttc attgcaaaat atagtttaca ggactacgcg tttgaacacg ttgtttatgg 20520
tagttttaac cagaagatta ttggaggttt gcatttgctt attggcttag cccgtaggca 20580
gcaaaaatcc aatctggtaa ttcaagagtt cgtgacatac gactctagca ttcattcgta 20640
ctttatcact gacgagaaca gtggtagtag taagagtgtg tgcactgtta ttgatttatt 20700
gttagatgat tttgtggaca ttgtaaagtc cctgaatcta aagtgtgtga gtaaggttgt 20760
taatgttaat gttgatttta aagatttcca gtttatgttg tggtgcaatg aggagaaggt 20820
catgactttc tatcctcgtt tgcaggctgc tgctgactgg aaacctggtt atgttatgcc 20880
tgtcttatat aagtatttgg aatcgcctct ggaaagagta aacctctgga attatggcaa 20940
gccgattact ttacctacag gatgtatgat gaatgttgct aagtatactc aattatgtca 21000
atatttgagc actacaacat tagcagttcc ggctaatatg cgtgtcttac accttggtgc 21060
cggttcggat aagggtgttg cccctgggtc tgcagttctt aggcagtggc taccagcggg 21120
aagtattctt gtagataatg atgtgaatcc atttgtgagt gacagtgtcg cctcatatta 21180
tggaaattgt ataaccttac cctttgattg tcagtgggat ctgataattt ctgatatgta 21240
cgaccctctt actaagaaca ttggggagta caacgtgagt aaagatggat tctttactta 21300
cctctgtcat ttaattcgtg acaagttggc tctgggtggc agtgttgcca taaaaataac 21360
agagttttct tggaacgctg agttatatag tttaatgggg aagtttgcgt tctggacaat 21420
cttttgcacc aacgtaaacg cctcttcaag tgaaggattt ttgattggca taaattggtt 21480
gaataagacc cgtaccgaaa ttgacggtaa aaccatgcat gccaattatc tgttttggag 21540
aaatagtaca atgtggaatg gaggggctta cagtctcttt gacatgagta agttcccttt 21600
gaaagcggct ggtacggctg ttgttagcct taaaccagac caaataaatg acttagtcct 21660
ctccttgatt gagaagggca agttattagt gcgtgataca cgcaaagaag tttttgttgg 21720
cgatagccta gtaaatgtca aataaatcta tacttgtcgt ggctgtgaaa atggcctttg 21780
ctgacaagcc taatcatttc ataaactttc ccctggccca atttagtggc tttatgggta 21840
agtatttaaa gctacagtct caacttgtgg aaatgggttt agactgtaaa ttacagaagg 21900
caccacatgt tagtattacc ctgcttgata ttaaagcaga ccaatacaaa caggtggaat 21960
ttgcaataca agaaataata gatgatctgg cggcatatga gggagatatt gtctttgaca 22020
accctcacat gcttggcaga tgccttgttc ttgatgttag aggatttgaa gagttgcatg 22080

aagatattgt tgaaattctc cgcagaaggg gttgcacggc agatcaatcc agacactgga 22140
ttccgcactg cactgtggcc caatttgacg aagaaagaga aacaaaagga atgcaattct 22200
atcataaaga acccttctac ctcaagcata acaacctatt aacggatgct gggcttgagc 22260
tcgtgaagat aggttcttcc aaaatagatg ggttttattg tagtgaactg agtgtttggt 22320
gtggtgagag gctttgttat aagcctccaa cacccaaatt cagtgatata tttggctatt 22380
gctgcataga taaaatacgt ggtgatttag aaataggaga cctaccgcag gatgatgagg 22440
aagcgtgggc cgagctaagt taccactatc aaagaaacac ctacttcttc agacatgtgc 22500
acgataatag catctatttt cgtaccgtgt gtagaatgaa gggttgtatg tgttgatttg 22560
tttttacact attagtgtaa taagcttatt attttgttga aaagggcagg atgtgcatag 22620
ctatggctcc tcgcacactg cttttgctga tttgatgtca gctggtgttt gggttcaatg 22680
aacctcttaa catcgtttca catttaaatg atgactggtt tctatttggt gacagtcgtt 22740
ctgactgtac ctatgtagaa aataacggtc atcctaaatt agattggctt gacctcgacc 22800
caaagttgtg taattcagga aagatttccg caaagagtgg taactctctc tttaggagtt 22860
ttcacttcac tgatttttac aattatacgg gtgagggaga ccaaattgta ttttatgaag 22920
gagttaattt tagtcccagc catggcttta aatgcctggc tcatggagat aataaaagat 22980
ggatgggcaa taaagctcga ttttatgccc gagtgtatga gaagatggcc caatatagga 23040
gcctatcgtt tgttaatgtg tcttatgcct atggaggtaa tgcaaagccc gcctccattt 23100
gcaaagacaa tactttaaca ctcaataacc ccaccttcat atcgaaggag tctaattatg 23160
ttgattatta ctatgagagt gaggctaatt tcacactaga aggttgtgat gaatttatag 23220
taccgctctg tggttttaat ggccattcca agggcagctc ttcggatgct gccaataaat 23280
attatactga ctctcagagt tactataata tggatattgg tgtcttatat gggttcaatt 23340
cgaccttgga tgttggcaac actgctaagg atccgggtct tgatctcact tgcaggtatc 23400
ttgcattgac tcctggtaat tataaggctg tgtccttaga atatttgtta agcttaccct 23460
caaaggctat ttgcctccat aagacaaagc gctttatgcc tgtgcaggta gttgactcaa 23520
ggtggagtag catccgccag tcagacaata tgaccgctgc agcctgtcag ctgccatatt 23580
gtttctttcg caacacatct gcgaattata gtggtggcac acatgatgcg caccatggtg 23640
attttcattt caggcagtta ttgtctggtt tgttatataa tgtttcctgt attgcccagc 23700
agggtgcatt tctttataat aatgttagtt cctcttggcc agcctatggg tacggtcatt 23760
gtccaacggc agctaacatt ggttatatgg cacctgtttg tatctatgac cctctcccgg 23820
tcatactgct aggtgtgtta ttgggtatag ctgtgttgat tattgtgttt ttgatgtttt 23880
attttatgac ggatagcggt gttagattgc atgaggcata atctaaacat gctgttcgtg 23940
tttattctat ttttgccctc ttgtttaggg tatattggtg attttagatg tatccagctt 24000
gtgaattcaa acggtgctaa tgttagtgct ccaagcatta gcactgagac cgttgaagtt 24060
tcacaaggcc tggggacata ttatgtgtta gatcgagttt atttaaatgc cacattattg 24120

cttactggtt actacccggt cgatggttct aagtttagaa acctcgctct tacgggaact 24180
aactcagtta gcttgtcgtg gtttcaacca ccctatttaa gtcagtttaa tgatggcata 24240
tttgcgaagg tgcagaacct taagacaagt acgccatcag gtgcaactgc atattttcct 24300
actatagtta taggtagttt gtttggctat acttcctata ccgttgtaat agagccatat 24360
aatggtgtta taatggcctc agtgtgccag tataccattt gtctgttacc ttacactgat 24420
tgtaagccta acactaatgg taataagctt atagggtttt ggcacacgga tgtaaaaccc 24480
ccaatttgtg tgttaaagcg aaatttcacg cttaatgtta atgctgatgc attttatttt 24540
catttttacc aacatggtgg tactttttat gcgtactatg cggataaacc ctccgctact 24600
acgtttttgt ttagtgtata tattggcgat attttaacac agtattatgt gttacctttc 24660
atctgcaacc caacagctgg tagcactttt gctccgcgct attgggttac acctttggtt 24720
aagcgccaat atttgtttaa tttcaaccag aagggtgtca ttactagtgc tgttgattgt 24780
gctagtagtt ataccagtga aataaaatgt aagacccaga gcatgttacc tagcactggt 24840
gtctatgagt tatccggtta tacggtccaa ccagttggag ttgtataccg gcgtgttgct 24900
aacctcccag cttgtaatat agaggagtgg cttactgcta ggtcagtccc ctcccctctc 24960
aactgggagc gtaagacttt tcagaattgt aattttaatt taagcagcct gttacgttat 25020
gttcaggctg agagtttgtt ttgtaataat atcgatgctt ccaaagtgta tggcaggtgc 25080
tttggtagta tttcagttga taagtttgct gtaccccgaa gtaggcaagt tgatttacag 25140
cttggtaact ctggatttct gcagactgct aattataaga ttgatacagc tgccacttcg 25200
tgtcagctgc attacacctt gcctaagaat aatgtcacca taaacaacca taacccctcg 25260
tcttggaata ggaggtatgg ctttaatgat gctggcgtct ttggcaaaaa ccaacatgac 25320
gttgtttacg ctcagcaatg ttttactgta agatctagtt attgcccgtg tgctcaaccg 25380
gacatagtta gcccttgcac tactcagact aagcctaagt ctgcttttgt taatgtgggt 25440
gaccattgtg aaggcttagg tgttttagaa gataattgtg gcaatgctga tccacataag 25500
ggttgtatct gtgccaacaa ttcatttatt ggatggtcac atgatacctg ccttgttaat 25560
gatcgctgcc aaatttttgc taatatattg ttaaatggca ttaatagtgg taccacatgt 25620
tccacagatt tgcagttgcc taatactgaa gtggttactg gcatttgtgt caaatatgac 25680
ctctacggta ttactggaca aggtgttttt aaagaggtta aggctgacta ttataatagc 25740
tggcaaaccc ttctgtatga tgttaatggt aatttgaatg gttttcgtga tcttaccact 25800
aacaagactt atacgataag gagctgttat agtggccgtg tttctgctgc atttcataaa 25860
gatgcacccg aaccggctct gctctatcgt aatataaatt gtagctatgt ttttagcaat 25920
aatatttccc gtgaggagaa cccacttaat tactttgata gttatttggg ttgtgttgtt 25980
aatgctgata accgcacgga tgaggcgctt cctaattgtg atctccgtat gggtgctggc 26040
ttatgcgttg attattcaaa atcacgcagg gctgaccgat cagtttctac tggctatcgg 26100

ttaactacat ttgagccata cactccgatg ttagttaatg atagtgtcca atccgttgat 26160
ggattatatg agatgcaaat accaaccaat tttactattg ggcaccatga ggagttcatt 26220
caaactagat ctccaaaggt gactatagat tgtgctgcat ttgtctgtgg tgataacact 26280
gcatgcaggc agcagttggt tgagtatggc tctttctgtg ttaatgttaa tgccattctt 26340
aatgaggtta ataacctctt ggataatatg caactacaag ttgctagtgc attaatgcag 26400
ggtgttacta taagctcgag actgccagac ggcatctcag gccctataga tgacattaat 26460
tttagtcctc tacttggatg cataggttca acatgtgctg aagacggcaa tggacctagt 26520
gcaatccgag ggcgttctgc tatagaggat ttgttatttg acaaggtcaa attatctgat 26580
gttggctttg tcgaggctta taataattgc accggtggtc aagaagttcg tgacctcctt 26640
tgtgtacaat cttttaatgg catcaaagta ttacctcctg tgttgtcaga gagtcagatc 26700
tctggctaca caaccggtgc tactgcggca gctatgttcc caccgtggtc agcagctgcc 26760
ggtgtgccat ttagtttaag tgttcaatat agaattaatg gtttaggtgt cactatgaat 26820
gtgcttagtg agaaccaaaa gatgattgct agtgctttta acaatgcgct gggtgctatc 26880
caggatgggt ttgatgcaac caattctgct ttaggtaaga tccagtccgt tgttaatgca 26940
aatgctgaag cactcaataa cttactaaat caactttcta acaggtttgg tgctattagt 27000
gcttctttac aagaaattct aactcggctt gaggctgtag aagcaaaagc ccagatagat 27060
cgtcttatta atggcaggtt aactgcactt aatgcgtata tatccaagca acttagtgat 27120
agtacgctta ttaaagttag tgctgctcag gccatagaaa aggtcaatga gtgcgttaag 27180
agccaaacca cgcgtattaa tttctgtggc aatggtaatc atatattatc tcttgtccag 27240
aatgcgcctt atggcttata ttttatacac ttcagctatg tgccaatatc ctttacaacc 27300
gcaaatgtga gtcctggact ttgcatttct ggtgatagag gattagcacc taaagctgga 27360
tattttgttc aagatgatgg agaatggaag ttcacaggca gttcatatta ctaccctgaa 27420
cccattacag ataaaaacag tgtcattatg agtagttgcg cagtaaacta cacaaaggca 27480
cctgaagttt tcttgaacac ttcaatacct aatccacccg actttaagga ggagttagat 27540
aaatggttta agaatcagac gtctattgcg cctgatttat ctctcgattt cgagaagtta 27600
aatgttactt tgctggacct gacgtatgag atgaacagga ttcaggatgc aattaagaag 27660
ttaaatgaga gctacatcaa cctcaaggaa gttggcacat atgaaatgta tgtgaaatgg 27720
ccttggtatg tttggttgct aattggatta gctggtgtag ctgtttgtgt gttgttattc 27780
tttatatgtt gctgcacagg ttgtggctca tgttgtttta agaagtgtgg aaattgttgt 27840
gatgagtatg gaggacacca ggacagtatt gtgatacata atatttcctc tcatgaggat 27900
tgactatcac agcctctcct ggaaagacag aaaatctaaa caatttatag cattctcatt 27960
gctacctggc cccgtaagag gcagtcatag ctatggccgt gttggtccta aggctacatt 28020
ggctgctgtc tttattggtc catttattgt agcatgtatg ctaggcattg gcctagttta 28080
tttattgcaa ttgcaagttc aaatttttca tgttaaggat accatacgtg tgactggcaa 28140

gccagccact gtgtcttata ctacaagtac accagtaaca ccgagcgcga cgacgctcga 28200
tggtactacg tatactttaa ttagacccac tagctcttat acaagagttt atcttggtac 28260
tccaagaggt tttgattata gtacatttgg gcctaagacc ctagattatg ttactaatct 28320
aaacctcatc ttaattctgg tcgtccatat acttttaagg cattgtccag gcatatgaga 28380
ccaacagcca catggatttg gcatgtgagt gatgcatggt tacgccgcac gcgggacttt 28440
ggtgtcattc gcctagaaga tttttgtttt caatttaatt atagccaacc ccgagttggt 28500
tattgtagag ttcctttaaa ggcttggtgt agcaaccagg gtaaatttgc agcgcagttt 28560
accctaaaaa gttgcgaaaa accaggtcac gaaaaattta ttactagctt cacggcctac 28620
ggcagaactg tccaacaggc cgttagcaag ttagtagaag aagctgttga ttttattctt 28680
tttagggcca cgcagctcga aagaaatgtt taatttattc cttacagaca cagtatggta 28740
tgtggggcag attattttta tattcgcagt gtgtttgatg gtcaccataa ttgtggttgc 28800
cttccttgcg tctatcaaac tttgtattca actttgcggt ttatgtaata ctttggtgct 28860
gtccccttct atttatttgt atgataggag taagcagctt tataagtatt ataatgaaga 28920
aatgagactg cccctattag aggtggatga tatctaatct aaacattatg agtagtacta 28980
ctcaggcccc agagcccgtc tatcaatgga cggccgacga ggcagttcaa ttccttaagg 29040
aatggaactt ctcgttgggc attatactac tctttattac tatcatacta cagttcggtt 29100
acacgagccg tagcatgttt atttatgttg tgaaaatgat aatcttgtgg ttaatgtggc 29160
cactgactat tgttttgtgt attttcaatt gcgtgtatgc gctaaataat gtgtatcttg 29220
gattttctat agtgtttact atagtgtcca ttgtaatctg gattatgtat tttgttaata 29280
gcataaggtt gtttatcagg actggtagct ggtggagctt caaccccgaa acaaacaacc 29340
ttatgtgtat agatatgaaa ggtaccgtgt atgttagacc cattattgag gattaccata 29400
cactaacagc cactattatt cgtggccacc tctacatgca aggtgttaag ctaggcaccg 29460
gtttctcttt gtctgacttg cccgcttatg ttacagttgc taaggtgtca cacctttgca 29520
cttataagcg cgcattctta gacaaggtag acggtgttag cggttttgct gtttatgtga 29580
agtccaaggt cggaaattac cgactgccct caaacaaacc gagtggcgcg gacaccgcat 29640
tgttgagaat ctaatctaaa ctttaaggat gtcttttgtt cctgggcaag aaaatgccgg 29700
tggcagaagc tcctctgtaa accgcgctgg taatggaatc ctcaagaaga ccacttgggc 29760
tgaccaaacc gagcgtggac caaataatca aaatagaggc agaaggaatc agccaaagca 29820
gactgcaact actcaaccca actccgggag tgtggttccc cattactcct ggttttctgg 29880
cattacccag ttccaaaagg gaaaggagtt tcagtttgca gaaggacaag gagtgcctat 29940
tgccaatgga atccccgctt cagagcaaaa gggatattgg tatagacaca accgccgttc 30000
ttttaaaaca cctgatgggc agcagaagca attactgccc agatggtatt tttactatct 30060
tggcacaggg ccccatgctg gagccagtta tggagacagc attgaaggtg tcttctgggt 30120

```
tgcaaacagc caagcggaca ccaataccccg ctctgatatt gtcgaaaggg acccaagcag   30180
tcatgaggct attcctacta ggtttgcgcc cggcacggta ttgcctcagg gcttttatgt   30240
tgaaggctct ggaaggtctg cacctgctag ccgatctggt tcgcggtcac aatcccgtgg   30300
gccaaataat cgcgctagaa gcagttccaa ccagcgccag cctgcctcta ctgtaaaacc   30360
tgatatggcc gaagaaattg ctgctcttgt tttggctaag ctcggtaaag atgccggcca   30420
gcccaagcaa gtaacgaagc aaagtgccaa agaagtcagg cagaaaattt taaacaagcc   30480
tcgccaaaag aggactccaa acaagcagtg cccagtgcag cagtgttttg gaaagagagg   30540
ccccaatcag aattttggag gctctgaaat gttaaaactt ggaactagtg atccacagtt   30600
ccccattctt gcagagttgg ctccaacagt tggtgccttc ttctttggat ctaaattaga   30660
attggtcaaa aagaattctg gtggtgctga tgaacccacc aaagatgtgt atgagctgca   30720
atattcaggt gcagttagat ttgatagtac tctacctggt tttgagacta tcatgaaagt   30780
gttgaatgag aatttgaatg cctaccagaa ggatggtggt gcagatgtgg tgagcccaaa   30840
gccccaaaga aaagggcgta gacaggctca ggaaaagaaa gatgaagtag ataatgtaag   30900
cgttgcaaag cccaaaagct ctgtgcagcg aaatgtaagt agagaattaa ccccagagga   30960
tagaagtctg ttggctcaga tccttgatga tggcgtagtg ccagatgggt tagaagatga   31020
ctctaatgtg taaagagaat gaatcctatg tcggcgctcg gtggtaaccc ctcgcgagaa   31080
agtcgggata ggacactctc tatcagaatg gatgtcttgc tgtcataaca gatagagaag   31140
gttgtggcag accctgtatc aattagttga aagagattgc aaaatagaga atgtgtgaga   31200
gaagttagca aggtcctacg tctaaccata agaacggcga taggcgcccc ctgggaagag   31260
ctcacatcag ggtactattc ctgcaatgcc ctagtaaatg aatgaagttg atcatggcca   31320
attggaagaa tcacaaaaaa aaaaaaaaaa aaaaaaa                             31357
```

<210> 35
<211> 25
<212> DNA
<213> Mus sp.

<220>
<221> misc_feature
<222> (1)..(25)
<223> Forward Primer

<400> 35
tgaacccacc aaagatgtgt atgag 25

<210> 36
<211> 25
<212> DNA
<213> Mus sp.

<220>
<221> misc_feature
<222> (1)..(25)

1> Reverse Primer

0> 36
tccttct ggtaggcatt caaat 25

10> 37
11> 16
12> DNA
13> Mus sp.

220>
221> misc_feature
222> (1)..(16)
223> Probe

<400> 37
ttgcacctga atattg 16

<210> 38
<211> 416
<212> DNA
<213> Mus sp.

<400> 38

ggcagctgct gctccgaggc ggtcaagagc gccatgagca ccattgacct ggactcgctg 60
atggcagagc acagcgctgc ctggtacatg cccgctgaca aggccctggt ggacagcgcg 120
gacgacgaca agacgttggc gccctgggag aaggccaaac cccagaaccc caacagcaaa 180
gaaggcttgc agccaattta ctggagcagg gatgacgtag cccagtggct caagtgggct 240
gaaaatgagt tttctttaag gccaattgac agcaacacgt ttgaaatgaa tggcaaagct 300
ctcctgctgc tgaccaaaga ggactttcgc tatcgatctc ctcattcagg tgatgtgctc 360
tatgaactcc ttcagcatat tctgaagcag aggaaacctc ggattctttt ttcacc 416

<210> 39
<211> 20
<212> DNA
<213> Mus sp.

<220>
<221> misc_feature
<222> (1)..(20)
<223> Forward Primer

<400> 39
aaaccccaga accccaacag 20

<210> 40
<211> 23
<212> DNA
<213> Mus sp.

<220>

<221> misc_feature
<222> (1)..(23)
<223> Reverse Primer

<400> 40
tcatccctgc tccagtaaat tgg 23

<210> 41
<211> 16
<212> DNA
<213> Mus sp.

<220>
<221> misc_feature
<222> (1)..(16)
<223> Probe

<400> 41
ctgcaagcct tctttg 16

<210> 42
<211> 792
<212> DNA
<213> Mus sp.

<400> 42

```
cattgaacaa gatggattgc acgcaggttc tccggccgct tgggtggaga ggctattcgg     60
ctatgactgg gcacaacaga caatcggctg ctctgatgcc gccgtgttcc ggctgtcagc    120
gcaggggcgc ccggttcttt ttgtcaagac cgacctgtcc ggtgccctga atgaactgca    180
ggacgaggca gcgcggctat cgtggctggc cacgacgggc gttccttgcg cagctgtgct    240
cgacgttgtc actgaagcgg gaagggactg gctgctattg ggcgaagtgc cggggcagga    300
tctcctgtca tctcaccttg ctcctgccga gaaagtatcc atcatggctg atgcaatgcg    360
gcggctgcat acgcttgatc cggctacctg cccattcgac caccaagcga aacatcgcat    420
cgagcgagca cgtactcgga tggaagccgg tcttgtcgat caggatgatc tggacgaaga    480
gcatcagggg ctcgcgccag ccgaactgtt cgccaggctc aaggcgcgca tgcccgacgg    540
cgaggatctc gtcgtgaccc atggcgatgc ctgcttgccg aatatcatgg tggaaaatgg    600
ccgcttttct ggattcatcg actgtggccg gctgggtgtg gcggaccgct atcaggacat    660
agcgttggct acccgtgata ttgctgaaga gcttggcggc gaatgggctg accgcttcct    720
cgtgctttac ggtatcgccg ctcccgattc gcagcgcatc gccttctatc gccttcttga    780
cgagttcttc tg                                                        792
```

<210> 43
<211> 15
<212> DNA
<213> Mus sp.

<220>

<221> misc_feature
<222> (1)..(15)
<223> Forward Primer

<400> 43
gggcgcccgg ttctt 15

<210> 44
<211> 21
<212> DNA
<213> Mus sp.

<220>
<221> misc_feature
<222> (1)..(21)
<223> Reverse Primer

<400> 44
cctcgtcctg cagttcattc a 21

<210> 45
<211> 15
<212> DNA
<213> Mus sp.

<220>
<221> misc_feature
<222> (1)..(15)
<223> Probe

<400> 45
acctgtccgg tgccc 15

<210> 46
<211> 1288
<212> DNA
<213> Mus sp.

<220>
<221> misc_feature
<222> (301)..(421)
<223> n is a, c, g, or t

<220>
<221> misc_feature
<222> (671)..(702)
<223> n is a, c, g, or t

<220>
<221> misc_feature
<222> (920)..(954)
<223> n is a, c, g, or t

<400> 46

ttaaagctca tgcctagacc tgatgctata gaaggtgtgc tcctcgcttc tctgccaatc 60
ttaaggtgcc ctggatggag ctgggtgacg tgtttaccct tgtagtctgt cctgtctata 120
tgcatggata tgcacagtgc ccttgaccca accctgccaa ccaggcacct gcagaaggtg 180
tagatgaccg tcagattgcc cagcatccct gtgagtccca ccagcaggat caccgtgcct 240
agggtatagt gagcatggtc tgggacatcg actgtgggga aggggaccca ggcagcagcc 300
nnnnnnnnnn nnnnnnnnnn nnnnnnnnnn nnnnnnnnnn nnnnnnnnnn nnnnnnnnnn 360

nnnnnnnnnn nnnnnnnnnn nnnnnnnnnn nnnnnnnnnn nnnnnnnnnn nnnnnnnnnn 420
nagcccatag aagaaagtgc aagtcttcca aaatttaacc ccacgcccat atatgtgtgg 480
atactgagct tctaagaggg agtgaaaggc tcagatggcc tgctggaggt taacaggaca 540
aatgcgtgcc tgcaggacag agcacagctt gggtgacctt aaggaatgag tagagccagg 600
tcctgggtac tgccctccca acgaatggat accccacagc aagcctccaa ggagaacttg 660
caacccctgt nnnnnnnnnn nnnnnnnnnn nnnnnnnnnn nnaaacgagg gaggagaact 720
ttccactaga aagagagttt aggttccccc aggctgctgg gaggccattt cccccatgag 780
gttagtacac agggactaag gatagctccc agggagaggc aggagtctgc ccaatgtcct 840
gcccagcatc ccactctggc ctgtacaagt ccagaagcct agggcatgcc tttcccccta 900
ggatactccc ccaggggatn nnnnnnnnnn nnnnnnnnnn nnnnnnnnnn nnnngaagag 960
caggtcagcc cctgcctttc tggttctcca gtggtctctg ccaacaaaga cattgcctgt 1020
gccctcttgt ctcagccact gtgtagagaa agcttagaga acttcagtga cgctcaaggt 1080
ccttcgtcta agctcagacc ttttctatct ccctgttaaa acaagggtgg ggacaggagt 1140
ctctgtgtac acacatgctc cccaaactta ccgtggggct aacagagaga agctgggctc 1200
ttacggagac gttctgagtg ccgttccaaa tgccttgcag ggcaggactg gttgtgaagc 1260
tgggatcctg agttaagctt gacaagac 1288

<210> 47
<211> 25
<212> DNA
<213> Mus sp.

<220>
<221> misc_feature
<222> (1)..(25)
<223> Forward Primer

<400> 47
tgggtgacct taaggaatga gtaga 25

<210> 48
<211> 20
<212> DNA
<213> Mus sp.

<220>
<221> misc_feature
<222> (1)..(20)
<223> Reverse Primer

<400> 48
gttctccttg gaggcttgct 20

<210> 49
<211> 16
<212> DNA
<213> Mus sp.

<220>
<221> misc_feature
<222> (1)..(16)
<223> Probe

<400> 49
ctgccctccc aacgaa 16

<210> 50
<211> 341
<212> DNA
<213> Mus sp.

<400> 50

```
gtgatgatga tgggcaacgt tcacgtagca gctcttctgc tcaactacgg tgcagattcg      60
aactgcgagg accccactac cttctcccgc ccggtgcacg acgcagcgcg ggaaggcttc     120
ctggacacgc tggtggtgct gcacgggtca ggggctcggc tggatgtgcg cgatgcctgg     180
ggtcgcctgc cgctcgactt ggcccaagag cggggacatc aagacatcgt gcgatatttg     240
cgttccgctg ggtgctcttt gtgttccgct gggtggtctt tgtgtaccgc tgggaacgtc     300
gcccagaccg acgggcatag cttcagctca agcacgccca g                         341
```

<210> 51
<211> 20
<212> DNA
<213> Mus sp.

<220>
<221> misc_feature
<222> (1)..(20)
<223> Forward Primer

<400> 51
cgaggacccc actaccttct 20

<210> 52
<211> 17
<212> DNA
<213> Mus sp.

<220>
<221> misc_feature
<222> (1)..(17)
<223> Reverse Primer

<400> 52
ccgctcttgg gccaagt 17

<210> 53
<211> 16
<212> DNA
<213> Mus sp.

<220>
<221> misc_feature
<222> (1)..(16)
<223> Probe

<400> 53
caggcatcgc gcacat 16

<210> 54
<211> 600
<212> DNA
<213> Mus sp.

<400> 54

atgaccgagt acaagcccac ggtgcgcctc gccacccgcg acgacgtccc ccgggccgta 60
cgcaccctcg ccgccgcgtt cgccgactac cccgccacgc gccacaccgt cgacccggac 120
cgccacatcg agcgggtcac cgagctgcaa gaactcttcc tcacgcgcgt cgggctcgac 180
atcggcaagg tgtgggtcgc ggacgacggc gccgcggtgg cggtctggac cacgccggag 240
agcgtcgaag cgggggcggt gttcgccgag atcggcccgc gcatggccga gttgagcggt 300
tcccggctgg ccgcgcagca acagatggaa ggcctcctgg cgccgcaccg gcccaaggag 360
cccgcgtggt tcctggccac cgtcggcgtc tcgcccgacc accagggcaa gggtctgggc 420
agcgccgtcg tgctccccgg agtggaggcg gccgagcgcg ccggggtgcc cgccttcctg 480
gagacctccg cgccccgcaa cctccccttc tacgagcggc tcggcttcac cgtcaccgcc 540
gacgtcgagt gcccgaagga ccgcgcgacc tggtgcatga cccgcaagcc cggtgcctga 600

<210> 55
<211> 17
<212> DNA
<213> Mus sp.

<220>
<221> misc_feature
<222> (1)..(17)
<223> Forward Primer

<400> 55
gcggtgttcg ccgagat 17

<210> 56
<211> 20
<212> DNA
<213> Mus sp.

<220>
<221> misc_feature
<222> (1)..(20)
<223> Reverse Primer

<400> 56
gaggccttcc atctgttgct 20

<210> 57
<211> 17
<212> DNA
<213> Mus sp.

<220>
<221> misc_feature
<222> (1)..(17)
<223> Probe

<400> 57
gcggtgttcg ccgagat 17

<210> 58
<211> 1008
<212> DNA
<213> Mus sp.

<400> 58

atgtctagat tagataaaag taaagtgatt aacagcgcat tagagctgct taatgaggtc 60
ggaatcgaag gtttaacaac ccgtaaactc gcccagaagc taggtgtaga gcagcctaca 120
ttgtattggc atgtaaaaaa taagcgggct ttgctcgacg ccttagccat tgagatgtta 180
gataggcacc atactcactt ttgccctttta gaaggggaaa gctggcaaga tttttacgt 240
aataacgcta aaagttttag atgtgcttta ctaagtcatc gcgatggagc aaaagtacat 300
ttaggtacac ggcctacaga aaaacagtat gaaactctcg aaaatcaatt agcctttta 360
tgccaacaag gtttttcact agagaatgca ttatatgcac tcagcgctgt ggggcatttt 420
actttaggtt gcgtattgga agatcaagag catcaagtcg ctaaagaaga aagggaaaca 480
cctactactg atagtatgcc gccattatta cgacaagcta tcgaattatt tgatcaccaa 540
ggtgcagagc cagccttctt attcggcctt gaattgatca tatgcggatt agaaaaacaa 600
cttaaatgtg aaagtgggtc cgcgtacagc cgcgcgcgta cgaaaaacaa ttacgggtct 660
accatcgagg gcctgctcga tctcccggac gacgacgccc ccgaagaggc ggggctggcg 720
gctccgcgcc tgtcctttct ccccgcggga cacacgcgca gactgtcgac ggcccccccg 780
accgatgtca gcctggggga cgagctccac ttagacggcg aggacgtggc gatggcgcat 840
gccgacgcgc tagacgattt cgatctggac atgttggggg acggggattc cccgggtccg 900
ggatttaccc cccacgactc cgccccctac ggcgctctgg atatggccga cttcgagttt 960
gagcagatgt ttaccgatgc ccttggaatt gacgagtacg gtgggtag 1008

<210> 59
<211> 25
<212> DNA
<213> Mus sp.

<220>
<221> misc_feature
<222> (1)..(25)
<223> Forward Primer

<400> 59
tgccaacaag gtttttcact agaga 25

<210> 60
<211> 26
<212> DNA
<213> Mus sp.

<220>
<221> misc_feature
<222> (1)..(26)
<223> Reverse Primer

<400> 60
ctcttgatct tccaatacgc aaccta 26

<210> 61
<211> 16

<212> DNA
<213> Mus sp.

<220>
<221> misc_feature
<222> (1)..(16)
<223> Probe

<400> 61
ccacagcgct gagtgc 16

<210> 62
<211> 1139
<212> DNA
<213> Mus sp.

<400> 62

```
gggtgaggct gttgcgacgc ctcttattta aaaaaaaagg gaggggtgtc tcacactttt      60
tctcttgaag gctccttctg tccccctctt ttcctttcct gaaaggcacc cccttaaacg     120
gtcctccgcc ttcccttcta ctcccttcct tccccacttc ggtcctcctc ttttcttcga     180
gggcccccac ccagccccct ccttcggggt cctcctcctc ctctgctctt tgggcgtccg     240
ccccgtcaat caccgccgtc tcggggcccc agcccggctc ctctccgcct cccgggctct     300
gggagtgcct ggggctcccg tctcggccaa cctccgctct gtgcagagcc ggggcgatct     360
gtcagcggag ctggccgagg ggggcggggg tgggagccgc ccgggccgcc ggggctcggg     420
ttaccggtga ctgacagcgt ctccatggcg aataatttga ctcgactatt gtctggcgcg     480
ggcaggcccc gggtcagata acccgaccaa tcagggcgcg ggccgccgcg cctcatgccc     540
gcttagaata atattattaa aaaagctgca agcgagctag acgggaggga gagcgaacga     600
gcgaggagcc ggcgagcgag cggcgggcgg gcgcggagca tgcggagcgg cgccccgggc     660
ggcctccggg cttgggcgcg ggcgaggcgc gcgggcggcg gggcgcgga gctgcgcggg     720
gccggcggcg ggagcgagga cggatcgttg tgactcagga gtcgctcggg agccggcgcc     780
tggccagggg gccccgcccg cctgtcggcc ggccggggcc ggcggggagg cgcccatgcg     840
gggccgcgaa gcgcggtgag ggcgcgcgcg ggcgggcggg cgcgcagccg ccaccatgtc     900
catgctgccc accttcggct tcacgcagga gcaagtggcg tgcgtgtgcg aggtgctgca     960
gcagggcggc aacatcgagc ggctgggtcg cttcctgtgg tcgctgcccg cctgcgagca    1020
cctccacaag aatgaaagcg tgctcaaggc caaggccgtg gtggccttcc accggggcaa    1080
cttccgcgag ctctacaaaa tcctggagag ccaccagttc tcgccgcaca accacgcca     1139
```

<210> 63
<211> 19
<212> DNA
<213> Mus sp.

<220>

<221> misc_feature
<222> (1)..(19)
<223> Forward Primer

<400> 63
gggttaccgg tgactgaca 19

<210> 64
<211> 18
<212> DNA
<213> Mus sp.

<220>
<221> misc_feature
<222> (1)..(28)
<223> Reverse Primer

<400> 64
cccgcgccag acaatagt 18

<210> 65
<211> 16
<212> DNA
<213> Mus sp.

<220>
<221> misc_feature
<222> (1)..(16)
<223> Probe

<400> 65
ccatggcgaa taattt 16

<210> 66
<211> 1443
<212> DNA
<213> Mus sp.

<400> 66

atgaacgacg tagccattgt gaaggagggc tggctgcaca aacgaggggga atatattaaa 60
acctggcggc cacgctactt cctcctcaag aacgatggca cctttattgg ctacaaggaa 120
cggcctcagg atgtggatca gcgagagtcc ccactcaaca acttctcagt ggcacaatgc 180

cagctgatga agacagagcg gccaaggccc aacacccttta tcatccgctg cctgcagtgg 240
accacagtca ttgagcgcac cttccatgtg gaaacgcctg aggagcggga agaatgggcc 300
accgccattc agactgtggc cgatggactc aagaggcagg aagaagagac gatggacttc 360
cgatcaggct cacccagtga caactcaggg gctgaagaga tggaggtgtc cctggccaag 420
cccaagcacc gtgtgaccat gaacgagttt gagtacctga aactactggg caagggcacc 480
tttgggaaag tgattctggt gaaagagaag gccacaggcc gctactatgc catgaagatc 540
ctcaagaagg aggtcatcgt cgccaaggat gaggttgccc acacgcttac tgagaaccgt 600
gtcctgcaga actctaggca tcccttcctt acggccctca agtactcatt ccagacccac 660
gaccgcctct gctttgtcat ggagtatgcc aacgggggcg agctcttctt ccacctgtct 720
cgagagcgcg tgttctccga ggaccgggcc cgcttctatg gtgcggagat tgtgtctgcc 780
ctggactact tgcactccga gaagaacgtg gtgtaccggg acctgaagct ggagaacctc 840
atgctggaca aggacgggca catcaagata acggacttcg ggctgtgcaa ggaggggatc 900
aaggatggtg ccactatgaa gacattctgc ggaacgccgg agtacctggc ccctgaggtg 960
ctggaggaca acgactacgg ccgtgcagtg gactggtggg ggctgggcgt ggtcatgtat 1020
gagatgatgt gtggccgcct gcccttctac aaccaggacc acgagaagct gttcgagctg 1080
atcctcatgg aggagatccg cttcccgcgc acactcggcc ctgaggccaa gtccctgctc 1140
tccgggctgc tcaagaagga ccctacacag aggctcggtg gggctctga ggatgccaag 1200
gagatcatgc agcaccggtt ctttgccaac atcgtgtggc aggatgtgta tgagaagaag 1260
ctgagcccac ctttcaagcc ccaggtcacc tctgagactg acaccaggta tttcgatgag 1320
gagttcacag ctcagatgat caccatcacg ccgcctgatc aagatgacag catggagtgt 1380
gtggacagtg agcggaggcc gcacttcccc cagttctcct actcagccag tggcacagcc 1440
tga 1443

<210> 67
<211> 17
<212> DNA
<213> Mus sp.

<220>
<221> misc_feature
<222> (1)..(17)
<223> Forward Primer

<400> 67
ggaacgccgg agtacct 17

<210> 68
<211> 17
<212> DNA
<213> Mus sp.

<220>
<221> misc_feature

<222> (1)..(17)
<223> Reverse Primer

<400> 68
actgcacggc cgtagtc 17

<210> 69
<211> 18
<212> DNA
<213> Mus sp.

<220>
<221> misc_feature
<222> (1)..(18)
<223> Probe

<400> 69
ctgaggtgct ggaggaca 18

<210> 70
<211> 725
<212> DNA
<213> Mus sp.

<400> 70

```
ccatggtgag caagggcgag gagctgttca ccggggtggt gcccatcctg gtcgagctgg      60
acggcgacgt aaacggccac aagttcagcg tgtccggcga gggcgagggc gatgccaccc     120
tacggcaagc tgaccctgaa gttcatctgc accaccggca agctgcccgt gccctggccc     180
accctcgtga ccaccctgac ctacggcgtg cagtgcttca gccgctaccc cgaccacatg     240
aagcagcacg acttcttcaa gtccgccatg cccgaaggct acgtccagga gcgcaccatc     300
ttcttcaagg acgacggcaa ctacaagacc cgcgccgagg tgaagttcga gggcgacacc     360
ctggtgaacc gcatcgagct gaagggcatc gacttcaagg aggacggcaa catcctgggg     420
cacaagctgg agtacaacta caacagccac aacgtctata tcatggccga caagcagaag     480
aacggcatca aggtgaactt caagatccgc cacaacatcg aggacggcag cgtgcagctc     540
gccgaccact accagcagaa cacccccatc ggcgacggcc ccgtgctgct gcccgacaac     600
cactacctga gcacccagtc cgccctgagc aaagacccca acgagaagcg cgatcacatg     660
gtcctgctgg agttcgtgac cgccgccggg atcactctcg gcatggacga gctgtacaag     720
taaag                                                                 725
```

<210> 71
<211> 22
<212> DNA
<213> Mus sp.

<220>
<221> misc_feature
<222> (1)..(22)
<223> Forward Primer

<400> 71
cgtcgtcctt gaagaagatg gt 22

<210> 72
<211> 20
<212> DNA
<213> Mus sp.

<220>
<221> misc_feature
<222> (1)..(20)
<223> Reverse Primer

<400> 72
cacatgaagc agcacgactt 20

<210> 73
<211> 16
<212> DNA
<213> Mus sp.

<220>
<221> misc_feature
<222> (1)..(16)
<223> Probe

<400> 73
catgcccgaa ggctac 16

<210> 74
<211> 8831
<212> DNA
<213> Mus sp.

<400> 74

gagcttcggg cgaaggcccg ggagcagcgg accgaggctg gcgcgatgct gttcccgggg 60
agcgcagtcg gctaccgttg aggaaggtgg agtgaggagt ggcccttcca gcgcccccta 120
tgtacgcctt cctgcgctcg gggccggtcg ccgcgttgcc cgcctccgta ccgcccgtga 180
ctctcggggc ccggagctcc ggcggcggcc ggggtcgagt cccgggggag gggaggcgcc 240
cgggcggcgc ccgagcttgc ggccgcggag cgagcgtctg gcaggtccaa gggtagccaa 300
ggatggctgc agcttcatat gatcagttgt taaagcaagt tgaggcactg aagatggaga 360
actcaaatct tcgacaagag ctagaagata attccaatca tcttacaaaa ctggaaactg 420
aggcatctaa tatgaaggaa gtacttaagc agctacaggg aagtattgaa gatgagacta 480
tgacttctgg acagattgac ttactagagc gtcttaaaga atttaactta gatagtaatt 540
tccccggagt gaaactacgc tcaaaaatgt cccttcgctc ctacggaagt cgggaaggat 600
ctgtatccag ccgttcagga gaatgcagtc ctgtccccat ggggtcattc ccaagaagaa 660
catttgtaaa tggaagcaga gagagtactg ggtatctaga agagcttgaa aaagaaagat 720
cattactcct tgctgatctt gacaaagaag agaaggaaaa ggactggtat tatgctcaac 780
ttcagaacct cacaaaaaga atagatagcc tgcctttaac tgaaaatttt tccttacaga 840

cagacatgac aagacggcag ctggagtatg aagcaaggca gatcagggct gcaatggagg 900
agcagcttgg cacctgccag gacatggaga agcgtgcaca gcgaagaata gccaggatcc 960
agcaaataga aaaggacata ctgcgcgtgc gccagctttt acagtcccag gcggcggaag 1020
cggagaggtc atctcagagc aggcatgatg ctgcctccca tgaagctggc cggcagcacg 1080
aaggccacgg agtggcagaa agcaacaccg cagcctccag tagtggtcag agtccagcta 1140
cacgtgtgga tcacgaaaca gccagtgttt tgagttctag cggcacgcac tctgctcctc 1200
gaaggttgac aagtcatctg gggacaaagg tggaaatggt gtattccttg ttgtcaatgc 1260
ttggtactca tgataaggac gatatgtcac gaactttgct agctatgtcc agctcccaag 1320
acagctgtat atccatgcgg cagtctggat gtcttcctct cctcatccag cttttacatg 1380
gcaatgacaa agactctgta ttgttgggaa attcccgggg cagtaaagag gctcgggcca 1440
gggccagtgc agcactccac aacatcattc actcacagcc tgatgacaag agaggcaggc 1500
gtgaaatccg agtccttcat cttttggaac agatacgagc ttactgtgaa acctgttggg 1560
agtggcagga agcccacgaa caaggcatgg accaggacaa aaacccaatg ccagctcctg 1620
ttgagcatca gatctgtcct gctgtgtgtg ttctaatgaa gctttcattt gatgaagagc 1680
ataggcatgc aatgaatgaa cttgggggac tgcaggccat tgcagagtta ttgcaggtgg 1740
actgtgagat gtatgggctt actaatgacc actacagtgt tactttaaga cggtatgctg 1800
gaatggcttt gacaaacttg acctttggag atgttgccaa caaggctacg ctgtgttcta 1860
tgaaaggctg catgagagca cttgtggccc agttaaaatc tgagagtgaa gacttacagc 1920
aggttattgc aagtgttttg aggaatttgt cttggcgagc agatgtaaat agcaaaaaga 1980
cgttgagaga agttggaagt gtgaaagcat tgatggaatg tgctttggaa gttaaaaagg 2040
aatcaaccct caaaagcgtt ttgagtgcct tatggaacct gtctgcacac tgcactgaga 2100
ataaggctga catctgtgct gtggatggag cactggcatt tctggttggc accctcactt 2160
accggagcca gacaaatact ttagccatta ttgaaagtgg aggtgggata ttacggaatg 2220
tgtccagctt gatagctaca aacgaagacc acaggcaaat cctaagagag aacaattgcc 2280
tacaaacttt attacagcac ttgaaatctc acagcttgac aatagtcagt aatgcatgtg 2340
gaactttgtg gaatctctca gcaagaaatc ctaaagacca ggaagccttg tgggacatgg 2400
gggcagtgag catgctcaag aacctcattc attccaagca caaaatgatt gccatgggaa 2460
gtgcagcagc tttaaggaat ctcatggcaa acagacctgc aaagtataag gatgccaata 2520
tcatgtctcc cggctcaagt ctgccatccc ttcacgttag gaaacagaaa gctctagaag 2580
ctgagctaga tgctcagcat ttatcagaaa ccttcgacaa cattgacaac ctaagtccca 2640
aggcctctca ccggagtaag cagagacaca agcagaatct ttatggtgac tatgcttttg 2700
acgccaatcg acatgatgat agtaggtcag acaatttcaa tactggaaac atgactgttc 2760
tttcaccata tttaaatact acggtattgc ccagctcttc ttcctcaagg ggaagtttag 2820

acagttctcg ttctgagaaa gacagaagtt tggagagaga gcgaggtatt ggcctcagtg 2880
cttaccatcc aacaacagaa aatgcaggaa cctcatcaaa acgaggtctg cagatcacta 2940
ccactgcagc ccagatagcc aaagttatgg aagaagtatc agccattcat acctcccagg 3000
acgacagaag ttctgcttct accaccgagt tccattgtgt ggcagacgac aggagtgcgg 3060
cacgaagaag ctctgcctcc cacacacact caaacacata caacttcact aagtcggaaa 3120
attcaaatag gacatgctct atgccttatg ccaaagtgga atataaacga tcttcaaatg 3180
acagtttaaa tagtgtcact agtagtgatg gatatggtaa aagaggccaa atgaaaccct 3240
cagttgaatc ctattctgaa gatgatgaaa gtaaattttg cagttatggt cagtatccag 3300
ctgacctagc ccataagata cacagtgcaa atcatatgga tgataatgat ggagaactgg 3360
atacaccaat aaattacagt cttaaatatt cagatgagca gttgaactca ggaaggcaga 3420
gtccctcaca gaatgaaagg tgggcaagac caaagcatgt gatagaagat gaaataaagc 3480
aaaacgagca aagacaagca agaagccaga acaccagtta tcctgtctat tctgagaata 3540
ccgatgacaa acacctcaaa ttccaaccac attttggaca acaagaatgt gtttccccat 3600
ataggtcaag gggaaccagt ggttcagaaa caaatcgaat gggttctagt catgcaatta 3660
atcaaaatgt aaaccagtct ctgtgtcagg aagatgatta tgaagatgat aaacctacca 3720
actacagtga acgttattct gaggaagaac aacatgaaga agaagaagag agaccgacaa 3780
attatagcat aaaatataat gaagagaaac atcatgtgga tcagcctatt gattatagtt 3840
taaaatatgc cactgacatt tcttcctcac aaaaaccatc attttcattc tcaaagaatt 3900
catcagcaca aagcactaaa cctgaacatc tctctccaag cagcgagaat acagctgtac 3960
ctccatctaa tgccaaaagg cagaatcagc tgcgtccaag ttcagcacaa agaaatggcc 4020
agactcaaaa aggcactact tgcaaagtcc cctccatcaa ccaagaaaca atacagactt 4080
actgcgtaga agacacccca atatgttttt caaggtgcag ttcattatca tcactgtcat 4140
cagctgacga tgaaatagga tgtgatcaga caacacagga agcagattct gctaatactc 4200
tgcagacagc agaagtaaaa gagaatgatg taactcggtc agctgaagat cctgcaactg 4260
aagttccagc agtgtcccag aatgctagag ccaaacccag ccgactccag gcttctggct 4320
tatcttcaga atcaaccagg cataataaag ctgttgagtt ttcttcagga gccaagtctc 4380
cctccaaaag tggtgctcag acacccaaaa gtcccccaga acactatgtc caggagactc 4440
cgctcgtatt cagcaggtgt acttctgtca gctcccttga cagttttgag agtcgctcca 4500
ttgccagctc tgttcagagt gagccatgta gtggaatggt gagtggcatc ataagcccca 4560
gtgaccttcc agatagtcct gggcagacca tgccaccaag cagaagcaaa acccctccac 4620
ctcctccaca gacagtgcag gccaagagag aggtgccaaa aagtaaagtc cctgctgctg 4680
agaagagaga gagtgggcct aagcagactg ctgtaaatgc tgccgtgcag agggtgcagg 4740
tccttccaga cgtggatact ttgttacact tcgccacaga aagtactcca gacgggtttt 4800
cttgttcctc cagcctaagt gctctgagcc tggatgagcc atttatacag aaagatgtag 4860

aattaagaat catgcctcca gttcaggaaa acgacaatgg gaatgaaact gaatcagaac 4920
agcctgagga atcaaatgaa aaccaggata aagaggtaga aaagcctgac tctgaaaaag 4980
acttattaga tgattctgat gacgatgata ttgaaatatt agaagaatgt attatttcag 5040
ccatgccaac aaagtcatca cgcaaagcca aaaaactagc ccagactgct tcaaaattac 5100
ctccacctgt ggcaaggaaa ccaagtcagc tacctgtgta taaacttctg ccagcacaga 5160
ataggctgca ggcacaaaaa catgttagct ttacaccagg ggatgatgtg ccccgggtgt 5220
actgtgtaga agggacacct ataaactttt ccacagcaac gtctctaagt gatctgacaa 5280
tagagtcccc tccaaatgaa ttggctactg gagatggggt cagagcgggt atacagtcag 5340
gtgaatttga aaaacgagat accattccta cagaaggcag aagtacagat gatgctcagc 5400
gaggaaaaat ctcatctata gttacaccag acctggatga caacaaagca gaggaaggag 5460
atattcttgc agaatgtatc aattctgcta tgcccaaagg aaaaagccac aagcctttcc 5520
gagtgaaaaa gataatggac caagtccaac aagcatcctc gacttcatct ggagctaaca 5580
aaaatcaagt agacactaag aaaaagaagc ctacttcacc agtaaagccc atgccacaaa 5640
atactgaata tagaacgcgt gtgagaaaga atacagactc aaaagttaat gtaaatactg 5700
aagaaacttt ctcagacaac aaagactcaa agaaaccaag cttacaaacc aatgccaagg 5760
ccttcaatga aaagctacct aacaatgaag acagagtgcg gggggagcttc gccttggact 5820
caccgcatca ctacacccct attgagggga cgccgtactg cttttcccga aatgactcct 5880
tgagttctct ggattttgat gatgacgatg ttgacctttc cagggaaaag gccgagttaa 5940
gaaagggcaa agaaagcaag gattccgaag ccaaagttac ctgccgccca gaaccaaact 6000
caagccagca ggcagctagt aagtcacaag ccagtataaa acatccagca aacagagcac 6060
agtccaaacc agtgctgcag aaacagccca ctttccccca gtcctccaaa gacggaccag 6120
atagaggggc agcaactgac gaaaaactgc agaattttgc tattgaaaat actccagttt 6180
gcttttctcg aaattcctct ctgagttccc ttagtgacat tgaccaggaa aacaacaata 6240
acaaagaaag tgaaccaatc aaagaagctg aacctgccaa ctcacaagga gagcccagta 6300
agcctcaggc atccgggtat gctcccaagt ccttccacgt cgaagacacc cctgtctgtt 6360
tctcaagaaa cagctctctc agttctctta gcattgactc tgaggacgac ctgttacagg 6420
agtgtataag ttctgccatg ccaaaaaaga aaaggccttc aagactcaag agtgagagcg 6480
aaaagcagag ccctagaaaa gtgggtggca tattagctga agacctgacg cttgatttga 6540
aagatctaca gaggccagat tcagaacacg ctttctcccc cgactcagaa aattttgact 6600
ggaaagctat tcaggaaggc gcaaactcca tagtaagtag tttgcaccaa gctgctgcag 6660
ccgccgcgtg cttatctaga caagcgtcat ccgactcaga ttccattctg tcactaaagt 6720
ccggcatttc tctgggatcg ccttttcatc ttacacctga tcaagaggaa aagccattca 6780
caagcaataa aggcccaaga attctcaaac ctggagagaa aagcacatta gaagcaaaaa 6840

aaatagaatc tgaaaacaaa ggaatcaaag gcgggaaaaa ggtttataaa agcttgatta 6900
cgggaaagat tcgctccaat tcagaaattt ccagccaaat gaaacaaccc ctcccgacaa 6960
acatgccttc aatctcaaga ggcaggacga tgattcacat cccagggctt cggaatagct 7020
cctctagtac aagccctgtc tctaagaaag gcccacccct caagactcca gcctctaaaa 7080
gccccagtga agggccggga gctaccactt ctcctcgagg aactaagcca gcaggaaagt 7140
cagagcttag ccctatcacc aggcaaactt cccaaatcag tgggtcaaat aaggggtctt 7200
ctagatcagg atctagagac tccactccct caagacctac acagcaacca ttaagtaggc 7260
caatgcagtc tccagggcga aactcaattt cccctggtag aaatggaata agccctccta 7320
acaaactgtc tcagctgccc agaacatcat ctcccagtac tgcttcaact aagtcctccg 7380
gttctgggaa aatgtcatat acatccccag gtagacagct gagccaacaa aatcttacca 7440
aacaagcaag tttatccaag aatgccagca gtatccccag aagtgagtcg gcatctaaag 7500
gactgaatca gatgagtaac ggcaatgggt caaataaaaa ggtagaactt tctagaatgt 7560
cttcaactaa atcaagtgga agtgaatcag acagatcaga aaggcctgca ttagtacgcc 7620
agtctacttt catcaaagaa gccccaagcc caaccctgag gaggaaactg gaggaatctg 7680
cctcatttga atccctttct ccatcttcta gaccagattc tcccaccagg tcgcaggcac 7740
agaccccagt tttaagccct tcccttcctg atatgtctct gtccacacat ccatctgttc 7800
aggcaggtgg gtggcgaaag ctcccgccta atctcagccc cactatcgag tataatgacg 7860
gaaggcccac aaaacggcat gatattgcac gctcccattc tgaaagtcct tccagactac 7920
caatcaaccg ggcgggaacc tggaagcgtg aacacagcaa acattcctcg tcccttcctc 7980
gagtgagtac ttggagaaga actggaagct catcttctat tctttctgct tcatcagagt 8040
ccagtgaaaa agcaaaaagt gaggatgaaa ggcatgtgag ctccatgcca gcacccagac 8100
agatgaagga aaaccaggtg cccaccaaag gaacatggag gaaaatcaag gaaagtgaca 8160
tttctcccac aggcatggct tctcagagcg cttcctcagg tgctgccagt ggtgctgaat 8220
ccaagcctct gatctatcag atggcacctc ctgtctctaa aacagaggat gtttgggtga 8280
gaattgagga ctgccccatt aacaacccta gatctggacg gtcccccaca ggcaacaccc 8340
ccccagtgat tgacagtgtt tcagagaagg gaagttcaag cattaaagat tcaaaagaca 8400
cccatgggaa acagagtgtg ggcagtggca gtcctgtgca aaccgtgggt ctggaaaccc 8460
gcctcaactc ctttgttcag gtagaggccc cagaacagaa aggaactgag gcaaaaccag 8520
gacagagtaa cccagtctct atagcagaga ctgctgagac gtgtatagca gagcgtaccc 8580
ctttcagttc cagtagctcc agcaagcaca gctcacctag cgggactgtt gctgccagag 8640
tgacaccttt taattacaac cctagcccta ggaagagcag cgcagacagc acttcagccc 8700
ggccgtctca gatccctacg ccagtgagca ccaacacgaa gaagagagat tcgaagactg 8760
acagcacaga atccagtgga gcccaaagtc ctaaacgcca ttccgggtct tacctcgtga 8820
cgtctgttta a 8831

<210> 75
<211> 52138
<212> DNA
<213> Mus sp.

<220>
<221> misc_feature
<222> (32166)..(33122)
<223> n is a, c, g, or t

<220>
<221> misc_feature
<222> (38299)..(38398)
<223> n is a, c, g, or t

<400> 75

```
gttccagaca gtcccacagg tgcagattag gagtcgccca ctcgggccac tactttctaa      60
ggctggttcc cagtaccact aaccatttcc cccaagtttg ctcccacccc gcgcctccca     120
gtaccttgcc cagagagaga aggtttactc attttgtgaa gaaaccaaca ttcaagtttc     180
cttggggtcc accgtggagc tacaggtact ctccttgtgg gcttcagacc ccctgctcta     240
agtgtaacta ttgcatacgc tgtgtgctgc aactgaatga ggacacaggt agttcttgag     300
ctgacagtga gggacactga aacaggcaag aagccataaa gatggaagaa ataagggatt     360
aattgactaa tgaaaacaaa aatgcatgag ggataaaagc taggtagaga tggggcagag     420
gaacaagggc ttcagcctat cagagatcac tgtccctcca gtgccacagg agagaaggat     480
gcgttggaag gtggggtcct gggactggcc agagacaggg gcggagccag cgcctgaagg     540
cagggccggt cgcagggtgg agccagaccc aagcgcaagg ctggcccgct gctggccact     600
gtggcaggga gctgccgcga gtgacagggt caagaagttg gtgggctgcc acgccgagct     660
tcgcgggctc ctgctcccac accagcagca ctcacttgcc cggagtcatg ccctcctaca     720
cggtcaccgt ggccaccggc agccagtggt tcgcgggcac cgacgactac atctacctca     780
gcctcattgg ctctgcgggc tgtagcgaga agcatctgct ggacaaggca ttctacaatg     840
acttcgaacg gggcgcggtg agaatgcgcg cttgggaccg acggctggca gcaaagagcg     900
ggagggcggc ggggcaggac aggcaggcac ctgagaactg tctgtcccag cgcgctcgtg     960
accctatgtg agcgcatctg gggatctgat gcagcgccag agtcggtgca tgccagggca    1020
agcgaggcac cctagacttc ctgatgactc gggtatctta agggacaaat gacttccaat    1080
gtgggggact gatgtggctg gcctcttatg tgagagatgg cacaaacttt caccaacagg    1140
acccagaatt tgtgagaggc cccttcacct cgggagttct gaggtcctag tgccccagag    1200
tccagtacca ctgcaaagca tagaaagccc tgttcgacag ctagccattt tgtgtagaca    1260
gtgtaagtcc agggtaagtc agagatccag gatcgggagt caacttgggc ataatcactc    1320
ttctatccct actgtggacc ttcgtttacc aaactaaaaa ttggttaggt ttaatcttac    1380
ccatgagtca tgagggagcc cagtcccatt tgggggctag gaatgagtcc agggattgcc    1440
```

ccccacactt aggtacccta catttcctgc actcagtccc agtggaggaa tgtaaaggaa 1500
ggaaggtctg gcccgagcag cctttggaaa gactgtcagc agggtgcgtg caagaggaca 1560
cttcctccct gagtattagc ttctgaagga aaaaaggaag aaagatttcc tttcttccct 1620
cctaatacag actttggagt ctggcagccc cacaggcact gtgggaggcc attccgtttg 1680
gatgctgctg gtgtgtaaag tctagcccca tgactttctc ttaacacagg caggtcatgg 1740
ttttcagtga cccactaagt gcctagtaca tcagacttgc tcagtaagtg agtctgtaat 1800
agacaggcac gctgcagacc cttggggtgg gggtgggggg ttcctcttcc ttctactacc 1860
tccaggtcta aactaggctt aggtttgatt ttaccaggct gccttcttat catttagttt 1920
actattgagt cgacccacat gaacttgctt agaattaacc atttgtgata agcacaatga 1980
caatttcata tgcttccact agatagatct gttcagccta aaccaaggaa atgatagtta 2040
agcccttttc tgggtcccaa actctaagca cgacgcttac attcctatcc tgggacctgg 2100
tactttgccc tgattagcaa gcttctaacc aggcttgaac aagcaagcgt gggtgttgac 2160
ctcagaatgg catcttttgc tcagtttcac cagagctggc cagagaatgg tgctgccaaa 2220
gaccaagcat ggcatccgtt tgggatgctg acaccctgtg caacctccaa agcacttgtg 2280
tttattttca gtaactgggg cttctcccag tatacagggg gaggaagaga ggacagaatg 2340
cttcctcttt ataaatggac tacagggggc cttctccaca aatctagcta tcagtgggtt 2400
cagtctaggt gcagcacagg acaccttatg tgtcatttcc tccaggagga gaatggcaat 2460
gtggccatca taaatgactg gacaggaagt aagaggcctg tcctgttcat catttgcctt 2520
tctgtcctgc ctcccaacct gaaaagtcat tcaggtgaca ttaatttaac accttagcaa 2580
gaaccccaga ggcaaatttc aggagagatt tgcatacata tttccattgt ggggagggaa 2640
ccacagctca agtgaactgc tgtcttctgg ctggatgcaa aaagagcctt aaaaaaagaa 2700
aaagaaaaca gcctttgaga aagttcctgt tgatgcaaag ttactccata ctttgtcttg 2760
cacagtccag agccacttcc tcattctgtg gccagtgtat ctttaaagtc cagatgtccc 2820
ttttgggtaa aggtagaaaa gaaaccttaa gggaagtgtt agctaagaag ataaggttat 2880
cccactgttc ttagaaaagt gccacattgt ttccatgaat agcagccacc gtgaaggccg 2940
gccagccact tccagcaccc cttcctaatg tacgaccgaa taatggtcag tgctaacctg 3000
ttttaataca ttccactatt gcccatcccc catgattccc acatgagttc tgtgtagcga 3060
ttcaggtcag gagcctttgc agatgggatt ctcctgagtg tcaggtgtca ggcatcaggg 3120
tttccctgtt gagaacccag acggaacaga atggctttgt tccataagct agtcctacgt 3180
ggcacagctc taacaaacct tgaaatcttg agtgcacatt agttggctac tttaactgct 3240
caacagtact ttgaaaagct aaggataggc tgtcctgctg agtatatggt tcttgaacat 3300
attcagtaca tgtaaattca tcttacaggt aatacgcttc ttaaatacat ctaacaaata 3360
ttcttatatt tatagagagt aaattctata gtacctcttc atgaggaaga gaatactgat 3420
cgggaacaaa catttctttc ttcatcagcc ttcatatact agttctctct cactatccat 3480

ctcccctacc tctgcccttc gtcctcctct tcttccttcc tttatccccc aatggtcaat 3540
ttctctcctg ctgctcaccc cccaatctct ccttcctttt ctactttaat gcccccttacc 3600
tctctccttc agcttctcct aactgcaacc tcactttccc acttcggtcc ctacatcctc 3660
ttgactactg ctcccagtgt tctcattctc tgcaactttt gctcttctta ttgctgcctt 3720
tggtctctca ttccctctgc cccagtctct cttttgccct ccagcctccc accccccttct 3780
ctgctctaca gcctcttcct cctcccacca gtttctcata ccccgtccaa gatggacagc 3840
caagttggcc atgcggtcgc tccagaagag atccattttc tcttccaccc atccacagag 3900
aggtgatata actgaaggtg gacaatgctt gaggaatata gagttgtcct ctggcagaga 3960
tggaagtgta aatgaatagt tgagtgttta taaaatggtg atagagagct cagaggctga 4020
tcaacaccac tttgctgaga tatgcatgta ccacatgatg atgtttgtgt cacaacagtc 4080
cacgtatatg atggtgtcct tgtaagatta tgacagagct gaaatttcat ttttcctgga 4140
atattcagta tgtttaaaca cataaatact tattatatgt tacctaatgt attcaataca 4200
gcaatagtca gtacatatat attacttagg agcaattggc tataccatgt agcctgggtg 4260
tgcagtatga tatatatcta tgttcatgta aatatacact atgacagaat tgccttatac 4320
ttcattttct agaatgtacc cctgtcactg atgtatgatt gtgtttggat actttaaaac 4380
taaatataca acagagttgt cctgacttgg gctaatgaac tacattcttc ttgtgtctct 4440
gaccaatgca gtggcatctc tgtcttgaga gctgtgttct agccatgtcc tcctctgtgt 4500
ggattcccta aattgagaat attgttctgc caactccctg tatctgaaat ctcctgccca 4560
ctcctctctg ctctctgtac ctctgaggtg gtcagtcact cactaacaca tcgatacctg 4620
ttctctgagt ctcagataca cctgtaaaat ggagtgactg tccacatcaa catttacatg 4680
tgcagcattc cctgagtacc tggatcaagc cccctttata gtacccacca taaagaactc 4740
agtatgtcaa ggacatagac aacaaatgaa gtgtcgtccc aacacactca accataaaca 4800
aggacagaaa aggtcagcac atttggtggc tccacatgcc cttctgaggc cttctttttg 4860
tgagacacca cctatcaccc tgtattttcc tagagggaag cctctctctg ctttctgtga 4920
tcttgttcta aatacactct ggaactctag acccacaaac aacatacttt atctctaaga 4980
cttccggggt tctaactgaa cccttcaact cttgtttatc tcctgtcaca tccaggcctg 5040
agggcagagc agcaaggtct ctgggttgac aagagataaa ccagactaca ggcctgacct 5100
ccatcttctt tggaagatcc tactgattaa gtccctctaa gattgcacaa tggtctattt 5160
cagtgcagtt ctgaggcccc cagccaggtg gagttaagga cccatttcct gtgactatac 5220
tataggtgtg ctaaagggtc agcctcccct cccccagaaa agctactgtt tttgtatgtc 5280
tgtcttttgt ttgttttgtt tgtttgcttg cttgcttgct tgcttgcttg ctttgtttgg 5340
tttggtttaa tttagtttgg ttggattttt ttgagacagg atttctctgt ctagccctgg 5400
ctgtcttaga attacagctc tgtagaccat gccgccctca cactcacaga gattcatctg 5460

tctctgcctc ccaagtgctg ggattcaagg catgcaccac caccacccag ctgctcagtg 5520
tctttcttac aggtcaccag ttgtagctgc atagatggcc tcaaagtaca tggctttctc 5580
ctcggatctt tctcattact ttagaatggg tgttttctct tcattcactc ctgatgtcat 5640
ctttccaacc ctcaagaccg tcctctctag tctctcaatg ccacaggaaa accacttcct 5700
ctgaagcagc tgctctgggg ctgggtccag gcattcttgg tggctgccct ttggtcttga 5760
ccacaggctg acttccctct cctctgcatg cactataagt cccacccact gtcccttgtc 5820
cttacaagag ttctccatcc acccagtcca gggcttaaac acatagagtg tttctctcac 5880
tccacgggca cttggagcta tgaatgaatt gcagtgcaaa gggttggctc aggctggtcg 5940
tagctagttc taatatatat atatatacct agtggactat gcccttggag aaggtagcta 6000
ctcaagtgtc ccgccactct gatttctcaa tgatgctgca agccagctgt tatctcttct 6060
taaaagatga agaaacacag ctggggtgat actcaggcag tcaagtgttc accatgcaag 6120
cacaaggacc caagtttgat ccttagaccc catatttttt aaaagccagt aatcctgggg 6180
ctacattata attctagtcc tgagacaaag gagacaggtg gatctctgag gttttctggc 6240
tagccagcct agcctagtta gtcagttcta ggccagtaag agacattgat ttggtgtagg 6300
gagtgatagt gcctgaggaa tgacatccaa ggttgcccac tgtcctacac acacacacac 6360
acacacacac acacacacac acacacacac acatctcctc atatgtacat gtgtgagact 6420
gaacaatatt ccattgcatt catatacact atattctctg tactcattca tatgaatact 6480
cattcatata tctttagcca agattcttaa ggaatctctt cattaattta ggtagactca 6540
cctcctgcaa gaccctggac cctaacttta cacagatgcg gctccagtga cacaggaatg 6600
tcttcagctc aaaaactgat cacatagaaa tagagacaag agtcagtcag tagcatccag 6660
gaaggagtag gggtggtggt gttgagagga ggcaccaaaa taaagttaga taggagacat 6720
atgctttaat ttttcatggc acagtacagt gacaataact cataatgatt tttgtatatt 6780
tcaaagtctg aagagaaaat ttttaatgtt tcttagatga gtatttgagg taacagaaat 6840
gtcaattacc ctggtttgcc agtgtttttt ctatacttat attgagaggt catattgtac 6900
ttcacaaact tatgattatc atcagctata aaaattaatt atatagaaag aaaaactccc 6960
ttgtcccatt ttagataagt caagcttagg aattaaatta gcagaagcca aaaataatag 7020
gagaaaagtg gacaaatttg cctaatgcag caatgtgtgt catatgttac ttaagaccaa 7080
aagaagactc acaaaggcag tgggagtcca cagctcacac actaaattgt atgtaaaaca 7140
tgtgataaag agagttgggc tagggaagtc agatggagtg actgtggctg tttgagatgg 7200
ccaaccgggt ctaactgtac aggtttctgc tttccttcta aggaagacct cttcacacga 7260
aggcagccat tagaatgatc ttgaactgcc attttctatc acttaactca agtatctgct 7320
gtgctagagc agcacgttga ggaaggaggg ttgtccacag catcagtatt caggcccagg 7380
aggcagtggt ggttgattgt cctgttttcc tgcccaggtg gactcctacg atgtcaccgt 7440
ggatgaggag ctgggcgaga tctacctagt caaaattgag aagcgcaaat actggctcca 7500

tgatgactgg tacctgaagt acatcacact gaagacaccc cacggggact acatcgagtt 7560
cccatgttac cgctggatca caggcgaggg cgagattgtc ctgagggatg gacgtggtaa 7620
gctgctcgga cccctgacac tcgtaggctt cctggaaact agaaagtctc cccttctcag 7680
agagctctat ttctgggcgt gagaattccc tcttgggtag agcacacctc tgtatcttgt 7740
tccctcatgg gagatgagat cttcctctgc tctaacgagc tctagagtgg agctaatagc 7800
ctgaggagtc ataccaacta tggcttccct ctgcacactg tcctggaaag gtgtggaaac 7860
aggccactag gtagtgaggg cctcttaaca ggcacccgtg acaagaatcc tgcaaatggg 7920
gagcgctgct agttagcaca tgacctctta agccaaattc tctggctgtc ctctctaagc 7980
caggggagtg gggaggtgtt cagggccata ggacccctcc tgaaggcctg gacgctaaag 8040
gtttggacta agattgccaa tataatgcca gctctagggg ccagaggcaa gctggagttc 8100
actacccccct gttttacttg tttgctttga aacaaagcct cagtttcact acatagctca 8160
ggctggcctt gaactttcag ttctgctgat ttagcctccc aagatcctag atatgcaggg 8220
tccaacttgg tacttccata agaggccatt ctttttcaga ccccactccc acagagggtc 8280
agagaaggaa gatgtcaaag atcagctgtt tgttgtttgc actcccccct cacctccctc 8340
tgagtgacac ccctcacccc ctctgagtaa caccatttgc acactagctg cttatatgaa 8400
tgggtcaaac taagccttgg caaacttcta tcttgtagtc gtaacccttg acttcgcttt 8460
cagtctgccg acagccactg cagtgaggat gactgctgat gcttatgaca gcaatgagca 8520
tctctcagat aaggatcttc tgccgttgtt catcttcaca gttgggaggg gcacacaaga 8580
tgagattgat aattaaacca ccaccatttt gatagaaaaa taagacagag gggaagaaga 8640
aggggaagaa gaggaagaaa gaggaggaga aagaaggagg aagaaggagg aggaaggagg 8700
aggaaggagg aggaaggagg aggaaggagg aggaaggagg agaaggagga ggaggaggaa 8760
gaggaagaag aagaagaaga agaagaagaa gaagaagaag aagaagaaga agaagaagaa 8820
gaagaagaag aagaagaagg aagaagaaga agaagaagaa gaagaagaag aagaagaaga 8880
agaagaagaa gaagaagaag aaggaagaag aagaagaagg aagaagaagg aagaagaaga 8940
agaagaagaa gaagaagaag aagaagaaga agaagaagaa gaagaagaag aagaagaaga 9000
agaagaagaa ggaagaggaa gaggaagagg aagaggaaga ggaggaagaa gaagaagaag 9060
aagaagaaga agaagaagaa gaagaagaag aagaagaaga agaagaagaa gaagaagaag 9120
aagaagaaga agaagaagaa gaagaagaag aagaagaaga agaagaagac cctctagggt 9180
cctttggctc actgtccttg atgtttaact tgacttacac caaattctgg gaaacctttt 9240
gacttggata cttgggttta gaaagatttt tatcacccct cccccaagct cagtgggaac 9300
ccccgcctcc actcacccac tcacccacag atcatttaag tcatcctgtg gctagaacaa 9360
ggaagggtgc ttctcttaac ccagggttta gcaggcctta gaatctattt tctgtctgct 9420
ccacccaaga tctcagtggg gaaatctttc ctgcctacag agtctgtagc tttgcacagc 9480

agcatgcttg ataacgcagg cagattggaa tgatttgagg aggtgttgag agagggacat 9540
caagaaggtg tgaaaatgtc cagaggggcc acttctgtat gtagggtcgg cagcttggcc 9600
aatcctgatg agctgggaga gaagaagagt ttgagaacaa tgtagtaagt ggcattttaa 9660
agataacctc tatcttatga gtcaatgcag agctgtgacc tcagtagaga agtggacatc 9720
cctggagatg aggaagagca gaatggagaa gacataacag ccccaccctc accacacaca 9780
cctcccctct gctctgtccc cagagctaaa tctaatggga agtccattga tgatatctat 9840
tcttatcagc tttattgaca agataaaatg gaaggggggt cttcagagac acaatctggc 9900
tgacaacttc cattaaccat ggctggtaac cgaccatcat agctttaatc agtcatttaa 9960
tttggctagt gatcatgtag gtctagaacc ttggaaggga tctgccaagt acttggtctg 10020
tgtcctgtgt gccattaact caatggactg caagcttcac cttgaagatg gttcttcttc 10080
atggccttca tctctgcata acaccccatt tttaaggagt ctcacaacat gagggactca 10140
gggtagcctc atttcttaca tagtagctca cttctttttt ccttcttctc tttttattag 10200
atattttctt tatttacatt tcaaatgcta tcccaaaagt cccctatact ctcccccccc 10260
ccgccctgct cccctaccca cccactccca cttcttggcg ctggcgttcc cctgtactga 10320
ggcatataaa gtttgcaaga ccaaggggcc tctcctccca atgatgacca gctaggccat 10380
cttctgctac atatgcagct agagacacga gctttggcgg tattggttag ttcatattgt 10440
tgttcctcct atagggttgc agaccccttg gtagctcact tctaagagca aatattccaa 10500
gaaacagaga atagatgctg ccaggatcca ggcctgtgcc tggaacaagt atagtgtaac 10560
tccaccaccc agccttgagg ggaagaacac caaaatatct caatttaaag aatgccagag 10620
gatttgggac cataattaac ctacatggca caacaaccaa cgccttgaac aatgtggcaa 10680
gaagagggcc cattccacaa gtgaccccat ttcccaccac tgctgggggc ctgcttagcc 10740
ttagaggagt gagctgaggt gggacacaga gttggatgaa ggccaaagac tgatgatctc 10800
tggccttgaa accctctctc ttgaaactca gtacagttag tactatcaat aacaagttca 10860
aagcagtatt tcatattcat tataagttca aagcagcagt ttttacatgg cctggtctta 10920
tcatagtgca cacctgtggc ttcatccttg gacctgaatg tatttccttg aacacaaatt 10980
tgtctcaagc cttatttctc tttttagtgt agttatgaaa gctcactgta tgtcttatta 11040
tgcagccttt tcttgctatt atgaggagtg ggtacattct attcttgatt ctaactttat 11100
tatatctttc tggcaaaaca actttcttgg aaccttcttc ctataattat ttaagttgaa 11160
tcatgagttc tatataaaat ataaaatcat taattcattt aaacagcatt aattcataaa 11220
agtccatctt catgttgatc tgcaggaaat ttgcccaata gcatgcactg atgcccagaa 11280
gttgttagac tgtataatag tgatatgagt gacatatgat tagcaagaag ccattctggg 11340
atgagtcttt tgggacttca cctcccagag ctcacccatc gcaaaccatg caaaaaaacc 11400
agtgagccat ctccaggaaa gtcatccaat agccttagcc cctcctagat atcttctgac 11460
aagccacatg atggcctaca tctcatattt taccccttca cctcagtaat cataaaagtg 11520

ataggactat caatgtggtc ccagaagcta tagcagatgt gtgtacaaat ggttcattcc 11580
agagctcatg cgagcccctg gggatgggtt ctgccatact cagttcagaa gcagctgaat 11640
cggtcatcct gcagcctggg aagccccatc cattgtaagc cggagtctca taactgggga 11700
gactgagtca gtctcttgag tcaacttgtt tttagttcct tggaactcta ttcataccaa 11760
acgcaggaaa aaaaaaaaaa gaatcgtctt agagtcaaga tgtccaagga atattctgac 11820
catctaaggc atttagcaaa gaaaatgtca aatgttcaca acagtgtcta gcacagtcag 11880
tgacaacagg cattgccaca gtttagactc acctcaacct cggggctatt gatcctgctc 11940
tgttgtcttg atcctcccct ttgggatcac ttctatactc gaggctggac atacagtgac 12000
ctagagagct ctggggtttt ctgagaactg aggggaatgg atctgctgta agggaaacga 12060
gaactgagag ttggtgccta agaaccattc acggcgcctt gaaacaatca tcttgtggta 12120
gctaagccta atgaaataaa aaaaatgact tgtaatcttt catgtttttt atttaacttt 12180
tccatcaatg ttaatatgtt gttcaaaata taaatgttta gtgcaattaa atatttaact 12240
caggtagcta gggagtatag tatactacag tacactgcag tacagaataa tacagtatat 12300
attataatca ccttttcaga ttcttacttg cgaccctgtt tgctgaattt accttagatt 12360
acagtatagc tgattaccat ttcatctgtg ctgaatgctc tgagaaaggg cctttagcaa 12420
cttgtcaaag ttcctgaaat ggcagatggc atgacaggcc aagtgacaaa atgctgtgac 12480
tcttaggcct gcaggcaagc aggaaaccct tgaggttatg ggttacccat tatgggggta 12540
agacataata accattattc tcaaatatta gacacataag aagaccaatg ctttgtagaa 12600
tgctttacaa tgtttttaca ttaacattct cctaagatcc caagaagtag gcgttatcat 12660
ctatatttta tatgaggcag tcaaggtagc ttgctctggg tcacatgact ttaaaccaca 12720
gatcccaagt ccatgcctgc tgtatctctt ttacataccc ccacagccta gatgagcaca 12780
tttgttttag agtagagcct gtcttctgtg taaatagcac aagggactgg acagtgtctt 12840
gcatattggg acttacagca tgcatttctc catgaagcaa ccaaatggac ttaaaagcat 12900
gagccatgag tcctgctgtt ttctctggcg gtatgaaagt cagctgctct ttcacctcaa 12960
tatctcaaaa taacagttat tggatcactc taaagtggct tattaatatc caccaaagcc 13020
tgtcatgagg tgtatactta taatcccagc acttggaagg cagaagtaag aaaatcggga 13080
gttcacagcc agctttgggt aaatagaaag ttccaggtca gccgagttat gtgagatctt 13140
gtctcaaaca aacaaacaaa aaacaaaaaa tctaaacaaa acaagggtat ctaataaacc 13200
ccacaaaata cctctcagct tgtcttggca gacaggtcta gaccaagagc cccaggattc 13260
ctgcataagc agcatgctga aggctaatgg aaaaggctgg gaaaacctga ccacggggca 13320
ttgtgtggac attgttgctc ttcctcaggg tgctctcata tttgtccttc ctctgcagca 13380
aaattggccc gagatgacca aattcacatc ctcaagcagc acagacgtaa agaactggag 13440
gcacggcaaa aacagtatcg gtgagttgtg gcatggacca agtggccgtg agtagcccct 13500

tctggaggag gaccatggta ggagacaagc ttgccaatgt cacatggacc atattcttac 13560
ctccgccttc aggcagtcag agtgaagggg gtcaaagaaa agactccttg gggagaatgg 13620
gtccaagaga gtgagtcgcc tttcccatta ttgtatgggc tgcctcaact atgtatgtct 13680
tcatgtaata ttttaactat aaaagtgata cataatctac aacagcatca aaaagtcact 13740
agacgtgatc aagaaaaaag ggtccaaatt attcaaatta aacataagga tgtaatccta 13800
gcactactag aaagatggtg gtggtgacaa agagcagcca gcctggaata cagagcccag 13860
cagtagaaac aaccccaaaa ggtgaaaagc cattaccagc tcccaaaagt tgacctctga 13920
cctccacaca cagtgttgtg tgtgcatgcc tgaacataga tgcacacaga cacccacata 13980
cataccacaa acacaataca atatttttaa gaaaatagag aatctgaata agccaatccc 14040
atgcaatata atttaattaa caattttcag atgtcctata aataaaagct tggatagctt 14100
catgagtaaa ttctagcaaa cattaaggaa ttaatatcaa tcttatatat atattcccac 14160
aaataggtga aaaggcaata ttttccaaca tatcgtatgg taccaatgtt gtacaaaaat 14220
taggcaaaga catcattaaa aaataaagca atacacaaat acctcacaaa aatacaactg 14280
caattatttt ctacaaaata ttagcaagcc aagttcatat ggtattaaca aataagattt 14340
ttccctagga atacagtgct tttgaacatt caaaactttg ttaatctaat atatatgtaa 14400
aaaatgaacc aaagtactta tacacatata taaaatatat aatatagata cacaaaaagg 14460
aatttgagaa aactcaacat tctttcataa tgaaacattc aaaatactgg aattgaagag 14520
aatgtcctca acacaataca gaacgtctat ggaaaactcc caagacaaag tgcttttccc 14580
caagatctag aaatgatagg acacccactt gtgctgcttc tgttcgatat ttcactgaag 14640
ggtctatttg gtcagctagg aaagaagaag aaaatgtttt acacaatcac ctttcataga 14700
tgacacagtc ttttatataa ctaataaatt caccaaaatt taattattta tgaaaaacta 14760
ataagttagc aaggttatga gatagaaact aaatcaaaaa attgatatat tataacaaaa 14820
gaagaaaaaa atctacttac agtaagttca aaatcaatag aatatttatt aggatgttta 14880
acaaaagcat tgtaagacta gtgacaaaac cctagaacac agttgaaaag aataaagaaa 14940
atctaagtaa gtcgaaaata tctcatgtcc ccttagcttt gctaaagtgg caatccttct 15000
cctgggaatg acggatgatg gagatccaat gcaatcgctg ttaaaatctc agctagtgtt 15060
ttttaaagca agattgataa gatggtcctc aaatccatat ggaaatgtat ggaaaggcat 15120
acaaagttac cagaccaatc ttgaaaatag gaaaactaag ttagagaagc tatatttccc 15180
aacatccaaa tttctgcata atcatagtat tcggacgaaa tgtagccgta tcatgagcaa 15240
tgacatagac ttccaagtcc aggaaaaaaa atcccttata cttacagtga attgaatgtg 15300
gtgggatcag caagacaaat ctgtatgtaa agaataacct tttaaataaa tggagccaag 15360
gaaactagat aggcaaagta caaacaacca tgacattgtg cacacatgta caaagtacat 15420
gatgttggat ccttacctaa caccaaattg attacctaaa aggtggaatt aaaaccatat 15480
aatcctgtga aaatacaaaa gtaaacatcc atggctttga gttggcaaag gattctgaga 15540

cagaacacca catcacacat agcaagagaa aaacggatga cttggcatca ccaaagtttc 15600
aggcttttat gtttcaaaga accccatgaa gaaaatgaaa gactgttggg gaagtgtttg 15660
cctaatgagc agaaagagct gagtccaacc ccagaatcca catttaaaaa aaaaaaaaac 15720
aaaaaacaga aaactgtggg agctgcagag accacccagc aaataaaatg tctgccacac 15780
aagatggagg acctaagttt caatctttgg catccactta aaagcccaga gctacagtgc 15840
atgtctatca ccccagcact gttggagtag agacaggtgg aatatcaggg ctcactggtc 15900
agccagtcta gctaaatcag tgaatccagg ttcagtaaag agatccagtc tgaagaaata 15960
aagtggagag tgactgaaga cacccaacat cagcctctgt cctctacatt cacacacaca 16020
cacacacaca cacacacaca cacacacatg cacacgcaca cacatacaca atcagacata 16080
gtaatgtata tgtgtgatca tagtgctgag gatgaagaga caggtagatc cctgaggctc 16140
tctaaccagc catcctagcc tacttggtga gcacctatct agagagaaag catatttcaa 16200
aaaccaggtg gatggctcct aaaatatgac agctgagttg tcctctggcc tccgcatgaa 16260
tggatacact tgtgcacaca aacataccta cacacaaaga gcccacagaa tgagaaagaa 16320
tatctgcaga acagttatgt ccaaactata taaagaattc ttaaaactcc agaaagaaat 16380
aatacaattt tattttttat gaagttattt acttacttta catcccaatc acatcttccc 16440
ctcccttttc tcctcccagt tcctctctct catcttctct ccccacccct ctctcctact 16500
ccccttcttc tcagaaaagg gcaggcctcc aatggaaatt aatcagcttt ggcatatcaa 16560
gttgcaataa gattaaaccc aacttctcct atagaggcta gacaaggcag cccagttagg 16620
ggaaagggat ccaaaggcag gcagtgtagt cagagacagc caatgctccc actttagtag 16680
tctcatatga agacccagct gcgcaactat tacatgtgtg cagagggcct acatctatcc 16740
tatgtgtgct ctctggttgg catttcagtc tctatgagtc cttatgggcc caggttagtt 16800
gagtctatgg gttttgttgt agtgtcctta actctttagg ctcctacaat ccttccttcc 16860
catcttctgc aggatttccc aagctccact taatgtttgt ctgtgggtct ctgtatcagt 16920
gtcaatcagt tgctgggtga actccctctg atgacagtta tgtaggatcc tgtctaaaag 16980
tatatcagaa tatcatgaat agtgttgggg gggtccctct catggcatgg gtctcaagtg 17040
ggccaatcat tggttggctc ttccttcaaa ttctgctcaa aaatacaatt ttaaaagggg 17100
taagaactca aagtccaaag aatggacatt tctccaagaa aaacatataa ctgggtaaac 17160
acataaaaag tcaggtgcta cattagttgt ctgggaaatg ctaattcaag cccagtacag 17220
cagcaagaat caaaaagtca tattattaca aattttggtg aaattacaac actcatatac 17280
cactgttgag aatgttaaat ggtgcatctg ctttggaaaa tggcttagtc attccccaaa 17340
ctaacaaata gagtaattat gttgcccagc caccgcactc ttgattatgt acctattaaa 17400
aaggaagtca tatgttctga tggttactgc tcactgccat ctaaactgga gaagcacatg 17460
caggtcagtg tgcctgcaag ctccagggag gattaagtga gtgagaaaga cccaacttga 17520

atgtgagtgg ctccatccta caaccctaga gtcctagatc taacataggg agaaaggggg 17580
aaagcaagga cctctggcac tccccttct ccacttcgtg gaccaccatg atgtgaatgg 17640
ctctattaca cctcacccat cccaccagaa tggacaagat ctctcaatct gaacaaaaca 17700
taactttcta ccctgaagtt gtattgaagt cacagtgaca aaaaagacac ctaatacata 17760
tgtctactct aaaatttcta tctgcatgat tgctgttcta ataaccgcac taacacaaag 17820
atgcgtacaa cctaaatgtc tattaatgga cgaatggata aaacgtcata tatctgtgta 17880
atttgatgtg gattccaggg gtcagacgcc actcatcagg cttggtggtg gcacctttac 17940
ctgatggcca tcttgtcagt cctactatat agtctttaaa agcttttgaa gtttgttagt 18000
gggaggaaca tataaaagtt tgaatctttg ggctagagaa acccaataat gcttcaaaca 18060
gagcttactg caccatcttg gtggggcttc agaaggctga ggtgccaaga gaagcacaga 18120
gagtgaagac ctgcttcatg agcttcagtg aggaatcagg actctgctgg tattgggctg 18180
taggccatca tgtggtattc tggcatgaga tctggcttca ctctacccgt ggcctaagaa 18240
ctcgaatgag gcttaattag tgaataatag attaatatgt ttggcagcaa aaatgtgaag 18300
acacgatagc acctagcatg tgctacagct actgtctgct gctcttaccc aggtgtccaa 18360
tgagagagat tccttgagtc ttagaagagg ctttggagtt ttgaatgagc attccaaaga 18420
ctctggaact tttaaagctt tgagggcttt tagagatgaa caaaacttat tttccattat 18480
aagattggca tgaagctaca gagaatagga tgggagttta tggcttaaat ttgtttgaat 18540
gtcaaactga caaggatgct ggacttgtga tggctaatct tgtcaacttg acaggatgta 18600
gaattatcta ggagacaaat ctctgggtgt gtctgtgagg gagatttcag atcgggtgaa 18660
ttaaggtggt aattcaccct ctgtgtgttt ccccaccaag tgaaccacag taaaccttct 18720
actaggaagt tgcttttatc aaatgctgtc acaataaaga tagaaataaa taatacctaa 18780
actagcatag cagccaaaca tagactagtg tctttaaaga ttacccttat ctgaacattc 18840
ctcataaaat gaatgataca attaatgaca tgacatttat gactggcttg ttttacttaa 18900
catgatagtt tctaggctca tgcatgttgt agcagctatt agtatttcat tcactatctg 18960
tagaaagaaa agactcccaa aaattgtcct ctgaccttca cacacatgtc ataacaggtg 19020
cacagagaga gagagagaga gagagagaat tcatatagag gaaaagaacc acatgatcag 19080
cattgccatg gcgatacctt acttcccaat cttagatatt atctattgtt ctcatcactg 19140
tgacaaaatg cttgaaaaca gcaactaaaa tgagattgtt tcaccttgga ctttggtttg 19200
tttgctgttt tgtatttttg tacagtctga gagaagacat agaggcaaag taggaggagc 19260
tgatgacatt gagcccatag tcggggaaca gagagagatg aagtctggta ctctacttgc 19320
tttctccttt gtatttagtc taggacccca tctcatggaa cggtactatt tacatttggg 19380
gtgggtcttc ccatgtcagt taatggcact ttagcaacta cctcacaagc atgctggagg 19440
tttatcaccc aaatgattct aggtcccatc aagctaactg tcaacattag ccaccattgt 19500
tagcttttc ttagattgaa aagtaccacc gggcctggtg gcgcatgcct ttaatcccag 19560

tactcaggag gcagaggcag gcagatttct gagttcgagg ccagcctggt ctacaaagtg 19620
agttccagga cagccatggc tatacagaga aaccctgtct cgaaaaaacc aaaccaaacc 19680
aaaacaaaca aaaaacaaaa aaacaaacaa acaaacaaaa accagaaaag tatctgagtt 19740
aaaataactt ataggaaatg tttattttag atcattgttt tggaagattg ggtttacagc 19800
caggaggacc tatggttttg ccttgtgtca aagaagcaca ccatttacag tcattgtggt 19860
agaacacaaa gctacttaat ctcacagcag ctggaagaca gacaaaaagg cccatggtcc 19920
aacattcctt caatgtgtgt caagtgactg aacttctggc taccatttct caggaagacc 19980
attagcagga gtcaagcttt gcctttatta tccaaagtat agatgttgcc taggggcaga 20040
ggggccagta atagcaatga tgactaaaga tatgtgttcc tttggggaaa tcataaaaat 20100
gttcaaatat tgactatgga gatggttacc cctatgtgat aagggtaaaa aagactttaa 20160
ttattgacta tcattgggta aattttatgg tataaaaatt atatgcctat aaatctgtaa 20220
aaatgtttaa ttaattcaaa ttgtaatata gacttcaaca tagcaacaaa atctatacaa 20280
catatagaga acagaagagc aaatcatgat aaccaggctc agcaaaacct cttaggtatg 20340
acaccaagca cacacagcac aaggacaatc gccacactgg actttgtcaa aacataaatc 20400
ttctatactt cactgaactc caccaaaaca gtaaaatgat aaaatgggcc aacgtatgca 20460
gactgcatcc agcaagaggc ttaaattcaa aatatataga gaacactaac aaaaggtaaa 20520
aagacaggtt ataacaaatg ctgccaatga gggatggagg tcaaacccca gcactctggt 20580
aaggtcaaaa agccacagcc attccgggag acatatcagt tcttcctcag aattatagat 20640
agtgtcacct cgtgacccag cagcacttct tatcacaagt gtttccaaaa ataatacaac 20700
cagaattagg ggtgtagctc atggcagagc acttacctag tatatagaag agccagggac 20760
tcagtgcact cagtgcacac ccctgtactg caatattaac attaataact aagccatgtg 20820
tctacaccaa atacttgtat gcaaatattc gtggcaaccg tcttccttgg ggtttctatt 20880
ggtgtaataa gacaccatga ccaaacgaaa cttgggaagg gaagggttta tttcacttca 20940
tactgccagg taatatctac cagtgagcca ctgagaccaa tatagttgat catacaatga 21000
aggcaagatg ccagaatcgc atgcagctgt cccataactc tggattctgt tgctaaggga 21060
aacatgtggc agtggagcta tgaccaataa caaagaagtg acatcacatc ccaggggtgg 21120
ctgggcttgg tgccgtgcct attgtcctag atgatttggg aggctaaaca gaagaatcat 21180
ttagtccccg atgttcaaga ccaacctatt tcaaaaagaa aattcagtat gtcaatgcta 21240
agagtaaggg ttgagggtac tctcactgat gtgatagata gctagagaga tgtttagggt 21300
ctgaaaggga gcagggaaga aagagagaga gggagaaaga gaaaaacagg cagaaagaca 21360
gacaggttga gagagataga gacaaatgca tatgtctaca taccctagtt cacagtttaa 21420
aagttaaaaa ctcttgcgcc atccctgcat ggtgtaagag cataacctga ttatactgta 21480
gtcaggccaa aaattcaaaa attcaatgtg tatgcaagcc tttccgctca gcaccctatg 21540

atgtagtttg gaacttctgt acttttacag ggcatagatc attgtcccag agtctgcctt 21600
cccttccatc cggctgagga aatcttcagc tctcttctcc atttcaactc cctgtgacaa 21660
agccctttat agagttctac aggacttcta gtctgtggag tgggttagga caaaactcca 21720
gagtgagtca atttgttgtc agccactatt atcttagtgt ggtttggtgc acacctaatt 21780
agacttctta cccattacac ctaagagatc acaatctagc ctgatctgga agccgtttac 21840
ttcatcacaa cctgggtctt gtgaagagga tgagaagggc actgtgaaat atcttgtccc 21900
tttcttaagc cctattttac tccctccttc caagccgctg ctctcttcag cagcccgcct 21960
cccacccatg ccccatttcc ggtcagaaaa cactgaactt tgcactgtct gcacaattgt 22020
gaagttcccc tttcacatac actttcaggc ttccagatta tggaagctga gcaagagaat 22080
acttccagga atgctgctgg cttgactcag tagcccatct gggaaagggc acagaaaggg 22140
cccaggtcag agctccgtgg cccctggccc tcactcgctg cactgtcagt gagtatgaca 22200
cagtatactg tggtctgagg gctctcacat ccctgaccta atacgtcagt atcttcagtt 22260
ttcaatcatc attatgctct gaagaccagc cattgacaca actcgacagc ctttcatttt 22320
aattcctgca tagttaaatg catcacacat ttttggtttt gtgttctgaa aactgagcgc 22380
aggacctatt aaatggtaag caagcaaaca ctctatcccc agctccggag gcatcattct 22440
tgggtaccac tttgctatat tcatttggct acctgctgat gaacacatgg gtacctccta 22500
gtgctttcag actgagccac agtgagcttt ctgctaacgt gagagaatta ctctggggaa 22560
catgtctaga tgaagatctg tgagatgacc aagtgtgtgc gtcatctgac ttctctagat 22620
cttggtaatt gtttgcccag ctgtccagac actttccacc cttagctggt gttgctgtaa 22680
gatgccattc ccctgcatca ccaacccatt cgacatgaga catttttgtt ctcgactgtc 22740
taatgggtag gattgtgtct catttcttcg tcataagatg ctacatattc atggattaca 22800
cataagcact acaggctgag ccaatgactc ccctactggt gaacattcag ctcgattccc 22860
tacttctgtt tgttttgaat attacagaga agcaacttgt atgcatgata tataagtcat 22920
ttaatcattg gcagatctgt ctagagaaca ttaatcactt gtctcaaagc agctctcatc 22980
tcacataaaa tgagataatg tattgtgaca caaatatgaa tgtccaggcc tggctggctt 23040
gggtgttctg catgctgaat ttccacacag aagcaattgc ataaacatcc atacccacag 23100
gataagagag agcattaaac caaagcattt tttccagcac atcagtggga acaagagttg 23160
catgagctac atccaattgg ggaaaatctg ctgcaggtgt gagagttacc tacctggtgg 23220
tagtcttagc tttggggtga gagttacctg gtggtagtct cagctttggg gttagtaagg 23280
aagctgctgg tgtgtttata cggtccaaag atttcacctg gtatttggaa ggataaaaag 23340
tcagacatag aggcagacaa tgattggcta ttaaagagac aaaaggtgac ccaagataac 23400
ttttctctag atctgcacac caactcttta taataacaaa gttcccataa gctgatcaac 23460
cttgtaaaac ctttagagca agtgcagctt tcttttctaa gaacattatt agttcacagt 23520
tgtgtcgggc aagtgtcaca ttttgcgcgt gcagaaatgt tgttttatgt cctctgatgc 23580

taagaaggct cattctgagg aactgataaa caggaggcgg agcagccagg agagcagtct 23640
ccagaagaga gaagagggta aagaagagga tgtctttcag aagacatcct ttatttatag 23700
cagttttact ggctatcatg atctcaatat gacctttctt tctccctctg catctgacaa 23760
aacatataca tataacataa aatggtaata ttatcccaaa agttaaaaca attaaacaca 23820
gagcctaaag gagaaaaagg taatgggtga aggaagcagg cagctagcca gccaaaaagt 23880
ctttctaaaa aaaaaaaata tgtataattc tatttgtcaa ttaaatgaat gaaagcatta 23940
aaataattaa attaaaataa ctattttaat cttttcataa tttaattaaa atatgattat 24000
atcatttctc tcctctccta actcctcccc ttccaatgtc tacacatccc ccaattcacc 24060
gcctcctctt ttatctaacc cttgttggca aatatacata aaaaccagtt aacatacaaa 24120
tacaacctgc tgagcccctg tcccctaagt gttgcctgca tgtgtatgtt tctagggctt 24180
gggattagat gacaaaccaa gccaggcatc tctgggtgag actgactctc ctctccacag 24240
ctgttaactg cttgtagctc ttcaactagg agtgggaccc ctgagatttt tccccagtct 24300
gtgttggaat gacaattatt ttggaattat ttgggttctt ttttggcagc catgttgtgg 24360
gcgtagcttt tctgtcatag ctagaagaga ctatcacaca gcagaccttc taattttcta 24420
gttcttatga tctttctgac tcctcttctt cgatgctttc tgagactcag gtgctggagt 24480
tgtgtggtag atacatcccc tgggtaccac acaaccattt cttctcttca ttttgactag 24540
atgtggcttt ctgtaacggt ctccttctgc tgcaaaaaga agttcctttg atgagggttg 24600
aaagttacac ttgcttgtgg gtgtctggat aagtattcag aattccatta agaattattc 24660
tggttagaga aattggcagt agtacaatct atgacctccc tagccaaaag cagttggcta 24720
agtttccagg gccaagaagg atgtctttcc tgttaagcag gctttgagtc caagtagaca 24780
gttgttggtt actgccaaga tataggcacc actactgccc tgtagggata tcttgccatg 24840
atggtcattg tggtttatat tcaatgacag ctggctaaga ctattgactc tttccttccc 24900
tgcatatgaa acacagctta tcactttctg gtcctatgaa agctggtcct cgagggactc 24960
ttttggttcc tttccagctc aaatcctcca cattttatgt gtggagtgca tggtgtcctc 25020
agcaataagg acttgctttt aacttctggg aggcaatcaa ggacaagcaa tagcctatat 25080
tgtttcaaga gctctcttgg atttccctga tcaaaagctc aaagggggct tgtcatgcat 25140
ggtaccgaac tttttgttag ctacgcttgg ctcttgttgg agcgaatcat ccctccatat 25200
gttgtaattt tttatattgc atgttttctc ttgtttttga gaaattgaga aatttcccac 25260
agggtattaa tatattatta tattggcatt acatatagtg attcactgtg aggcttccat 25320
ggtcataaag actgcacact attcaacatt acgtatatga tgttggctga agagagaagg 25380
tactattctg catttgtaat ttagaaggtt ggattctctg gatgattttt ttcttttttt 25440
ttaagcctaa gtatacttta atttggtact gatatagtag ttcataaaga tgataccact 25500
atggtgtaga actcaaagtt atgatattaa gttcaggaag cgaatgtatg taattcattg 25560

caaatttgca atgcttgatt acacatgttc aagattcaac acggagaggc ttctgatact 25620
acggtggcac cagcagggaa gtcacaagtt cagactgtct gagacatgca ataaggttct 25680
ttcttaaaga aaacaaaatg tggaaaaaca gtcttgaaat ttttcacagc tctttcttcc 25740
tttcaatttt cttgtgcaga aatttgtgtg atgataagaa tcagaatgtt gctctgcatt 25800
atctagtaat actaactatc ttgtggagtg atcctgtgca ttcaccagaa tgcaaatact 25860
gcttccttac taaatcactc ggagccactg attcccatca gctagaaaca aacctgagca 25920
acagagtcag tgggcagaac accataatcc tcctaagagc atggagttag tactcttcgc 25980
atacatccct ttatttatta tttatgtatt tattttcggg gaaagggata gcattttatt 26040
ttttaatctt cttatatctg ttgaggcccg ttttgtgacc aattatatgg tcaattttgg 26100
agaaggtacc atgaggtgct gaaaagaagg tatattcttt tgttttagga tgaagtagtc 26160
tataaatatc tgttaagttc atttggttca taacttctgt tagtttcact gtgtctctgt 26220
ttggtttctg tttctatgat ctgtccattg atgagagtgg ggtggcgaag tctcccacta 26280
ctattgtgtg cagtacaatg tgtactttga gttttactaa cgtttctttt atgaatgtgg 26340
atgcccttgc atttcgagaa tagatgttca gaattgagag tttatcttgg tagatttttc 26400
ctttgatgtg tatgaagtgt ccttccttat ctttttaaat aacttttggt tgaaagtgaa 26460
ttttattcaa tattagaatg gctactccag cttgtttctt gggaccattt gcttggaaaa 26520
ttgttttcca gccctttact ctgaggtaat gttttagcca ttgaagtgca tttcctgtag 26580
gcagcaaaat tctgggttct gtttatgtat ccagtctgtt attctatgtc tttttatttg 26640
ggaatctggt ccatcaatgt taagatattg aggaataatg attgttactt cctgttattt 26700
ttgttgttag aggtagaatt atgattgtgt gactatcttc tattgggttt gttgaaagaa 26760
gattactttc ttgctttttc tatggtatag ttttcctcct tgttttggtg ttttccatct 26820
attatccttt gtagggctgg atttgcgaaa agatattgtg taaatttggt tttgtcatgg 26880
aatatctcgt tttctccatc tatgataatt gagagttttg ctgggtatag tagcctgggc 26940
tggcatttgt gttctcttag ggtctgtatg acatgtgccc aggatcttct agctttcata 27000
gtctctggtg agaagtctgg tataattttg ataagtctgc ctttatatgt tacttgacct 27060
ttttccatta ctgcttttaa tattctttct ttgtttagtg catttggtgt tttgattatt 27120
atgtgatggg aggaatttct tttctggtcc cctctatttg gagctctgta ggcttcttgt 27180
atgttcatgg ggatctctct ctttaggtta gggaagtttt cttctataat tttgttgaag 27240
atatttactg gccctttaag ttgaaaatct tcactctctt ctatacctgt catcctttgg 27300
tttggtcttc tcattgtgtc ctggatttcc tggatgtttt tggttaggag ctttttgctt 27360
tttgcatttt ctttgcgtgt tgtgtcaatg ttttctatgg tatcttctgc acctgagatt 27420
ctctcttcta tctcttgtat tctgttggtg atgcttgcat ctatgactcc tgatatcttt 27480
ccaatgtttt ctaacttcag ggttgtctcc ctttgtgatt tctttattgt ttctagttcc 27540
atttttagat cctgggtggt tttgttcatt cccttcacct gtttgattgt gttttcccat 27600

aattctttaa gggatttttg tgtttcttct ttaagggctt ctacttgttt acccatgttc 27660
tcctgtattt tttaagggag ttatttacgt ccttcttaaa gtcctctatc atcatcatga 27720
gaagtgattt aaggtccaaa tctttctttt ccagtgtgat ggtgtatcca ggacttgcta 27780
tgttggagca gtgggttctg attatgctaa ataaccttgg tttctgttgc ttacgttctt 27840
atgcttgcct cttgctatct gattatctct agagctacat gccctcactg tatctgactg 27900
gagcctgtct ttcctatgat cctggttgtg tcaaaactcc ttagagtttg gctgtctctg 27960
tggtcctgtg attctgggat cttgtgatcc tgagatcctg gatgtgtcag agctcctggg 28020
agtcaagctg cctctgggac cctgaagatc ctggtgtgac caagctcctg agattctgtg 28080
atcctgtgat cctaggcatg ttagagagcc tgggagttga gctttctctg ggtgttgtgg 28140
ggctggctct catgttttct cttatatgta gatattagat tttaagcttt ccatatgtgt 28200
gtttcattta aaatagccac agaggttggg tagcttttat gagaccagag agaggaagga 28260
aactcccccc aaaagagaca tagaatatag tgttatgggt aatcgtaagg gaaactaaaa 28320
tgggaggttt aaatggggag aggatgggaa tgtgttacaa aggagaaatt atgggaggga 28380
caactaatac taaaagcctt ttgactagcc atatggaaac tcacaactct caaagcttcc 28440
taaaatgtat aaaatggaat taccgtataa tggaagagac aatgccccaa ctagacatca 28500
tatgctagca agtaaaactg ccagtatcag gaatgggtta catcttgttg tgtccctgat 28560
caaaggtgac ccacagacag tcccccataa acaatatagg ctattgtcta ttattagtta 28620
tccttcagaa cctgacagaa aatgagtgtt gtgtccggcc agcagaccac aacctgggtt 28680
ctagcctgga aaggcatttt ggaaacctgg aagagaagag gggctaggtg gcgagagaaa 28740
agaatgtagc caagacagtt actctgatca aggctcaaat tttattgttg cgacactagt 28800
tatgaaggaa gggggagggg acccgattcc cgccgaataa tctctggtcc agtagaaagg 28860
tgcacgtgtg tggctccgca ggttccagca gtgggcgtgg cagaacgaat gagcaggaag 28920
ctccacccct gagcaagcag gtttcaggct aggggagggg agactacaaa tgaggacatc 28980
acttacttat gtcatcaaac atgagaaaat taacctggtg cccaacccga agctttagcc 29040
atactgacaa gcactcacaa tgatgaaagg tgctatatat acatgtgacc agatgagcaa 29100
cagcatcatc attctttccc agctacaaac cctgagacct ataactgtgt cctgccttgc 29160
aagatatact ggtacaataa tggcacaaac gttatggaaa aaccaaccac ttcctaaaaa 29220
tcacatttaa agcttattcc atgagatgaa acccatatcc agaaactatt aatgaggcca 29280
caaacttgaa acaagatgag gcatggaccc taggggaaca actactactg ctatcctgct 29340
aacggagcat gccacgccta aagacatact gccataccca taagctaata tgtatgacaa 29400
gccccatcag agaagtttct tcttggagta gatgagaatt agcacagaaa cacacacact 29460
cccaaatgga ggatgggcag acaatgtcag ttcttgaaat gctcaatcct aaatggagtg 29520
tctttgtcaa acccctttcc tccagccttg gggatctagg cagacgtggc agaaagagct 29580

gaaggtggtg gatgactccg aaaatacagt gtctttcaga cacaaagggg ttgatggaca 29640
ttgaaactca cagagacatt aacactatgt acaagacttt gcaaattcaa gctagataaa 29700
tctcagtacc gagagaagtg ggcacaagtc ccaccctcaa ccaggatgct atttaaaatt 29760
gatacctgct cagagaggga aaatccattt tctccaatgg agtgacactg ggtatatatc 29820
aactgtactg cagggcaggg ctcatgctca agggtagttg gtcaacacaa catgggctcc 29880
gtagttctct ttgggggagg gtgtgtctct ttttgtattg tttatatttt tctgagagag 29940
agagagtaaa agtgtggatt ttgggtgggt aggaaggtat agaaaactta ggagtcgagg 30000
aagtgaaaga tatggtgaaa atatgttgta tgaaaatttc aaataatttt taaaatgtaa 30060
taaaaagaaa acagccttat tccaaaagaa aaaaaaatcc ataaaccctt tacagtgtgt 30120
aacactttaa gcctgagaaa tttattaaat tttctgattt ggcattcaag catctagaga 30180
gttttcaacc atagctcttc tgcatttcct ttggagcttt actgcccact tgtgttacat 30240
tgcctctgga ttgcagaaag tcaagagcac ttcatggacc aaagatggca cttagatagt 30300
tcattatgga atgaatacac tttgtccttt aagagcctga tcagttctca tgctgattgt 30360
aatcttctcc tatttctttg ttgaagtcat actttatcaa tcatgtcttt atgaggtcag 30420
atatcttttt tacagtaaat tgcaccatct acacctcctt aagctctctc cttccaaacc 30480
agatgtcttt gctgcctctt ccttatttct gtgtagttga tccttgtggc cttcctccat 30540
ggagtgtgag gtttttgttc ccccaccccc acccccacca cctggcctat ttatgtctgt 30600
gggtctggag gcctttggct tctgtgggtt gacttgtctg tatgtgtcat tcagtggaag 30660
ggacatgcca gtagcaagct agctggcaaa ctgatccact ctcatctgca aggctctcat 30720
ctgcaaagga ggtaacggag agccccttgg tgtcatatct tgagaaagtt ctttctgagc 30780
tgacaaaaca atttctaaac tctgcagaaa atttgtatgg acatttgggg caggtgaaat 30840
ggctcagcta gtaaaagcac tcgccgtgca agccaatgac ctgagtcttt cctctacttt 30900
cctgacttgc tgcaggagag aaccaactcc caaagcagtc ctcacaggga ggtcctgtct 30960
gatcaagaag ctgctttagg aacctgtttc ttagttttaa gaaactaagc agcctggaga 31020
gctgcaagtg cacctgctga aaccgtgcct gaggtactga cagcacagtg cctcgggtac 31080
tgacagcaca gtgcctcagg gactgacagc acagtgcctc agggactgac agcacagtgc 31140
ctcagggact gacagcacag tgcctcaggg actgacagca cagtgcctca gggactgaca 31200
gcacagtgcc tcggatactg acaacacagt gcctcaggta ctgacagcac agtgcctcag 31260
gtactgacag cacagtgcct caggtactgg cagcacagtg cctcaggtac tggcagcaca 31320
gtgcctcagg tactggcagc acagtgcctc agggactgac agcacagtgc ctcggatact 31380
gacaaaggtg ctttctctct tccggctgca aacaagccca gtcttcctac taactcccaa 31440
aggtcaggga cacttcgaat tgtggcccac ttcttatcaa ctcttgcctg aattctggat 31500
caaagtacct catatagcac ctagcccttc cgggaaaaca cataagtaaa gactcctctt 31560
tgttcctcat tcttccttca acagcaatct gggattgagt ccccactatg gtggtcagca 31620

atattgaagg atctgtttac ctgaaacaca tctcaatgtt gtttttgtgt tatttttgtt 31680
ttcttttgtt tctgagatag agtctcactg tagagcctta gctggcctgg aagtagctct 31740
gtagagcagg ctggcctgag actcacagag acccgtctgg ctctgcctct tgagtgctgg 31800
gattaaagtg caccacttgg atggctctgc ctatctattt ttaaaccaga actcaaagct 31860
atgcacacaa ctacactcac atgtcctcac acatatcatg tacacacaag ttaacaataa 31920
atataatttt aaaaataaaa taataaaatg tgaatgctat tgttggaaac taagaaaggg 31980
gggcctgggg aagagggggg aaagggaaaa cccacacccc accagagttt tgcctattct 32040
ctggtctgtc aggcgtggga gagctgctat ctaccttcca ctcatccctg ggtgggcatt 32100
caagcctctg acccgctctt cgaggggggct gcaaggggca gctctacctg ggattcccgg 32160
agctannnnn nnnnnnnnnn nnnnnnnnnn nnnnnnnnnn nnnnnnnnnn nnnnnnnnnn 32220
nnnnnnnnnn nnnnnnnnnn nnnnnnnnnn nnnnnnnnnn nnnnnnnnnn nnnnnnnnnn 32280
nnnnnnnnnn nnnnnnnnnn nnnnnnnnnn nnnnnnnnnn nnnnnnnnnn nnnnnnnnnn 32340
nnnnnnnnnn nnnnnnnnnn nnnnnnnnnn nnnnnnnnnn nnnnnnnnnn nnnnnnnnnn 32400
nnnnnnnnnn nnnnnnnnnn nnnnnnnnnn nnnnnnnnnn nnnnnnnnnn nnnnnnnnnn 32460
nnnnnnnnnn nnnnnnnnnn nnnnnnnnnn nnnnnnnnnn nnnnnnnnnn nnnnnnnnnn 32520
nnnnnnnnnn nnnnnnnnnn nnnnnnnnnn nnnnnnnnnn nnnnnnnnnn nnnnnnnnnn 32580
nnnnnnnnnn nnnnnnnnnn nnnnnnnnnn nnnnnnnnnn nnnnnnnnnn nnnnnnnnnn 32640
nnnnnnnnnn nnnnnnnnnn nnnnnnnnnn nnnnnnnnnn nnnnnnnnnn nnnnnnnnnn 32700
nnnnnnnnnn nnnnnnnnnn nnnnnnnnnn nnnnnnnnnn nnnnnnnnnn nnnnnnnnnn 32760
nnnnnnnnnn nnnnnnnnnn nnnnnnnnnn nnnnnnnnnn nnnnnnnnnn nnnnnnnnnn 32820
nnnnnnnnnn nnnnnnnnnn nnnnnnnnnn nnnnnnnnnn nnnnnnnnnn nnnnnnnnnn 32880
nnnnnnnnnn nnnnnnnnnn nnnnnnnnnn nnnnnnnnnn nnnnnnnnnn nnnnnnnnnn 32940
nnnnnnnnnn nnnnnnnnnn nnnnnnnnnn nnnnnnnnnn nnnnnnnnnn nnnnnnnnnn 33000
nnnnnnnnnn nnnnnnnnnn nnnnnnnnnn nnnnnnnnnn nnnnnnnnnn nnnnnnnnnn 33060
nnnnnnnnnn nnnnnnnnnn nnnnnnnnnn nnnnnnnnnn nnnnnnnnnn nnnnnnnnnn 33120
nnccaattcc ccacatgcta tccctggtac acatgttgcc tggtacactg gatcaatgaa 33180
aactttagct ccactcaacc ctttgaaggc attgcatcct ttgaactgcc aagcatgaag 33240
agaagaacat ttatgtcatt gctgatagtt gactggtggg tggtcataca gtggagtccc 33300
tcaataactg gcttgctcgt ggatgtgtat tggagacccc tctattcatt tttccaagta 33360
aacaacattg tacactattc ttatttttat gcttattaat gtacttgtaa aggtcacaga 33420
aacaataaaa atggaaggtt ttcataaagc tcgctgagga gggtctagaa gttagacgga 33480
tctctgatag acagtgggac cacaaataga atttttcaat aaaaggcaaa catccagctt 33540
tttcctcaca ctcttgacct gaaaaagcca taaaggcctg gagagtattt ctcccaccag 33600

tagggcagaa gggcccctg ctggtggact ggccaacaaa tcgaaagcct gctcaggata 33660
tttaccctga gacaaggacc tatcaatcag ccctctactc tttgttcaaa ctgatgaacc 33720
ttaaactggg ggaagcacgt tcctcaaaga cccagaaacc cccaattatg gaggagagac 33780
ttgatttccg acaatgctct tggacctata atatttcaaa ctccacaaac tcatcaaagt 33840
ttcaatcagt ccatcagtcc catttgtaca ctcagtttct ttccagggat gttcatttct 33900
aagagggtcc ctgggctagc cagacaccaa ggagaagacg aggagtctgc accagtggga 33960
gcacaaggtc atgttaaagt gctgaagact tcttgatgcc caaaacctgc atccctaaag 34020
ggagctttaa tggcaaggaa aatcgggaat atccatgaaa actgagctca gggtgaatat 34080
ttcagtgttt aagcaaaact gatttatttt ctctgtccca aatgaatgaa catgattttg 34140
ttttcattag tttgttctca gagaatgttc agtaagtgtt caatgcatga aggctgtaag 34200
aatttaaacg tgctcactgg agttggtgtc tgcttctctc cagagggaac aactttggtt 34260
gtggctacct ctaaccgggc tggaggccag atgttatagg ggcattccct cagtggtctg 34320
cacccaatgt tctttctcac ttcctttaat gccctctgat tccaccgtga aaactatctc 34380
cttccccaac caccacacac agaatcagga tgtgcattga ccaagctggc aggacatttg 34440
acactggctg agtgtggcat aggagcctta agccctcttc ctttgactgt ctagacagac 34500
ccctcttggg ggctggggct gttcaaagtc acctgtatta agtgaggtct ctaggatctc 34560
cctcgctcct ttctattcat gagtgcacat ctgttccact cactaactct ttggtataag 34620
ccagtctcat agctctactg ttgtgatcat gccatggcac aaatgtttgc cattcagcat 34680
tggaaagatt cctggcactt ccacttatag tgcattgagc ttataatggg aaatagacca 34740
ccatatacaa ctttgtctgc aacatggcag cctcagctca ggggatagcc aaaagggcca 34800
cctctaacat agtgttttca cttttctcat gctagatgga tggagtggaa ccccggcttc 34860
cctttgagta ttgatgccaa atgccacaag gatctgcccc gagatatcca gtttgatagt 34920
gaaaaaggag tggactttgt tctgaactac tcaaaagcgt aagttttaag aacttcaggg 34980
tatgagtggc tcatctgata gctggggatg ggagagtgtt aggacttcag gaccacaaag 35040
aaagagcaca ccattccacc tcaagtgtct ggaccaggaa cagctgtact cttgatcaga 35100
agggcagcca tacaccaaga cagacattgt atttgcttat tctaactcag gtagtgtctg 35160
tattctcccc attgactggg atccaattca cggccttaat gagccaacct gaacagaatt 35220
ggtacacaag aaatgaagga aggggaaaag ggtcactgat ccaggtcatc aaatgcctag 35280
aatacagcaa tggaccttgg tgtaaataaa gatttcgttg gggaagggga aattaaagca 35340
tttccataga agtccaaggt tatttgggga gggataaaaa atgagtttat tccttgtctg 35400
aaggcaagtt gtagagcatc tgatagaaaa gactcgtatt tgttctttct tatagtagga 35460
aaggcctttt ttataggact tgggctgggg gagcagttca gctgaacacc attccccatg 35520
cgtttgctct acccaggcca ggaggtagtc tctcatcgaa cagttgcaag atgacttata 35580
tctaaacagc cttgacccaa aagtcttcta gcacctacag ccccgtgctt tcagctaaaa 35640

gcagaggctg ttcttactta caacactcca tctccacttc cagttcactc ctgtggccag 35700
tccatcagaa gatggagtcc agactgatgc caagaggcag gttccacagt aacagtgact 35760
cagatgacac tgttggtccc atgacttaga ttcttgttca tttgtcttaa ggaccccatg 35820
aagtaggcat atgtgtttat cactgttctg gtgctgtgaa gagacactaa gaccaaagca 35880
attcttataa aagaaagcat ttaagtggga gcttgctcac agttccagag gttaagttca 35940
ttatcatcct ggtggggagc acggtggcac acacgatgct ggagaagtag ctcagaactt 36000
tacatgctga accacaggca gacacagaga aagacctgga tcaggtgtag gcttttgaaa 36060
cctcaaagcc caaccccagt ttcctccaac aaggacacat ctattccaac aaggcaacat 36120
ctcctaatcc ttttaatcct ttcaaatagg cccattccct gatgactaag cattcaaata 36180
taggagccta tgggggccat ccaagcacca taggacatat tgtcctctct gcagttgagg 36240
aaccgagaag gaataagaag gttgaggacc ttgcctaaag tcctttgcta ataaacaaca 36300
gagacactca actctgggtt acatgtctgc actagagttg ctgcaggatg ccagcacaac 36360
catggatact ctgtcaaaga gtagactctt tcagcaccag accaaggtta gcaaggacac 36420
ctttgatgtt atactttgac tcttagagaa cttttatact catacaaacc tagaaaaccc 36480
taaactcttg aagcccaact gagccttata ctcccccagg aacttttagc acttcccagc 36540
acacagcaca tccacagaca agtctcagag agaaggactt ggctacctac acacccaaca 36600
ggcagccagg gctgaggacc acaaatcagt ctgctccatc tgggtgtttt cacagctatt 36660
gttggtcatt tctaggtaag gaaatcaaga gtccagtggg ttaagataaa gtaatttaca 36720
tatatatatt cccatacata aatagatgat ttatatataa actaactctg gtgacctgaa 36780
tgagaaatga ccctgtaagc ttgagtatat gaacacttag tacccagttg gtgctggctt 36840
tagaggcatg aaggatgcaa gaatgaaggg attgtggagt cttcccccat ggttttagaa 36900
agccactaag gcaaggcact gtgtggtacg gatgtccctg tgtgaagacc acaagagcaa 36960
gaggctctga gaggctattg atcaagctat acaagtgtgg ccttggctgc agtggagaca 37020
ccaagagatt ggaggtgtca gagctgagag atacatacat aggagagctg ctcacataga 37080
ttggaatcag ccaaagagag aaatatatgt tgtagtcagc aaagttggaa gggaagagcc 37140
atttaagccc tttgacaata ggcatagggc taaaggattt ggagtgtgcc ctgctgggtt 37200
tcagtcttgc tttcatccag tatttcctct ctattcctgc tttcctctct tgtggaacgg 37260
taatgtatat tctttgtgga agtatgtagt tttttttttt aaatgggggt tataattaag 37320
agattgccta gagtctcaga agagacttta acacagaact gagcttgcaa gactctgggg 37380
acatctgcag ttggactaaa agattttgaa ttatgatatg gcacaaacct acagggaacc 37440
aggagttgag tgtggtttaa gtgagaaatg tctatagaac tggcgtaggt aatatttggt 37500
gctctatttg ggaaagttta ggacgggcag ccttgctgca gaaagaatga actaggggca 37560
ggtttttaaa gtttaaagtc ttgccccatt tatcatttgt gctctgcaaa ggaatgaatg 37620

aacacatgca gaggtgggat tcagagacat tccagagggc ataggcctgg tctcttacac 37680
agctggggtg cagtaggaag agcgtctgcc acaggataag ttctatcctg gtcccacaag 37740
gccagcacct acctcttgga actgcctggg ctatgaggag gttcccaagt atgaagtaaa 37800
aacactctgc gatggcatag tgggtgggca aggtgacctg tcccctggtt tgcaggatgg 37860
agaacctgtt catcaaccgc ttcatgcaca tgttccagtc ttcctggcac gactttgctg 37920
actttgagaa aatcttcgtc aaaatcagca acactatatc tggtaagagg gttctgtgga 37980
cgggagagtt tatttttctg tctgagactt gcaaacatcc aacccatgca ccccctcgac 38040
atcatctcac cagaagtgtc aaggccaatg ggactttttc tttacataca taaatcattt 38100
gtggctcttc tgctcagacc cgaggcagga ctgggctaca tcaacctagc attggaggct 38160
gaaatgatgg ctgagcaatt ctgctcttgc aaaagactca agtgcagttt caaacatcca 38220
cactgggtgg ctcacaacca ccactaactt cagctacagg gcatccaaag tctctggtct 38280
ctggagacac atgtgcatnn nnnnnnnnnn nnnnnnnnnn nnnnnnnnnn nnnnnnnnnn 38340
nnnnnnnnnn nnnnnnnnnn nnnnnnnnnn nnnnnnnnnn nnnnnnnnnn nnnnnnnntt 38400
acacacacac acacacacac acacacacac acacacttaa tagtagtaaa aacaaaattt 38460
acagcaatgg ggtccttaag accaaagttc atttagttgg agccctggca ctgtcctgga 38520
cagtaataga tggagagtca tgacagccat cactcttcca agaggcagga atggttaaag 38580
atgctgacct cagaacaagg gatcaacatt ctcccctctc ttttcatgct gggcggaagg 38640
accggaaacc ttgagaagag caagtgcctg gaaatcttac tgttcactaa ggcatagctc 38700
taggaaaact agctctatag cctgactcag caatgagtag ggacacagca agtagggaca 38760
tggcaagtga gaagatcccg atacctacaa agatttgtcc tcctgcagga tcgtgggatc 38820
ctgcccagcg gtcctatcta gaggtcattc tctccacaga gcgagtcaag aaccactggc 38880
aggaagacct catgtttggc taccagttcc tgaatggctg caacccagta ctcatcaagc 38940
gctgcacagc gttgcccccg aagctcccag tgaccacaga gatggtggag tgcagcctag 39000
agcggcagct cagtttagaa caggaagtac aggtaggcca gcttgctgaa gacctagtct 39060
ttccacagta gccacttcct gctgtccaat gcttgctggt ccccagactt ttactggctt 39120
gtctggttga ttgattgtgt gaaatcagta gtttcttcat ttttctggct gcagcatcca 39180
ggaggatctt gggcactgtg caagccacat gcagtcacag gaggggctgc atctacacaa 39240
tcctcacatc tgcaaacaca gtgtcagtgt tgtgttcaat gtccatatca ataattactt 39300
gacattccct gtgataggtg tgttctacac atagtcaccc aactgtcatc tgctttgcaa 39360
aggtgaggaa actagagaaa gaagtagact ttgtgtaggt agaacttact gctaattaga 39420
aacataggca ctacctgtcc ctcacacact ttcccaagcc agtctttcct gactttcact 39480
tgcaggctca agccctcatc cccagttcca aggctgatct tgtcttgagt cggatagagt 39540
ttaaaccaaa tcggcttgca tctgatggaa tcttccaggc attaccgtgc tagattttat 39600
tttgcttgct gagagtccca acaaccctca gcatactccc atctccccat tgcagctagc 39660

accactgggc ctccatctcc agctgttcat gtaacctcca ttgcctctgt tcaccctgtc 39720
taggtaccca gcccacatgg agactccaaa cacataccat tcaggcctaa cttcctgcct 39780
acatgacttt gggggttact taaccaactg aagttggggt cctgatccac atggaaagtc 39840
cttccccact catcaggacc ttcatgggat ctgacatatt tctaggcatg cagacagcca 39900
gcaacaacaa ctccttacac ccaccccatg cacagaaata agcatgtctt agaactaaga 39960
gggacctaaa cctctgcaat tgcccctagc tacccagaag taggaccttg cctgttctcc 40020
aagacaagcc ccctaccaac tccaggggag gtggcttatg ggttatcttc actttgagct 40080
gacccatcaa acacatgata gtgatgatgt tctgctgtgg tttagatcat gaggcaataa 40140
tctaacaatc taacagccac tcctgattag actcagactg actaccttgt ctcttatact 40200
gtcaccaact aacacaccag aacagttcac agggccctcc atcattcttg acacctttct 40260
ttgacagcct cagtgacatg ttaaacacca agcccacgag ttgctttgtt tacagtattg 40320
agaagtggcc ccagagcctc acatgtctta gaaagccctc ttccactgag ctatattacc 40380
aacccctaaa tattttttaa aaatattttc cagtgaaatt tggttttgga acatgattgg 40440
aagaaccttt caaggaccct tttcccccca tatatgcaga gagtagcccc cccccccat 40500
ctcttccttt ccaggagaac cctgtcctgg atgacaatgg ctagtttgcc cagtgttgaa 40560
cttgatctaa atgaagtcct ccagctgcat ctgcatgtct gacttctcct ctctcactct 40620
cagagtggtc tttccgcttc tttcctgagc attaagcagc tttcaggcac aatgaacctg 40680
agtcagttgt ggccagtctt tcattttagc tttgaaaata attagaaagc agaaaataaa 40740
tatgtaagcc agctttggag ctggagaacc agacccatgc attctgaatt ccatttgtac 40800
ccaaagcttt agggtaaaag cctgggttcc cgaggcccag atagactagg tttggccacc 40860
cagccccaca cacaaacaaa agagataggt agaaagggaa aaaacagaga tttaatcaat 40920
atggacacac agggggaaga gataatgagg tccagtgacc ccctaaatcc atcttggggc 40980
actgtcatga gttttaagtt taaatagaag aagttctggc ctaggggaca agatgtggct 41040
aaataagaag tcccagtctt tttgacctat tttttgatac tgcaagtatc aaacaatagc 41100
aagtcttcat tactgattga acatacggat ctagtttcca caagattact tctgccccag 41160
gctctgtagt attgctatcc tggtacaagt gtgtgtgtgt gtgtgtgtgt gtatctgtct 41220
gtctgtcctg gtaggtgtta cctgtttacc tgtttctggg acacaagact gtgacatatt 41280
ttaaattttt gcagcaaata ttttggttca aataaacctc tgaaccatga aggaggaagc 41340
tcagaacttc tgttctatat gatacatgaa tggatcactg cagaaacatt cctgtgttga 41400
atgtaaatgt aaaatgtatt atgttaatat actgcaggag agaatgaggg cttacctttg 41460
taccettggc cttgaagaga ggaaagtagg ttagacagac tcttccagtg accagcaggc 41520
tgacttgcag agtctgcagg ggtgtgacaa aggcaggagt agcaggctgt cgtcctgcat 41580
ttagttacta cgtggtcaca tgaagaatcg tccactctta ctaaaacagt ttctagtgtt 41640

tgttacacac caagttacag ggaaggtgcg tgcatgtggg tgtggtgcac atgtcctgaa 41700
gaccgtcagt cacatcccac ccctggcctg gctcccgggt ccgtttctgc ttcaagtagc 41760
ttgaatatat ttggctgcct cctgcaggaa gggaacattt tcatcgttga ttacgagcta 41820
ctggatggca tcgatgctaa caaaactgac ccctgtacac accagttcct ggctgccccc 41880
atctgcctgc tatataagaa cctagccaac aagattgttc ccattgccat ccaggtaggt 41940
ggctgctcag ccccagccct tctgtacgac ttgtacctct agggctatgt gtgtgtgtgt 42000
gtgtggtggg ggttctgggg cagcaccatc cactccactg agaggcacca gaagctctca 42060
ctccagcttt ccctggggtg ggggagaaaa atgagctact gggggtcgct gctgccatga 42120
gaaggctgtc cacaccacgg cagacagtgc ctttcccagg tacctttgag acattcccta 42180
ctgaggctaa gcactgggga gggcttgtcc tgcagtgacc tgcttttcct cctaccacct 42240
cctgacgact gtttccaaat tgacatgccc acacaagcca gatgggtgaa gctaggggtg 42300
aagttgcaaa cagaagccac tgagctgtgt cggagacctc accagtcctg gaacattgtc 42360
ttccatacaa gtagattctt gcagccccga ggacagtgga gggccacggg agcagtctca 42420
tggggatgga aagtctgtga cactgggtaa tgggaataat atcagtgata tgctggtgtc 42480
tacacagtct aaaagccttt gcctgggaaa gcttagaaat catgaaaact gctgtatgtt 42540
aagtgcctaa gaacagacac tatgcttgag cttggccaaa agaccaagaa gcggtaaaac 42600
tgtgacacat tttaaatttt ccagcaactg tattagtttc aaatagacct ctgaaccaga 42660
ggagaagtag tttagagctt ttgttctgtt caatatgatt cccgaatggg tcactgcaga 42720
aacatctctg tgttgactgt agatataaaa taccttctgt tgtctcagac ctggaggagg 42780
gacttggcac ataaagaagc gtgtatatcg ctggcatcct aaatattctg gatcctggag 42840
cctatgatca ttgggtctca cacagcatct gtctccattt gtgtaactgt caccttactg 42900
ctctcaacca tcttacccat gcaaggagac taaggtacag gtgctcatag ttagccagcc 42960
acggagctga aattcaagcc agctgtctca agcctgaagt ttaacttatg tttataatcc 43020
caggctgtcg gttcaaggac agcctgggct acagagtggt gcccagtgtc aatttgttta 43080
actaattaat caaatcagtt agctgcatat ctataccccc acccctctgc actccaggag 43140
tcctgagcct attatcagag aaagctctat gtgtaagaag ctgaagcatc aaatgaaggc 43200
acaggggatg tgggaaatca ccactgaaag atgccaggag agggatggat taccactgac 43260
aggtgttctg aaatagcatg tcccagacat gtatttatta gagccagagg cctggctcaa 43320
atctcagctc tttccattgt tagcaggaat taggcattgg acaagccaca ggacatggtt 43380
aaccctttct ctatagctgc ccaggagaat taatgcaccc cagggcagtg gtagctgcag 43440
ttactgggac aacccttgaa cagggcccac agctgctgta acagatggat tctaggctgg 43500
gctgtaagaa ggcccatggg tctccgtgtt gaggctggca aggccaggac aattgaaagc 43560
agtgctgaga ggttctctgc caagactgaa ctgaaccagc ctttttgagg atcaacagag 43620
caacatgcta gaggatggag atcatggttc tggcttgggt gtgaggctgc agacagacag 43680

aacataggag aggttgaggg gatgggaccg tgtccaaagc aggaaacact acagcactgt 43740
acaccttcaa agaggactgc atctgctctc agctgacttg gcccttccca gggctagtac 43800
caaagcagaa aagcaaaaca tggaatatgg atgatatcag ctcatttatg caaaaatgaa 43860
tgcaagaaca cagcatgcca caaaccatat gctttacatg ggtagacatt ggtgttggct 43920
tggtgagaca caggacacag ggtcaccacg ctgcatacag aacaggttgg agatagtaag 43980
gataggtcta agttcctcat aataaggttc tgtttatgaa aagtaaacac aaatacacac 44040
atatgcaaag acctagggaa gcagcgacct cttgtggctg gacaagaggt acattctcac 44100
tagttcttgg actcttctgt aaatgctgga gtagtttaca ataataatca tatattattt 44160
tctactcaga aacaaaagat ggcctccttc aaaagcattt aaaataatgc aagcacgttg 44220
tcctgggcat attctagaag agctgggaca aggacaagtc tggaggggtg ggaggaggtg 44280
tggcatgctc tgtataaaag ctcaccatgg gagcccctgt gtggcatggt cagacagaga 44340
ggaaggaact gcagggtgat ggagttacag caggcaaagc aacagagcat ccctacttag 44400
ctccatccag acctctgtgt ggcctggaga gaggctgagt cactcagaac ctcacacttt 44460
atctctttgc aaaataggaa ggatgaggaa gaactgttgg tcaggctgtg gtgaggattt 44520
tgtgatgaca catacgtcaa accttacgac acagcagaaa gtctctggac tcagtgcctg 44580
aggatacctg agtaggacag gtggcccgtg tgctctagga agcaggccac agggtggctg 44640
tctgtgggcc acacagctat ggaaacagag cgatcactgt gaggagcagt cctctgagaa 44700
ccttgctcac taggaccctc tggaaacggc aagcctgttg tcctctgagg acacatgtgc 44760
tgacttctga cttaaacctg atcacttaag gaatctgaaa ttctctccta aaggtagagg 44820
acaaatccat tagcgaacaa gtgggaagga tactgtgggg gtcaggagtg tctccctggc 44880
ctccttgctc atcctcaggc ggtaggggag agctgcttcc ctagtagtcc tgccacctcc 44940
cccacagcac agtggcttag gaagcttagg acgcagtact ccttgctctg gtgttctcga 45000
gacgcactta atccaaacca agacccggag taaattctta gtgtccataa cccaaagact 45060
tagaagaaga gtaaaagaat gaaacctgcc aggactattt cagaatggtt ttgtgttgta 45120
gaaaagtggg tgaggcaagg agctaaaggg gtctgcaacc ctataggtgg aacaacaata 45180
tgaactaacc agtacccccg gagctcgtgt ctctagctgc atatgtagca gaagacggcc 45240
tagtcggcca tcattgggaa gagaggcccc ttggtcttgc aaactttata tgcctcagta 45300
caggggaatg ccagggccaa gaagtgggag tggggggggg agtgggggga gggtctgggg 45360
gacttttggg atagcatttg aaatgtaaat gaagaaaata cctaattaaa aaataaataa 45420
ataaaattct caaagattaa aaaagaaaga aagaaagaaa agtaaagaaa agaaaagtgg 45480
gtgaggtaaa tattttaccc tggggttacg tatttctttt ttcttttaaa aaatatggct 45540
gactaaaaaa aaaaaaatgt aatttaaaaa aaggtggcct cctagaaatt tgaatttgtg 45600
ctgcttgcat tatatttcta ttggctagtg ctactttgga tggagagaca aatcagaaag 45660

gtgcttataa cctctctaat gaaaacaact taaggaaggg agagttattt tggtgctcag 45720
tttaagggaa tacagtccat cgcagagggt gaggcatggc tgtagaaata ggaagtggtt 45780
ggttccattc ataatcagga agcaaataga caggaagtgg ggccagtatg tggaacctcc 45840
aagcccaagt ctcctgctcc taaaagttcc aaatcttcca aaacagcact gccagctagc 45900
gatcaagtgt tcaaacacac gagcctaccg ggacatcaca tttaaaccgc aaaaccatct 45960
tccagattag ttaggggggt gcagctaact ctgctcaggg gccttatctt tctggcttta 46020
aagaaatgag tttttaaaga acagataaca aatttcttta cattttagag aaaaatcaca 46080
tgacccaagc aaggaaactg gaaatgcaaa cgcaagctgg gcccctgggt acctggtttc 46140
agagttaggg ctcagagcag ggctccagca gcaaatcctg atttgcagct ggagcaaaag 46200
ggaactgggc agcatgcagc cccagccttg gtccagaagg agcacggcac tcaaggtcag 46260
ggtgtctgtc agggaggagg atggagggca ctgctgagca gaagtggtaa gctggaggat 46320
gccaggctct ggggagggca caggtcaccg acccttgcag ctgctcgctc cttccccgag 46380
gtctgtacca ctgtcctcaa gttacagaca aggaacctac agctcacaaa caggtcaaaa 46440
gacatgtcac tagggccctc caccactccc cacgtgcttt ttgtgatctg aaagtcatgg 46500
ccggttgatg tttgctttct tgtggaactt ccccagagtt ctcccacagc tcccaatgag 46560
aaataaagtg gagcgttctc tttctctaat gcaccagctc aaccaaaccc ctggagagag 46620
taacccaatc ttcctcccta cggattcaaa gtacgactgg cttttggcca aaatctgggt 46680
gcgttccagt gacttccacg tccatcaaac gatcacccac cttctgcgca cgcatctggt 46740
gtctgaggtg tttggtatcg ccatgtaccg ccagctgcct gctgtgcatc ccctttttcaa 46800
ggtacaacca gccagggctc cacctacaag gaaagattat ctaggagagt agctggcatc 46860
ccagggtgtg tgcgagtgga gtggtgatag ctaggagtag gttgcaaaga ggggcctagg 46920
acagacattc aagggccaag tcacaggagc ctgttacaac cctgctgtcc agaaagcaga 46980
gttcaaaagg gcagttaagt cattttcctt ctcttcccaa atgctgccag cacttgattg 47040
ttgggtcaac tacaggctaa aaaaaattac tgctgctaag ccaggtggtg gtggtggcag 47100
cacttgtctt taatgccagt acttgggagg cagaagcagg cagatctctg agttcaaggc 47160
tagcctggtc tacagagtga gttctggtac agccagggct atatacctag aggaactctt 47220
tgtctagaaa aaaaaataaa aataaaaaaa aataaaacaa aaaagaagag agctggtgct 47280
ttggtctctt cgttgtaaat tagttcaaag caggacgtct gccttagaga ctgataacca 47340
ggatctgttc ctagtgtagt aggagacaag cgccagggcc cttactcctc agcataacag 47400
tggtggccac aggcctccta tagctacagg gcaggaaggt ctacacagag ccctttcact 47460
tcctctctgg ggccagagtg acccccgcac tgtgggctgg cctggcctgg gctgggagtg 47520
ctgagtgagc atggagaccc tcagtgagat ttctcctcca tccagctgct ggtagcccat 47580
gtgaggttca ccattgctat caacactaag gcccgggaac agcttatctg cgagtatggc 47640
ctttttgaca aggtgagtgc cctctcttca tgtggagcct ggacaagctc tgccttgtgg 47700

ctccatccct atctgaggtg tgaaaagtgt gggaaactct ggtccttcaa ccaacaccac 47760
agccagctct cccactctgt gttctctgtc accttttat gtatcctgtc cagtttcagt 47820
gtctgagcct cctccacaca agccactaac ctctacctta caaaacatct gttatttctc 47880
ccatactacc atatccctgt caccctgcct gtggccttcg aggtatttga acagaactgc 47940
ccaataattg cctacccatg tccttgatgt tcccagttct tcaaagctga ttggatgccc 48000
aaagctatgt tctgcacaaa atcctgctct ctccgtttgc agctatacag tgtccggtgc 48060
acgtcacctc atcgtgggag tgggagcgtg ggcctactta aagctgaaca gctctcccac 48120
tccacctcac aggacagact ggcctctcta tacccagcca cccactggcc tccaagtccc 48180
tcacagatga atctacagta tggatatgga cagcttttgg ggaaagcaac caaactcagg 48240
ctctctgctc tcttaccagg ccaatgccac cgggggtgga gggcacgtgc agatggtgca 48300
gagggctgtc caggatctga cctattcctc cctgtgtttc ccggaggcca tcaaggcccg 48360
gggcatggac agcacggaag acatcccctt ctacttctat cgtgatgatg gactgctcgt 48420
gtgggaggct atccagtcgt gagtgtgact gggttctgtg ggaaggggaa accctaaaga 48480
agagtatgat gaggcaagct tgctcctggg ttggctgctg atggagggaa ggggcagagt 48540
ccgacattga gaactgatgg gactgggaga gggacctgct ggatacgcac tcctgatagc 48600
cccctgaccc aggttcacaa tggaggtggt gagcatctac tatgagaacg accaggtggt 48660
ggaggaggac caggaactgc aggacttcgt gaaggatgtt tacgtgtacg gcatgcgggg 48720
caaaaaggcc tcaggtaggc tacagggcaa gtgtgcatct ccaggtcatg aggaacagaa 48780
ggcaggtgac tctgctctcg ggtacccacc agtctcagag cgtccctcgg agcatccagc 48840
ctccctttct ctgagcatct tgctaagtgt gtgtggggag ctaagataga ggcagaggtg 48900
ggtgtccctc acagagatga gctcacggtc ggtggtcggt ggctctgtct taggtttccc 48960
caagtccatc aagagcaggg agaagctgtc cgagtacctg acggtggtga tcttcacggc 49020
ctctgcccag catgcagctg taaacttcgg ccaggtaggc caggctagcc ctttctgggg 49080
gagagtctta ggtacctcac agtgggaacc acctccaatt ccacacccct ggcccaggct 49140
cttgccctac tggtcctccc atctccatgc agcctctgag cctggaacca gcagcagcag 49200
gcatgaggca cccaccaggc tggggcagtt ggaagttctg gaaaaggagg gaaagggtct 49260
gaggagggag gtctggggac cctggaagaa aggcaaggta ggtaacaaag gagggggaca 49320
ctagtggtat ggtgacatgt caaaggtgtg gcctgggaaa ccaggagagg agagggccaa 49380
gacaggtgca gtggggacat cagagaggtg gatggtgtcc acagggcggg gtggagcctg 49440
ctgagctccc tatgaaccaa ggaaaagcag gcctgtggat ccgaggtggg cagccactgg 49500
gcttcttggg cacccacgct gttgatgttg atgttgctct cacagtatga ctggtgctcc 49560
tggatcccca acgctcctcc aactatgcgg gccccaccac ccacggccaa gggtgtggtc 49620
accatcgagc agatcgtgga tactctacca gaccgtggcc gatcatgttg gcatctaggt 49680

gcagtgtggg ccttgagcca gtttcaagaa aatgaggtga gaccaggcac tgttggaaac 49740
accgtagatc actctagttt taacccattc tccagcgcac ggctttgggg ctctgactca 49800
agctagaaac ctgcagtcag aaatcctgat ttctaaggtg gagctattag gagttggggg 49860
tggggttact ccaccccagg tcctcagcgt gggtctgagc cctgggcagg atggcagtgg 49920
gaggcacagg ctctgctcgg agtcaccaga ggggtgtgtg tgtgtgtgtg tgtgtgtgtg 49980
tgtgtgtgtg tgtgtgtgtg ctatcaccct gtaccccata ctcagttgat aaatctattc 50040
cacatggttt cctgactccc cacaaggaga attagagctt cctagtttca cttaggtatc 50100
cttcacagct atgctaagtg caggtcttgg tggaatcagc cctttgcaat gtctcagcgc 50160
tgttcctatc aaagtacctt cagcttgtcc cagacctgct aatgtgctgg ctctatgctc 50220
agtaggacca gaggagttgt tcctaggaga ctaatttcct ggtcaaagga tgtcaagtct 50280
gacttagctt ctccaaacta caccccagat ctcccacccc cttccagcca ctgaacactt 50340
tgcagacatt ggtgtaagca cattcccctg caggatggcc acctctccat gcccaggacc 50400
cctgccggtc tcacgcctta aaaggaagat gagaaagaca aagggcaggc cagcaggacc 50460
ctgtggccac agaacttcag agctgggagc ttccaggttc ccttgcacct taccttgtct 50520
aaaaaaaaac acctggcaac aaggaagtaa cttcctttaa gatctccagc ccttagcttt 50580
tcatagggca aaggaggtag catttgcata atctaagttt taaaaaagaa acaaacttaa 50640
tttgcatatt tatgagatct aagtgtgata tgtgtatgca ttggtgctgc tcagttatgg 50700
catctgatct atcacctcaa acaagttact tccttgtgtc cttgtgtaac attcagactc 50760
ttctagttta gagatatact acagcattaa ctatttaccc taccatgctg tagaacactg 50820
cttaggcctc ctttgaatat actaatttgc catttttcct cctctgtggt aacttctttg 50880
agatgaccag gcaggactgc cttactgttt tagatagggt ctcactatgt agccctggct 50940
ggcctggaac tctcagagac ctgctagcgt ttgcctccca aatgctgaga ttaaagacat 51000
gtatgtgcca ctgcacctgt tgagacagca gtttttagta tcctgtatat ggtatttgca 51060
tctgcttctt tcccagttca tctaagttcc aaagacagga ttttgtatgg ccacgtagtg 51120
ttccatcgag cattctcagt gggtagcaaa ggtcaaaggt gataagagta ggtcctcctc 51180
cttccccccc cctcccccca aaggaagcct cttagagtta gacaaggcct atcagtctat 51240
aaggtaccct cttaaactct ttccatgtct gtcgcagctg tttctaggca tgtacccaga 51300
ggagcatttc attgagaagc cagtgaagga agccatgatc cgattccgca agaacctgga 51360
ggccatcgtc agcgtgatcg ccgagcgcaa taagaacaaa aagctcccct actactacct 51420
gtcaccagac aggattccca acagcgtagc catctaaggc cttgcctccc tacccagcag 51480
ctctctggga aggccagtgg ctttattagc cagatcccag cttgcctggc aggctctggg 51540
tcgatcttcc tgcagctggt gcctcttcca agctcgaagt gctgctcttg ggcctaggtg 51600
gtctggttga aactgaaggc tgttgtagga tggggagaca tcacagagcc tcagcatgtg 51660
ctacttcttc agtggacaca gttgaggaac ctcccaggca gggcagagat gtgcagctgt 51720

```
gtcccccagc ccagctcagt gcctcgtcac tcggtagcat cagaataagt gacaactgtt  51780
ctggctgggt caggggtact ttattctatt tatgcttcct ccaattgctt gcatagagta  51840
ggtgcttaga gaaagttctt ggattaagag tttgttataa aataaacttc atttaaaaca  51900
ggtgtcatac cacatgctga ggtccagtca cccccactcc cacccccacc aaaatcactg  51960
ttctctttcg atcaacaata aaaaagctgg tctactacct cctccaactg acaaggtctt  52020
tgccccccac tccataccag tggccctttc tgcttttgta gacaatacag gtattctaaa  52080
ttaaacacac aaatctaaag atctgaattt atgctatgat tcatatatga gaacacat    52138
```

## Claims

1. A method for detecting at least one designated genetic sequence in a biological sample, comprising:

    a) treating said biological sample to obtain a lysate containing cellular debris including a genomic nucleic acid, wherein said genomic nucleic acid includes at least a portion of an intact nucleic acid;
    b) separating a standard concentration of genomic nucleic acid from a measured amount of magnetic particles saturated with the genomic nucleic acid; and
    c) screening said standard concentration of genomic nucleic acid to detect said designated genetic sequence, wherein this comprises:

        i) adding at least one probe and primer set corresponding to one of said at least one designated genetic sequence to said biological sample in at least one well of a microwell container;
        ii) adding at least one probe and primer set corresponding to a reference sequence to said biological nucleic acid sample in said at least one well of a microwell container;
        iii) screening said biological sample in said at least one well of a microwell container to obtain screening results, wherein one of said screening results is a probe value; and
        iv) comparing the probe values for said probe corresponding to said designated genetic sequence with said probe value corresponding to said reference sequence to detect said at least one designated genetic sequence.

2. The method of claim 1 wherein said biological sample is from a tissue biopsy.

3. The method of claim 1 wherein said biological sample is from a human.

4. The method of claim 1 wherein said biological sample is murine.

5. A method to screen a biological sample for at least one designated genetic sequence within 24 hours of receiving the biological sample at a screening laboratory comprising:

    a) acquiring the identity of at least one designated genetic sequence for a plurality of samples;
    b) acquiring positive and negative controls for said designated genetic sequence;
    c) obtaining at least one probe and primer set to determine the presence of said designated genetic sequence;
    d) receiving said biological sample from a remote user at a screening laboratory and detecting the designated genetic sequence according to the method of claim 1; and
    e) providing screening result to the remote user with 24 hours of receiving said sample at said screening laboratory.

6. The method of claim 5 wherein said biological sample is murine.

7. The method of claim 5 wherein said biological sample is obtained from a human.

8. The method of claim 1 wherein said biological sample is from blood.

**9.** The method of claim 1 wherein said probe values are in the form of CT values.

**10.** The method of claim 1 wherein comparing the probe values for said probe corresponding to said designated genetic sequence with said probe value corresponding to said reference sequence is in the form of RCN values.

**11.** A method for evaluating the validity of data obtained from genotype screening of a strain including at least one designated genetic sequence according to claim 1, said method comprising:

a) dispensing an aliquot of a standard concentration of a biological nucleic acid sample separated from a measured amount of magnetic particles saturated with the nucleic acid from the strain into at least one well of a microwell container;
b) adding at least one probe and primer set corresponding to a designated genetic sequence to said biological nucleic acid sample and at least one probe and primer set corresponding to a reference sequence to said at least one well of a microwell container;
c) screening said biological nucleic acid sample in said at least one well of said microwell container to obtain screening results wherein one of said screening results is a probe value; and
d) comparing the probe values for said probe corresponding to said designated genetic sequence with said probe value corresponding to said reference sequence to detect said at least one designated genetic sequence.

**12.** The method of claim 11 wherein said screening results are probe values.

**13.** The method of claim 11 wherein said probe signal values are in the form of CT values.

**14.** The method of claim 11 wherein comparing the probe values for said probe corresponding to said designated genetic sequence with said probe value corresponding to said reference sequence is in the form of RCN values.

**Patentansprüche**

**1.** Verfahren zur Detektion mindestens einer vorbestimmten genetischen Sequenz in einer biologischen Probe, umfassend:

a) Behandeln der biologischen Probe, um ein Lysat zu erhalten, das Zelltrümmer enthält, umfassend eine genomische Nukleinsäure, wobei die genomische Nukleinsäure mindestens einen Teil einer intakten Nukleinsäure umfasst;
b) Trennen einer Standardkonzentration der genomischen Nukleinsäure von einer abgemessenen Menge magnetischer Partikel, die der genomischen Nukleinsäure gesättigt sind; und
c) Screenen der Standardkonzentration der genomischen Nukleinsäure, um die vorbestimmte genetische Sequenz zu detektieren, wobei dies umfasst:

i) Zugegeben mindestens einer Sonde und einer Primersatzes, entsprechend mindestens einer der mindestens einen vorbestimmten genetischen Sequenz, zu der biologischen Probe in mindestens einer Vertiefung eines Mikrovertiefungsbehälters;
ii) Zugeben mindestens einer Sonde und eines Primersatzes, entsprechend einer Referenzsequenz, zu der biologischen Nukleinsäureprobe in der mindestens einen Vertiefung eines Mikrovertiefungsbehälters;
iii) Screenen der biologischen Probe in der mindestens einen Vertiefung eines Mikrovertiefungsbehälters, um Screeningergebnisse zu erhalten, wobei eines der Screeningergebnisse ein Sondenwert ist, und
iv) Vergleichen der Sondenwerte für die Sonde, die der vorbestimmten genetischen Sequenz entspricht mit dem Sondenwert, der der Referenzsequenz entspricht, um die mindestens eine vorbestimmte genetische Sequenz zu detektieren.

**2.** Verfahren nach Anspruch 1, wobei die biologische Probe von einer Gewebebiopsie ist.

**3.** Verfahren nach Anspruch 1, wobei die biologische Probe von einem Menschen ist.

**4.** Verfahren nach Anspruch 1, wobei die biologische Probe murin ist.

**5.** Verfahren zum Screenen einer biologischen Probe auf mindestens eine vorbestimmte genetische Sequenz innerhalb von 24 Stunden nach Empfang der biologischen Probe in einem Screeninglabor, umfassend

a) Erlangen der Identität mindestens einer vorbestimmten genetischen Sequenz für eine Vielzahl von Proben;
b) Erlangen positiver und negativer Kontrollen für die vorbestimmte genetische Sequenz;
c) Erhalten mindestens einer Sonde und eines Primersatzes zur Bestimmung der Anwesenheit der vorbestimmten genetischen Sequenz;
d) Empfang einer biologischen Probe von einem entfernten Benutzer in einem Screeninglabor und Detektieren der vorbestimmten genetischen Sequenz gemäß dem Verfahren nach Anspruch 1; und
e) Übermitteln der Screeningergebnisse an den entfernten Benutzer innerhalb von 24 Stunden nach Empfang der Probe in dem Screeninglabor.

**6.** Verfahren nach Anspruch 5, wobei die biologische Probe murin ist.

**7.** Verfahren nach Anspruch 5, wobei die biologische Probe von einem Menschen erhalten wurde.

**8.** Verfahren nach Anspruch 1, wobei die biologische Probe aus Blut ist.

**9.** Verfahren nach Anspruch 1, wobei die Sondenwerte in Form von CT-Werten sind.

**10.** Verfahren nach Anspruch 1, wobei Vergleichen der Sondenwerte für die Sonde, die der vorbestimmten genetischen Sequenz entspricht mit dem Sondenwert, der der Referenzsequenz entspricht in der Form von RCN-Werten ist.

**11.** Verfahren zur Evaluierung der Validität von Daten, die durch Genotypscreenen eines Stammes erhalten wurden, umfassend mindestens eine vorbestimmte genetische Sequenz nach Anspruch 1, wobei das Verfahren umfasst:

a) Zuführen eines Aliquots einer Standardkonzentration einer biologischen Nukleinsäureprobe, die von einer abgemessenen Menge mit der Nukleinsäure aus dem Stamm gesättigter magnetischer Partikel getrennt ist, in mindestens eine Vertiefung eines Mikrovertiefungsbehälters;
b) Zugeben mindestens einer Sonde und eines Primersatzes, entsprechend einer vorbestimmten genetischen Sequenz, zu der biologischen Nukleinsäure Probe und mindestens einer Sonde und eines Primersatzes, entsprechend einer Referenzsequenz, zu der mindestens einen Vertiefung eines Mikrovertiefungsbehälters;
c) Screenen der biologischen Nukleinsäureprobe in der mindestens einen Vertiefung des Mikrovertiefungsbehälters, um Screeningergebnisse zu erhalten, wobei eines der Screeningergebnisse ein Sondenwert ist; und
d) Vergleich der Sondenwerte für die Sonde, die der vorbestimmten genetischen Sequenz entspricht, mit dem Sondenwert, der der Referenzsequenz entspricht, um die mindestens eine vorbestimmte genetische Sequenz zu detektieren.

**12.** Verfahren nach Anspruch 11, wobei die Screeningergebnisse Sondenwerte sind.

**13.** Verfahren nach Anspruch 11, wobei die Sondensignalwerte in Form von CT-Werten sind.

**14.** Verfahren nach Anspruch 11, wobei Vergleichen der Sondenwerte für die Sonde, die der vorbestimmten genetischen Sequenz entspricht mit dem Sondenwert, der der Referenzsequenz entspricht, in der Form von RCN-Werten ist.

## Revendications

**1.** Procédé pour détecter au moins une séquence génétique désignée dans un échantillon biologique, comprenant le fait :

a) de traiter ledit échantillon biologique pour obtenir un lysat contenant des débris cellulaires comportant un acide nucléique génomique, où ledit acide nucléique génomique comporte au moins une partie d'un acide nucléique intact ;
b) de séparer une concentration standard d'acide nucléique génomique d'une quantité mesurée de particules magnétiques saturées avec l'acide nucléique génomique ; et
c) de cribler ladite concentration standard de l'acide nucléique génomique pour détecter ladite séquence génétique désignée, où ceci comprend le fait :

i) d'ajouter au moins une sonde et un ensemble d'amorces correspondant à l'une de ladite au moins une séquence génétique désignée audit échantillon biologique dans au moins un puits d'un récipient à micro-puits ;
ii) d'ajouter au moins une sonde et un ensemble d'amorces correspondant à une séquence de référence audit échantillon d'acide nucléique biologique dans ledit au moins un puits d'un récipient à micro-puits ;
iii) de cribler ledit échantillon biologique dans ledit au moins un puits d'un récipient à micro-puits pour obtenir des résultats de criblage, où l'un desdits résultats de criblage est une valeur de sonde ; et
iv) de comparer les valeurs de sonde pour ladite sonde correspondant à ladite séquence génétique désignée à ladite valeur de sonde correspondant à ladite séquence de référence pour détecter ladite au moins une séquence génétique désignée.

**2.** Procédé de la revendication 1, dans lequel ledit échantillon biologique provient d'une biopsie tissulaire.

**3.** Procédé de la revendication 1, dans lequel ledit échantillon biologique provient d'un être humain.

**4.** Procédé de la revendication 1, dans lequel ledit échantillon biologique est murin.

**5.** Procédé pour cribler un échantillon biologique pour au moins une séquence génétique désignée dans les 24 heures qui suivent la réception de l'échantillon biologique dans un laboratoire de criblage comprenant le fait :

a) d'acquérir l'identité d'au moins une séquence génétique désignée pour une pluralité d'échantillons ;
b) d'acquérir de témoins positif et négatif pour ladite séquence génétique désignée ;
c) d'obtenir au moins une sonde et un ensemble d'amorces pour déterminer la présence de ladite séquence génétique désignée ;
d) de recevoir ledit échantillon biologique à partir d'un utilisateur distant dans un laboratoire de criblage et de détecter la séquence génétique désignée selon le procédé de la revendication 1 ; et
e) de fournir le résultat de criblage à l'utilisateur distant dans les 24 heures qui suivent la réception dudit échantillon dans ledit laboratoire de criblage.

**6.** Procédé de la revendication 5, dans lequel ledit échantillon biologique est murin.

**7.** Procédé de la revendication 5, dans lequel ledit échantillon biologique est obtenu à partir d'un être humain.

**8.** Procédé de la revendication 1, dans lequel ledit échantillon biologique provient du sang.

**9.** Procédé de la revendication 1, dans lequel lesdites valeurs de sonde sont sous forme de valeurs de CT.

**10.** Procédé de la revendication 1, dans lequel la comparaison des valeurs de sonde pour ladite sonde correspondant à ladite séquence génétique désignée avec ladite valeur de sonde correspondant à ladite séquence de référence est sous forme de valeurs de RCN.

**11.** Procédé pour évaluer la validité de données obtenues à partir de criblage de génotype d'une souche comportant au moins une séquence génétique désignée selon la revendication 1, ledit procédé comprenant le fait :

a) de distribuer une aliquote d'une concentration standard d'un échantillon d'acide nucléique biologique séparée d'une quantité mesurée de particules magnétiques saturées avec l'acide nucléique de la souche dans ledit au moins un puits d'un récipient à micro-puits ;
b) d'ajouter au moins une sonde et un ensemble d'amorces correspondant à une séquence génétique désignée audit échantillon d'acide nucléique biologique et au moins une sonde et un ensemble d'amorces correspondant à une séquence de référence audit au moins un puits d'un récipient à micro-puits ;
c) de cribler ledit échantillon d'acide nucléique biologique dans ledit au moins un puits dudit récipient à micro-puits pour obtenir des résultats de criblage où l'un desdits résultats de criblage est une valeur de sonde ; et
d) de comparer les valeurs de sonde pour ladite sonde correspondant à ladite séquence génétique désignée à ladite valeur de sonde correspondant à ladite séquence de référence pour détecter ladite au moins une séquence génétique désignée.

**12.** Procédé de la revendication 11, dans lequel lesdits résultats de criblage sont des valeurs de sonde.

**13.** Procédé de la revendication 11, dans lequel lesdites valeurs de signal de sonde sont sous forme de valeurs de CT.

**14.** Procédé de la revendication 11, dans lequel la comparaison des valeurs de sonde pour ladite sonde correspondant à ladite séquence génétique désignée avec ladite valeur de sonde correspondant à ladite séquence de référence est sous forme de valeurs de RCN.

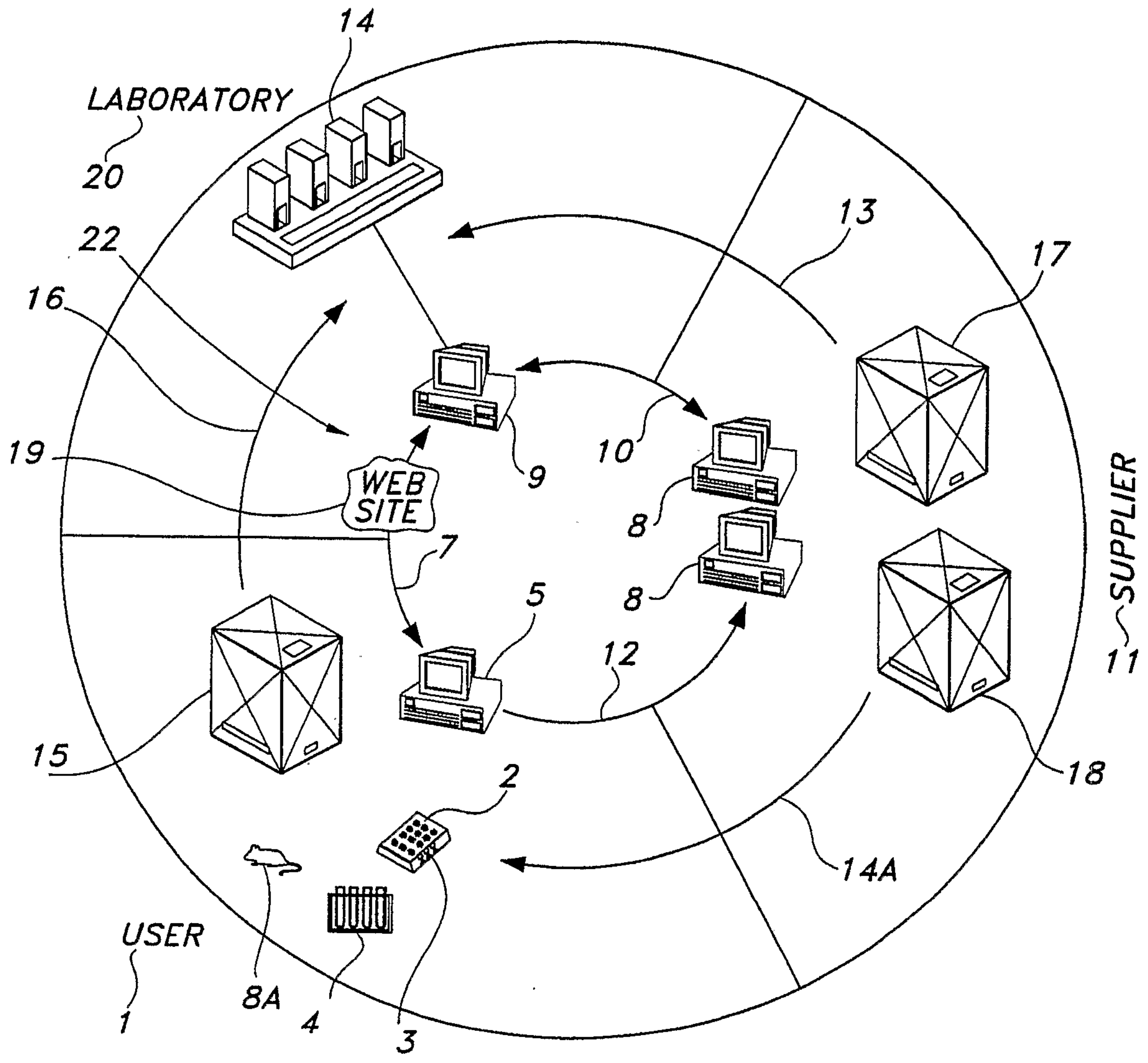
14
LABORATORY
20
22
13
17
16
19
WEB SITE
9
10
8
8
7
5
SUPPLIER
11
12
15
18
2
14A
USER
1
8A
4
3

FIG. 1

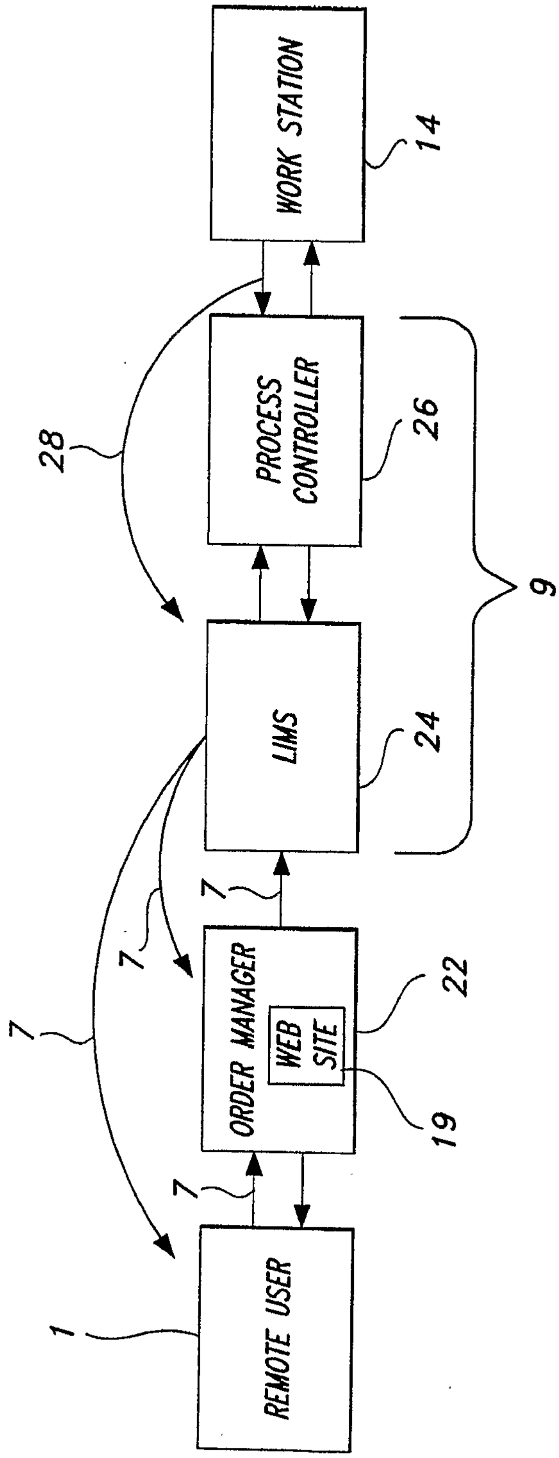
WORK STATION
14
PROCESS CONTROLLER
26
28
LIMS
24
9
ORDER MANAGER
WEB SITE
22
19
7
7
7
7
REMOTE USER
1

FIG. 2

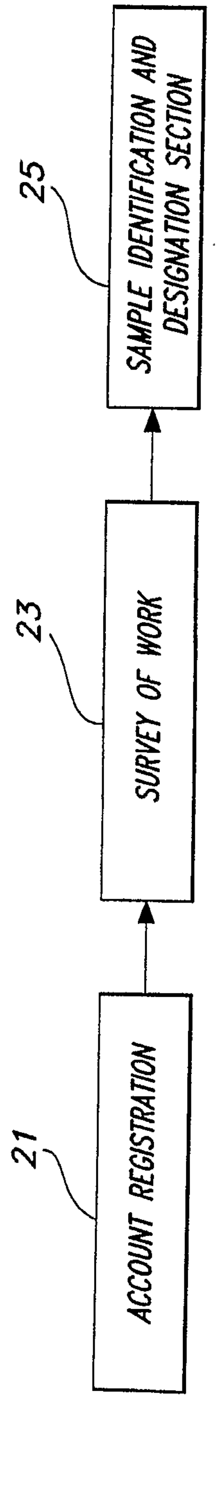
25
SAMPLE IDENTIFICATION AND DESIGNATION SECTION
23
SURVEY OF WORK
21
ACCOUNT REGISTRATION

FIG. 3

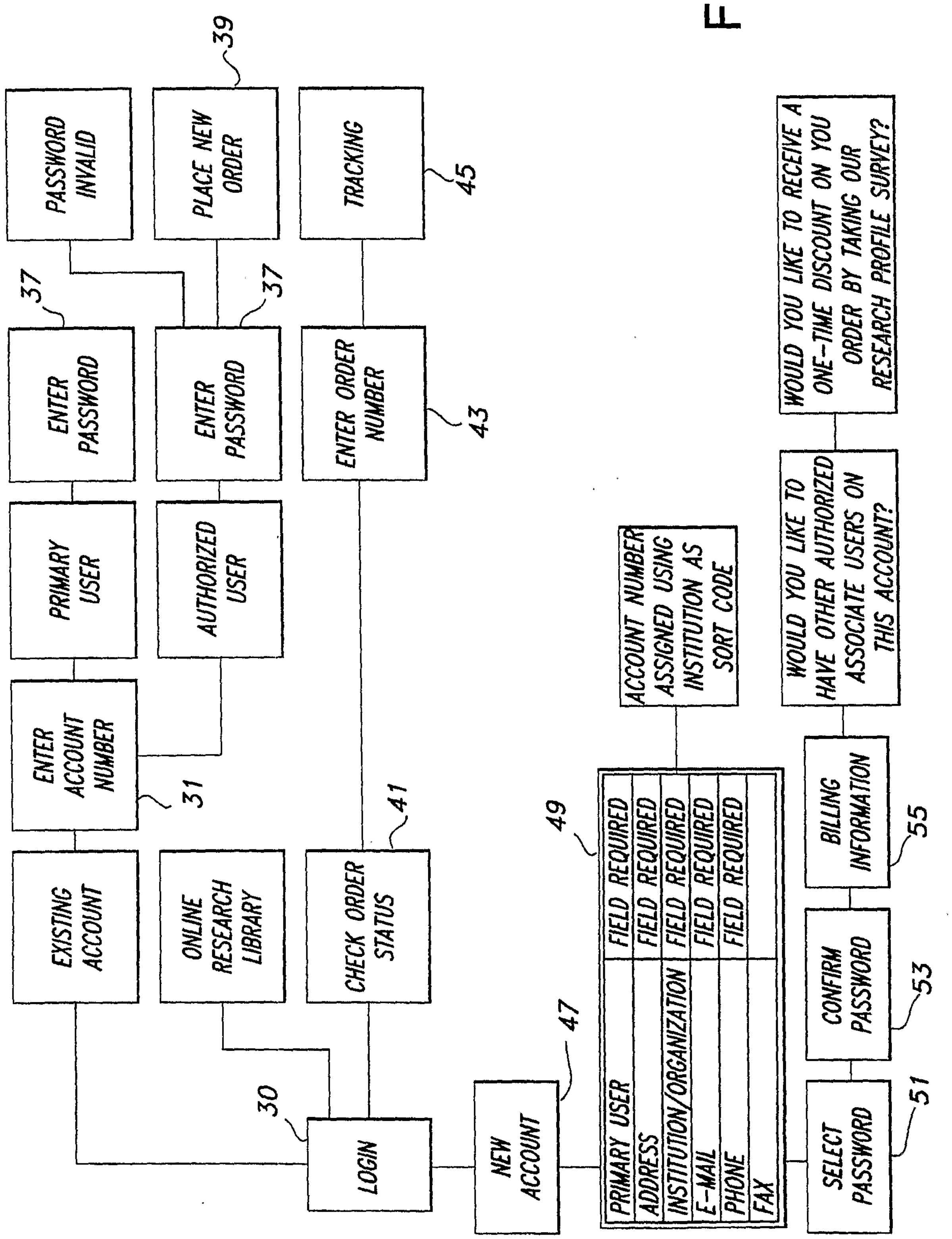
PASSWORD INVALID
PLACE NEW ORDER
39
TRACKING
45
37
ENTER PASSWORD
ENTER PASSWORD
37
ENTER ORDER NUMBER
43
PRIMARY USER
AUTHORIZED USER
ENTER ACCOUNT NUMBER
31
EXISTING ACCOUNT
ONLINE RESEARCH LIBRARY
CHECK ORDER STATUS
41
LOGIN
30
NEW ACCOUNT
47
49
PRIMARY USER FIELD REQUIRED
ADDRESS FIELD REQUIRED
INSTITUTION/ORGANIZATION FIELD REQUIRED
E-MAIL FIELD REQUIRED
PHONE FIELD REQUIRED
FAX
ACCOUNT NUMBER ASSIGNED USING INSTITUTION AS SORT CODE
SELECT PASSWORD
51
CONFIRM PASSWORD
53
BILLING INFORMATION
55
WOULD YOU LIKE TO HAVE OTHER AUTHORIZED ASSOCIATE USERS ON THIS ACCOUNT?
WOULD YOU LIKE TO RECEIVE A ONE-TIME DISCOUNT ON YOU ORDER BY TAKING OUR RESEARCH PROFILE SURVEY?

FIG. 4

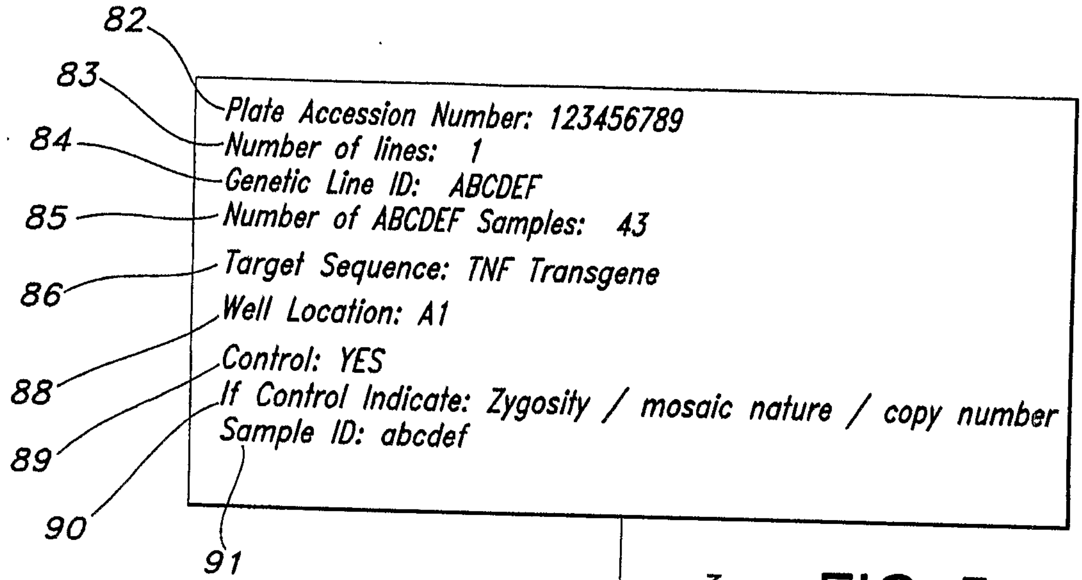
82
83
84
85
86
88
89
90
91
Plate Accession Number: 123456789
Number of lines: 1
Genetic Line ID: ABCDEF
Number of ABCDEF Samples: 43
Target Sequence: TNF Transgene
Well Location: A1
Control: YES
If Control Indicate: Zygosity / mosaic nature / copy number
Sample ID: abcdef

FIG. 5

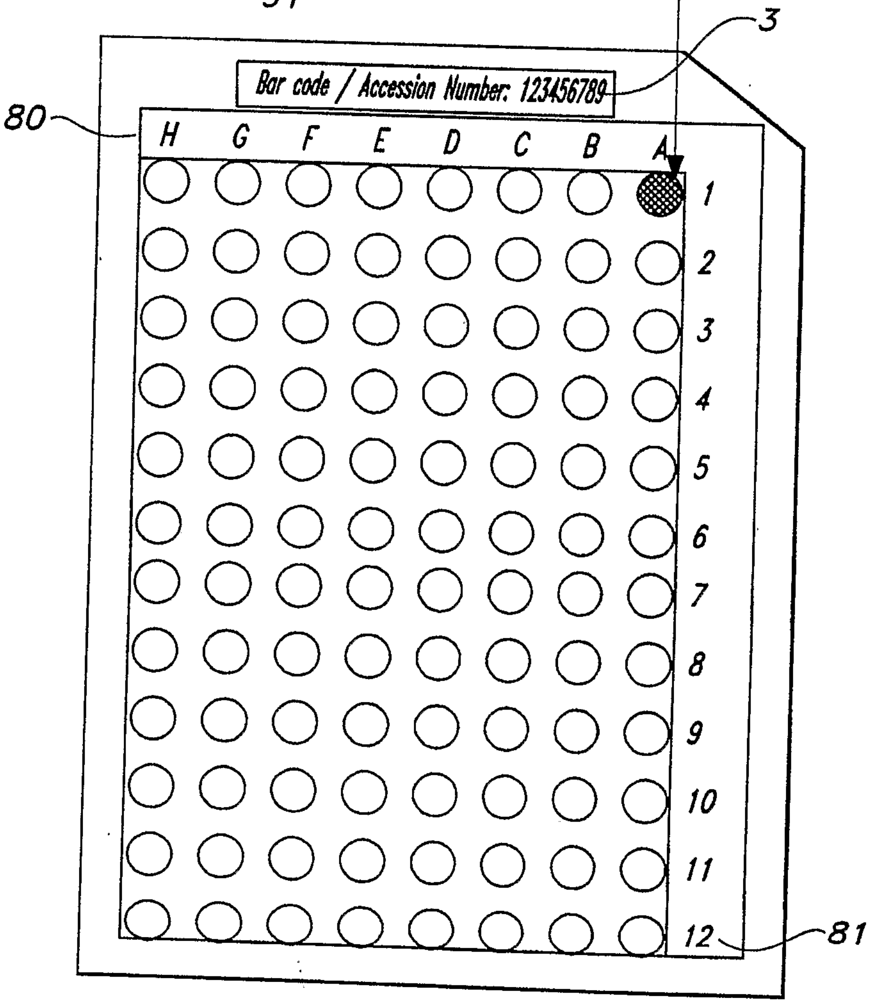
3
80
81
Bar code / Accession Number: 123456789
H G F E D C B A
1
2
3
4
5
6
7
8
9
10
11
12

FIG. 6

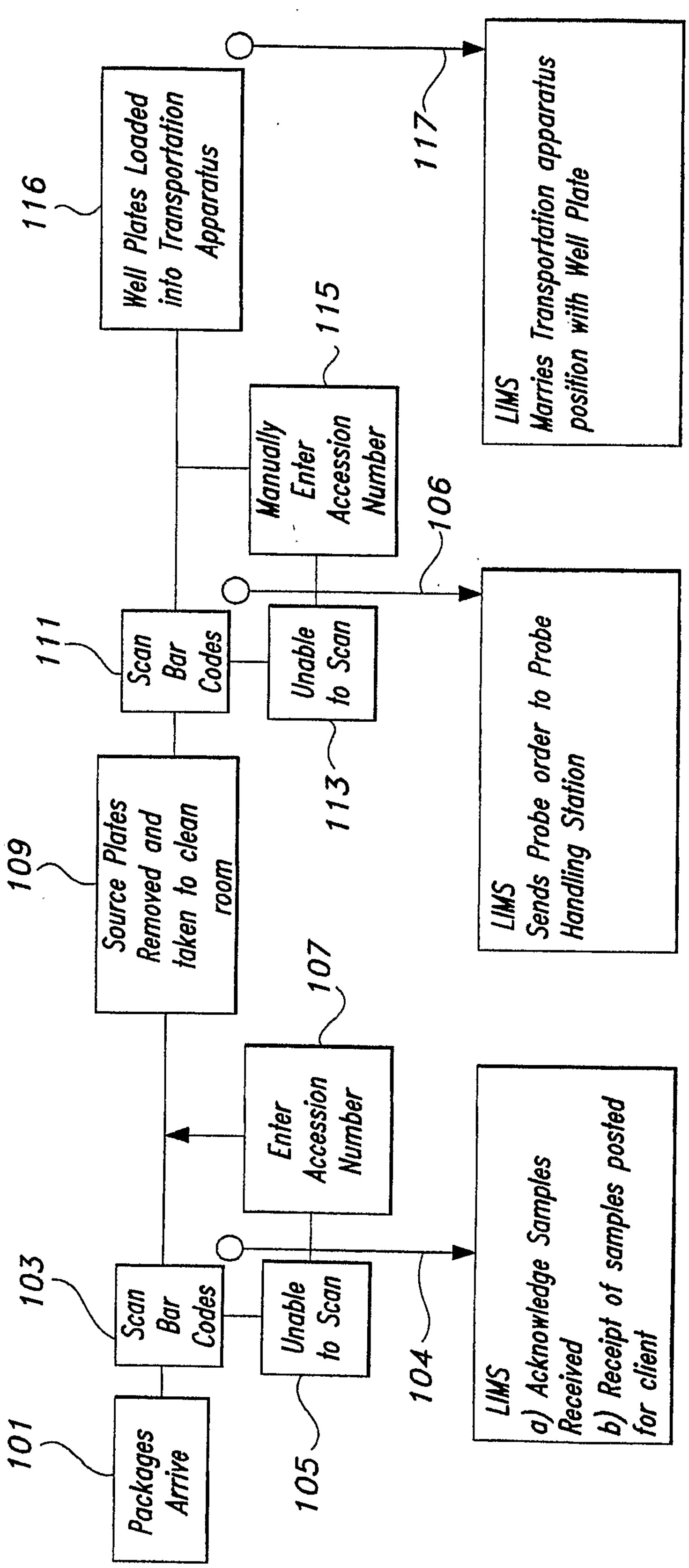
101
Packages Arrive
103
Scan Bar Codes
105
Unable to Scan
104
LIMS
a) Acknowledge Samples Received
b) Receipt of samples posted for client
107
Enter Accession Number
109
Source Plates Removed and taken to clean room
111
Scan Bar Codes
113
Unable to Scan
106
LIMS
Sends Probe order to Probe Handling Station
115
Manually Enter Accession Number
116
Well Plates Loaded into Transportation Apparatus
117
LIMS
Marries Transportation apparatus position with Well Plate

FIG. 7A

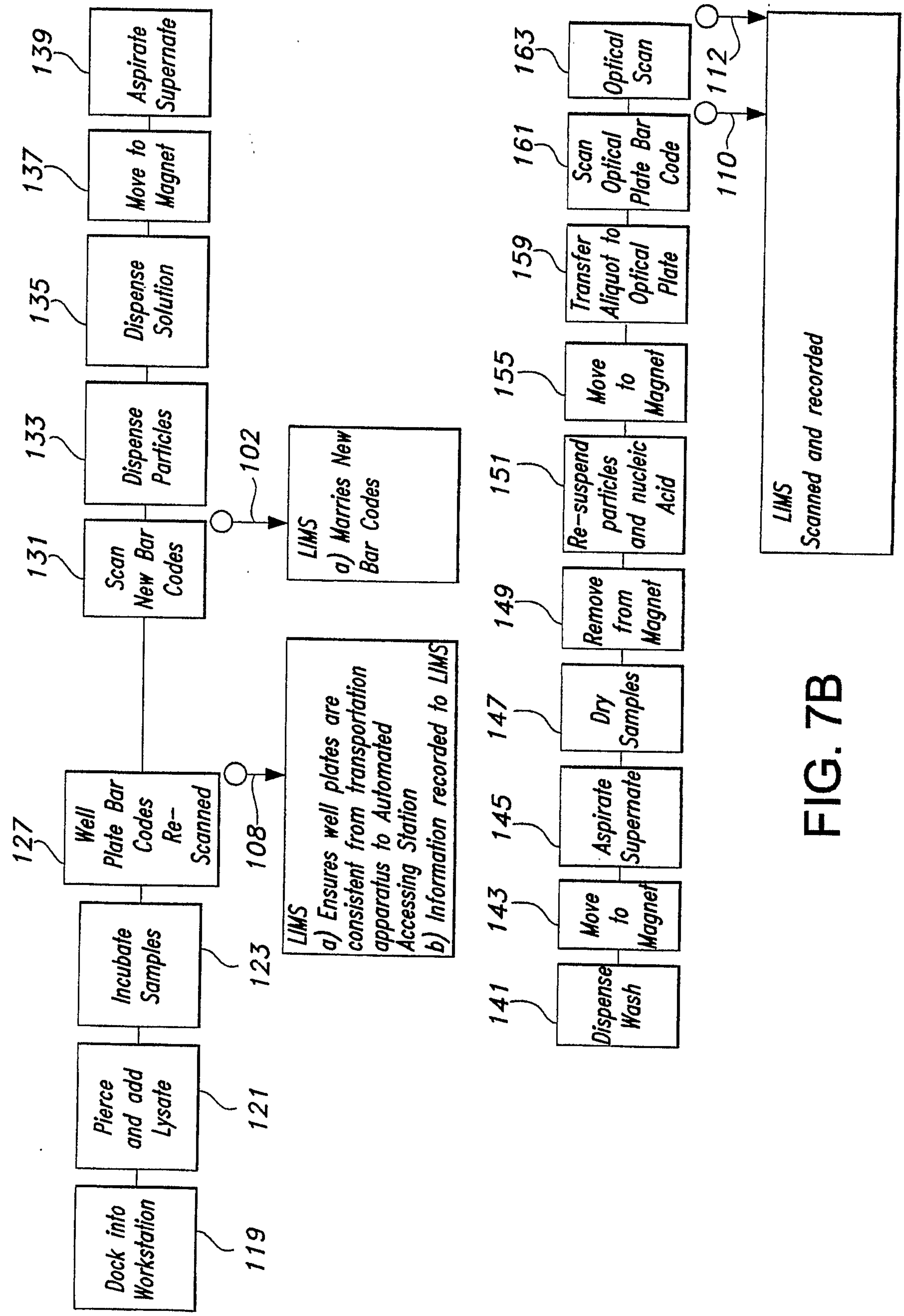
Dock into Workstation
119
Pierce and add Lysate
121
Incubate Samples
123
Well Plate Bar Codes Re-Scanned
127
108
LIMS
a) Ensures well plates are consistent from transportation apparatus to Automated Accessing Station
b) Information recorded to LIMS
Scan New Bar Codes
131
102
LIMS
a) Marries New Bar Codes
Dispense Particles
133
Dispense Solution
135
Move to Magnet
137
Aspirate Supernate
139
Dispense Wash
141
Move to Magnet
143
Aspirate Supernate
145
Dry Samples
147
Remove from Magnet
149
Re-suspend particles and nucleic Acid
151
Move to Magnet
155
Transfer Aliquot to Optical Plate
159
Scan Optical Plate Bar Code
161
Optical Scan
163
110
112
LIMS
Scanned and recorded

FIG. 7B

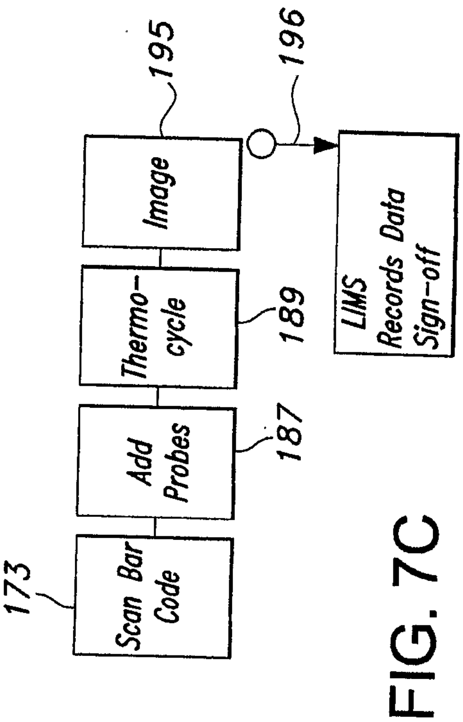
173
Scan Bar Code
187
Add Probes
189
Thermo-cycle
195
Image
196
LIMS Records Data Sign-off

FIG. 7C

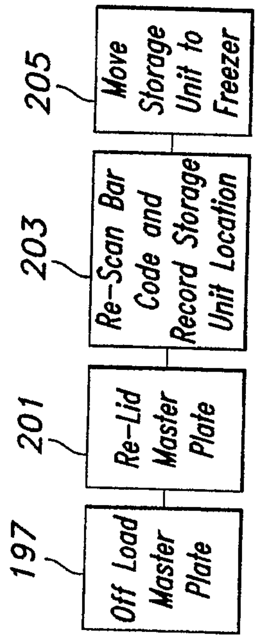
197
Off Load Master Plate
201
Re-Lid Master Plate
203
Re-Scan Bar Code and Record Storage Unit Location
205
Move Storage Unit to Freezer

FIG. 7D

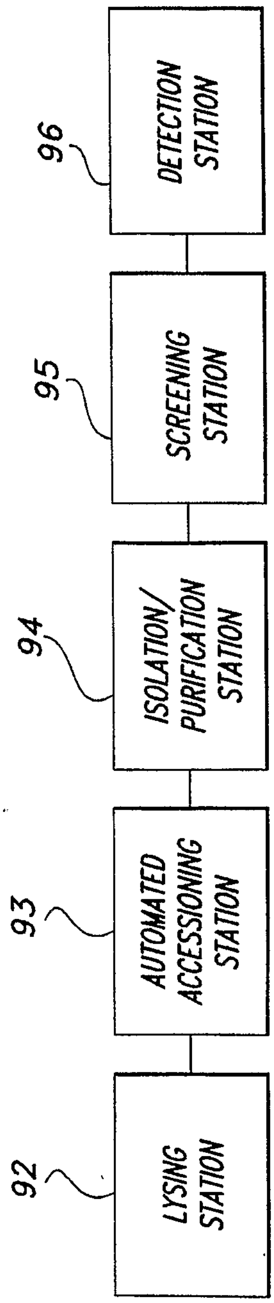
92
LYSING STATION
93
AUTOMATED ACCESSIONING STATION
94
ISOLATION/ PURIFICATION STATION
95
SCREENING STATION
96
DETECTION STATION

FIG. 8

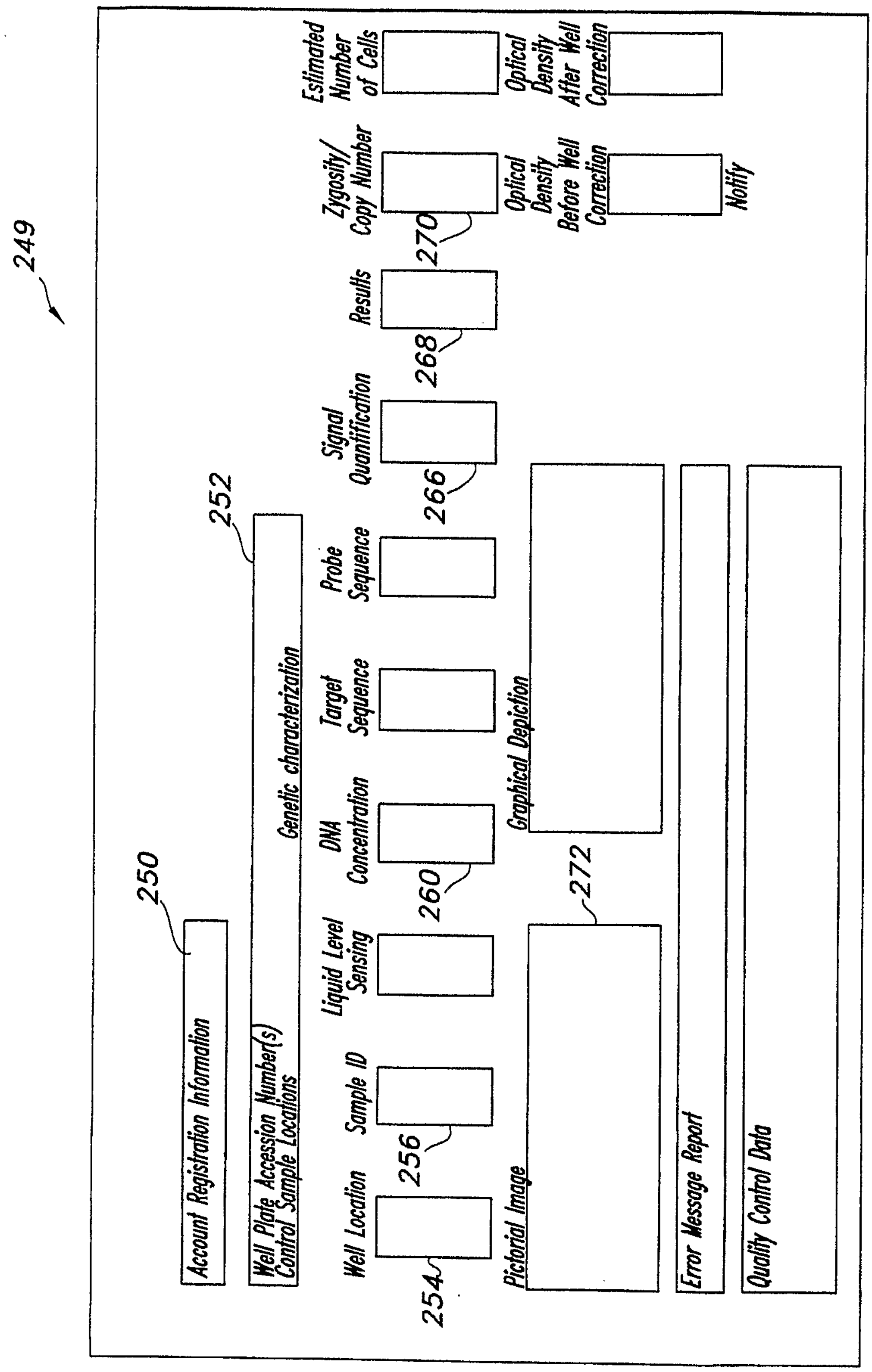
249
Account Registration Information
250
Well Plate Accession Number(s)
Control Sample Locations
Genetic characterization
252
Well Location
Sample ID
Liquid Level Sensing
DNA Concentration
Target Sequence
Probe Sequence
Signal Quantification
Results
Zygosity/ Copy Number
Estimated Number of Cells
254
256
260
266
268
270
Optical Density Before Well Correction
Optical Density After Well Correction
Notify
Pictorial Image
Graphical Depiction
272
Error Message Report
Quality Control Data

FIG. 9

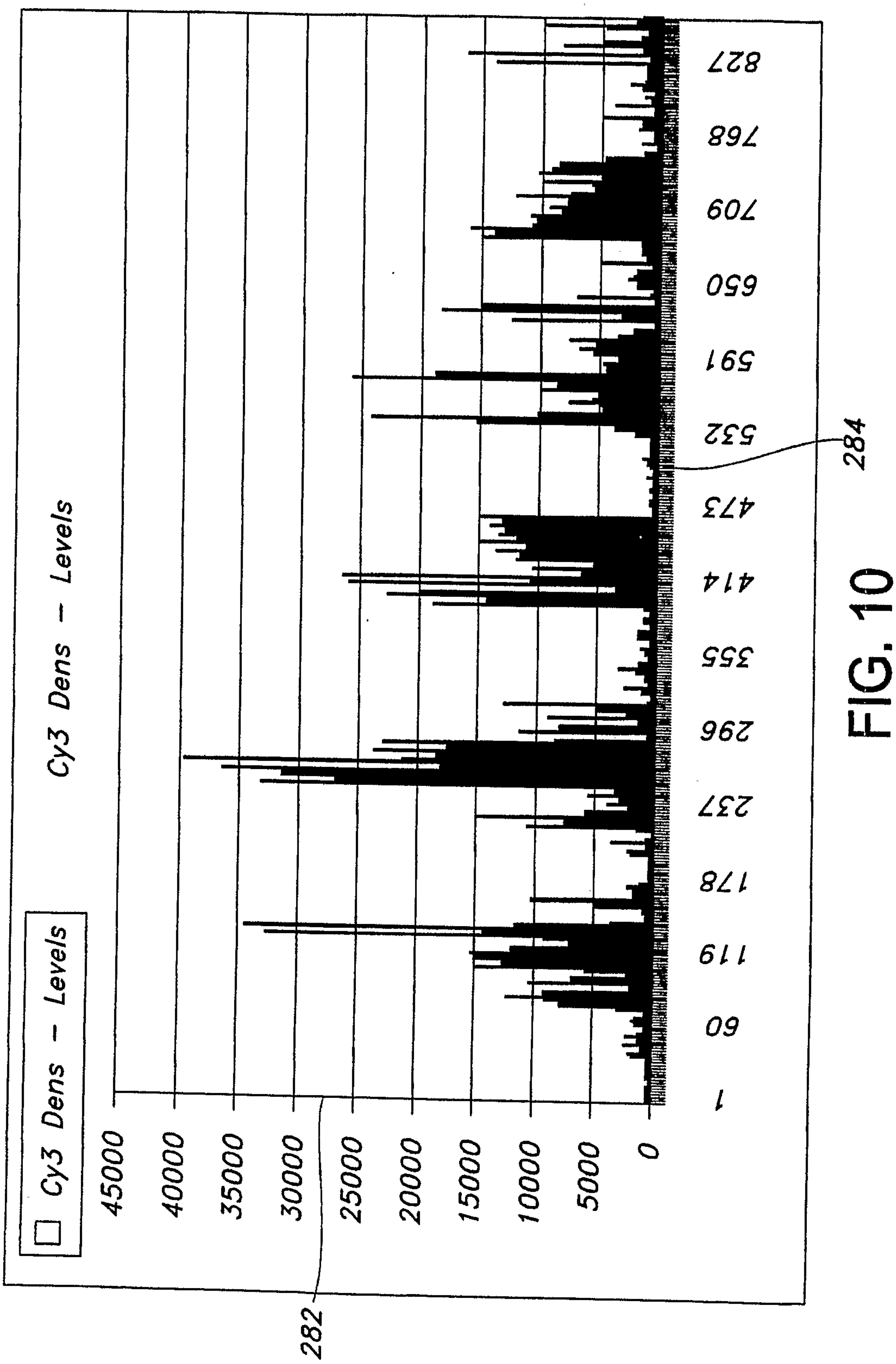
Cy3 Dens - Levels
Cy3 Dens - Levels
45000
40000
35000
30000
25000
20000
15000
10000
5000
0
1
60
119
178
237
296
355
414
473
532
591
650
709
768
827
282
284

FIG. 10

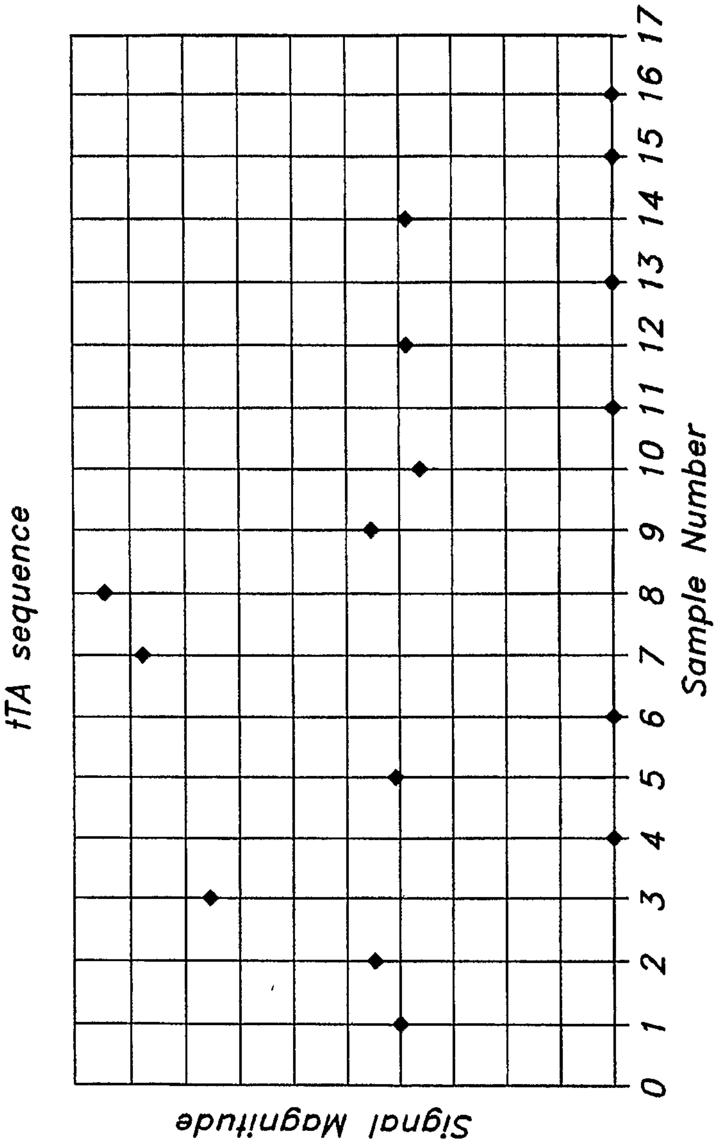
tTA sequence
Signal Magnitude
Sample Number
0 1 2 3 4 5 6 7 8 9 10 11 12 13 14 15 16 17

FIG. 11

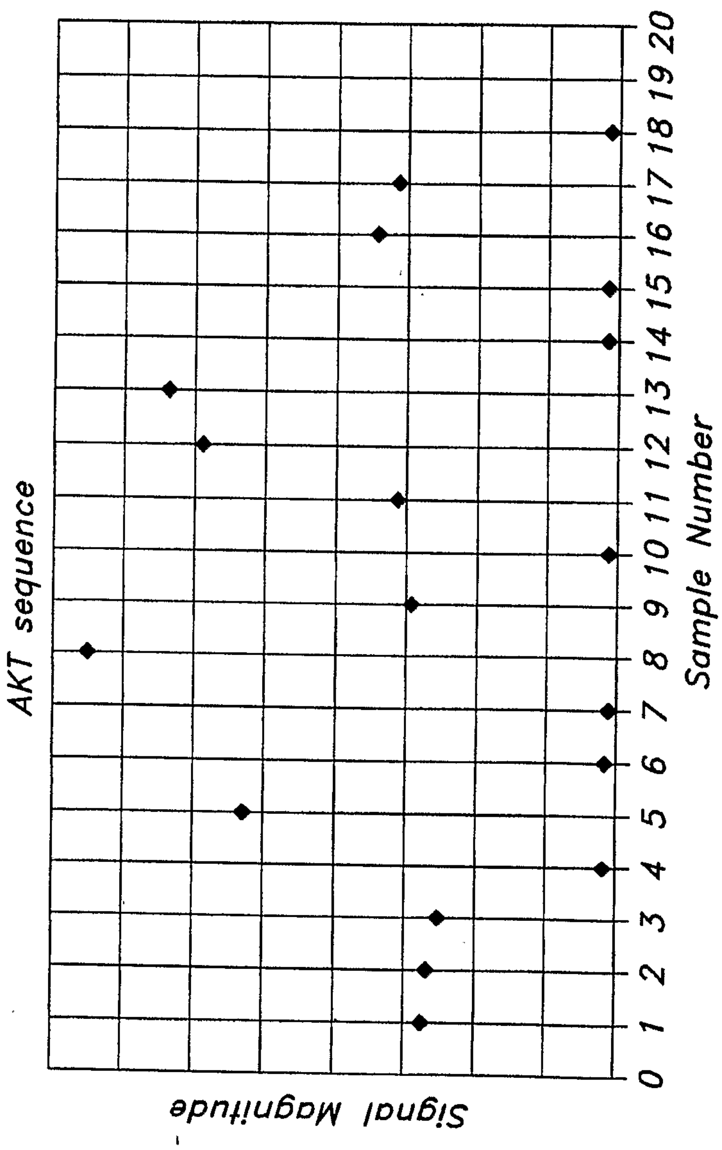
AKT sequence
Signal Magnitude
0 1 2 3 4 5 6 7 8 9 10 11 12 13 14 15 16 17 18 19 20
Sample Number

FIG. 12

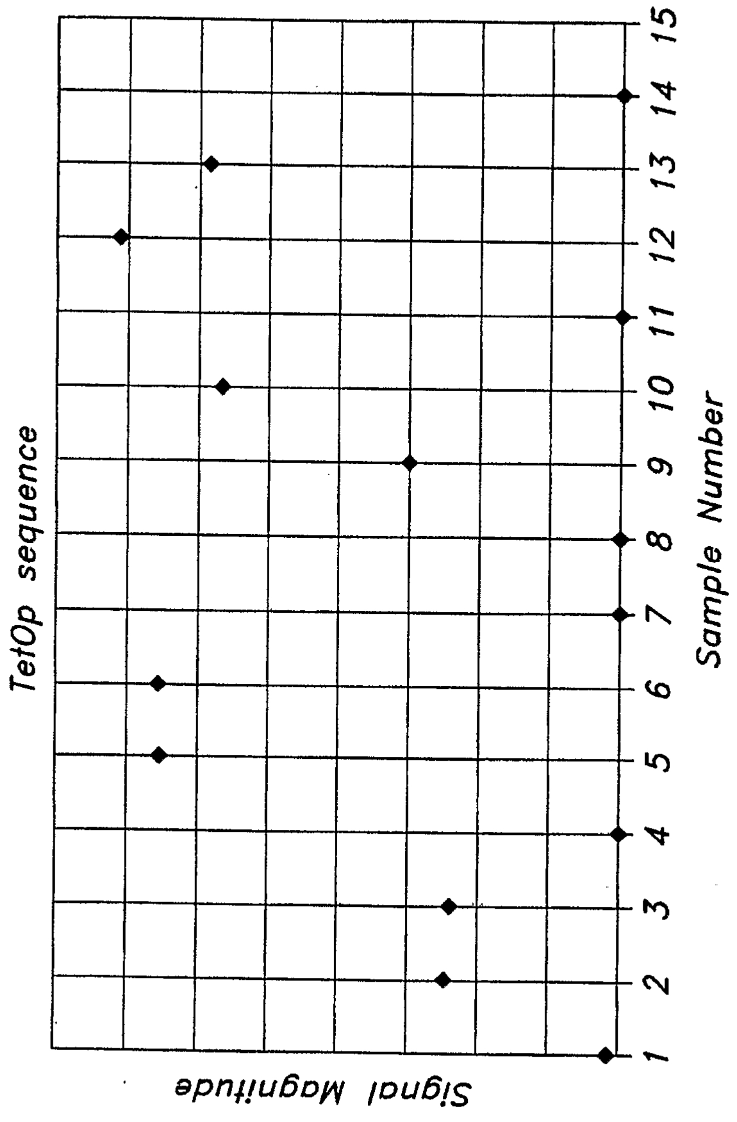
TetOp sequence
Signal Magnitude
Sample Number
1 2 3 4 5 6 7 8 9 10 11 12 13 14 15

FIG. 13

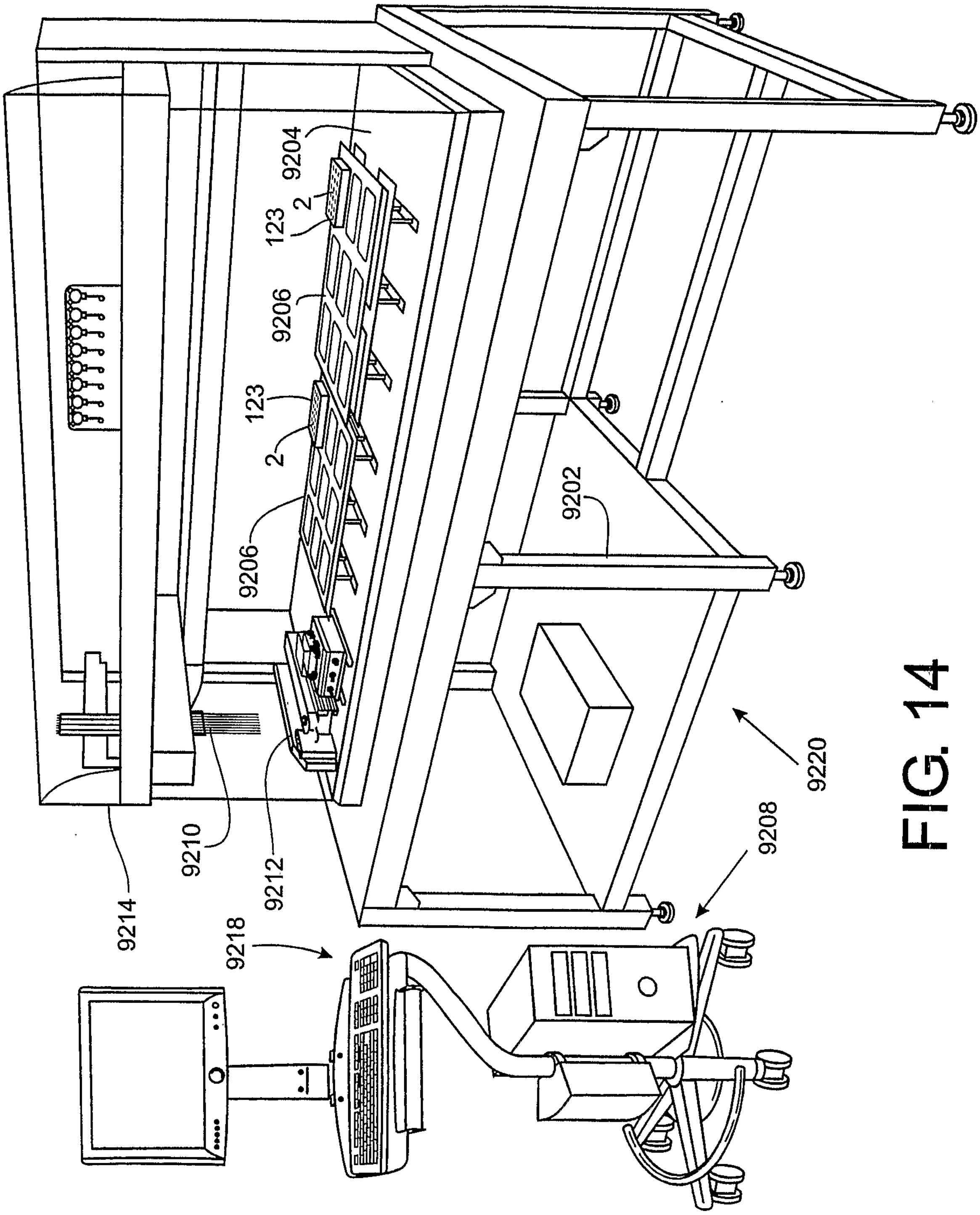


FIG. 14

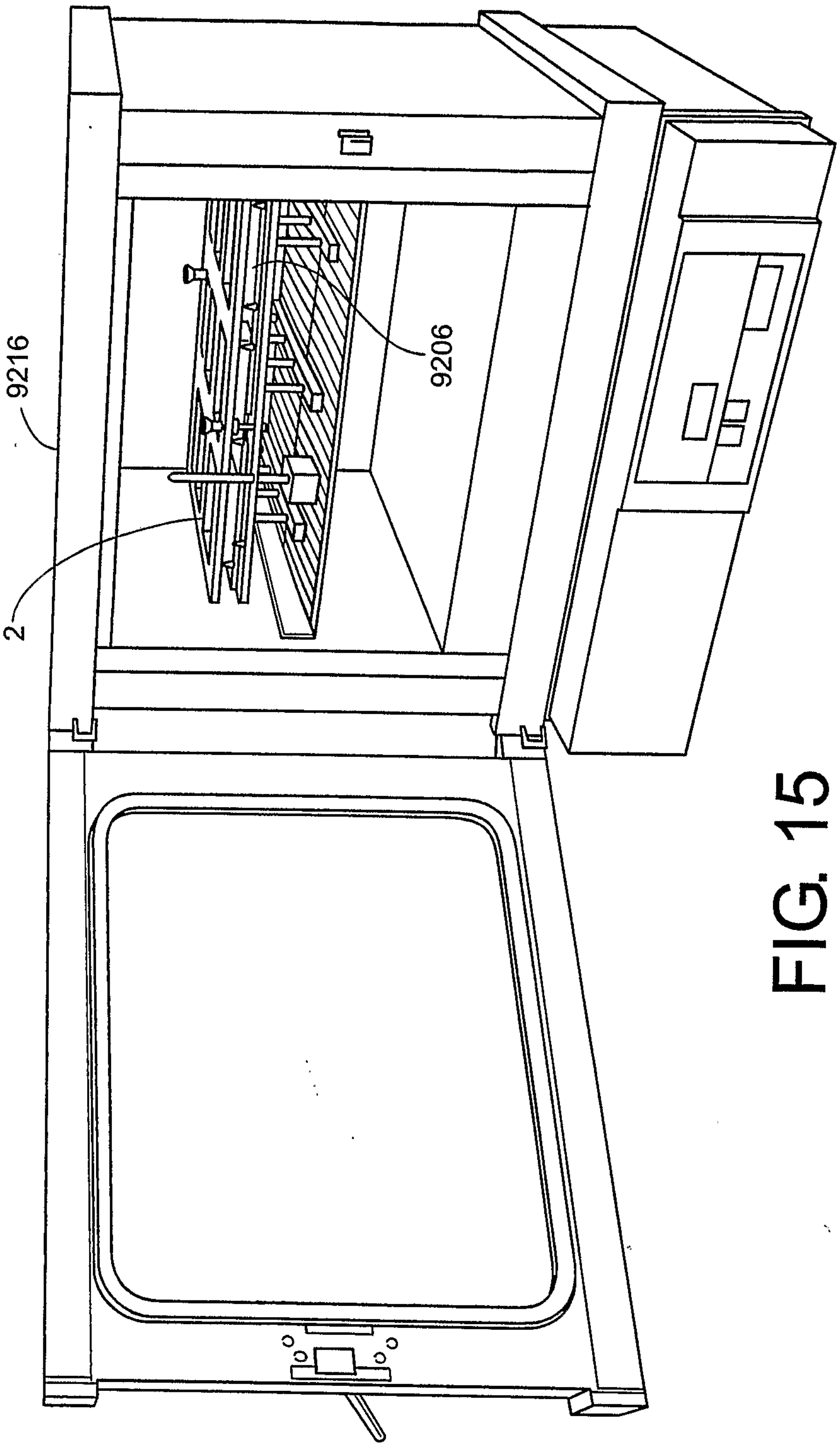


FIG. 15

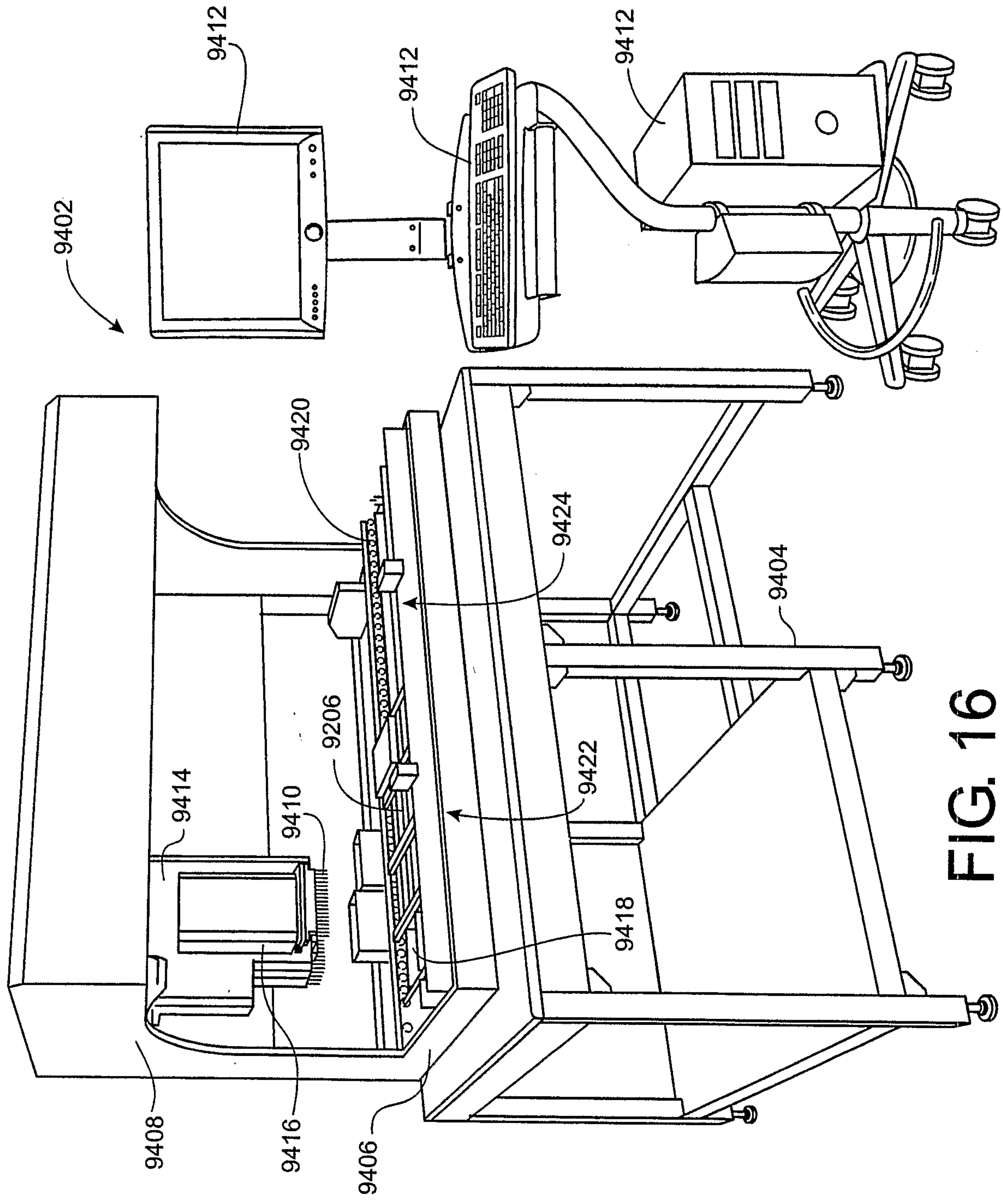
9402
9412
9412
9412
9412
9420
9424
9404
9206
9422
9414
9410
9418
9408
9416
9406

FIG. 16

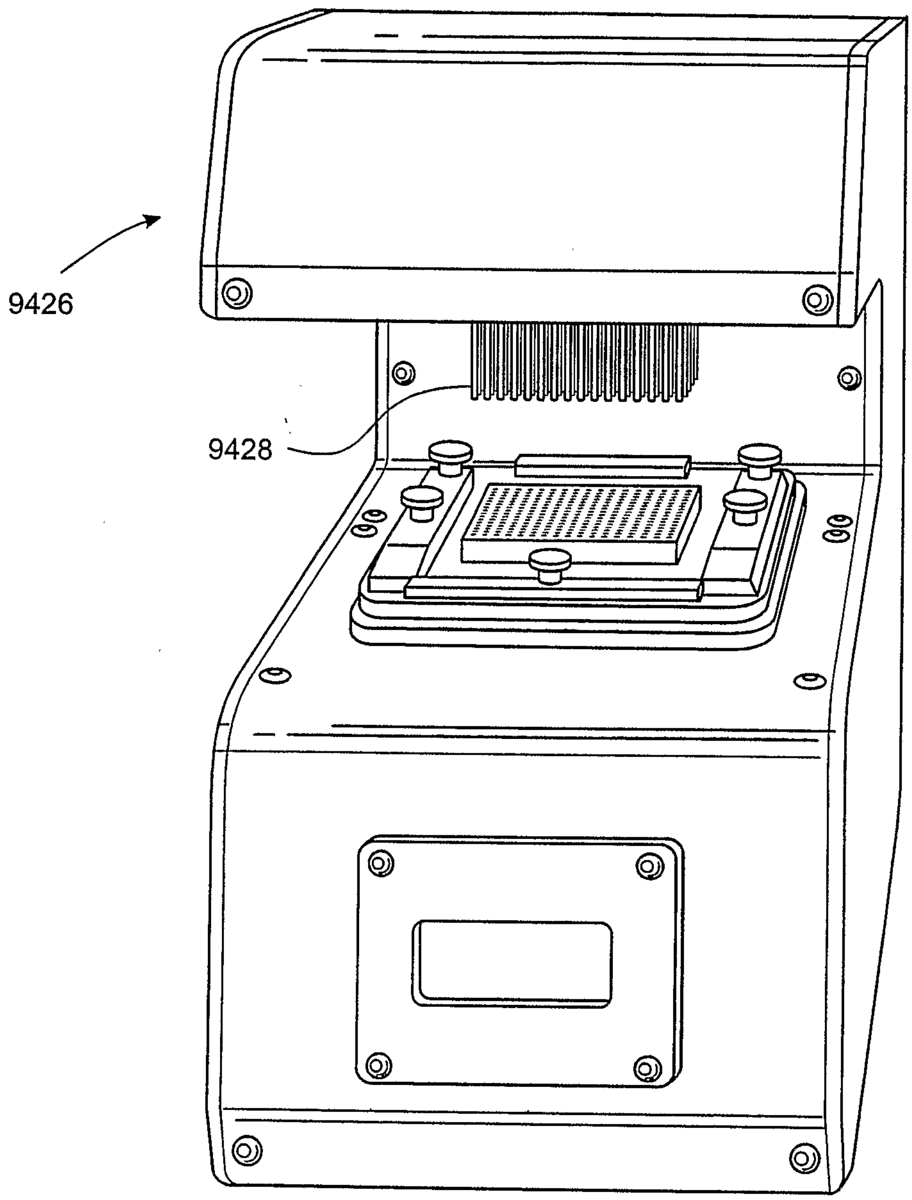


FIG. 17

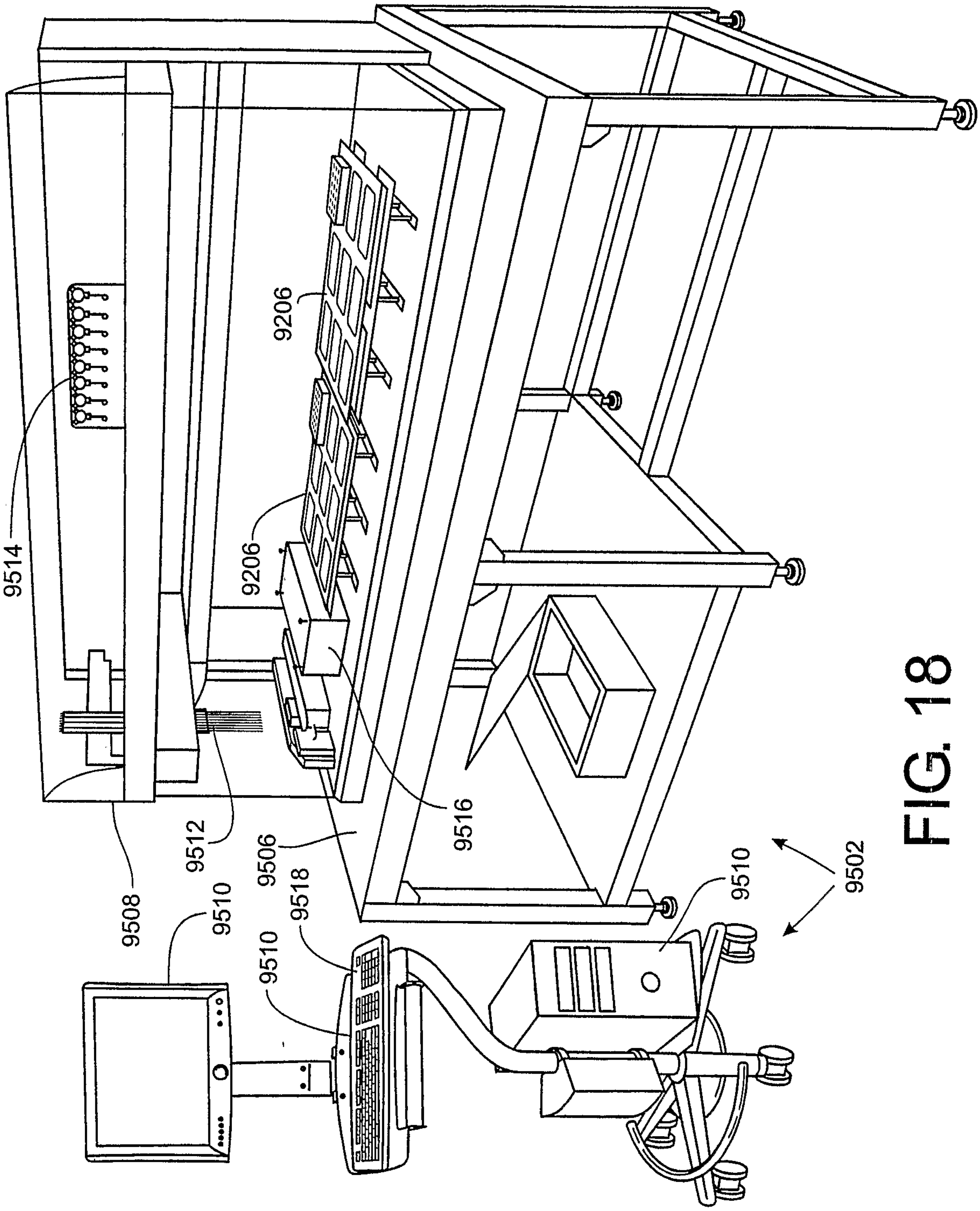
9514
9206
9206
9508
9512
9506
9516
9510
9510
9518
9510
9510
9502

FIG. 18

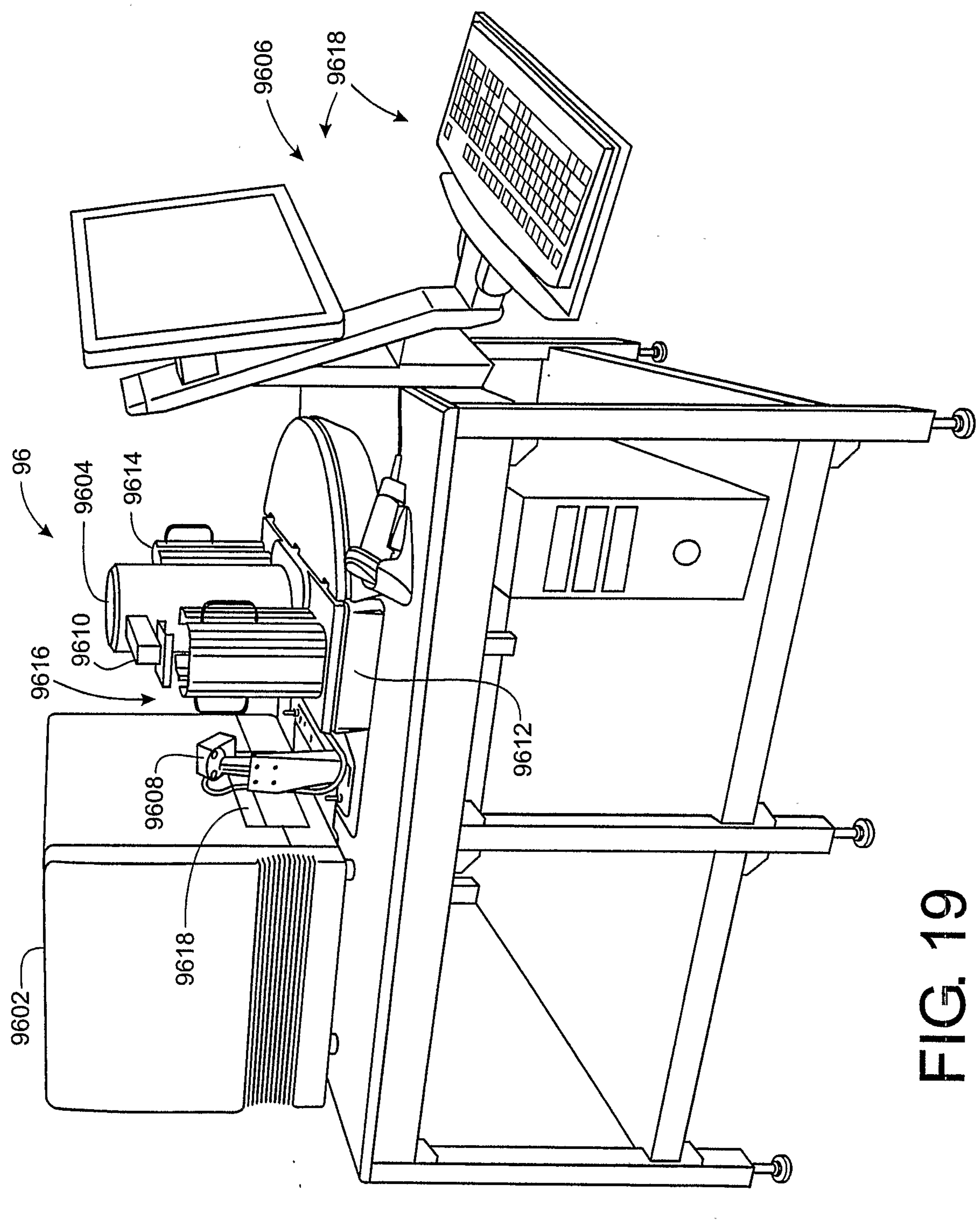


FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5654182 A
- US 5677177 A
- US 5527695 A
- WO 0220842 A
- US 20020177137 A
- US 5705628 A


### Non-patent literature cited in the description


- **SHENA, M.** DNA Microarrays: A Practical Approach. Oxford University Press, 2000
- **TURNER et al.** *Promega Application Notes,* 2003, 1-4
- **GREENSPOON et al.** *Journal of Forensic Sciences,* 2004, vol. 49, 29-39
- **KLUG, W.S. ; CUMMINGS, M.R.** Concepts of Genetics. Prentice-Hall, 1997
- *Oxford Dictionary of Biochemistry and Molecular Biology,* 182
- Oxford Dictionary of Biochemistry and Molecular Biology. 577
- **HAHN CN ; DEL PILAR MARTIN M ; SCHRODER M ; VANIER MT ; HARA Y ; SUZUKI K ; SUZUKI K ; D'AZZO A.** Generalized CNS disease and massive GM1-ganglioside accumulation in mice defective in lysosomal acid beta-galactosidase. *Hum Mol Genet,* February 1997, vol. 6 (2), 205-11